# EUROPEAN PATENT APPLICATION

(11) **EP 3 782 997 A1**
(43) Date of publication of application: **24.02.2021**
(21) Application number: 19192263.2
(22) Date of filing: 19.08.2019
(51) Int. Cl.: C07D 487/04, C07D 471/04, C07D 519/00, A61K 31/519, A61P 9/00, A61P 11/00, A61P 17/00, A61P 29/00, A61P 35/00

(54) **FUSED PYRIMIDINE COMPOUNDS AND PHARMACEUTICAL COMPOSITIONS THEREOF FOR THE TREATMENT OF FIBROTIC DISEASES**

(71) Applicant: Galapagos N.V., 2800 Mechelen (BE)
(72) Inventor: TEMAL-LAIB, Taoues, 93230 Romainville (FR); HECKMANN, Bertrand, 93230 Romainville (FR); COUTY, Sylvain, 93230 Romainville (FR); RICHARD, Sébastien, 93230 Romainville (FR); ANNOOT, Denis Maurice, 93230 Romainville (FR); TIRERA, Amynata, 93230 Romainville (FR); TRIBALLEAU, Nicolas, 93230 Romainville (FR); MESIC, Milan, 10000 Zagreb (HR)
(74) Representative: Bar, Grégory

(57) **Abstract**

The present invention discloses compounds according to Formula I:

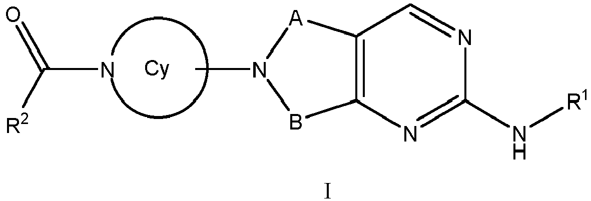

Wherein A, B, R¹, R², and Cy are as defined herein.

The present invention relates to compounds inhibiting autotaxin (NPP2 or ENPP2), methods for their production, pharmaceutical compositions comprising the same, and methods of treatment using the same, for the prophylaxis and/or treatment of fibrotic diseases, proliferative diseases, inflammatory diseases, autoimmune diseases, respiratory diseases, cardiovascular diseases, neurodegenerative diseases, dermatological disorders, pain, and/or abnormal angiogenesis associated diseases by administering the compounds of the invention.

## Description

### FIELD OF THE INVENTION

The present invention relates to compounds that may be useful in the prophylaxis and/or treatment of fibrotic diseases, proliferative diseases, inflammatory diseases, autoimmune diseases, respiratory diseases, cardiovascular diseases, neurodegenerative diseases, dermatological disorders, pain, and/or abnormal angiogenesis associated diseases. In particular, the compound of the invention may inhibit autotaxin, also known as ectonucleotide pyrophosphatase/phosphodiesterase 2 (NPP2 or ENPP2). The present invention also provides methods for the production of the compound of the invention, pharmaceutical compositions comprising the compound of the invention, methods for the prophylaxis and/or treatment of fibrotic diseases, proliferative diseases, inflammatory diseases, autoimmune diseases, respiratory diseases, cardiovascular diseases, neurodegenerative diseases, dermatological disorders, pain, and/or abnormal angiogenesis associated diseases by administering the compounds of the invention.

### BACKGROUND OF THE INVENTION

Autotaxin (ATX; also known as ENPP2 (ectonucleotide pyrophosphatase / phosphodiesterase 2) or lysophospholipase D) is a ∼120 kDa protein that belongs to the ENPP family of enzymes which is composed of seven members, out of which ENPP1 and ENPP3 are the closest to ATX. Whereas ENPP1 and ENPP3 are active in converting ATP into pyrophosphate (a regulator of mineralization and calcification processes in bone), ATX is the only ENPP enzyme with lysophospholipase D (lysoPLD) activity and is responsible for the hydrolysis of lysophosphatidylcholine (LPC) to produce the bioactive lipid lysophosphatidic acid (LPA). Several pieces of evidence have established ATX as the main source of LPA in blood. For example, blood LPA and ATX levels have been shown to be strongly correlated in humans. In addition, LPA levels are reduced by 50% in mice carrying a heterozygous null mutation of ATX (Tanaka et al. 2006).

Several physiological events playing a crucial role in a plethora of cellular functions, such as cell proliferation, survival, migration, and motility are promoted by LPA (Law et al. 2019). LPA binds to specific G-protein-coupled receptors (LPA1-6), resulting in the initiation and/or activation of specific cellular signaling cascades including calcium mobilization, activation of Ras, and extracellular-signal-regulated kinase (ERK) pathways participating in cell proliferation, activation of phosphoinositide 3 (P13)-protein kinase B (AKT) pathway which is among others responsible for apoptosis, and stimulation of Rho pathway involved in cell shape change and migration.

This autotaxin activity/LPA signaling has been linked to a variety of human pathologies such as cancer, chronic inflammation, cardiovascular disorders, Alzheimer's disease, fibroproliferative diseases, autoimmune diseases, and metabolic syndrome (Law et al. 2019; Matralis et al. 2019).

Compounds targeting the autotaxin/LPA axis have entered the clinic, for example GLPG1690 (Maher et al. 2019).

However, the current therapies are not satisfactory and therefore there remains a need to identify further compounds that may be of use in the treatment of fibrotic diseases, proliferative diseases, inflammatory diseases, autoimmune diseases, respiratory diseases, cardiovascular diseases, neurodegenerative diseases, dermatological disorders, pain, and/or abnormal angiogenesis associated diseases.

The present invention therefore provides compounds, methods for their manufacture and pharmaceutical compositions comprising a compound of the invention together with a suitable pharmaceutical carrier. The present invention also provides for the use of a compound of the invention in the preparation of a medicament for the treatment of fibrotic diseases, proliferative diseases, inflammatory diseases, autoimmune diseases, respiratory diseases, cardiovascular diseases, neurodegenerative diseases, dermatological disorders, pain, and/or abnormal angiogenesis associated diseases.

### SUMMARY OF THE INVENTION

The present invention relates to compounds that may be useful in the prophylaxis and/or treatment of fibrotic diseases, proliferative diseases, inflammatory diseases, autoimmune diseases, respiratory diseases, cardiovascular diseases, neurodegenerative diseases, dermatological disorders, pain, and/or abnormal angiogenesis associated diseases. In particular, the compound of the invention may inhibit autotaxin, also known as ectonucleotide pyrophosphatase/phosphodiesterase 2 (NPP2 or ENPP2). The present invention also provides methods for the production of the compound of the invention, pharmaceutical compositions comprising the compound of the invention, methods for the prophylaxis and/or treatment of fibrotic diseases, proliferative diseases, inflammatory diseases, autoimmune diseases, respiratory diseases, cardiovascular diseases, neurodegenerative diseases, dermatological disorders, pain, and/or abnormal angiogenesis associated diseases.by administering the compounds of the invention.

Accordingly, in a first aspect of the invention, the compounds of the invention are provided having a Formula I: 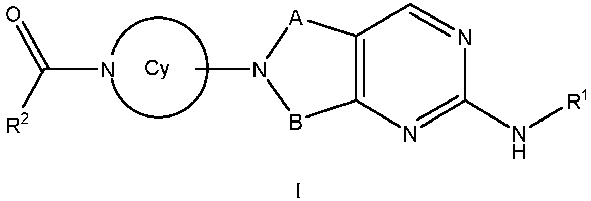 wherein
each A and B is independently C₁₋₄ alkylenyl optionally substituted with one oxo;
R¹ is:
   - 6-10 membered monocyclic or fused bicyclic aryl, optionally substituted with one or more independently selected R^{3a} groups, or
   - 5-10 membered monocyclic or fused bicyclic heteroaryl comprising one or more heteroatoms independently selected from N, O and S, which heteroaryl is optionally substituted with one or more independently selected R^{3b} groups;
R² is
   - phenyl optionally substituted with one or more independently selected R⁵ groups, or
   - 5-10 membered monocyclic or fused bicyclic heteroaryl comprising one or more heteroatoms independently selected from N, O and S, which heteroaryl is optionally substituted with one or more independently selected R⁵ groups;
each R^{3a} and R^{3b} is independently selected from:
   - halo,
   - C₁₋₄ alkyl optionally substituted with one or more independently selected halo,
   - C₁₋₄ alkoxy optionally substituted with one or more independently selected halo,
   - -SF₅,
   - -CN,
   - -O-C₃₋₇ cycloalkyl, and
   - -C(=O)NH₂;
Cy is:
   - 4-10 membered monocyclic, bridged bicyclic, or spiro bicyclic heterocycloalkyl comprising one N atom, which heterocycloalkyl is optionally substituted with one or more independently selected R⁴ groups, or
   - 4-10 membered monocyclic, bridged bicyclic, or spiro bicyclic heterocycloalkenyl comprising one double bond and one N atom, which heterocycloalkenyl is optionally substituted with one or more independently selected R⁴ groups;
each R⁴ is independently selected from:
   - C₁₋₄ alkyl optionally substituted with one or more independently selected halo,
   - -C(=O)O-C₁₋₄ alkyl,
   - halo,
   - -CN, and
   - -OH;
each R⁵ is independently selected from:
   - -OH,
   - -SH,
   - halo,
   - C₁₋₄ alkyl optionally substituted with one or more independently selected -OH, or halo,
   - C₁₋₄ alkoxy optionally substituted with one or more independently selected -OH, or halo,
   - -NH-S(=O)₂-C₁₋₄ alkyl, which alkyl is optionally substituted with one or more independently selected halo or C₁₋₄ alkoxy,
   - -NH-S(=O)₂-C₃₋₇ cycloalkyl,
   - -NH-C(=O)C₁₋₄ alkyl,
   - -C(=O)OH,
   - -C(=O)NH₂,
   - -C(=O)O-C₁₋₄ alkyl, which alkyl is optionally substituted with one 5-6 membered monocyclic heterocycloalkenyl comprising one double bond and one or two O atoms, which heterocycloalkenyl is optionally substituted with one or more independently selected C₁₋₄ alkyl or oxo,
   - -C(=O)-NH-S(=O)₂-C₁₋₄ alkyl,
   - -S(=O)₂NH₂, and
   - 5-6 membered monocyclic heteroaryl comprising one or more heteroatoms independently selected from N, O, and S.

In a particular aspect, the compounds of the invention are provided for use in the prophylaxis and/or treatment of fibrotic diseases, proliferative diseases, inflammatory diseases, autoimmune diseases, respiratory diseases, cardiovascular diseases, neurodegenerative diseases, dermatological disorders, pain, and/or abnormal angiogenesis associated diseases.

In a further aspect, the present invention provides pharmaceutical compositions comprising a compound of the invention, and a pharmaceutical carrier, excipient or diluent. In a particular aspect, the pharmaceutical composition may additionally comprise further therapeutically active ingredients suitable for use in combination with the compounds of the invention. In a more particular aspect, the further therapeutically active ingredient is an agent for the treatment of fibrotic diseases, proliferative diseases, inflammatory diseases, autoimmune diseases, respiratory diseases, cardiovascular diseases, neurodegenerative diseases, dermatological disorders, pain, and/or abnormal angiogenesis associated diseases.

Moreover, the compounds of the invention, useful in the pharmaceutical compositions and treatment methods disclosed herein, are pharmaceutically acceptable as prepared and used.

In a further aspect, this invention provides a method of treating a mammal, in particular humans, afflicted with a condition selected from among those listed herein, and particularly fibrotic diseases, proliferative diseases, inflammatory diseases, autoimmune diseases, respiratory diseases, cardiovascular diseases, neurodegenerative diseases, dermatological disorders, pain, and/or abnormal angiogenesis associated diseases, which method comprises administering an effective amount of the pharmaceutical composition or compounds of the invention as described herein.

The present invention also provides pharmaceutical compositions comprising a compound of the invention, and a suitable pharmaceutical carrier, excipient or diluent for use in medicine. In a particular aspect, the pharmaceutical composition is for use in the prophylaxis and/or treatment of fibrotic diseases, proliferative diseases, inflammatory diseases, autoimmune diseases, respiratory diseases, cardiovascular diseases, neurodegenerative diseases, dermatological disorders, pain, and/or abnormal angiogenesis associated diseases.

In additional aspects, this invention provides methods for synthesizing the compounds of the invention, with representative synthetic protocols and pathways disclosed later on herein.

Other objects and advantages will become apparent to those skilled in the art from a consideration of the ensuing detailed description.

### DETAILED DESCRIPTION OF THE INVENTION

### Definitions

The following terms are intended to have the meanings presented therewith below and are useful in understanding the description and intended scope of the present invention.

When describing the invention, which may include compounds, pharmaceutical compositions containing such compounds and methods of using such compounds and compositions, the following terms, if present, have the following meanings unless otherwise indicated. It should also be understood that when described herein any of the moieties defined forth below may be substituted with a variety of substituents, and that the respective definitions are intended to include such substituted moieties within their scope as set out below. Unless otherwise stated, the term "substituted" is to be defined as set out below. It should be further understood that the terms "groups" and "radicals" can be considered interchangeable when used herein.

The articles 'a' and 'an' may be used herein to refer to one or to more than one (*i.e.* at least one) of the grammatical objects of the article. By way of example 'an analogue' means one analogue or more than one analogue.

'Alkyl' means straight or branched aliphatic hydrocarbon having the specified number of carbon atoms. Particular alkyl groups have 1 to 6 carbon atoms or 1 to 4 carbon atoms. Branched means that one or more alkyl groups such as methyl, ethyl or propyl is attached to a linear alkyl chain. Particular alkyl groups are methyl (-CH₃), ethyl (-CH₂-CH₃), n-propyl (-CH₂-CH₂-CH₃), isopropyl (-CH(CH₃)₂), n-butyl (-CH₂-CH₂-CH₂-CH₃), tert-butyl (-CH₂-C(CH₃)₃), sec-butyl (-CH₂-CH(CH₃)₂), n-pentyl (-CH₂-CH₂-CH₂-CH₂-CH₃), n-hexyl (-CH₂-CH₂-CH₂-CH₂-CH₂-CH₃), and 1,2-dimethylbutyl (-CHCH₃)-C(CH₃)H₂-CH₂-CH₃). Particular alkyl groups have between 1 and 4 carbon atoms.

'Alkenyl' refers to monovalent olefinically (unsaturated) hydrocarbon groups with the number of carbon atoms specified. Particular alkenyl has 2 to 8 carbon atoms, and more particularly, from 2 to 6 carbon atoms, which can be straight-chained or branched and having at least 1 and particularly from 1 to 2 sites of olefinic unsaturation. Particular alkenyl groups include ethenyl (-CH=CH₂), n-propenyl (-CH₂CH=CH₂), isopropenyl (-C(CH₃)=CH₂) and the like.

'Alkylene' refers to divalent alkene radical groups having the number of carbon atoms specified, in particular having 1 to 6 carbon atoms and more particularly 1 to 4 carbon atoms which can be straight-chained or branched. This term is exemplified by groups such as methylene (-CH₂-), ethylene (-CH₂-CH₂-), or -CH(CH₃)- and the like.

'Alkynylene' refers to divalent alkyne radical groups having the number of carbon atoms and the number of triple bonds specified, in particular 2 to 6 carbon atoms and more particularly 2 to 4 carbon atoms which can be straight-chained or branched. This term is exemplified by groups such as -C≡C-, -CH₂-C≡C-, and -C(CH₃)H-C≡CH-.

'Alkoxy' refers to the group O-alkyl, where the alkyl group has the number of carbon atoms specified. In particular the term refers to the group -O-C₁₋₆ alkyl. Particular alkoxy groups are methoxy, ethoxy, n-propoxy, isopropoxy, n-butoxy, tert-butoxy, sec-butoxy, n-pentoxy, n-hexoxy, and 1,2-dimethylbutoxy. Particular alkoxy groups are lower alkoxy, i.e. with between 1 and 6 carbon atoms. Further particular alkoxy groups have between 1 and 4 carbon atoms.

'Amino' refers to the radical -NH₂.

'Aryl' refers to a monovalent aromatic hydrocarbon group derived by the removal of one hydrogen atom from a single carbon atom of a parent aromatic ring system. In particular aryl refers to an aromatic ring structure, monocyclic or fused polycyclic, with the number of ring atoms specified. Specifically, the term includes groups that include from 6 to 10 ring members. Particular aryl groups include phenyl, and naphthyl.

'Cycloalkyl'refers to a non-aromatic hydrocarbyl ring structure, monocyclic, fused polycyclic, bridged polycyclic, or spirocyclic, with the number of ring atoms specified. A cycloalkyl may have from 3 to 12 carbon atoms, in particular from 3 to 10, and more particularly from 3 to 7 carbon atoms. Such cycloalkyl groups include, by way of example, single ring structures such as cyclopropyl, cyclobutyl, cyclopentyl, cyclohexyl, and cycloheptyl.

'Cyano' refers to the radical -CN.

'Halo' or 'halogen' refers to fluoro (F), chloro (CI), bromo (Br) and iodo (I). Particular halo groups are either fluoro or chloro.

'Hetero' when used to describe a compound or a group present on a compound means that one or more carbon atoms in the compound or group have been replaced by a nitrogen, oxygen, or sulfur heteroatom. Hetero may be applied to any of the hydrocarbyl groups described above such as alkyl, *e.g.* heteroalkyl, cycloalkyl, *e.g.* heterocycloalkyl, aryl, *e.g.* heteroaryl, and the like having from 1 to 4, and particularly from 1 to 3 heteroatoms, more typically 1 or 2 heteroatoms, for example a single heteroatom.

'Heteroaryl' means an aromatic ring structure, monocyclic or fused polycyclic, that includes one or more heteroatoms independently selected from O, N and S and the number of ring atoms specified. In particular, the aromatic ring structure may have from 5 to 9 ring members. The heteroaryl group can be, for example, a five membered or six membered monocyclic ring or a fused bicyclic structure formed from fused five and six membered rings or two fused six membered rings or, by way of a further example, two fused five membered rings. Each ring may contain up to four heteroatoms typically selected from nitrogen, sulphur and oxygen. Typically the heteroaryl ring will contain up to 4 heteroatoms, more typically up to 3 heteroatoms, more usually up to 2, for example a single heteroatom. In one embodiment, the heteroaryl ring contains at least one ring nitrogen atom. The nitrogen atoms in the heteroaryl rings can be basic, as in the case of an imidazole or pyridine, or essentially non-basic as in the case of an indole or pyrrole nitrogen. In general the number of basic nitrogen atoms present in the heteroaryl group, including any amino group substituents of the ring, will be less than five.

Examples of five membered monocyclic heteroaryl groups include but are not limited to pyrrolyl, furanyl, thiophenyl, imidazolyl, furazanyl, oxazolyl, oxadiazolyl, oxatriazolyl, isoxazolyl, thiazolyl, isothiazolyl, pyrazolyl, triazolyl and tetrazolyl groups.

Examples of six membered monocyclic heteroaryl groups include but are not limited to pyridinyl, pyrazinyl, pyridazinyl, pyrimidinyl and triazinyl.

Particular examples of bicyclic heteroaryl groups containing a five membered ring fused to another five-membered ring include but are not limited to imidazothiazolyl and imidazoimidazolyl.

Particular examples of bicyclic heteroaryl groups containing a six membered ring fused to a five membered ring include but are not limited to benzofuranyl, benzothiophenyl, benzoimidazolyl, benzoxazolyl, isobenzoxazolyl, benzisoxazolyl, benzothiazolyl, benzoisothiazolyl, isobenzofuranyl, indolyl, isoindolyl, indolizinyl, purinyl (*e.g.* adenine, guanine), indazolyl, pyrazolopyrimidinyl, triazolopyrimidinyl, and pyrazolopyridinyl groups.

Particular examples of bicyclic heteroaryl groups containing two fused six membered rings include but are not limited to quinolinyl, isoquinolinyl, pyridopyridinyl, quinoxalinyl, quinazolinyl, cinnolinyl, phthalazinyl, naphthyridinyl, and pteridinyl groups. Particular heteroaryl groups are those derived from thiophenyl, pyrrolyl, benzothiophenyl, benzofuranyl, indolyl, pyridinyl, quinolinyl, imidazolyl, oxazolyl and pyrazinyl.

Examples of representative heteroaryls include the following: 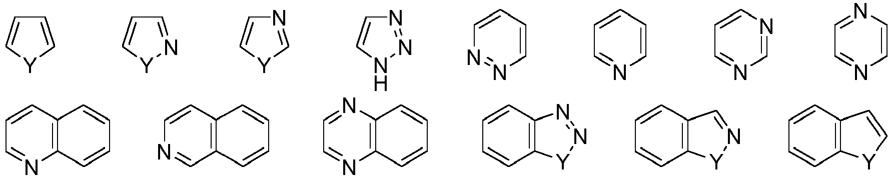 wherein each Y is selected from >C=O, NH, O and S.

'Heterocycloalkyl' means a non-aromatic fully saturated ring structure, monocyclic, fused polycyclic, spirocyclic, or bridged polycyclic, that includes one or more heteroatoms independently selected from O, N and S and the number of ring atoms specified. The heterocycloalkyl ring structure may have from 4 to 12 ring members, in particular from 4 to 10 ring members and more particularly from 4 to 7 ring members. Each ring may contain up to four heteroatoms typically selected from nitrogen, sulphur and oxygen. Typically the heterocycloalkyl ring will contain up to 4 heteroatoms, more typically up to 3 heteroatoms, more usually up to 2, for example a single heteroatom. Examples of heterocyclic rings include, but are not limited to azetidinyl, oxetanyl, thietanyl, pyrrolidinyl (*e.g.* 1-pyrrolidinyl, 2-pyrrolidinyl and 3-pyrrolidinyl), tetrahydrofuranyl (*e.g.* 1-tetrahydrofuranyl, 2-tetrahydrofuranyl and 3-tetrahydrofuranyl), tetrahydrothiophenyl (*e.g.* 1-tetrahydrothiophenyl, 2-tetrahydrothiophenyl and 3-tetrahydrothiophenyl), piperidinyl (*e.g.* 1-piperidinyl, 2-piperidinyl, 3-piperidinyl and 4-piperidinyl), tetrahydropyranyl (*e.g.* 4-tetrahydropyranyl), tetrahydrothiopyranyl (*e.g.* 4-tetrahydrothiopyranyl), morpholinyl, thiomorpholinyl, dioxanyl, or piperazinyl.

As used herein, the term 'heterocycloalkenyl' means a 'heterocycloalkyl', which comprises at least one double bond. Particular examples of heterocycloalkenyl groups are shown in the following illustrative examples: 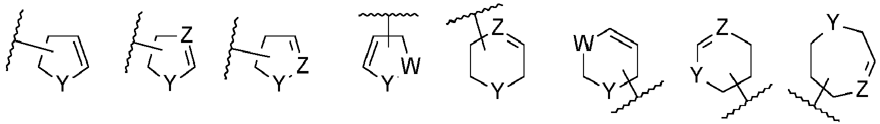 wherein each W is selected from CH₂, NH, O and S; each Y is selected from NH, O, C(=O), SO₂, and S; and each Z is selected from N or CH.

Particular examples of monocyclic rings are shown in the following illustrative examples: 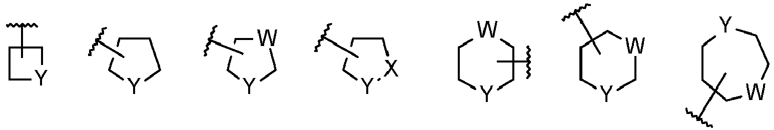 wherein each W and Y is independently selected from -CH₂-, -NH-, -O- and -S-.

Particular examples of fused bicyclic rings are shown in the following illustrative examples: 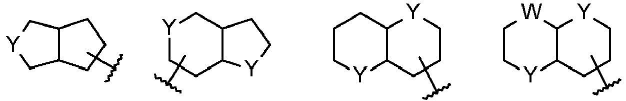 wherein each W and Y is independently selected from -CH₂-, -NH-, -O- and -S-.

Particular examples of bridged bicyclic rings are shown in the following illustrative examples: 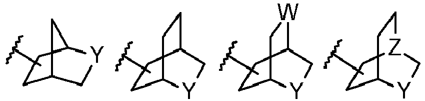 wherein each W and Y is independently selected from -CH₂-, -NH-, -O- and -S- and each Z is selected from N or CH.

Particular examples of spirocyclic rings are shown in the following illustrative examples: 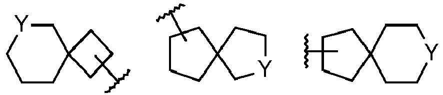 wherein each Y is selected from -CH₂-, -NH-, -O- and -S-.

'Hydroxyl' refers to the radical -OH.

'Oxo' refers to the radical =O.

'Substituted' refers to a group in which one or more hydrogen atoms are each independently replaced with the same or different substituent(s).

'Sulfo' or 'sulfonic acid' refers to a radical such as -SO₃H.

'Thiol' refers to the group -SH.

As used herein, term 'substituted with one or more' refers to one to four substituents. In one embodiment it refers to one to three substituents. In further embodiments it refers to one or two substituents. In a yet further embodiment it refers to one substituent.

'Thioalkoxy' refers to the group -S-alkyl where the alkyl group has the number of carbon atoms specified. In particular the term refers to the group -S-C₁₋₆ alkyl. Particular thioalkoxy groups are thiomethoxy, thioethoxy, n-thiopropoxy, isothiopropoxy, n-thiobutoxy, tert-thiobutoxy, sec-thiobutoxy, n-thiopentoxy, n-thiohexoxy, and 1,2-dimethylthiobutoxy. Particular thioalkoxy groups are lower thioalkoxy, *i.e.* with between 1 and 6 carbon atoms. Further particular alkoxy groups have between 1 and 4 carbon atoms.

One having ordinary skill in the art of organic synthesis will recognize that the maximum number of heteroatoms in a stable, chemically feasible heterocyclic ring, whether it is aromatic or non-aromatic, is determined by the size of the ring, the degree of unsaturation and the valence of the heteroatoms. In general, a heterocyclic ring may have one to four heteroatoms so long as the heteroaromatic ring is chemically feasible and stable.

'Pharmaceutically acceptable' means approved or approvable by a regulatory agency of the Federal or a state government or the corresponding agency in countries other than the United States, or that is listed in the U.S. Pharmacopoeia or other generally recognized pharmacopoeia for use in animals, and more particularly, in humans.

'Pharmaceutically acceptable salt' refers to a salt of a compound of the invention that is pharmaceutically acceptable and that possesses the desired pharmacological activity of the parent compound. In particular, such salts are non-toxic may be inorganic or organic acid addition salts and base addition salts. Specifically, such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, *e.g.* an alkali metal ion, an alkaline earth ion, or an aluminum ion; or coordinates with an organic base such as ethanolamine, diethanolamine, triethanolamine, N-methylglucamine and the like. Salts further include, by way of example only, sodium, potassium, calcium, magnesium, ammonium, tetraalkylammonium, and the like; and when the compound contains a basic functionality, salts of non-toxic organic or inorganic acids, such as hydrochloride, hydrobromide, tartrate, mesylate, acetate, maleate, oxalate and the like. The term 'pharmaceutically acceptable cation' refers to an acceptable cationic counter-ion of an acidic functional group. Such cations are exemplified by sodium, potassium, calcium, magnesium, ammonium, tetraalkylammonium cations, and the like.

'Pharmaceutically acceptable vehicle' refers to a diluent, adjuvant, excipient or carrier with which a compound of the invention is administered.

'Prodrugs' refers to compounds, including derivatives of the compounds of the invention, which have cleavable groups and become by solvolysis or under physiological conditions the compounds of the invention which are pharmaceutically active *in vivo.* Such examples include, but are not limited to, choline ester derivatives and the like, N-alkylmorpholine esters and the like.

'Solvate' refers to forms of the compound that are associated with a solvent, usually by a solvolysis reaction. This physical association includes hydrogen bonding. Conventional solvents include water, EtOH, acetic acid and the like. The compounds of the invention may be prepared *e.g.* in crystalline form and may be solvated or hydrated. Suitable solvates include pharmaceutically acceptable solvates, such as hydrates, and further include both stoichiometric solvates and non-stoichiometric solvates. In certain instances the solvate will be capable of isolation, for example when one or more solvent molecules are incorporated in the crystal lattice of the crystalline solid. 'Solvate' encompasses both solution-phase and isolable solvates. Representative solvates include hydrates, ethanolates and methanolates.

'Subject' includes humans. The terms 'human', 'patient' and 'subject' are used interchangeably herein.

'Effective amount' means the amount of a compound of the invention that, when administered to a subject for treating a disease, is sufficient to effect such treatment for the disease. The "effective amount" can vary depending on the compound, the disease and its severity, and the age, weight, etc., of the subject to be treated.

'Preventing' or 'prevention' refers to a reduction in risk of acquiring or developing a disease or disorder (*i.e.* causing at least one of the clinical symptoms of the disease not to develop in a subject that may be exposed to a disease-causing agent, or predisposed to the disease in advance of disease onset.

The term 'prophylaxis' is related to 'prevention', and refers to a measure or procedure the purpose of which is to prevent, rather than to treat or cure a disease. Non-limiting examples of prophylactic measures may include the administration of vaccines; the administration of low molecular weight heparin to hospital patients at risk for thrombosis due, for example, to immobilization; and the administration of an anti-malarial agent such as chloroquine, in advance of a visit to a geographical region where malaria is endemic or the risk of contracting malaria is high.

'Treating' or 'treatment' of any disease or disorder refers, in one embodiment, to ameliorating the disease or disorder (*i.e.* arresting the disease or reducing the manifestation, extent or severity of at least one of the clinical symptoms thereof). In another embodiment 'treating' or 'treatment' refers to ameliorating at least one physical parameter, which may not be discernible by the subject. In yet another embodiment, 'treating' or 'treatment' refers to modulating the disease or disorder, either physically, (*e.g.* stabilization of a discernible symptom), physiologically, (*e.g.* stabilization of a physical parameter), or both. In a further embodiment, "treating" or "treatment" relates to slowing the progression of the disease.

As used herein the term 'fibrotic diseases' refers to diseases characterized by excessive scarring due to excessive production, deposition, and contraction of extracellular matrix, and are that are associated with the abnormal accumulation of cells and/or fibronectin and/or collagen and/or increased fibroblast recruitment and include but are not limited to fibrosis of individual organs or tissues such as the heart, kidney, liver, joints, lung, pleural tissue, peritoneal tissue, skin, cornea, retina, musculoskeletal and digestive tract. In particular, the term fibrotic diseases refers to idiopathic pulmonary fibrosis (IPF); cystic fibrosis, other diffuse parenchymal lung diseases of different etiologies including iatrogenic drug-induced fibrosis, occupational and/or environmental induced fibrosis, granulomatous diseases (sarcoidosis, hypersensitivity pneumonia), collagen vascular disease, alveolar proteinosis, langerhans cell granulomatosis, lymphangioleiomyomatosis, inherited diseases (Hermansky-Pudlak Syndrome, tuberous sclerosis, neurofibromatosis, metabolic storage disorders, familial interstitial lung disease); radiation induced fibrosis; chronic obstructive pulmonary disease (COPD); scleroderma; bleomycin induced pulmonary fibrosis; chronic asthma; silicosis; asbestos induced pulmonary fibrosis; acute respiratory distress syndrome (ARDS); kidney fibrosis; tubulointerstitium fibrosis; glomerular nephritis; focal segmental glomerular sclerosis; IgA nephropathy; hypertension; Alport; gut fibrosis; liver fibrosis; cirrhosis; alcohol induced liver fibrosis; toxic/drug induced liver fibrosis; hemochromatosis; nonalcoholic steatohepatitis (NASH); biliary duct injury; primary biliary cirrhosis; infection induced liver fibrosis; viral induced liver fibrosis; and autoimmune hepatitis; corneal scarring; hypertrophic scarring; Dupuytren disease, keloids, cutaneous fibrosis; cutaneous scleroderma; systemic sclerosis, spinal cord injury/fibrosis; myelofibrosis; vascular restenosis; atherosclerosis; arteriosclerosis; Wegener's granulomatosis; Peyronie's disease, or chronic lymphocytic. More particularly, the term 'fibrotic diseases' refers to idiopathic pulmonary fibrosis (IPF), or systemic sclerosis.

As used herein the term 'proliferative disease(s)' refers to conditions such as cancer (*e.g.* uterine leiomyosarcoma or prostate cancer), myeloproliferative disorders (*e.g.* polycythemia vera, essential thrombocytosis and myelofibrosis), leukemia (*e.g.* acute myeloid leukaemia, acute and chronic lymphoblastic leukemia), multiple myeloma, psoriasis, restenosis, scleroderma or fibrosis. In particular the term refers to cancer, leukemia, multiple myeloma and psoriasis.

As used herein, the term 'cancer' refers to a malignant or benign growth of cells in skin or in body organs, for example but without limitation, breast, prostate, lung, kidney, pancreas, stomach or bowel. A cancer tends to infiltrate into adjacent tissue and spread (metastasise) to distant organs, for example to bone, liver, lung or the brain. As used herein the term cancer includes both metastatic tumour cell types (such as but not limited to, melanoma, lymphoma, leukaemia, fibrosarcoma, rhabdomyosarcoma, and mastocytoma) and types of tissue carcinoma (such as but not limited to, colorectal cancer, prostate cancer, small cell lung cancer and non-small cell lung cancer, breast cancer, pancreatic cancer, bladder cancer, renal cancer, gastric cancer, glioblastoma, primary liver cancer, ovarian cancer, prostate cancer and uterine leiomyosarcoma). In particular, the term "cancer' refers to acute lymphoblastic leukemia, acute myeloidleukemia, adrenocortical carcinoma, anal cancer, appendix cancer, astrocytomas, atypical teratoid/rhabdoid tumor, basal cell carcinoma, bile duct cancer, bladder cancer, bone cancer (osteosarcoma and malignant fibrous histiocytoma), brain stem glioma, brain tumors, brain and spinal cord tumors, breast cancer, bronchial tumors, Burkitt lymphoma, cervical cancer, chronic lymphocytic leukemia, chronic myelogenous leukemia, colon cancer, colorectal cancer, craniopharyngioma, cutaneous T-Cell lymphoma, embryonal tumors, endometrial cancer, ependymoblastoma, ependymoma, esophageal cancer, ewing sarcoma family of tumors, eye cancer, retinoblastoma, gallbladder cancer, gastric (stomach) cancer, gastrointestinal carcinoid tumor, gastrointestinal stromal tumor (GIST), gastrointestinal stromal cell tumor, germ cell tumor, glioma, hairy cell leukemia, head and neck cancer, hepatocellular (liver) cancer, hodgkin lymphoma, hypopharyngeal cancer, intraocular melanoma, islet cell tumors (endocrine pancreas), Kaposi sarcoma, kidney cancer, Langerhans cell histiocytosis, laryngeal cancer, leukemia, Acute lymphoblastic leukemia, acute myeloid leukemia, chronic lymphocytic leukemia, chronic myelogenous leukemia, hairy cell leukemia, liver cancer, non-small cell lung cancer, small cell lung cancer, Burkitt lymphoma, cutaneous T-celllymphoma, Hodgkin lymphoma, non-Hodgkin lymphoma, lymphoma, Waldenstrom macroglobulinemia, medulloblastoma, medulloepithelioma, melanoma, mesothelioma, mouth cancer, chronic myelogenous leukemia, myeloid leukemia, multiple myeloma, asopharyngeal cancer, neuroblastoma, non-small cell lung cancer, oral cancer, oropharyngeal cancer, osteosarcoma, malignant fibrous histiocytoma of bone, ovarian cancer, ovarian epithelial cancer, ovarian germ cell tumor, ovarian low malignant potential tumor, pancreatic cancer, papillomatosis, parathyroid cancer, penile cancer, pharyngeal cancer, pineal parenchymal tumors of intermediate differentiation, pineoblastoma and supratentorial primitive neuroectodermal tumors, pituitary tumor, plasma cell neoplasm/multiple myeloma, pleuropulmonary blastoma, primary central nervous system lymphoma, prostate cancer, rectal cancer, renal cell (kidney) cancer, retinoblastoma, rhabdomyosarcoma, salivary gland cancer, sarcoma, Ewing sarcoma family of tumors, sarcoma, kaposi, Sezary syndrome, skin cancer, small cell Lung cancer, small intestine cancer, soft tissue sarcoma, squamous cell carcinoma, stomach (gastric) cancer, supratentorial primitive neuroectodermal tumors, T -cell lymphoma, testicular cancer, throat cancer, thymoma and thymic carcinoma, thyroid cancer, urethral cancer, uterine cancer, uterine sarcoma, vaginal cancer, vulvar cancer, Waldenstrom macroglobulinemia, and Wilms tumor

As used herein the term 'leukemia' refers to neoplastic diseases of the blood and blood forming organs. Such diseases can cause bone marrow and immune system dysfunction, which renders the host highly susceptible to infection and bleeding. In particular the term leukemia refers to acute myeloid leukaemia (AML), and acute lymphoblastic leukemia (ALL) and chronic lymphoblastic leukaemia (CLL).

As used herein the term 'inflammatory diseases' refers to the group of conditions including, rheumatoid arthritis, osteoarthritis, juvenile idiopathic arthritis, psoriasis, psoriatic arthritis, allergic airway disease (*e.g.* asthma, rhinitis), chronic obstructive pulmonary disease (COPD), inflammatory bowel diseases (*e.g.* Crohn's disease, ulcerative colitis), endotoxin-driven disease states (*e.g.* complications after bypass surgery or chronic endotoxin states contributing to *e.g.* chronic cardiac failure), and related diseases involving cartilage, such as that of the joints. Particularly the term refers to rheumatoid arthritis, osteoarthritis, allergic airway disease (*e.g.* asthma), chronic obstructive pulmonary disease (COPD) and inflammatory bowel diseases (*e.g.* Crohn's disease and ulcerative colitis). More particularly the term refers to rheumatoid arthritis, and chronic obstructive pulmonary disease (COPD).

As used herein the term 'autoimmune disease(s)' refers to the group of diseases including obstructive airways disease, including conditions such as COPD, asthma (e.g intrinsic asthma, extrinsic asthma, dust asthma, infantile asthma) particularly chronic or inveterate asthma (for example late asthma and airway hyperreponsiveness), bronchitis, including bronchial asthma, systemic lupus erythematosus (SLE), cutaneous lupus erythrematosis, lupus nephritis, dermatomyositis, Sjogren's syndrome, multiple sclerosis, psoriasis, dry eye disease, type I diabetes mellitus and complications associated therewith, atopic eczema (atopic dermatitis), thyroiditis (Hashimoto's and autoimmune thyroiditis), contact dermatitis and further eczematous dermatitis, inflammatory bowel disease (*e.g.* Crohn's disease and ulcerative colitis), atherosclerosis and amyotrophic lateral sclerosis. Particularly the term refers to COPD, asthma, systemic lupus erythematosis, type I diabetes mellitus and inflammatory bowel disease.

As used herein , the term 'respiratory disease' refers to diseases affecting the organs that are involved in breathing, such as the nose, throat, larynx, eustachian tubes, trachea, bronchi, lungs, related muscles (*e.g.,* diaphram and intercostals), and nerves. In particular, examples of respiratory diseases include asthma, adult respiratory distress syndrome and allergic (extrinsic) asthma, non-allergic (intrinsic) asthma, acute severe asthma, chronic asthma, clinical asthma, nocturnal asthma, allerGen-induced asthma, aspirin-sensitive asthma, exercise-induced asthma, isocapnic hyperventilation, child onset asthma, adult-onset asthma, cough-variant asthma, occupational asthma, steroid-resistant asthma, seasonal asthma, seasonal allergic rhinitis, perennial allergic rhinitis, chronic obstructive pulmonary disease, including chronic bronchitis or emphysema, pulmonary hypertension, interstitial lung fibrosis and/or airway inflammation, cystic fibrosis, and hypoxia.

As used herein the term 'asthma' as used herein refers to any disorder of the lungs characterized by variations in pulmonary gas flow associated with airway constriction of whatever cause (intrinsic, extrinsic, or both; allergic or non-allergic). The term asthma may be used with one or more adjectives to indicate the cause.

As used herein the term 'cardiovascular disease' refers to diseases affecting the heart or blood vessels or both. In particular, cardiovascular disease includes arrhythmia (atrial or ventricular or both); atherosclerosis and its sequelae; angina; cardiac rhythm disturbances; myocardial ischemia; myocardial infarction; cardiac or vascular aneurysm; vasculitis, stroke; peripheral obstructive arteriopathy of a limb, an organ, or a tissue; reperfusion injury following ischemia of the brain, heart, kidney or other organ or tissue; endotoxic, surgical, or traumatic shock; hypertension, valvular heart disease, heart failure, abnormal blood pressure, vasoconstriction (including that associated with migraines); vascular abnormality, inflammation, insufficiency limited to a single organ or tissue.

As used herein the term 'neurodegenerative diseases' refers to disorders that are associated with atrophy of the affected central or peripheral structures of the nervous system. In particular, the term 'neurodegenerative diseases' refers to diseases such as Alzheimer's disease and other dementias, degenerative nerve diseases, encephalitis, epilepsy, genetic brain disorders, head and brain malformations, hydrocephalus, stroke, Parkinson's disease, multiple sclerosis, amyotrophic lateral sclerosis (ALS or Lou Gehrig's Disease), Huntington's disease, and prion diseases.

As used herein the term 'dermatological disorder' refers to a skin disorder. In particular, dermatological disorders include proliferative or inflammatory disorders of the skin such as, atopic dermatitis, bullous disorders, collagenoses, psoriasis, psoriatic lesions, dermatitis, contact dermatitis, eczema, pruritus, scleroderma, wound healing, scarring, hypertrophic scarring, keloids, Kawasaki disease, rosacea, Sjögren-Larsson syndrome, or urticaria.

As used herein the term 'pain' refers to diseases or disorders characterized by unpleasant feeling often caused by intense or damaging stimuli, and include but is not limited to nociceptive pain, inflammatory pain (associated with tissue damage and inflammatory cell infiltration) and neuropathic or dysfunctional pain (caused by damage to or abnormal function of the nervous system), and/or pain associated or caused by the conditions mentioned herein. Pain can be acute or chronic. In particular, the term refers to neuropathic pain.

As used herein the term 'abnormal angiogenesis associated disease' refers to diseases caused by the dysregulation of the processes mediating angiogenesis. In particular, abnormal angiogenesis associated disease refers to atherosclerosis, hypertension, tumor growth, inflammation, rheumatoid arthritis, wet-form macular degeneration, choroidal neovascularization, retinal neovascularization, and diabetic retinopathy.

'Compound(s) of the invention', and equivalent expressions, are meant to embrace compounds of the Formula(e) as herein described, which expression includes the pharmaceutically acceptable salts, and the solvates, *e.g.* hydrates, and the solvates of the pharmaceutically acceptable salts where the context so permits. Similarly, reference to intermediates, whether or not they themselves are claimed, is meant to embrace their salts, and solvates, where the context so permits.

When ranges are referred to herein, for example but without limitation, C₁-₈ alkyl, the citation of a range should be considered a representation of each member of said range.

Other derivatives of the compounds of this invention have activity in both their acid and acid derivative forms, but in the acid sensitive form often offers advantages of solubility, tissue compatibility, or delayed release in the mammalian organism (Bundgaard 1991). Prodrugs include acid derivatives well know to practitioners of the art, such as, for example, esters prepared by reaction of the parent acid with a suitable alcohol, or amides prepared by reaction of the parent acid compound with a substituted or unsubstituted amine, or acid anhydrides, or mixed anhydrides. Simple aliphatic or aromatic esters, amides and anhydrides derived from acidic groups pendant on the compounds of this invention are particularly useful prodrugs. In some cases it is desirable to prepare double ester type prodrugs such as (acyloxy)alkyl esters or ((alkoxycarbonyl)oxy)alkylesters. Particular such prodrugs are the C₁-₈ alkyl, C₂-₈ alkenyl, C₆-₁₀ optionally substituted aryl, and (C₆-₁₀ aryl)-(C₁₋₄ alkyl) esters of the compounds of the invention.

The present disclosure includes all isotopic forms of the compounds of the invention provided herein, whether in a form (i) wherein all atoms of a given atomic number have a mass number (or mixture of mass numbers) which predominates in nature (referred to herein as the "natural isotopic form") or (ii) wherein one or more atoms are replaced by atoms having the same atomic number, but a mass number different from the mass number of atoms which predominates in nature (referred to herein as an "unnatural variant isotopic form"). It is understood that an atom may naturally exists as a mixture of mass numbers. The term "unnatural variant isotopic form" also includes embodiments in which the proportion of an atom of given atomic number having a mass number found less commonly in nature (referred to herein as an "uncommon isotope") has been increased relative to that which is naturally occurring e.g. to the level of >20%, >50%, >75%, >90%, >95% or> 99% by number of the atoms of that atomic number (the latter embodiment referred to as an "isotopically enriched variant form"). The term "unnatural variant isotopic form" also includes embodiments in which the proportion of an uncommon isotope has been reduced relative to that which is naturally occurring. Isotopic forms may include radioactive forms (i.e. they incorporate radioisotopes) and non-radioactive forms. Radioactive forms will typically be isotopically enriched variant forms.

An unnatural variant isotopic form of a compound may thus contain one or more artificial or uncommon isotopes such as deuterium (²H or D), carbon-11 (¹¹C), carbon-13 (¹³C), carbon-14 (¹⁴C), nitrogen-13 (¹³N), nitrogen-15 (¹⁵N), oxygen-15 (¹⁵O), oxygen-17 (¹⁷O), oxygen-18 (¹⁸O), phosphorus-32 (³²P), sulphur-35 (³⁵S), chlorine-36 (³⁶Cl), chlorine-37 (³⁷Cl), fluorine-18 (¹⁸F) iodine-123 (¹²³I), iodine-125 (¹²⁵I) in one or more atoms or may contain an increased proportion of said isotopes as compared with the proportion that predominates in nature in one or more atoms.

Unnatural variant isotopic forms comprising radioisotopes may, for example, be used for drug and/or substrate tissue distribution studies. The radioactive isotopes tritium, i.e. ³H, and carbon-14, i.e. ¹⁴C, are particularly useful for this purpose in view of their ease of incorporation and ready means of detection. Unnatural variant isotopic forms which incorporate deuterium i.e ²H or D may afford certain therapeutic advantages resulting from greater metabolic stability, for example, increased in vivo half-life or reduced dosage requirements, and hence may be preferred in some circumstances. Further, unnatural variant isotopic forms may be prepared which incorporate positron emitting isotopes, such as ¹¹C, ¹⁸F, ¹⁵0 and ¹³N, and would be useful in Positron Emission Topography (PET) studies for examining substrate receptor occupancy.

It is also to be understood that compounds that have the same molecular formula but differ in the nature or sequence of bonding of their atoms or the arrangement of their atoms in space are termed 'isomers'. Isomers that differ in the arrangement of their atoms in space are termed 'stereoisomers'.

Stereoisomers that are not mirror images of one another are termed 'diastereomers' and those that are non-superimposable mirror images of each other are termed 'enantiomers'. When a compound has an asymmetric center, for example, it is bonded to four different groups, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory (i.e. as (+) or (-)-isomers respectively). A chiral compound can exist as either individual enantiomer or as a mixture thereof. A mixture containing equal proportions of the enantiomers is called a 'racemic mixture'.

'Tautomers' refer to compounds that are interchangeable forms of a particular compound structure, and that vary in the displacement of hydrogen atoms and electrons. Thus, two structures may be in equilibrium through the movement of π electrons and an atom (usually H). For example, enols and ketones are tautomers because they are rapidly interconverted by treatment with either acid or base. Another example of tautomerism is the aci- and nitro- forms of phenylnitromethane, that are likewise formed by treatment with acid or base.

Tautomeric forms may be relevant to the attainment of the optimal chemical reactivity and biological activity of a compound of interest.

The compounds of the invention may possess one or more asymmetric centers; such compounds can therefore be produced as individual (R)- or (S)- stereoisomers or as mixtures thereof.

Unless indicated otherwise, the description or naming of a particular compound in the specification and claims is intended to include both individual enantiomers and mixtures, racemic or otherwise, thereof. The methods for the determination of stereochemistry and the separation of stereoisomers are well-known in the art.

It will be appreciated that compounds of the invention may be metabolized to yield biologically active metabolites.

### THE INVENTION

The present invention relates to compounds that may be useful in the prophylaxis and/or treatment of fibrotic diseases, proliferative diseases, inflammatory diseases, autoimmune diseases, respiratory diseases, cardiovascular diseases, neurodegenerative diseases, dermatological disorders, pain, and/or abnormal angiogenesis associated diseases. In particular, the compound of the invention may inhibit autotaxin, also known as ectonucleotide pyrophosphatase/phosphodiesterase 2 (NPP2 or ENPP2). The present invention also provides methods for the production of the compound of the invention, pharmaceutical compositions comprising the compound of the invention, methods for the prophylaxis and/or treatment of fibrotic diseases, proliferative diseases, inflammatory diseases, autoimmune diseases, respiratory diseases, cardiovascular diseases, neurodegenerative diseases, dermatological disorders, pain, and/or abnormal angiogenesis associated diseases.by administering the compounds of the invention.

Accordingly, in a first aspect of the invention, the compounds of the invention are provided having a Formula I: 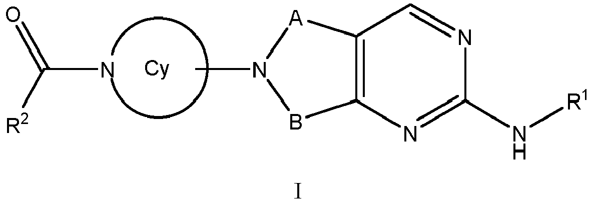 wherein
each A and B is independently C₁₋₄ alkylenyl optionally substituted with one oxo;
R¹ is:
   - 6-10 membered monocyclic or fused bicyclic aryl, optionally substituted with one or more independently selected R^{3a} groups, or
   - 5-10 membered monocyclic or fused bicyclic heteroaryl comprising one or more heteroatoms independently selected from N, O and S, which heteroaryl is optionally substituted with one or more independently selected R^{3b} groups,
R² is
   - phenyl optionally substituted with one or more independently selected R⁵ groups, or
   - 5-10 membered monocyclic or fused bicyclic heteroaryl comprising one or more heteroatoms independently selected from N, O and S, which heteroaryl is optionally substituted with one or more independently selected R⁵ groups;
each R^{3a} and R^{3b} is independently selected from:
   - halo,
   - C₁₋₄ alkyl optionally substituted with one or more independently selected halo,
   - C₁₋₄ alkoxy optionally substituted with one or more independently selected halo,
   - -SF₅,
   - -CN,
   - -O-C₃₋₇ cycloalkyl, and
   - -C(=O)NH₂;
Cy is:
   - 4-10 membered monocyclic, bridged bicyclic, or spiro bicyclic heterocycloalkyl comprising one N atom, which heterocycloalkyl is optionally substituted with one or more independently selected R⁴ groups, or
   - 4-10 membered monocyclic, bridged bicyclic, or spiro bicyclic heterocycloalkenyl comprising one double bond and one N atom, which heterocycloalkenyl is optionally substituted with one or more independently selected R⁴ groups;
each R⁴ is independently selected from:
   - C₁₋₄ alkyl optionally substituted with one or more independently selected halo,
   - -C(=O)O-C₁₋₄ alkyl,
   - halo,
   - -CN, and
   - -OH;
each R⁵ is independently selected from:
   - -OH,
   - -SH,
   - halo,
   - C₁₋₄ alkyl optionally substituted with one or more independently selected -OH, or halo,
   - C₁₋₄ alkoxy optionally substituted with one or more independently selected -OH, or halo,
   - -NH-S(=O)₂-C₁₋₄ alkyl, which alkyl is optionally substituted with one or more independently selected halo, or C₁₋₄ alkoxy,
   - -NH-S(=O)₂-C₃₋₇ cycloalkyl,
   - -NH-C(=O)C₁₋₄ alkyl,
   - -C(=O)OH,
   - -C(=O)NH₂,
   - -C(=O)O-C₁₋₄ alkyl which alkyl is optionally substituted with one 5-6 membered monocyclic heterocycloalkenyl comprising one double bond and one or two O atoms, which heterocycloalkenyl is optionally substituted with one or more independently selected C₁₋₄ alkyl or oxo,
   - -C(=O)-NH-S(=O)₂-C₁₋₄ alkyl,
   - -S(=O)₂NH₂, and
   - 5-6 membered monocyclic heteroaryl comprising one or more heteroatoms independently selected from N, O and S.

In one embodiment the compound of the invention is according to Formula I, wherein A is C₁₋₄ alkylenyl optionally substituted with one oxo. In a particular embodiment, A is -CH₂-, -C(=O)-, -CH₂-CH₂-, -CH(CH₃)-, or -C(CH₃)₂-. In a more particular embodiment, A is -CH₂-.

In one embodiment the compound of the invention is according to Formula I, wherein B is C₁₋₄ alkylenyl. In a particular embodiment, B is -CH₂-, -CH₂-CH₂-, -CH(CH₃)-, or -C(CH₃)₂-. In a more particular embodiment, B is -CH₂- or -CH₂-CH₂-. In a most particular embodiment, B is -CH₂-.

In one embodiment the compound of the invention is according to Formula IIa or IIb: 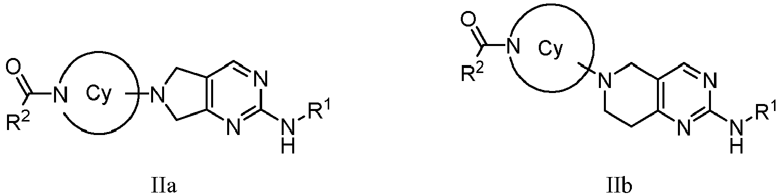 wherein R¹, R², and Cy are as previously defined.

In one embodiment the compound of the invention is according to any one of Formulae I-IIb, wherein Cy is 4-10 membered monocyclic, bridged bicyclic, or spiro bicyclic heterocycloalkyl comprising one N atom. In a particular embodiment, Cy is azetidinyl, pyrrolidinyl, piperidinyl, azabicyclo[3.1.1]heptanyl, azaspiro[3.3]heptanyl, or azabicyclo[3.2.1]octanyl. In a more particular embodiment, Cy is azetidinyl, or piperidinyl. In a most particular embodiment, Cy is piperidinyl.

In one embodiment the compound of the invention is according to any one of Formulae I-IIb, wherein Cy is 4-10 membered monocyclic, bridged bicyclic, or spiro bicyclic heterocycloalkyl comprising one N atom, which heterocycloalkyl is substituted with one or more independently selected R⁴ groups. In a particular embodiment, Cy is azetidinyl, pyrrolidinyl, piperidinyl, azabicyclo[3.1.1]heptanyl, azaspiro[3.3]heptanyl, or azabicyclo[3.2.1]octanyl, each of which is substituted with one or more independently selected R⁴ groups. In a more particular embodiment, Cy is azetidinyl, or piperidinyl, each of which is substituted with one or more independently selected R⁴ groups. In a most particular embodiment, Cy is piperidinyl substituted with one or more independently selected R⁴ groups.

In one embodiment the compound of the invention is according to any one of Formulae I-IIb, wherein Cy is 4-10 membered monocyclic, bridged bicyclic, or spiro bicyclic heterocycloalkenyl comprising one double bond and one N atom. In a more particular embodiment, Cy is dihydro-1H-pyrrole or tetrahydropyridinyl.

In one embodiment the compound of the invention is according to any one of Formulae I-IIb, wherein Cy is 4-10 membered monocyclic, bridged bicyclic, or spiro bicyclic heterocycloalkenyl comprising one double bond and one N atom, which heterocycloalkenyl is substituted with one or more independently selected R⁴ groups. In a particular embodiment, Cy is dihydro-1H-pyrrole or tetrahydropyridinyl, each of which is substituted with one or more independently selected R⁴ groups.

In one embodiment the compound of the invention is according to any one of Formulae I-IIb, wherein Cy is as previously defined, and one or more independently selected R⁴ is C₁₋₄ alkyl optionally substituted with one or more independently selected halo. In a particular embodiment, one or more independently selected R⁴ is -CH₃, -CH₂CH₃, or -CF₃. In a more particular embodiment, one or more independently selected R⁴ is -CH₃.

In one embodiment the compound of the invention is according to any one of Formulae I-IIb, wherein Cy is as previously defined, and one or more independently selected R⁴ is -C(=O)O-C₁₋₄ alkyl. In a particular embodiment, one or more independently selected R⁴ is -C(=O)O-CH₃.

In one embodiment the compound of the invention is according to any one of Formulae I-IIb, wherein Cy is as previously defined, and one or more independently selected R⁴ is halo. In a particular embodiment, one or more independently selected R⁴ is F.

In one embodiment the compound of the invention is according to any one of Formulae I-IIb, wherein Cy is as previously defined, and one or more independently selected R⁴ is -CN.

In one embodiment the compound of the invention is according to any one of Formulae I-IIb, wherein Cy is as previously defined, and one or more independently selected R⁴ is -OH.

In one embodiment, the compound of the invention is according to any one of Formulae I-IIb, wherein Cy is:

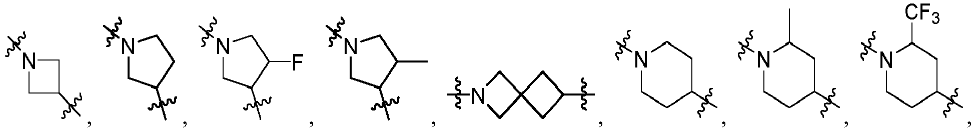

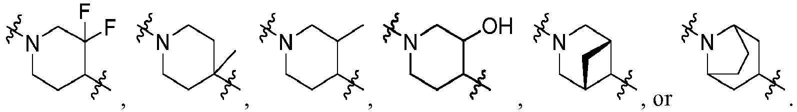

In one embodiment, the compound of the invention is according to any one of Formulae I-IIb, wherein R¹ is 6-10 membered monocyclic or fused bicyclic aryl. In a particular embodiment, R¹ is phenyl.

In one embodiment, the compound of the invention is according to any one of Formulae I-IIb, wherein R¹ is 6-10 membered monocyclic or fused bicyclic aryl substituted with one or more independently selected R^{3a} groups. In a particular embodiment, R¹ is phenyl substituted with one or more independently selected R^{3a} groups. In a more particular embodiment, R¹ is phenyl substituted with one or two independently selected R^{3a} groups.

In one embodiment, the compound of the invention is according to any one of Formulae I-IIb, wherein R¹ is as defined previously, and one or more independently selected R^{3a} is halo. In a particular embodiment, one or more independently selected R^{3a} is F or Cl. In a more particular embodiment, one or more independently selected R^{3a} is F.

In one embodiment, the compound of the invention is according to any one of Formulae I-IIb, wherein R¹ is as defined previously, and one or more independently selected R^{3a} is C₁₋₄ alkyl optionally substituted with one or more independently selected halo. In a particular embodiment, one or more independently selected R^{3a} is -CH₃, -CH₂CH₃, -CH(CH₃)₂, -CF₃, or -CH₂CF₃. In a more particular embodiment, one or more independently selected R^{3a} is -CH₃.

In one embodiment, the compound of the invention is according to any one of Formulae I-IIb, wherein R¹ is as defined previously, and one or more independently selected R^{3a} is C₁₋₄ alkoxy optionally substituted with one or more independently selected halo. In a particular embodiment, one or more independently selected R^{3a} is -OCH₃, -OCH₂CH₃, -OCH(CH₃)₂, -OCF₃, or -OCH₂CF₃.

In one embodiment, the compound of the invention is according to any one of Formulae I-IIb, wherein R¹ is as defined previously, and one or more independently selected R^{3a} is -SF₅.

In one embodiment, the compound of the invention is according to any one of Formulae I-IIb, wherein R¹ is as defined previously, and one or more independently selected R^{3a} is -CN.

In one embodiment, the compound of the invention is according to any one of Formulae I-IIb, wherein R¹ is as defined previously, and one or more independently selected R^{3a} is -O-C₃₋₇ cycloalkyl. In a particular embodiment, one or more independently selected R^{3a} is -O-cyclopropyl.

In one embodiment, the compound of the invention is according to any one of Formulae I-IIb, wherein R¹ is as defined previously, and one or more independently selected R^{3a} is -C(=O)NH₂.

In one embodiment, the compound of the invention is according to any one of Formulae I-IIb, wherein R¹ is 5-10 membered monocyclic or fused bicyclic heteroaryl comprising one or more heteroatoms independently selected from N, O and S. In a particular embodiment, R¹ is pyridinyl, or pyrazolyl.

In one embodiment, the compound of the invention is according to any one of Formulae I-IIb, wherein R¹ is 5-10 membered monocyclic or fused bicyclic heteroaryl comprising one or more heteroatoms independently selected from N, O and S, which heteroaryl is optionally substituted with one or more independently selected R^{3b} groups. In a particular embodiment, R¹ is pyridinyl, or pyrazolyl, each of which is substituted with one or more independently selected R^{3b} groups. In a particular embodiment, R¹ is pyridinyl, or pyrazolyl, each of which is substituted with one or two independently selected R^{3b} groups.

In one embodiment, the compound of the invention is according to any one of Formulae I-IIb, wherein R¹ is as defined previously, and one or more independently selected R^{3b} is halo. In a particular embodiment, one or more independently selected R^{3b} is F or Cl. In a more particular embodiment, one or more independently selected R^{3b} is F.

In one embodiment, the compound of the invention is according to any one of Formulae I-IIb, wherein R¹ is as defined previously, and one or more independently selected R^{3b} is C₁₋₄ alkyl optionally substituted with one or more independently selected halo. In a particular embodiment, one or more independently selected R^{3b} is -CH₃, -CH₂CH₃, -CH(CH₃)₂, -CF₃, or -CH₂CF₃. In a more particular embodiment, one or more independently selected R^{3b} is -CH₃.

In one embodiment, the compound of the invention is according to any one of Formulae I-IIb, wherein R¹ is as defined previously, and one or more independently selected R^{3b} is C₁₋₄ alkoxy optionally substituted with one or more independently selected halo. In a particular embodiment, one or more independently selected R^{3b} is -OCH₃, -OCH₂CH₃, -OCH(CH₃)₂, -OCF₃, or -OCH₂CF₃.

In one embodiment, the compound of the invention is according to any one of Formulae I-IIb, wherein R¹ is as defined previously, and one or more independently selected R^{3b} is -SF₅.

In one embodiment, the compound of the invention is according to any one of Formulae I-IIb, wherein R¹ is as defined previously, and one or more independently selected R^{3b} is -CN.

In one embodiment, the compound of the invention is according to any one of Formulae I-IIb, wherein R¹ is as defined previously, and one or more independently selected R^{3b} is -O-C₃₋₇ cycloalkyl. In a particular embodiment, one or more independently selected R^{3b} is -O-cyclopropyl.

In one embodiment, the compound of the invention is according to any one of Formulae I-IIb, wherein R¹ is as defined previously, and one or more independently selected R^{3b} is -C(=O)NH₂.

In one embodiment, the compound of the invention is according to any one of Formulae I-IIb, wherein R¹ is:

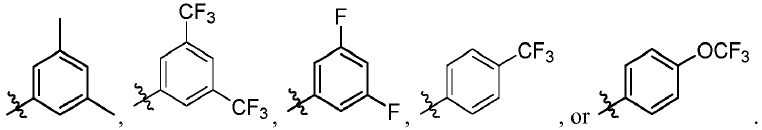

In one embodiment the compound of the invention is according to Formula IIIa or IIIb: 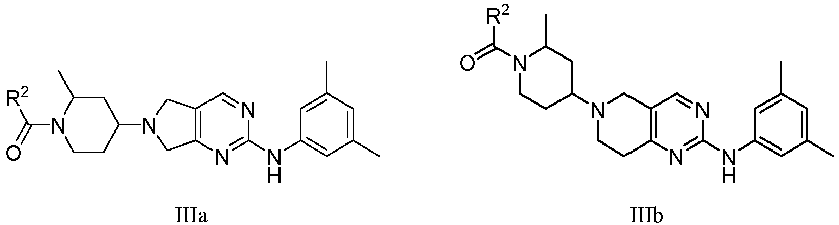 wherein R² is as previously defined.

In one embodiment, the compound of the invention is according to any one of Formulae I-IIIb, wherein R² is phenyl.

In one embodiment, the compound of the invention is according to any one of Formulae I-IIIb, wherein R² is phenyl substituted with one or more independently selected R⁵ groups. In a particular embodiment, R² is phenyl substituted with one or two independently selected R⁵ groups. In a more particular embodiment, R² is phenyl substituted with one R⁵ group.

In one embodiment, the compound of the invention is according to any one of Formulae I-IIIb, wherein R² is 5-10 membered monocyclic or fused bicyclic heteroaryl comprising one or more heteroatoms independently selected from N, O and S. In a particular embodiment, R² is indazolyl, benzoimidazolyl, or benzotriazolyl. In a more particular embodiment, R² is benzotriazolyl. In a most particular embodiment, R² is

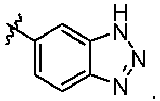

In one embodiment, the compound of the invention is according to any one of Formulae I-IIIb, wherein R² is 5-10 membered monocyclic or fused bicyclic heteroaryl comprising one or more heteroatoms independently selected from N, O and S, which heteroaryl is substituted with one or more independently selected R⁵ groups. In a particular embodiment, R² is pyridinyl, benzotriazolyl, benzoxazolyl, benzothiazolyl, benzoimidazolyl, or indazolyl, each of which is substituted with one or more independently selected R⁵ groups. In a more particular embodiment, R² is pyridinyl, benzotriazolyl, benzoxazolyl, benzothiazolyl, benzoimidazolyl, or indazolyl, each of which is substituted with one or two independently selected R⁵ groups. In a further more particular embodiment, R² is pyridinyl, benzotriazolyl, benzoxazolyl, benzothiazolyl, benzoimidazolyl, or indazolyl, each of which is substituted with one R⁵ group. In a most particular embodiment, R² is pyridinyl substituted with one R⁵ group.

In one embodiment, the compound of the invention is according to any one of Formulae I-IIIb, wherein R² is as previously described, and one or more independently selected R⁵ is -OH.

In one embodiment, the compound of the invention is according to any one of Formulae I-IIIb, wherein R² is as previously described, and one or more independently selected R⁵ is -SH.

In one embodiment, the compound of the invention is according to any one of Formulae I-IIIb, wherein R² is as previously described, and one or more independently selected R⁵ is halo. In a particular embodiment, one or more independently selected R⁵ is F.

In one embodiment, the compound of the invention is according to any one of Formulae I-IIIb, wherein R² is as previously described, and one or more independently selected R⁵ is C₁₋₄ alkyl optionally substituted with one or more independently selected -OH, or halo. In a particular embodiment, one or more independently selected R⁵ is -CH₃, -CH₂CH₃, or -CH(CH₃)₂, each of which is optionally substituted with one or more independently selected -OH, or halo. In a more particular embodiment, one or more independently selected R⁵ is -CH₃, -CF₃, -CH₂CH₃, -CH₂OH, or -C(CH₃)₂OH.

In one embodiment, the compound of the invention is according to any one of Formulae I-IIIb, wherein R² is as previously described, and one or more independently selected R⁵ is C₁₋₄ alkoxy optionally substituted with one or more independently selected -OH, or halo. In a particular embodiment, one or more independently selected R⁵ is -OCH₃, -OCH₂CH₃, or -OCH(CH₃)₂, each of which is optionally substituted with one or more independently selected -OH, or halo. In a more particular embodiment, one or more independently selected R⁵ is -OCH₃, -OCF₃, -OCH₂CH₃.

In one embodiment, the compound of the invention is according to any one of Formulae I-IIIb, wherein R² is as previously described, and one or more independently selected R⁵ is -NH-S(=O)₂-C₁₋₄ alkyl, which alkyl is optionally substituted with one or more independently selected halo, or C₁₋₄ alkoxy. In a more particular embodiment, one or more independently selected R⁵ is -NH-S(=O)₂-CH₃, or -NH-S(=O)₂-CH₂CH₃, which alkyl is optionally substituted with one or more independently selected halo, or C₁₋₄ alkoxy. In a more particular embodiment, one or more independently selected R⁵ is -NH-S(=O)₂-CH₃, or -NH-S(=O)₂-CH₂CH₃, which alkyl is optionally substituted with one or more independently selected F, -OCH₃ or -OCH₂CH₃. In a most particular embodiment, one or more independently selected R⁵ is -NH-S(=O)₂-CH₃, -NH-S(=O)₂-CH₂CH₃, -NH-S(=O)₂-CH₂CF₃, or -NH-S(=O)₂-CH₂CH₂OCH₃.

In one embodiment, the compound of the invention is according to any one of Formulae I-IIIb, wherein R² is as previously described, and one or more independently selected R⁵ is -NH-S(=O)₂-C₃₋₇ cycloalkyl. In a particular embodiment, one or more independently selected R⁵ is -NH-S(=O)₂-cyclopropyl.

In one embodiment, the compound of the invention is according to any one of Formulae I-IIIb, wherein R² is as previously described, and one or more independently selected R⁵ is -NH-C(=O)C₁₋₄ alkyl. in a particular embodiment, one or more independently selected R⁵ is -NH-C(=O)CH₃, -NH-C(=O)CH₂CH₃ or -NH-C(=O)CH(CH₃)₂.

In one embodiment, the compound of the invention is according to any one of Formulae I-IIIb, wherein R² is as previously described, and one or more independently selected R⁵ is -C(=O)OH.

In one embodiment, the compound of the invention is according to any one of Formulae I-IIIb, wherein R² is as previously described, and one or more independently selected R⁵ is -C(=O)NH₂.

In one embodiment, the compound of the invention is according to any one of Formulae I-IIIb, wherein R² is as previously described, and one or more independently selected R⁵ is -C(=O)O-C₁₋₄ alkyl which alkyl is optionally substituted with one 5-6 membered monocyclic heterocycloalkenyl comprising one double bond and one or two O atoms, which heterocycloalkenyl is optionally substituted with one or more independently selected C₁₋₄ alkyl or oxo In a particular embodiment, one or more independently selected R⁵ is -C(=O)O-CH₃, or -C(=O)O-CH₂CH₃, each of which is optionally substituted with one 5-6 membered monocyclic heterocycloalkenyl comprising one double bond and one or two O atoms, which heterocycloalkenyl is optionally substituted with one or more independently selected C₁₋₄ alkyl or oxo. In a more particular embodiment, one or more independently selected R⁵ is -C(=O)O-CH₃, or -C(=O)O-CH₂CH₃, each of which is optionally substituted with

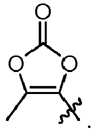

In one embodiment, the compound of the invention is according to any one of Formulae I-IIIb, wherein R² is as previously described, and one or more independently selected R⁵ is -C(=O)-NH-S(=O)₂-C₁₋₄ alkyl. In a particular embodiment, one or more independently selected R⁵ is -C(=O)-NH-S(=O)₂-CH₃, -C(=O)-NH-S(=O)₂-CH₂CH₃ or -C(=O)-NH-S(=O)₂-CH(CH₃)₂.

In one embodiment, the compound of the invention is according to any one of Formulae I-IIIb, wherein R² is as previously described, and one or more independently selected R⁵ is -S(=O)₂NH₂.

In one embodiment, the compound of the invention is according to any one of Formulae I-IIIb, wherein R² is as previously described, and one or more independently selected R⁵ is 5-6 membered monocyclic heteroaryl comprising one or more heteroatoms independently selected from N, O and S. In a particular embodiment, one or more independently selected R⁵ is tetrazolyl.

In one embodiment, the compound of the invention is according to Formula I, wherein the compound is selected from:
6-[3-[2-(3,5-dimethylanilino)-7,8-dihydro-5H-pyrido[4,3-d]pyrimidin-6-yl]azetidine-1-carbonyl]-3H-1,3-benzoxazol-2-one,
1H-benzotriazol-5-yl-[3-[2-(3,5-dimethylanilino)-7,8-dihydro-5H-pyrido[4,3-d]pyrimidin-6-yl]azetidin-1-yl]methanone,
6-[3-[2-(3,5-dimethylanilino)-5,7-dihydropyrrolo[3,4-d]pyrimidin-6-yl]azetidine-1-carbonyl]-3H-1,3-benzoxazol-2-one,
6-[3-[2-(3,5-dimethylanilino)-7,8-dihydro-5H-pyrido[4,3-d]pyrimidin-6-yl]pyrrolidine-1-carbonyl]-3H-1,3-benzoxazol-2-one,
1H-benzotriazol-5-yl-[3-[2-(3,5-dimethylanilino)-7,8-dihydro-5H-pyrido[4,3-d]pyrimidin-6-yl]pyrrolidin-1-yl]methanone,
6-[3-[2-[3,5-bis(trifluoromethyl)anilino]-5,7-dihydropyrrolo[3,4-d]pyrimidin-6-yl]azetidine-1-carbonyl]-3H-1,3-benzoxazol-2-one,
6-[4-[2-(3,5-dimethylanilino)-7,8-dihydro-5H-pyrido[4,3-d]pyrimidm-6-yl]piperidme-1-carbonyl]-3H-1,3-benzoxazol-2-one,
6-[3-[2-[3,5-bis(trifluoromethyl)anilino]-7,8-dihydro-5H-pyrido[4,3-d]pyrimidin-6-yl]azetidine-1-carbonyl]-3H-1,3-benzoxazol-2-one,
1H-benzotriazol-5-yl-[3-[2-[3,5-bis(trifluoromethyl)anilino]-5,7-dihydropyrrolo[3,4-d]pyrimidin-6-yl]azetidin-1-yl]methanone,
1H-benzotriazol-5-yl-[4-[2-(3,5-dimethylanilino)-5,7-dihydropyrrolo[3,4-d]pyrimidin-6-yl]-1-piperidyl]methanone,
6-[4-[2-(3,5-dimethylanilino)-5,7-dihydropyrrolo[3,4-d]pyrimidin-6-yl]piperidine-1-carbonyl]-3H-1,3-benzoxazol-2-one,
6-[3-[2-(3,5-dimethylanilino)-5,7-dihydropyrrolo[3,4-d]pyrimidin-6-yl]pyrrolidine-1-carbonyl]-3H-1,3-benzoxazol-2-one,
1H-benzotriazol-5-yl-[3-[2-(3,5-dimethylanilino)-5,7-dihydropyrrolo[3,4-d]pyrimidin-6-yl]pyrrolidin-1-yl]methanone,
6-[3-[2-[3-chloro-5-(trifluoromethyl)anilino]-7,8-dihydro-5H-pyrido[4,3-d]pyrimidin-6-yl]azetidine-1-carbonyl]-3H-1,3-benzoxazol-2-one,
6-[3-[2-(3-chloro-5-methyl-anilino)-7,8-dihydro-5H-pyrido[4,3-d]pyrimidin-6-yl]azetidine-1-carbonyl]-3H-1,3-benzoxazol-2-one,
1H-benzotriazol-5-yl-[3-[2-(3-chloro-5-methyl-anilino)-7,8-dihydro-5H-pyrido[4,3-d]pyrimidin-6-yl]azetidin-1-yl]methanone,
6-[3-[2-[3-chloro-5-(trifluoromethyl)anilino]-5,7-dihydropyrrolo[3,4-d]pyrimidin-6-yl]azetidine-1-carbonyl]-3H-1,3-benzoxazol-2-one,
1H-benzotriazol-5-yl-[3-[2-(3,5-dimethylanilino)-5,7-dihydropyrrolo[3,4-d]pyrimidin-6-yl]azetidin-1-yl]methanone,
[3-[2-(3,5-dimethylanilino)-5,7-dihydropyrrolo[3,4-d]pyrimidin-6-yl]azetidin-1-yl]-(1H-indazol-5-yl)methanone,
3-[3-[2-(3,5-dimethylanilino)-5,7-dihydropyrrolo[3,4-d]pyrimidin-6-yl]azetidine-1-carbonyl]benzenesulfonamide,
6-[3-[2-(3,5-dimethoxyanilino)-5,7-dihydropyrrolo[3,4-d]pyrimidin-6-yl]azetidine-1-carbonyl]-3H-1,3-benzoxazol-2-one,
1H-benzotriazol-5-yl-[3-[2-[3-chloro-5-(trifluoromethyl)anilino]-5,7-dihydropyrrolo[3,4-d]pyrimidin-6-yl]azetidin-1-yl]methanone,
6-[3-[2-(3-methoxy-5-methyl-anilino)-5,7-dihydropyrrolo[3,4-d]pyrimidin-6-yl]azetidine-1-carbonyl]-3H-1,3-benzoxazol-2-one,
1H-benzotriazol-5-yl-[3-[2-(3-methoxy-5-methyl-anilino)-5,7-dihydropyrrolo[3,4-d]pyrimidin-6-yl]azetidin-1-yl]methanone,
3-[3-[2-(3,5-dimethylanilino)-5,7-dihydropyrrolo[3,4-d]pyrimidin-6-yl]azetidine-1-carbonyl]benzoic acid,
5-[3-[2-(3,5-dimethylanilino)-5,7-dihydropyrrolo[3,4-d]pyrimidin-6-yl]azetidine-1-carbonyl]-1H-pyridin-2-one,
1H-benzotriazol-5-yl-[4-[2-(3,5-dimethoxyanilino)-5,7-dihydropyrrolo[3,4-d]pyrimidin-6-yl]-1-piperidyl]methanone,
6-[3-[2-[3-methyl-5-(trifluoromethyl)anilino]-5,7-dihydropyrrolo[3,4-d]pyrimidin-6-yl]azetidine-1-carbonyl]-3H-1,3-benzoxazol-2-one,
3H-benzotriazol-5-yl-[3-[2-[3-methyl-5-(trifluoromethyl)anilino]-5,7-dihydropyrrolo[3,4-d]pyrimidin-6-yl]azetidin-1-yl]methanone,
1H-benzotriazol-5-yl-[3-[2-[4-(trifluoromethyl)anilino]-5,7-dihydropyrrolo[3,4-d]pyrimidin-6-yl]azetidin-1-yl]methanone,
1H-benzotriazol-5-yl-[3-[2-(3,5-dimethoxyanilino)-5,7-dihydropyrrolo[3,4-d]pyrimidin-6-yl]azetidin-1-yl]methanone,
4-[3-[2-(3,5-dimethylanilino)-5,7-dihydropyrrolo[3,4-d]pyrimidin-6-yl]azetidine-1-carbonyl]-1H-pyridin-2-one,
1H-benzotriazol-5-yl-[3-[2-(3,5-dimethylanilino)-5,7-dihydropyrrolo[3,4-d]pyrimidin-6-yl]-1-piperidyl]methanone,
1H-benzotriazol-5-yl-[(2S,4S/2R,4R)-4-[2-(3,5-dimethylanilino)-5,7-dihydropyrrolo[3,4-d]pyrimidin-6-yl]-2-methyl-1-piperidyl]methanone,
6-[4-[2-(3,5-dimethylanilino)-5,7-dihydropyrrolo[3,4-d]pyrimidin-6-yl]-2-methyl-piperidine-1-carbonyl]-3H-1,3-benzoxazol-2-one,
1H-benzotriazol-5-yl-[(3R,4S/3S,4R)-4-[2-(3,5-dimethylanilino)-5,7-dihydropyrrolo[3,4-d]pyrimidin-6-yl]-3-methyl-1-piperidyl]methanone,
1H-benzotriazol-5-yl-[4-[2-(3,5-dimethylanilino)-5,7-dihydropyrrolo[3,4-d]pyrimidin-6-yl]-3,3-difluoro-1-piperidyl]methanone,
4-[3-[2-(3,5-dimethylanilino)-5,7-dihydropyrrolo[3,4-d]pyrimidin-6-yl]azetidine-1-carbonyl]benzenesulfonamide,
6-[3-[2-(3,5-dimethylanilino)-5,7-dihydropyrrolo[3,4-d]pyrimidin-6-yl]piperidine-1-carbonyl]-3H-1,3-benzoxazol-2-one,
1H-benzotriazol-5-yl-[4-[2-(3,5-dimethylanilino)-5,7-dihydropyrrolo[3,4-d]pyrmidin-6-yl]-3-fluoro-1-piperidyl]methanone,
1H-benzotriazol-5-yl-[6-[2-(3,5-dimethylanilino)-5,7-dihydropyrrolo[3,4-d]pyrmidin-6-yl]-2-azaspiro[3.3]heptan-2-yl]methanone,
1H-benzotriazol-5-yl-[3-[2-(3,5-dimethoxyanilino)-5,7-dihydropyrrolo[3,4-d]pyrimidin-6-yl]pyrrolidin-1-yl]methanone,
1H-benzotriazol-5-yl-[3-[2-[(5-methyl-3-pyridyl)ammo]-5,7-dihydropyrrolo[3,4-d]pyrimidin-6-yl]azetidin-1-yl]methanone,
1H-benzotriazol-5-yl-[3-[2-[(5-methyl-3-pyridyl)amino]-5,7-dihydropyrrolo[3,4-d]pyrimidin-6-yl]pyrrolidin-1-yl]methanone,
4-[3-[2-[(5-methyl-3 -pyridyl)amino]-5,7-dihydropyrrolo[3,4-d]pyrimidin-6-yl]pyrrolidine-1-carbonyl]benzenesulfonamide,
1H-benzotriazol-5-yl-[3-[2-[(5-tert-butyl-2-methyl-pyrazol-3-yl)amino]-5,7-dihydropyrrolo[3,4-d]pyrimidin-6-yl] azetidin-1 -yl]methanone,
4-[4-[2-[(5-methyl-3-pyridyl)amino]-5,7-dihydropyrrolo[3,4-d]pyrimidin-6-yl]piperidine-1-carbonyl]benzenesulfonamide,
4-[3-[2-[(5-methyl-3-pyridyl)amino]-5,7-dihydropyrrolo[3,4-d]pyrimidin-6-yl]azetidine-1-carbonyl]benzenesulfonamide,
1H-benzotriazol-5-yl-[4-[2-[(5-methyl-3-pyridyl)amino]-5,7-dihydropyrrolo[3,4-d]pyrimidin-6-yl]-1-piperidyl]methanone,
1H-benzotriazol-5-yl-[4-[2-[(5-tert-butyl-2-methyl-pyrazol-3-yl)amino]-5,7-dihydropyrrolo[3,4-d]pyrimidin-6-yl]-1-piperidyl]methanone,
1H-benzotriazol-5-yl-[3-[2-[(4,6- dimethyl-2-pyridyl)amino]-5,7-dihydropyrrolo[3,4-d]pyrimidin-6-yl]azetidin-1-yl]methanone,
6-[3-[2-[(4,6-dimethyl-2-pyridyl)amino]-5,7-dihydropyrrolo[3,4-d]pyrimidin-6-yl]azetidine-1-carbonyl]-3H-1,3-benzoxazol-2-one,
1H-benzotriazol-5-yl-[3-[2-(3,5-difluoroanilino)-5,7-dihydropyrrolo[3,4-d]pyrimidin-6-yl]azetidin-1-yl]methanone,
1H-benzotriazol-5-yl-[4-[2-(3,5-difluoroanilino)-5,7-dihydropyrrolo[3,4-d]pyrimidin-6-yl]-1-piperidyl]methanone,
6-[4-[2-(3,5-difluoroanilino)-5,7-dihydropyrrolo[3,4-d]pyrimidin-6-yl]piperidine-1-carbonyl]-3H-1,3-benzoxazol-2-one,
3H-benzotriazol-5-yl-[4-[2-[(4,6-dimethyl-2-pyridyl)amino]-5,7-dihydropyrrolo[3,4-d]pyrimidin-6-yl]-1-piperidyl]methanone,
6-[3-[2-(3,5-difluoroanilino)-5,7-dihydropyrrolo[3,4-d]pyrimidin-6-yl]azetidine-1-carbonyl]-3H-1,3-benzoxazol-2-one,
4-[[6-[1-(1H-benzotriazole-5-carbonyl)azetidin-3-yl]-5,7-dihydropyrrolo[3,4-d]pyrimidin-2-yl] amino]benzonitrile,
4-[[6-[1-(1H-benzotriazole-5-carbonyl)-4-piperidyl]-5,7-dihydropyrrolo[3,4-d]pyrimidin-2-yl]amino]benzonitrile,
6-[4-[2-[(4,6-dimethyl-2-pyridyl)amino]-5,7-dihydropyrrolo[3,4-d]pyrimidin-6-yl]piperidine-1-carbonyl]-3H-1,3-benzoxazol-2-one,
[4-[2-(3,5-dimethylanilino)-5,7-dihydropyrrolo[3,4-d]pyrimidin-6-yl]-1-piperidyl]-(2-thioxo-3H-1,3-benzoxazol-6-yl)methanone,
[3-[2-(3,5-dimethylanilino)-5,7-dihydropyrrolo[3,4-d]pyrimidin-6-yl] azetidin-1-yl]-(2-thioxo-3H-1,3-benzoxazol-6-yl)methanone,
1H-benzotriazol-5-yl-[5-[2-(3,5-dimethylanilino)-5,7-dihydropyrrolo[3,4-d]pyrimidin-6-yl]-2-azabicyclo[2.2.1]heptan-2-yl]methanone,
6-[5-[2-(3,5-dimethylanilino)-5,7-dihydropyrrolo[3,4-d]pyrimidin-6-yl]-2-azabicyclo[2.2.1]heptane-2-carbonyl]-3H-1,3-benzoxazol-2-one,
[4-[2-(3,5-dimethylanilino)-5,7-dihydropyrrolo[3,4-d]pyrimidin-6-yl]-3-methyl-1-piperidyl]-(3H-triazolo[4,5-b]pyridin-6-yl)methanone,
[4-[2-(3,5-dimethylanilino)-5,7-dihydropyrrolo[3,4-d]pyrimidin-6-yl]-1-piperidyl]-(3H-triazolo[4,5-b]pyridin-6-yl)methanone,
[3-[2-(3,5-dimethylanilino)-5,7-dihydropyrrolo[3,4-d]pyrimidin-6-yl]azetidin-1-yl]-(1H-triazolo[4,5-b]pyridin-6-yl)methanone,
1H-benzotriazol-5-yl-[4-[2-(3,5-dimethylanilino)-5,7-dihydropyrrolo[3,4-d]pyrimidin-6-yl]-2,6-dimethyl-1-piperidyl]methanone,
6-[4-[2-(3,5-dimethylanilino)-5,7-dihydropyrrolo[3,4-d]pyrimidin-6-yl]piperidine-1-carbonyl]-3H-1,3-benzothiazol-2-one,
6-[3-[2-(3,5-dimethylanilino)-5,7-dihydropyrrolo[3,4-d]pyrimidin-6-yl]azetidine-1-carbonyl]-3H-1,3-benzothiazol-2-one,
1H-benzotriazol-5-yl-[(2S,4R/2R,4S)-4-[2-(3,5-dimethylanilino)-5,7-dihydropyrrolo[3,4-d]pyrimidin-6-yl]-2-methyl-1-piperidyl]methanone,
N-[4-[4-[2-(3,5-dimethylanilino)-5,7-dihydropyrrolo[3,4-d]pyrimidin-6-yl]piperidine-1-carbonyl]phenyl]methanesulfonamide,
1H-benzotriazol-5-yl-[(2R,4R)-4-[2-(3,5-dimethylanilino)-5,7-dihydropyrrolo[3,4-d]pyrimidin-6-yl]-2-methyl-1-piperidyl]methanone,
1H-benzotriazol-5-yl-[(2S,4S)-4-[2-(3,5-dimethylanilino)-5,7-dihydropyrrolo[3,4-d]pyrimidin-6-yl]-2-methyl-1-piperidyl]methanone,
3H-benzotriazol-5-yl-[4-[2-(3,5-dimethylanilino)-5,7,8,9-tetrahydropyrimido[5,4-c]azepin-6-yl]-1-piperidyl]methanone,
[4-[2-(3,5-dimethylanilino)-5,7,8,9-tetrahydropyrimido[5,4-c]azepin-6-yl]-1-piperidyl]-(3H-triazolo[4,5-b]pyridin-6-yl)methanone,
3H-benzotriazol-5-yl-[3-[2-(3,5-dimethylanilino)-5,7-dihydropyrrolo[3,4-d]pyrimidin-6-yl]-4-methyl-pyrrolidin-1-yl]methanone,
1H-benzotriazol-5-yl-[(1R,5S)-6-[2-(3,5-dimethylanilino)-5,7-dihydropyrrolo[3,4-d]pyrimidin-6-yl]-3-azabicyclo[3.1.1]heptan-3-yl]methanone,
[4-[2-(3,5-dimethylanilino)-5,7-dihydropyrrolo[3,4-d]pyrimidin-6-yl]-1-piperidyl]-(6-fluoro-3H-benzotriazol-5-yl)methanone,
[3-[2-(3,5-dimethylanilino)-5,7-dihydropyrrolo[3,4-d]pyrimidin-6-yl]azetidin-1-yl]-(6-fluoro-3H-benzotriazol-5-yl)methanone,
[3-[2-(3,5-dimethylanilino)-5,7-dihydropyrrolo[3,4-d]pyrimidin-6-yl]azetidin-1-yl]-(6-methoxy-1H-benzotriazol-5-yl)methanone,
[3-[2-(3,5-dimethylanilino)-5,7-dihydropyrrolo[3,4-d]pyrimidin-6-yl]-4-methyl-pyrrolidin-1-yl]-(6-fluoro-1H-benzotriazol-5-yl)methanone,
[3-[2-(3,5-dimethylanilino)-5,7-dihydropyrrolo[3,4-d]pyrimidin-6-yl]-4-methyl-pyrrolidin-1-yl]-(6-methoxy-1H-benzotriazol-5-yl)methanone,
1-(3H-benzotriazole-5-carbonyl)-4-[2-(3,5-dimethylanilino)-5,7-dihydropyrrolo[3,4-d]pyrimidin-6-yl]-2,5-dihydropyrrole-3-carbonitrile,
[3-[2-(3,5-dimethylanilino)-5,7-dihydropyrrolo[3,4-d]pyrimidin-6-yl]azetidin-1-yl]-(1H-triazolo[4,5-b]pyridin-5-yl)methanone,
3H-benzotriazol-5-yl-[3-[2-(3,5-dimethylanilino)-7,8-dihydro-5H-pyrido[4,3-d]pyrimidin-6-yl]-4-methyl-pyrrolidin-1-yl]methanone,
[3-[2-(3,5-dimethylanilino)-7,8-dihydro-5H-pyrido[4,3-d]pyrimidin-6-yl]-4-methyl-pyrrolidin-1-yl]-(6-methoxy-1H-benzotriazol-5-yl)methanone,
1H-benzotriazol-5-yl-[(2R,4S)-4-[2-(3,5-dimethylanilino)-5,7-dihydropyrrolo[3,4-d]pyrimidin-6-yl]-2-methyl-1-piperidyl]methanone,
1H-benzotriazol-5-yl-[(2S,4R)-4-[2-(3,5-dimethylanilino)-5,7-dihydropyrrolo[3,4-d]pyrimidin-6-yl]-2-methyl-1-piperidyl]methanone,
1H-benzotriazol-5-yl-[3-[2-(3,5-dimethylanilino)-5,7,8,9-tetrahydropyrimido[5,4-c]azepin-6-yl] azetidin-1-yl]methanone,
1H-benzotriazol-5-yl-[3-[2-(3,5-dimethylanilino)-7,7-dimethyl-5H-pyrrolo[3,4-d]pyrimidin-6-yl] azetidin-1-yl]methanone,
1H-benzotriazol-5-yl-[(2S)-4-[2-(3,5-dimethylanilino)-7,8-dihydro-5H-pyrido[4,3-d]pyrimidin-6-yl]-2-methyl-1-piperidyl]methanone,
6-[(2R,4R)-1-(1H-benzotriazole-5-carbonyl)-2-methyl-4-piperidyl]-2-(3,5-dimethylanilino)-7,8-dihydropyrido[4,3-d]pyrimidin-5-one,
1H-benzotriazol-5-yl-[(2R,4R)-4-[2-(3,5-dimethylanilino)-7,8-dihydro-5H-pyrido[4,3-d]pyrimidin-6-yl]-2-methyl-1-piperidyl]methanone,
1H-benzotriazol-5-yl-[(3S,4S/3R,4R)-4-[2-(3,5-dimethylanilino)-5,7-dihydropyrrolo[3,4-d]pyrimidin-6-yl]-3-methyl-1-piperidyl]methanone,
1H-benzotriazol-5-yl-[4-[2-(3,5-dimethylanilino)-5,7-dihydropyrrolo[3,4-d]pyrimidin-6-yl]-2,2-dimethyl-1-piperidyl]methanone,
1H-benzotriazol-5-yl-[4-[2-(3,5-dimethylanilino)-5,7-dihydropyrrolo[3,4-d]pyrimidin-6-yl]-3,3-difluoro-pyrrolidin-1-yl]methanone,
1H-benzotriazol-5-yl-[(3R,4S/3S,4R)-3-[2-(3,5-dimethylanilino)-5,7-dihydropyrrolo[3,4-d]pyrimidin-6-yl]-4-fluoro-pyrrolidin-1-yl]methanone,
1H-benzotriazol-5-yl-[(3S,4R/3R,4S)-3-[2-(3,5-dimethylanilino)-7,8-dihydro-5H-pyrido[4,3-d]pyrimidin-6-yl]-4-fluoro-pyrrolidin-1-yl]methanone,
methyl (2S,4R)-1-(1H-benzotriazole-5-carbonyl)-4-[2-(3,5-dimethylanilino)-5,7-dihydropyrrolo[3,4-d]pyrimidin-6-yl]piperidine-2-carboxylate,
[(2R,4R)-4-[2-(3,5-dimethylanilino)-5,7-dihydropyrrolo[3,4-d]pyrimidin-6-yl]-2-methyl-1-piperidyl]-(6-methoxy-1H-benzotriazol-5-yl)methanone,
[4-[2-(3,5-dimethylanilino)-5,7-dihydropyrrolo[3,4-d]pyrimidin-6-yl]-1-piperidyl]-(6-methoxy-1H-benzotriazol-5-yl)methanone,
1H-benzotriazol-5-yl-[4-[2-(3,5-dimethylanilino)-5,7-dihydropyrrolo[3,4-d]pyrimidin-6-yl]-2-(trifluoromethyl)-1-piperidyl]methanone,
1H-benzimidazol-5-yl-[4-[2-(3,5-dimethylanilino)-5,7-dihydropyrrolo[3,4-d]pyrimidin-6-yl]-1-piperidyl]methanone,
1H-benzotriazol-5-yl-[4-[2-(3,5-dimethylanilino)-5,6,8,9-tetrahydropyrimido[4,5-d]azepin-7-yl]-1-piperidyl]methanone,
1H-benzotriazol-5-yl-[4-[2-(3,5-dimethylanilino)-5,7-dihydropyrrolo[3,4-d]pyrimidin-6-yl]-3-hydroxy-1-piperidyl]methanone,
1H-benzotriazol-5-yl-[3-[2-(3,5-dimethylanilino)-5,7-dihydropyrrolo[3,4-d]pyrimidin-6-yl]-4-hydroxy-1-piperidyl]methanone,
[(2S,4R)-4-[2-(3,5-dimethylanilino)-5,7-dihydropyrrolo[3,4-d]pyrimidin-6-yl]-2-methyl-1-piperidyl]-(6-methoxy-1H-benzotriazol-5-yl)methanone,
[(2S,4S)-4-[2-(3,5-dimethylanilino)-5,7-dihydropyrrolo[3,4-d]pyrimidin-6-yl]-2-methyl-1-piperidyl]-(6-methoxy-1H-benzotriazol-5-yl)methanone,
1H-benzotriazol-5-yl-[3-[2-(3,5-dimethylanilino)-5,7-dihydropyrrolo[3,4-d]pyrimidin-6-yl]-8-azabicyclo[3.2.1]octan-8-yl]methanone,
[(2R,4R)-4-[2-(3,5-dimethylanilino)-5,7-dihydropyrrolo[3,4-d]pyrimidin-6-yl]-2-methyl-1-piperidyl]-(6-fluoro-1H-benzotriazol-5-yl)methanone,
[(2R,4S)-4-[2-(3,5-dimethylanilino)-5,7-dihydropyrrolo[3,4-d]pyrimidin-6-yl]-2-methyl-1-piperidyl]-(6-fluoro-1H-benzotriazol-5-yl)methanone,
[4-[2-(3,5-dimethylanilino)-5,7-dihydropyrrolo[3,4-d]pyrimidin-6-yl]-2,2-dimethyl-1-piperidyl]-(6-fluoro-1H-benzotriazol-5-yl)methanone,
5-[4-[2-(3,5-dimethylanilino)-5,7-dihydropyrrolo[3,4-d]pyrimidin-6-yl]piperidine-1-carbonyl]-3H-1,3-benzoxazol-2-one,
[(3R,4S/3S,4R)-4-[2-(3,5-dimethylanilino)-5,7-dihydropyrrolo[3,4-d]pyrimidin-6-yl]-3-methyl-1-piperidyl]-(6-methoxy-1H-benzotriazol-5-yl)methanone,
[(2R,4S)-4-[2-(3,5-dimethylanilino)-5,7-dihydropyrrolo[3,4-d]pyrimidin-6-yl]-2-methyl-1-piperidyl]-(6-methoxy-1H-benzotriazol-5-yl)methanone,
1H-benzotriazol-5-yl-[4-[2-(3,5-dimethylanilino)-5-methyl-7,8-dihydro-5H-pyrido[4,3-d]pyrimidin-6-yl]-1-piperidyl]methanone / 1H-benzotriazol-5-yl-[4-[2-(3,5-dimethylanilino)-6-methyl-6,8-dihydro-5H-pyrido[3,4-d]pyrimidin-7-yl]-1-piperidyl]methanone mixture,
1H-benzotriazol-5-yl-[4-[2-(3,5-dimethylanilino)-7,8-dihydro-5H-pyrido[4,3-d]pyrimidin-6-yl]-3-hydroxy-1-piperidyl]methanone / 1H-benzotriazol-5-yl-[3-[2-(3,5-dimethylanilino)-7,8-dihydro-5H-pyrido[4,3-d]pyrimidin-6-yl]-4-hydroxy-1-piperidyl]methanone mixture,
[(2S,4S)-4-[2-(3,5-dimethylanilino)-5,7-dihydropyrrolo[3,4-d]pyrimidin-6-yl]-2-methyl-1-piperidyl]-[3-(1H-tetrazol-5-yl)phenyl]methanone,
4-[(2S,4S)-4-[2-(3,5-dimethylanilino)-5,7-dihydropyrrolo[3,4-d]pyrimidin-6-yl]-2-methyl-piperidine-1-carbonyl]benzenesulfonamide,
1H-benzotriazol-5-yl-[(2R,4R)-4-[2-(3-methoxy-5-methyl-anilino)-5,7-dihydropyrrolo[3,4-d]pyrimidin-6-yl]-2-methyl-1-piperidyl]methanone,
1H-benzotriazol-5-yl-[(2R,4R)-4-[2-(4-fluoro-3-methoxy-anilino)-5,7-dihydropyrrolo[3,4-d]pyrimidin-6-yl]-2-methyl-1-piperidyl]methanone,
1H-benzotriazol-5-yl-[(2R,4R)-4-[2-[3-methoxy-5-(trifluoromethyl)anilino]-5,7-dihydropyrrolo[3,4-d]pyrimidin-6-yl]-2-methyl-1-piperidyl]methanone,
3-[[6-[(2R,4S)-1-(1H-benzotriazole-5-carbonyl)-2-methyl-4-piperidyl]-5,7-dihydropyrrolo[3,4-d]pyrimidin-2-yl]amino]-5-(trifluoromethyl)benzonitrile,
3-[[6-[(2R,4R)-1-(1H-benzotriazole-5-carbonyl)-2-methyl-4-piperidyl]-5,7-dihydropyrrolo[3,4-d]pyrimidin-2-yl]amino]-5-(trifluoromethyl)benzamide,
1H-benzotriazol-5-yl-[4-[2-(3,5-dimethylanilino)-5,7-dihydropyrrolo[3,4-d]pyrmidin-6-yl]-4-methyl-1-piperidyl]methanone,
N-[4-[(2S,4S)-4-[2-(3,5-dimethylanilino)-5,7-dihydropyrrolo[3,4-d]pyrimidin-6-yl]-2-methylpiperidine-1-carbonyl]phenyl]sulfonyl-2-methyl-propanamide,
4-[(2S,4S)-4-[2-(3,5-dimethylanilino)-5,7-dihydropyrrolo[3,4-d]pyrimidin-6-yl]-2-methyl-piperidine-1-carbonyl]benzoic acid,
4-[(2S,4S)-4-[2-(3,5-dimethylanilino)-5,7-dihydropyrrolo[3,4-d]pyrimidin-6-yl]-2-methyl-piperidine-1-carbonyl]-N-methylsulfonyl-benzamide,
1H-benzotriazol-5-yl-[(2R,4R)-2-methyl-4-[2-(4-methylanilino)-5,7-dihydropyrrolo[3,4-d]pyrimidin-6-yl]-1-piperidyl]methanone,
1H-benzotriazol-5-yl-[(2R,4R)-4-[2-(4-chloroanilino)-5,7-dihydropyrrolo[3,4-d]pyrimidin-6-yl]-2-methyl-1-piperidyl]methanone,
methyl (3S,4S)-1-(1H-benzotriazole-5-carbonyl)-4-[2-(3,5-dimethylanilino)-5,7-dihydropyrrolo[3,4-d]pyrimidin-6-yl]piperidine-3-carboxylate,
methyl (3S,4R/3R,4S)-1-(1H-benzotriazole-5-carbonyl)-4-[2-(3,5-dimethylanilino)-5,7-dihydropyrrolo[3,4-d]pyrimidin-6-yl]piperidine-3-carboxylate,
[(2R,4S)-4-[2-(3,5-dimethylanilino)-5,7-dihydropyrrolo[3,4-d]pyrimidin-6-yl]-2-methyl-1-piperidyl]-(6-ethoxy-1H-benzotriazol-5-yl)methanone,
[(2R,4R)-4-[2-(3,5-dimethylanilino)-5,7-dihydropyrrolo[3,4-d]pyrimidin-6-yl]-2-methyl-1-piperidyl]-(6-ethoxy-1H-benzotriazol-5-yl)methanone,
[4-[2-(3,5-dimethylanilino)-5,7-dihydropyrrolo[3,4-d]pyrimidin-6-yl]-1-piperidyl]-(3H-triazolo[4,5-c]pyridin-6-yl)methanone,
[4-[2-(3,5-dimethylanilino)-5,7-dihydropyrrolo[3,4-d]pyrimidin-6-yl]-1-piperidyl]-(6-ethoxy-1H-benzotriazol-5-yl)methanone,
1H-benzotriazol-5-yl-[(2S)-4-[2-(3,5-dimethylanilino)-5,6,8,9-tetrahydropyrimido[4,5-d]azepin-7-yl]-2-methyl-1-piperidyl]methanone,
1H-benzotriazol-5-yl-[(2R,4S)-4-[2-(4-fluoroanilino)-5,7-dihydropyrrolo[3,4-d]pyrimidin-6-yl]-2-methyl-1-piperidyl]methanone,
1H-benzotriazol-5-yl-[(2R,4R)-4-[2-(4-fluoroanilino)-5,7-dihydropyrrolo[3,4-d]pyrimidin-6-yl]-2-methyl-1-piperidyl]methanone,
methyl 3-[(2R,4R)-4-[2-(3,5-dimethylanilino)-5,7-dihydropyrrolo[3,4-d]pyrimidin-6-yl]-2-methylpiperidine-1-carbonyl]benzoate,
3-[(2R,4R)-4-[2-(3,5-dimethylanilino)-5,7-dihydropyrrolo[3,4-d]pyrimidin-6-yl]-2-methyl-piperidine-1-carbonyl]benzoic acid,
[(2R,4R)-4-[2-(3,5-dimethylanilino)-5,7-dihydropyrrolo[3,4-d]pyrimidin-6-yl]-2-methyl-1-piperidyl]-(1H-triazol-4-yl)methanone,
[(3R,4S/3S,4R)-4-[2-(3,5-dimethylanilino)-5,7-dihydropyrrolo[3,4-d]pyrimidin-6-yl]-3-methyl-1-piperidyl]-(6-fluoro-1H-benzotriazol-5-yl)methanone,
1H-benzotriazol-5-yl-[(2R,4R)-4-[2-(4-methoxyanilino)-5,7-dihydropyrrolo[3,4-d]pyrimidin-6-yl]-2-methyl-1-piperidyl]methanone,
1H-benzotriazol-5-yl-[(2R,4R)-2-methyl-4-[2-[4-(pentafluoro-λ⁶-sulfanyl)anilino]-5,7-dihydropyrrolo[3,4-d]pyrimidin-6-yl]-1-piperidyl]methanone,
1H-benzotriazol-5-yl-[(2R,4R)-4-[2-(3,5-dimethoxyanilino)-5,7-dihydropyrrolo[3,4-d]pyrimidin-6-yl]-2-methyl-1-piperidyl]methanone,
1H-benzotriazol-5-yl-[(2R,4R)-2-methyl-4-[2-[4-(trifluoromethyl)anilino]-5,7-dihydropyrrolo[3,4-d]pyrimidin-6-yl]-1-piperidyl]methanone,
[(2R,4R)-4-[2-(3,5-dimethylanilino)-5,7-dihydropyrrolo[3,4-d]pyrimidin-6-yl]-2-methyl-1-piperidyl]-[4-(1H-tetrazol-5-yl)phenyl]methanone,
N-[4-[(2R,4R)-4-[2-(3,5-dimethylanilino)-5,7-dihydropyrrolo[3,4-d]pyrimidin-6-yl]-2-methylpiperidine-1-carbonyl]-2-pyridyl]methanesulfonamide,
N-[5-[(2R,4R)-4-[2-(3,5-dimethylanilino)-5,7-dihydropyrrolo[3,4-d]pyrimidin-6-yl]-2-methylpiperidine-1-carbonyl]-2-pyridyl]methanesulfonamide,
5-[(2R,4R)-4-[2-(3,5-dimethylanilino)-5,7-dihydropyrrolo[3,4-d]pyrimidin-6-yl]-2-methyl-piperidine-1-carbonyl]indolin-2-one,
5-[(2R,4R)-4-[2-(3,5-dimethylanilino)-5,7-dihydropyrrolo[3,4-d]pyrimidin-6-yl]-2-methyl-piperidine-1-carbonyl]-1,3-dihydrobenzimidazol-2-one,
1H-benzotriazol-5-yl-[4-[2-(3,5-dimethylanilino)-7,8-dihydro-5H-pyrido[4,3-d]pyrimidin-6-yl]-4-methyl-1-piperidyl]methanone,
5-[(2R,4R)-4-[2-(3,5-dimethylanilino)-5,7-dihydropyrrolo[3,4-d]pyrimidin-6-yl]-2-methyl-piperidine-1-carbonyl]pyridine-2-sulfonamide,
4-[[6-[(2R,4R)-1-(1H-benzotriazole-5-carbonyl)-2-methyl-4-piperidyl]-5,7-dihydropyrrolo[3,4-d]pyrimidin-2-yl]amino]benzonitrile,
[(2R,4R)-4-[2-(3,5-dimethylanilino)-5,7-dihydropyrrolo[3,4-d]pyrimidin-6-yl]-2-methyl-1-piperidyl]-(1-methyltriazol-4-yl)methanone,
[4-[2-(3,5-dimethylanilmo)-7,8-dihydro-5H-pyrido[4,3-d]pyrimidm-6-yl]-1-piperidyl]-(1H-triazol-4-yl)methanone,
[(2R,4R)-4-[2-(3,5-dimethylanilino)-5,7-dihydropyrrolo[3,4-d]pyrimidin-6-yl]-2-methyl-1-piperidyl]-(6-methyl-1H-benzotriazol-5-yl)methanone,
1H-benzotriazol-5-yl-[(2R,4S)-2-methyl-4-[2-[4-(trifluoromethoxy)anilino]-5,7-dihydropyrrolo[3,4-d]pyrimidin-6-yl]-1-piperidyl]methanone,
[(2R,4S)-4-(2-anilino-5,7-dihydropyrrolo[3,4-d]pyrimidin-6-yl)-2-methyl-1-piperidyl]-(1H-benzotriazol-5-yl)methanone,
[(2R,4R)-4-(2-anilino-S,7-dihydropyrrolo[3,4-d]pyrimidin-6-yl)-2-methyl-l-piperidyl]-(1H-benzotriazol-5-yl)methanone,
N-[4-[(3R,4S/3S,4R)-4-[2-(3,5-dimethylanilino)-5,7-dihydropyrrolo[3,4-d]pyrimidin-6-yl]-3-methylpiperidine-1-carbonyl]-2-pyridyl]methanesulfonamide,
[(2R,4R)-4-[2-(3,5-dimethylanilmo)-5,7-dihydropyrrolo[3,4-d]pyrimidm-6-yl]-2-methyl-1-piperidyl]-(4-methyl-1H-benzotriazol-5-yl)methanone,
1H-benzotriazol-5-yl-[(2R,4R)-2-methyl-4-[2-[4-(trifluoromethoxy)anilino]-5,7-dihydropyrrolo[3,4-d]pyrimidin-6-yl]-1-piperidyl]methanone,
1H-benzotriazol-5-yl-[(2R,4S)-4-[2-(3-methoxyanilino)-5,7-dihydropyrrolo[3,4-d]pyrimidin-6-yl]-2-methyl-1-piperidyl]methanone,
1H-benzotriazol-5-yl-[(2R,4R)-4-[2-(3-methoxyanilino)-5,7-dihydropyrrolo[3,4-d]pyrimidin-6-yl]-2-methyl-1-piperidyl]methanone,
[(3R,4S/3S,4R)-4-[2-(3,5-dimethylanilino)-5,7-dihydropyrrolo[3,4-d]pyrimidin-6-yl]-3-methyl-1-piperidyl]-(1H-triazol-4-yl)methanone,
[(2R,4R)-4-[2-(4-fluoroanilino)-5,7-dihydropyrrolo[3,4-d]pyrimidin-6-yl]-2-methyl-1-piperidyl]-(1H-triazol-4-yl)methanone,
N-[4-[4-[2-(3,5-dimethylanilino)-7,8-dihydro-5H-pyrido[4,3-d]pyrimidin-6-yl]piperidine-1-carbonyl]-2-pyridyl]methanesulfonamide,
[(2R,4R)-2-methyl-4-[2-[4-(trifluoromethyl)anilino]-5,7-dihydropyrrolo[3,4-d]pyrimidin-6-yl]-1-piperidyl]-(1H-triazol-4-yl)methanone,
[(2R,4R)-4-[2-(3,5-dimethylanilino)-5,7-dihydropyrrolo[3,4-d]pyrimidin-6-yl]-2-methyl-1-piperidyl]-(5-methyl-1H-triazol-4-yl)methanone,
N-[4-[(2R,4R)-2-methyl-4-[2-[4-(pentafluoro-λ⁶-sulfanyl)anilino]-5,7-dihydropyrrolo[3,4-d]pyrimidin-6-yl]piperidine-1-carbonyl]-2-pyridyl]methanesulfonamide,
N-[4-[(2R,4S)-2-methyl-4-[2-[4-(pentafluoro-λ⁶-sulfanyl)anilino]-5,7-dihydropyrrolo[3,4-d]pyrimidin-6-yl]piperidine-1-carbonyl]-2-pyridyl]methanesulfonamide,
N-[4-[(2R,4R)-4-[2-(4-chloroanilino)-5,7-dihydropyrrolo[3,4-d]pyrimidin-6-yl]-2-methyl-piperidine-1-carbonyl]-2-pyridyl]methanesulfonamide,
N-[4-[(2R,4S)-4-[2-(4-chloroanilino)-5,7-dihydropyrrolo[3,4-d]pyrimidin-6-yl]-2-methyl-piperidine-1-carbonyl]-2-pyridyl]methanesulfonamide,
N-[4-[(2R,4R)-2-methyl-4-[2-(4-methylanilino)-5,7-dihydropyrrolo[3,4-d]pyrimidin-6-yl]piperidine-1-carbonyl]-2-pyridyl]methanesulfonamide,
N-[4-[(2R,4S)-2-methyl-4-[2-(4-methylanilino)-5,7-dihydropyrrolo[3,4-d]pyrimidin-6-yl]piperidine-1-carbonyl]-2-pyridyl]methanesulfonamide,
(6-methyl-1H-benzotriazol-5-yl)-[(2R,4R)-2-methyl-4-[2-[4-(trifluoromethyl)anilino]-5,7-dihydropyrrolo[3,4-d]pyrimidin-6-yl]-1-piperidyl]methanone,
[(2R,4R)-4-[2-(3-methoxy-5-methyl-anilino)-5,7-dihydropyrrolo[3,4-d]pyrimidin-6-yl]-2-methyl-1-piperidyl]-(6-methyl-1H-benzotriazol-5-yl)methanone,
[(2R,4S)-4-[2-(3-methoxy-5-methyl-anilino)-5,7-dihydropyrrolo[3,4-d]pyrimidin-6-yl]-2-methyl-1-piperidyl]-(6-methyl-1H-benzotriazol-5-yl)methanone,
N-[4-[(2R,4R)-2-methyl-4-[2-[4-(trifluoromethyl)anilino]-5,7-dihydropyrrolo[3,4-d]pyrimidin-6-yl]piperidine-1-carbonyl]-2-pyridyl]methanesulfonamide,
N-[4-[(2R,4R)-4-[2-(3-methoxy-5-methyl-anilino)-5,7-dihydropyrrolo[3,4-d]pyrimidin-6-yl]-2-methyl-piperidine-1-carbonyl]-2-pyridyl]methanesulfonamide,
N-[4-[(2R,4R)-4-[2-(4-fluoroanilino)-5,7-dihydropyrrolo[3,4-d]pyrimidin-6-yl]-2-methyl-piperidine-1-carbonyl]-2-pyridyl]methanesulfonamide,
N-[4-[(2R,4R)-4-[2-(3-methoxyanilino)-5,7-dihydropyrrolo[3,4-d]pyrimidin-6-yl]-2-methylpiperidine-1-carbonyl]-2-pyridyl]methanesulfonamide,
N-[4-[(2R,4S)-2-methyl-4-[2-[4-(trifluoromethyl)anilino]-5,7-dihydropyrrolo[3,4-d]pyrimidin-6-yl]piperidine-1-carbonyl]-2-pyridyl]methanesulfonamide,
N-[4-[(2R,4S)-4-[2-(3-methoxy-5-methyl-anilino)-5,7-dihydropyrrolo[3,4-d]pyrimidin-6-yl]-2-methyl-piperidine-1-carbonyl]-2-pyridyl]methanesulfonamide,
N-[4-[(2R,4S)-4-[2-(4-fluoroanilino)-5,7-dihydropyrrolo[3,4-d]pyrimidin-6-yl]-2-methyl-piperidine-1-carbonyl]-2-pyridyl]methanesulfonamide,
N-[4-[(2R,4S)-4-[2-(3-methoxyanilino)-5,7-dihydropyrrolo[3,4-d]pyrimidin-6-yl]-2-methylpiperidine-1-carbonyl]-2-pyridyl]methanesulfonamide,
[4-[2-(3,5-dimethylanilino)-5,7-dihydropyrrolo[3,4-d]pyrimidin-6-yl]-4-methyl-1-piperidyl]-(6-fluoro-1H-benzotriazol-5-yl)methanone,
(6-fluoro-1H-benzotriazol-5-yl)-[(2R,4R)-2-methyl-4-[2-[4-(pentafluoro-λ⁶-sulfanyl)anilino]-5,7-dihydropyrrolo[3,4-d]pyrimidin-6-yl]-1-piperidyl]methanone,
(6-fluoro-1H-benzotriazol-5-yl)-[(2R,4S)-2-methyl-4-[2-[4-(pentafluoro-λ⁶-sulfanyl)anilino]-5,7-dihydropyrrolo[3,4-d]pyrimidin-6-yl]-1-piperidyl]methanone,
(6-fluoro-1H-benzotriazol-5-yl)-[(2R,4R)-4-[2-(3-fluoro-5-methoxy-anilino)-5,7-dihydropyrrolo[3,4-d]pyrimidin-6-yl]-2-methyl-1-piperidyl]methanone,
(2-amino-4-pyridyl)-[(2R,4R)-4-[2-(3,5-dimethylanilino)-5,7-dihydropyrrolo[3,4-d]pyrimidin-6-yl]-2-methyl-1-piperidyl]methanone,
[4-[2-(3,5-dimethylanilino)-7,8-dihydro-5H-pyrido[4,3-d]pyrimidin-6-yl]-1-piperidyl]-[1-(6-methyl-1H-benzotriazol-5-yl)-4-piperidyl]methanone,
N-[4-[(2R,4R)-4-[2-(3,5-dimethoxyanilino)-5,7-dihydropyrrolo[3,4-d]pyrimidin-6-yl]-2-methylpiperidine-1-carbonyl]-2-pyridyl]methanesulfonamide,
N-[4-[4-[2-[4-(trifluoromethyl)anilino]-5,7-dihydropyrrolo[3,4-d]pyrimidin-6-yl]piperidine-1-carbonyl]-2-pyridyl]methanesulfonamide,
N-[4-[4-[2-(3,5-dimethylanilino)-5,7-dihydropyrrolo[3,4-d]pyrimidin-6-yl]piperidine-1-carbonyl]-2-pyridyl]methanesulfonamide,
N-[4-[(2R,4R)-2-methyl-4-[2-(3-methylanilino)-5,7-dihydropyrrolo[3,4-d]pyrimidin-6-yl]piperidine-1-carbonyl]-2-pyridyl]methanesulfonamide,
N-[4-[(2R,4R)-4-[2-(3-fluoro-5-methoxy-anilino)-5,7-dihydropyrrolo[3,4-d]pyrimidin-6-yl]-2-methylpiperidine-1-carbonyl]-2-pyridyl]methanesulfonamide,
N-[4-[(2R,4R)-2-methyl-4-[2-[4-(trifluoromethoxy)anilino]-5,7-dihydropyrrolo[3,4-d]pyrimidin-6-yl]piperidine-1-carbonyl]-2-pyridyl]methanesulfonamide,
N-[3-[(2R,4R)-4-[2-(3,5-dimethylanilino)-5,7-dihydropyrrolo[3,4-d]pyrimidin-6-yl]-2-methylpiperidine-1-carbonyl]phenyl]methanesulfonamide,
N-[4-[(2R,4R)-4-[2-(4-fluoro-3-methyl-anilino)-5,7-dihydropyrrolo[3,4-d]pyrimidin-6-yl]-2-methylpiperidine-1-carbonyl]-2-pyridyl]methanesulfonamide,
N-[4-[(2R,4R)-4-[2-(3,5-dimethylanilino)-5,7-dihydropyrrolo[3,4-d]pyrimidin-6-yl]-2-methylpiperidine-1-carbonyl]-5-fluoro-2-pyridyl]methanesulfonamide,
N-[4-[(2R,4R)-4-[2-(3,5-dimethylanilino)-5,7-dihydropyrrolo[3,4-d]pyrimidin-6-yl]-2-methylpiperidine-1-carbonyl]-2-pyridyl]acetamide,
N-[4-[(2R,4R)-4-[2-(3,5-dimethylanilino)-5,7-dihydropyrrolo[3,4-d]pyrimidin-6-yl]-2-methylpiperidine-1-carbonyl]-2-pyridyl]ethanesulfonamide,
N-[5-[(2R,4R)-4-[2-(3,5-dimethylanilino)-5,7-dihydropyrrolo[3,4-d]pyrimidin-6-yl]-2-methylpiperidine-1-carbonyl]-3-pyridyl]methanesulfonamide,
N-[4-[(2R,4R)-4-[2-(3,5-dimethylanilino)-5,7-dihydropyrrolo[3,4-d]pyrimidin-6-yl]-2-methylpiperidine-1-carbonyl]-2-pyridyl]-1,1,1-trifluoro-methanesulfonamide,
N-[4-[(2R,4R)-4-[2-(3-chloro-5-fluoro-anilino)-5,7-dihydropyrrolo[3,4-d]pyrimidin-6-yl]-2-methylpiperidine-1-carbonyl]-2-pyridyl]methanesulfonamide,
N-[4-[(2R,4S)-4-[2-(3-chloro-5-fluoro-anilino)-5,7-dihydropyrrolo[3,4-d]pyrimidin-6-yl]-2-methylpiperidine-1-carbonyl]-2-pyridyl]methanesulfonamide,
N-[4-[(2R,4R)-2-methyl-4-[2-[3-methyl-4-(trifluoromethyl)anilino]-5,7-dihydropyrrolo[3,4-d]pyrimidin-6-yl]piperidine-1-carbonyl]-2-pyridyl]methanesulfonamide,
N-[4-[(2R,4S)-2-methyl-4-[2-[3-methyl-4-(trifluoromethyl)anilino]-5,7-dihydropyrrolo[3,4-d]pyrimidin-6-yl]piperidine-1-carbonyl]-2-pyridyl]methanesulfonamide,
N-[4-[(2R,4R)-4-[2-(4-chloro-3-methoxy-anilino)-5,7-dihydropyrrolo[3,4-d]pyrimidin-6-yl]-2-methyl-piperidine-1-carbonyl]-2-pyridyl]methanesulfonamide,
N-[4-[(2R,4R)-4-[2-(3,5-dimethylanilino)-5,7-dihydropyrrolo[3,4-d]pyrimidin-6-yl]-2-methylpiperidine-1-carbonyl]-5-methyl-2-pyridyl]methanesulfonamide,
N-[4-[(2R,4S)-4-[2-[3-(cyclopropoxy)anilino]-5,7-dihydropyrrolo[3,4-d]pyrimidin-6-yl]-2-methylpiperidine-1-carbonyl]-2-pyridyl]methanesulfonamide,
N-[4-[(2R,4S)-4-[2-(3,4-dimethylanilino)-5,7-dihydropyrrolo[3,4-d]pyrimidin-6-yl]-2-methylpiperidine-1-carbonyl]-2-pyridyl]methanesulfonamide,
N-[4-[(2R,4S)-4-[2-(4-ethylanilino)-5,7-dihydropyrrolo [3,4-d]pyrimidin-6-yl]-2-methyl-piperidine-1-carbonyl]-2-pyridyl]methanesulfonamide,
N-[4-[(2R,4R)-4-[2-[3-(cyclopropoxy)anilino]-5,7-dihydropyrrolo[3,4-d]pyrimidin-6-yl]-2-methylpiperidine-1-carbonyl]-2-pyridyl]methanesulfonamide,
N-[4-[(2R,4R)-4-[2-(3,4-dimethylanilino)-5,7-dihydropyrrolo[3,4-d]pyrimidin-6-yl]-2-methylpiperidine-1-carbonyl]-2-pyridyl]methanesulfonamide,
N-[4-[(2R,4R)-4-[2-(4-ethylanilino)-5,7-dihydropyrrolo[3,4-d]pyrimidin-6-yl]-2-methyl-piperidine-1-carbonyl]-2-pyridyl]methanesulfonamide,
N-[4-[(2R,4R)-4-[2-(3,5-dimethylanilino)-5,7-dihydropyrrolo[3,4-d]pyrimidin-6-yl]-2-methylpiperidine-1-carbonyl]-6-methyl-2-pyridyl]methanesulfonamide,
N-[4-[(2R,4R)-4-[2-(3-chloro-5-methoxy-anilino)-5,7-dihydropyrrolo[3,4-d]pyrimidin-6-yl]-2-methyl-piperidine-1-carbonyl]-2-pyridyl]methanesulfonamide,
N-[4-[(2R,4S)-4-[2-(3-chloro-5-methoxy-anilino)-5,7-dihydropyrrolo[3,4-d]pyrimidin-6-yl]-2-methylpiperidine-1-carbonyl]-2-pyridyl]methanesulfonamide,
N-[4-[(2R,4R)-4-[2-[4-chloro-3-(trifluoromethoxy)anilino]-5,7-dihydropyrrolo[3,4-d]pyrimidin-6-yl]-2-methyl-piperidine-1-carbonyl]-2-pyridyl]methanesulfonamide,
N-[4-[(2R,4S)-4-[2-[4-chloro-3-(trifluoromethoxy)anilino]-5,7-dihydropyrrolo[3,4-d]pyrimidin-6-yl]-2-methyl-piperidine-1-carbonyl]-2-pyridyl]methanesulfonamide,
N-[4-[(2R,4R)-2-methyl-4-[2-[2-methyl-4-(trifluoromethyl)anilino]-5,7-dihydropyrrolo[3,4-d]pyrimidin-6-yl]piperidine-1-carbonyl]-2-pyridyl]methanesulfonamide,
N-[4-[(2R,4S)-2-methyl-4-[2-[2-methyl-4-(trifluoromethyl)anilino]-5,7-dihydropyrrolo[3,4-d]pyrimidin-6-yl]piperidine-1-carbonyl]-2-pyridyl]methanesulfonamide,
N-[4-[(2R,4R)-4-[2-(3-methoxy-4-methyl-anilino)-5,7-dihydropyrrolo[3,4-d]pyrimidin-6-yl]-2-methyl-piperidine-1-carbonyl]-2-pyridyl]methanesulfonamide,
N-[4-[(2R,4S)-4-[2-(3-methoxy-4-methyl-anilino)-5,7-dihydropyrrolo[3,4-d]pyrimidin-6-yl]-2-methyl-piperidine-1-carbonyl]-2-pyridyl]methanesulfonamide,
N-[4-[(2R,4R)-2-methyl-4-[2-[4-(2,2,2-trifluoroethyl)anilino]-5,7-dihydropyrrolo[3,4-d]pyrimidin-6-yl]piperidine-1-carbonyl]-2-pyridyl]methanesulfonamide,
N-[4-[(2R,4S)-2-methyl-4-[2-[4-(2,2,2-trifluoroethyl)anilino]-5,7-dihydropyrrolo[3,4-d]pyrimidin-6-yl]piperidine-1-carbonyl]-2-pyridyl]methanesulfonamide,
N-[4-[(2R,4S)-2-methyl-4-[2-[4-methyl-3-(trifluoromethyl)anilino]-5,7-dihydropyrrolo[3,4-d]pyrimidin-6-yl]piperidine-1-carbonyl]pyridin-2-yl]methanesulfonamide,
N-[6-[(2R,4R)-4-[2-(3,5-dimethylanilino)-5,7-dihydropyrrolo[3,4-d]pyrimidin-6-yl]-2-methylpiperidine-1-carbonyl]-2-pyridyl]methanesulfonamide,
N-[4-[(2R,4R)-4-[2-(3-ethoxyanilino)-5,7-dihydropyrrolo[3,4-d]pyrimidin-6-yl]-2-methyl-piperidine-1-carbonyl]-2-pyridyl]methanesulfonamide,
N-[4-[(2R,4S)-4-[2-(3-ethoxyanilino)-5,7-dihydropyrrolo[3,4-d]pyrimidin-6-yl]-2-methyl-piperidine-1-carbonyl]-2-pyridyl]methanesulfonamide,
N-[4-[(2R,4S)-4-[2-(3-chloro-4-fluoro-anilino)-5,7-dihydropyrrolo[3,4-d]pyrimidin-6-yl]-2-methylpiperidine-1-carbonyl]-2-pyridyl]methanesulfonamide,
N-[4-[(2R,4R)-4-[2-(3,4-difluoroanilino)-5,7-dihydropyrrolo[3,4-d]pyrimidin-6-yl]-2-methylpiperidine-1-carbonyl]-2-pyridyl]methanesulfonamide,
N-[4-[(2R,4R)-4-[2-(3-chloro-4-fluoro-anilino)-5,7-dihydropyrrolo[3,4-d]pyrimidin-6-yl]-2-methylpiperidine-1-carbonyl]-2-pyridyl]methanesulfonamide,
N-[4-[(2R,4S)-4-[2-(3-isopropoxyanilino)-5,7-dihydropyrrolo[3,4-d]pyrimidin-6-yl]-2-methylpiperidine-1-carbonyl]-2-pyridyl]methanesulfonamide,
N-[4-[(2R,4S)-4-[2-(4-chloro-3-fluoro-anilino)-5,7-dihydropyrrolo[3,4-d]pyrimidin-6-yl]-2-methylpiperidine-1-carbonyl]-2-pyridyl]methanesulfonamide,
[(2R,4R)-4-[2-(3,5-dimethylanilino)-5,7-dihydropyrrolo[3,4-d]pyrimidin-6-yl]-2-methyl-1-piperidyl]-[2-(hydroxymethyl)-4-pyridyl]methanone,
N-[4-[(2R,4R)-4-[2-(3-isopropoxyanilino)-5,7-dihydropyrrolo[3,4-d]pyrimidin-6-yl]-2-methylpiperidine-1-carbonyl]-2-pyridyl]methanesulfonamide,
N-[4-[(2R,4R)-4-[2-(3,5-dimethylanilino)-7,8-dihydro-5H-pyrido[4,3-d]pyrimidin-6-yl]-2-methylpiperidine-1-carbonyl]-2-pyridyl]methanesulfonamide,
N-[4-[(2R,4S)-4-[2-(3,5-dimethylanilino)-7,8-dihydro-5H-pyrido[4,3-d]pyrimidin-6-yl]-2-methylpiperidine-1-carbonyl]-2-pyridyl]methanesulfonamide,
N-[4-[3-[2-(3,5-dimethylanilino)-7,8-dihydro-5H-pyrido[4,3-d]pyrimidin-6-yl]azetidine-1-carbonyl]-2-pyridyl]methanesulfonamide,
N-[5-[3-[2-(3,5-dimethylanilino)-7,8-dihydro-5H-pyrido[4,3-d]pyrimidin-6-yl]azetidine-1-carbonyl]-2-pyridyl]methanesulfonamide,
N-[4-[(2R,4S)-4-[2-(3-fluoro-4-methyl-anilino)-5,7-dihydropyrrolo[3,4-d]pyrimidin-6-yl]-2-methylpiperidine-1-carbonyl]-2-pyridyl]methanesulfonamide,
N-[4-[(2R,4R)-4-[2-(3-fluoro-4-methyl-anilino)-5,7-dihydropyrrolo[3,4-d]pyrimidin-6-yl]-2-methylpiperidine-1-carbonyl]-2-pyridyl]methanesulfonamide,
N-[4-[(2R,4R)-4-[2-(4-fluoro-3-methoxy-anilino)-5,7-dihydropyrrolo[3,4-d]pyrimidin-6-yl]-2-methylpiperidine-1-carbonyl]-2-pyridyl]methanesulfonamide,
N-[4-[(2R,4S)-4-[2-(4-fluoro-3-methoxy-anilino)-5,7-dihydropyrrolo[3,4-d]pyrimidin-6-yl]-2-methylpiperidine-1-carbonyl]-2-pyridyl]methanesulfonamide,
N-[4-[(2R,4R)-2-methyl-4-[2-[3-methyl-5-(trifluoromethyl)anilino]-5,7-dihydropyrrolo[3,4-d]pyrimidin-6-yl]piperidine-1-carbonyl]-2-pyridyl]methanesulfonamide,
N-[4-[(2R,4S)-4-[2-[3-fluoro-5-(trifluoromethyl)anilino]-5,7-dihydropyrrolo[3,4-d]pyrimidin-6-yl]-2-methyl-piperidine-1-carbonyl]-2-pyridyl]methanesulfonamide,
N-[4-[(2R,4R)-4-[2-[3-fluoro-5-(trifluoromethyl)anilino]-5,7-dihydropyrrolo[3,4-d]pyrimidin-6-yl]-2-methyl-piperidine-1-carbonyl]-2-pyridyl]methanesulfonamide,
N-[4-[(2R,4R)-2-methyl-4-[2-[3-(trifluoromethyl)anilino]-5,7-dihydropyrrolo[3,4-d]pyrimidin-6-yl]piperidine-1-carbonyl]-2-pyridyl]methanesulfonamide,
N-[4-[(2R,4S)-2-methyl-4-[2-[3-(trifluoromethyl)anilino]-5,7-dihydropyrrolo[3,4-d]pyrimidin-6-yl]piperidine-1-carbonyl]-2-pyridyl]methanesulfonamide,
N-[4-[(2R,4R)-2-methyl-4-[2-[3-(trifluoromethoxy)anilino]-5,7-dihydropyrrolo[3,4-d]pyrimidin-6-yl]piperidine-1-carbonyl]-2-pyridyl]methanesulfonamide,
N-[4-[(2R,4R)-4-[2-(4-chloro-3-fluoro-anilino)-5,7-dihydropyrrolo[3,4-d]pyrimidin-6-yl]-2-methylpiperidine-1-carbonyl]-2-pyridyl]methanesulfonamide,
N-[4-[(2R,4R)-4-[2-(3,5-dimethylanilino)-5,7-dihydropyrrolo[3,4-d]pyrimidin-6-yl]-2-methylpiperidine-1-carbonyl]-2-pyridyl]-2,2,2-trifluoro-ethanesulfonamide,
N-[4-[(2R,4R)-4-[2-(3,5-dimethylanilino)-5,7-dihydropyrrolo[3,4-d]pyrimidin-6-yl]-2-methylpiperidine-1-carbonyl]-2-pyridyl]cyclopropanesulfonamide,
N-[4-[(2R,4S)-4-[2-(3,5-dimethylanilino)-5,7-dihydropyrrolo[3,4-d]pyrimidin-6-yl]-2-methylpiperidine-1-carbonyl]-2-pyridyl]methanesulfonamide,
N-[4-[(2R,4R)-4-[2-[(2,6-dimethyl-4-pyridyl)amino]-5,7-dihydropyrrolo[3,4-d]pyrimidin-6-yl]-2-methyl-piperidine-1-carbonyl]-2-pyridyl]methanesulfonamide,
N-[4-[(2R,4S)-4-[2-[(2,6-dimethyl-4-pyridyl)amino]-5,7-dihydropyrrolo[3,4-d]pyrimidin-6-yl]-2-methyl-piperidine-1-carbonyl]-2-pyridyl]methanesulfonamide,
[(2R,4R)-4-[2-(3,5-dimethylanilino)-5,7-dihydropyrrolo[3,4-d]pyrimidin-6-yl]-2-methyl-1-piperidyl]-[2-(1-hydroxy-1-methyl-ethyl)-4-pyridyl]methanone,
N-[4-[(2R,4R)-4-[2-(3-cyano-4-methyl-anilino)-5,7-dihydropyrrolo[3,4-d]pyrimidin-6-yl]-2-methylpiperidine-1-carbonyl]-2-pyridyl]methanesulfonamide,
N-[4-[(2R,4S)-4-[2-(3-cyano-4-methyl-anilino)-5,7-dihydropyrrolo[3,4-d]pyrimidin-6-yl]-2-methylpiperidine-1-carbonyl]-2-pyridyl]methanesulfonamide,
N-[4-[(2R,4R)-2-methyl-4-[2-[(6-methyl-3-pyridyl)amino]-5,7-dihydropyrrolo[3,4-d]pyrimidin-6-yl]piperidine-1-carbonyl]-2-pyridyl]methanesulfonamide,
N-[4-[(2R,4S)-2-methyl-4-[2-[(6-methyl-3-pyridyl)amino]-5,7-dihydropyrrolo[3,4-d]pyrimidin-6-yl]piperidine-1-carbonyl]-2-pyridyl]methanesulfonamide,
N-[4-[(2R,4R)-4-[2-(3,5-dimethylanilino)-5,7-dihydropyrrolo[3,4-d]pyrimidin-6-yl]-2-methylpiperidine-1-carbonyl] oxazol-2-yl]methanesulfonamide,
N-[4-[(2R,4R)-4-[2-(3,5-dimethylanilino)-5,7-dihydropyrrolo[3,4-d]pyrimidin-6-yl]-2-methylpiperidine-1-carbonyl]-2-pyridyl]-2-methoxy-ethanesulfonamide,
N-[5-[3-[2-(3,5-dimethylanilino)-7,8-dihydro-5H-pyrido[4,3-d]pyrimidin-6-yl]pyrrolidine-1-carbonyl]-2-pyridyl]methanesulfonamide,
N-[5-cyano-4-[(2R,4R)-4-[2-(3,5-dimethylanilino)-5,7-dihydropyrrolo[3,4-d]pyrimidin-6-yl]-2-methyl-piperidine-1-carbonyl]-2-pyridyl]methanesulfonamide,
4-[(2R,4R)-4-[2-(3,5-dimethylanilino)-5,7-dihydropyrrolo[3,4-d]pyrimidin-6-yl]-2-methyl-piperidine-1-carbonyl]pyridine-2-carboxylic acid,
1H-benzotriazol-5-yl-[3-[2-(3,5-dimethylanilino)-7,8-dihydro-5H-pyrido[4,3-d]pyrimidin-6-yl]-1-piperidyl]methanone,
N-[5-[4-[2-(3,5-dimethylanilino)-7,8-dihydro-5H-pyrido[4,3-d]pyrimidin-6-yl]piperidine-1-carbonyl]-2-pyridyl]methanesulfonamide,
N-[5-[3-[2-(3,5-dimethylanilino)-7,8-dihydro-5H-pyrido[4,3-d]pyrimidin-6-yl]piperidine-1-carbonyl]-2-pyridyl]methanesulfonamide,
N-[4-[(2R,4S)-4-[2-(4-chloro-2-methyl-anilino)-5,7-dihydropyrrolo[3,4-d]pyrimidin-6-yl]-2-methylpiperidine-1-carbonyl]-2-pyridyl]methanesulfonamide,
N-[4-[(2R,4R)-4-[2-(4-chloro-2-methyl-anilino)-5,7-dihydropyrrolo[3,4-d]pyrimidin-6-yl]-2-methylpiperidine-1-carbonyl]-2-pyridyl]methanesulfonamide,
N-[4-[(2R,4S)-4-[2-(2,4-dimethylanilino)-5,7-dihydropyrrolo[3,4-d]pyrimidin-6-yl]-2-methylpiperidine-1-carbonyl]-2-pyridyl]methanesulfonamide,
methyl 4-[(2R,4R)-4-[2-(3,5-dimethylanilino)-5,7-dihydropyrrolo[3,4-d]pyrimidin-6-yl]-2-methylpiperidine-1-carbonyl]pyridine-2-carboxylate,
methyl 5-[4-[2-(3,5-dimethylanilino)-7,8-dihydro-5H-pyrido[4,3-d]pyrimidin-6-yl]piperidine-1-carbonyl]pyridine-2-carboxylate,
methyl 4-[4-[2-(3,5-dimethylanilino)-7,8-dihydro-5H-pyrido[4,3-d]pyrimidin-6-yl]piperidine-1-carbonyl]pyridine-2-carboxylate,
5-[4-[2-(3,5-dimethylanilino)-7,8-dihydro-5H-pyrido[4,3-d]pyrimidin-6-yl]piperidine-1-carbonyl]pyridine-2-carboxylic acid,
4-[4-[2-(3,5-dimethylanilino)-7,8-dihydro-5H-pyrido[4,3-d]pyrimidin-6-yl]piperidine-1-carbonyl]pyridine-2-carboxylic acid,
4-[(2R,4R)-4-[2-(3,5-dimethylanilino)-5,7-dihydropyrrolo[3,4-d]pyrimidin-6-yl]-2-methyl-piperidine-1-carbonyl]pyridine-2-carboxamide,
methyl 4-[4-[2-(3,5-dimethylanilino)-5,6,8,9-tetrahydropyrimido[4,5-d]azepin-7-yl]piperidine-1-carbonyl]pyridine-2-carboxylate,
methyl 4-[(2R,4R)-2-methyl-4-[2-[3-methyl-5-(trifluoromethyl)anilino]-5,7-dihydropyrrolo[3,4-d]pyrimidin-6-yl]piperidine-1-carbonyl]pyridine-2-carboxylate,
4-[(2R,4R)-2-methyl-4-[2-[3-methyl-5-(trifluoromethyl)anilino]-5,7-dihydropyrrolo[3,4-d]pyrimidin-6-yl]piperidine-1-carbonyl]pyridine-2-carboxylic acid,
methyl 4-[(2R,4R)-2-methyl-4-[2-[4-(trifluoromethyl)anilino]-5,7-dihydropyrrolo[3,4-d]pyrimidin-6-yl]piperidine-1-carbonyl]pyridine-2-carboxylate,
4-[(2R,4R)-2-methyl-4-[2-[4-(trifluoromethyl)anilino]-5,7-dihydropyrrolo[3,4-d]pyrimidin-6-yl]piperidine-1-carbonyl]pyridine-2-carboxylic acid,
methyl 4-[(2R,4R)-4-[2-[3,5-bis(trifluoromethyl)anilino]-5,7-dihydropyrrolo[3,4-d]pyrimidin-6-yl]-2-methyl-piperidine-1-carbonyl]pyridine-2-carboxylate,
methyl 4-[(2R,4S)-4-[2-[3,5-bis(trifluoromethyl)anilino]-5,7-dihydropyrrolo[3,4-d]pyrimidin-6-yl]-2-methyl-piperidine-1-carbonyl]pyridine-2-carboxylate,
4-[(2R,4R)-4-[2-[3,5-bis(trifluoromethyl)anilino]-5,7-dihydropyrrolo[3,4-d]pyrimidin-6-yl]-2-methyl-piperdine-1-carbonyl]pyridine-2-carboxylic acid,
4-[(2R,4S)-4-[2-[3,5-bis(trifluoromethyl)anilino]-5,7-dihydropyrrolo[3,4-d]pyrimidin-6-yl]-2-methyl-piperidine-1-carbonyl]pyridine-2-carboxylic acid,
4-[4-[2-(3,5-dimethylanilino)-5,6,8,9-tetrahydropyrimido[4,5-d]azepin-7-yl]piperidine-1-carbonyl]pyridine-2-carboxylic acid,
4-[(2R,4R)-4-[2-(3,5-dimethylanilino)-5,7-dihydropyrrolo[3,4-d]pyrimidin-6-yl]-2-methyl-piperidine-1-carbonyl]-N-methylsulfonyl-pyridine-2-carboxamide,
4-[(2R,4R)-4-[2-(3,5-dimethylanilino)-5,7-dihydropyrrolo[3,4-d]pyrimidin-6-yl]-2-methyl-piperidine-1-carbonyl]pyridme-2-sulfonamide,
1H-benzotriazol-5-yl-[(2R,4R)-2-methyl-4-[2-[(6-methyl-3-pyridyl)amino]-5,7 -dihydropyrrolo[3,4-d]pyrimidin-6-yl]-1 -piperidyl]methanone,
1H-benzotriazol-5-yl-[(2R,4S)-2-methyl-4-[2-[(6-methyl-3-pyridyl)ammo]-5,7-dihydropyrrolo[3,4-d]pyrimidin-6-yl] -1 -piperidyl]methanone,
N-[[4-[(2R,4R)-4-[2-(3,5-dimethylanilino)-5,7-dihydropyrrolo[3,4-d]pyrimidin-6-yl]-2-methyl-piperidine-1 -carbonyl] -2-pyridyl] sulfonyl] acetamide,
[(2R,4R)-4-[2-(3,5-dimethylanilino)-5,7-dihydropyrrolo[3,4-d]pyrimidin-6-yl]-2-methyl-1-piperidyl]-[2-(1H-tetrazol-5-yl)-4-pyridyl]methanone,
[(2R,4S)-4-[2-(3,5-dimethylanilmo)-5,7-dihydropyrrolo[3,4-d]pyrimidin-6-yl]-2-methyl-1-piperidyl]-[2-(1H-tetrazol-5-yl)-4-pyridyl]methanone,
[(2R,4R)-4-[2-(3,5-dimethylanilmo)-5,7-dihydropyrrolo[3,4-d]pyrimidm-6-yl]-2-methyl-1-piperidyl]-(4-pyridyl)methanone,
methyl 4-[(2R,4R)-2-methyl-4-[2-[(6-methyl-3-pyridyl)amino]-5,7-dihydropyrrolo[3,4-d]pyrimidin-6-yl]piperidine-1-carbonyl]pyridine-2-carboxylate,
[2-(hydroxymethyl)-4-pyridyl]-[(2R,4R)-2-methyl-4-[2-[(6-methyl-3-pyridyl)amino]-5,7-dihydropyrrolo [3,4-d]pyrimidin-6-yl] -1 -piperidyl]methanone,
4-[(2R,4R)-2-methyl-4-[2-[(6-methyl-3-pyridyl)amino]-5,7-dihydropyrrolo[3,4-d]pyrimidin-6-yl]piperidine-1-carbonyl]pyridine-2-carboxylic acid,
(5-methyl-2-oxo-1,3-dioxol-4-yl)methyl4-[(2R,4R)-2-methyl-4-[2-[4-(trifluoromethyl)anilino]-5,7-dihydropyrrolo[3,4-d]pyrimidin-6-yl]piperidine-1-carbonyl]pyridine-2-carboxylate,
1H-benzotriazol-5-yl-[(2R,4S)-2-methyl-4-[2-(4-methylanilino)-5,7-dihydropyrrolo[3,4-d]pyrimidin-6-yl]-1-piperidyl]methanone,
6-[1-(1H-benzotriazole-5-carbonyl)-4-piperidyl]-2-(3,5-dimethylanilino)-7,8-dihydropyrido[4,3-d]pyrimidin-5-one,
1H-benzotriazol-5-yl-[4-[2-(3,5-dimethylanilino)-7,8-dihydro-5H-pyrido[4,3-d]pyrimidin-6-yl]-1-piperidyl]methanone,
6-[1-(1H-benzotriazole-5-carbonyl)azetidin-3-yl]-2-(3,5-dimethylanilino)-7,8-dihydropyrido[4,3-d]pyrimidin-5-one, and
N-[4-[(2R)-4-[3-[2-(3,5-dimethylanilino)-7,8-dihydro-5H-pyrido[4,3-d]pyrimidin-6-yl]azetidin-1-yl]-2-methyl-piperidine-1-carbonyl]-2-pyridyl]methanesulfonamide.

In one embodiment, the compound of the invention is according to Formula I, wherein the compound is [(2R,4R)-4-[2-(3,5-dimethylanilino)-5,7-dihydropyrrolo[3,4-d]pyrimidin-6-yl]-2-methyl-1-piperidyl]-(6-fluoro-1H-benzotriazol-5-yl)methanone.

In one embodiment, the compound of the invention is according to Formula I, wherein the compound is not [(2R,4R)-4-[2-(3,5-dimethylanilino)-5,7-dihydropyrrolo[3,4-d]pyrimidin-6-yl]-2-methyl-1-piperidyl]-(6-fluoro-1H-benzotriazol-5-yl)methanone.

In one embodiment, the compounds of the invention are provided in a natural isotopic form.

In one embodiment, the compounds of the invention are provided in an unnatural variant isotopic form. In a specific embodiment, the unnatural variant isotopic form is a form in which deuterium (i.e. ²H or D) is incorporated where hydrogen is specified in the chemical structure in one or more atoms of a compound of the invention. In one embodiment, the atoms of the compounds of the invention are in an isotopic form which is not radioactive. In one embodiment, one or more atoms of the compounds of the invention are in an isotopic form which is radioactive. Suitably radioactive isotopes are stable isotopes. Suitably the unnatural variant isotopic form is a pharmaceutically acceptable form.

In one embodiment, a compound of the invention is provided whereby a single atom of the compound exists in an unnatural variant isotopic form. In another embodiment, a compound of the invention is provided whereby two or more atoms exist in an unnatural variant isotopic form.

Unnatural isotopic variant forms can generally be prepared by conventional techniques known to those skilled in the art or by processes described herein e.g. processes analogous to those described in the accompanying Examples for preparing natural isotopic forms. Thus, unnatural isotopic variant forms could be prepared by using appropriate isotopically variant (or labelled) reagents in place of the normal reagents employed in the illustrative example as examples.

In one aspect a compound of the invention according to any one of the embodiments herein described is present as the free base.

In one aspect a compound of the invention according to any one of the embodiments herein described is a pharmaceutically acceptable salt.

In one aspect a compound of the invention according to any one of the embodiments herein described is a solvate of the compound.

In one aspect a compound of the invention according to any one of the embodiments herein described is a solvate of a pharmaceutically acceptable salt of a compound.

While specified groups for each embodiment have generally been listed above separately, a compound of the invention includes one in which several or each embodiment in the above Formula, as well as other formulae presented herein, is selected from one or more of particular members or groups designated respectively, for each variable. Therefore, this invention is intended to include all combinations of such embodiments within its scope.

While specified groups for each embodiment have generally been listed above separately, a compound of the invention may be one for which one or more variables (for example, R groups) is selected from one or more embodiments according to any of the Formula(e) listed above. Therefore, the present invention is intended to include all combinations of variables from any of the disclosed embodiments within its scope.

Alternatively, the exclusion of one or more of the specified variables from a group or an embodiment, or combinations thereof is also contemplated by the present invention.

In certain aspects, the present invention provides prodrugs and derivatives of the compounds according to the formulae above. Prodrugs are derivatives of the compounds of the invention, which have metabolically cleavable groups and become by solvolysis or under physiological conditions the compounds of the invention, which are pharmaceutically active, *in vivo.* Such examples include, but are not limited to, choline ester derivatives and the like, N-alkylmorpholine esters and the like.

Other derivatives of the compounds of this invention have activity in both their acid and acid derivative forms, but the acid sensitive form often offers advantages of solubility, tissue compatibility, or delayed release in the mammalian organism (Bundgaard 1991). Prodrugs include acid derivatives well known to practitioners of the art, such as, for example, esters prepared by reaction of the parent acid with a suitable alcohol, or amides prepared by reaction of the parent acid compound with a substituted or unsubstituted amine, or acid anhydrides, or mixed anhydrides. Simple aliphatic or aromatic esters, amides and anhydrides derived from acidic groups pendant on the compounds of this invention are preferred prodrugs. In some cases it is desirable to prepare double ester type prodrugs such as (acyloxy)alkyl esters or ((alkoxycarbonyl)oxy)alkylesters. Particularly useful are the C₁ to C₈ alkyl, C₂-C₈ alkenyl, aryl, C₇-C₁₂ substituted aryl, and C₇-C₁₂ arylalkyl esters of the compounds of the invention.

### CLAUSES

1. A compound according to Formula I: 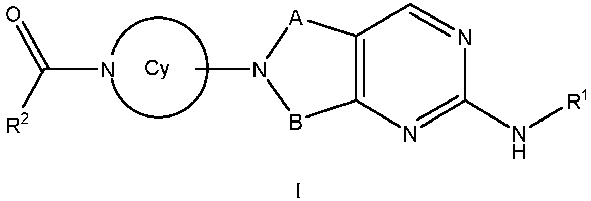 wherein
   each A and B is independently C₁₋₄ alkylenyl optionally substituted with one oxo;
   R¹ is:
      - 6-10 membered monocyclic or fused bicyclic aryl, optionally substituted with one or more independently selected R^{3a} groups, or
      - 5-10 membered monocyclic or fused bicyclic heteroaryl comprising one or more heteroatoms independently selected from N, O and S, which heteroaryl is optionally substituted with one or more independently selected R^{3b} groups,
   R² is
      - phenyl optionally substituted with one or more independently selected R⁵ groups, or
      - 5-10 membered monocyclic or fused bicyclic heteroaryl comprising one or more heteroatoms independently selected from N, O and S, which heteroaryl is optionally substituted with one or more independently selected R⁵ groups;
   each R^{3a} and R^{3b} is independently selected from:
      - halo,
      - C₁₋₄ alkyl optionally substituted with one or more independently selected halo,
      - C₁₋₄ alkoxy optionally substituted with one or more independently selected halo,
      - -SF₅,
      - -CN,
      - -O-C₃₋₇ cycloalkyl, and
      - -C(=O)NH₂;
   Cy is:
      - 4-10 membered monocyclic, bridged bicyclic, or spiro bicyclic heterocycloalkyl comprising one N atom, which heterocycloalkyl is optionally substituted with one or more independently selected R⁴ groups, or
      - 4-10 membered monocyclic, bridged bicyclic, or spiro bicyclic heterocycloalkenyl comprising one double bond and one N atom, which heterocycloalkenyl is optionally substituted with one or more independently selected R⁴ groups;
   each R⁴ is independently selected from:
      - C₁₋₄ alkyl optionally substituted with one or more independently selected halo,
      - -C(=O)O-C₁₋₄ alkyl,
      - halo,
      - -CN, and
      - -OH;
   each R⁵ is independently selected from:
      - -OH,
      - -SH,
      - halo,
      - C₁₋₄ alkyl optionally substituted with one or more independently selected -OH, or halo,
      - C₁₋₄ alkoxy optionally substituted with one or more independently selected -OH, or halo,
      - -NH-S(=O)₂-C₁₋₄ alkyl, which alkyl is optionally substituted with one or more independently selected halo, or C₁₋₄ alkoxy,
      - -NH-S(=O)₂-C₃₋₇ cycloalkyl,
      - -NH-C(=O)C₁₋₄ alkyl,
      - -C(=O)OH,
      - -C(=O)NH₂,
      - -C(=O)O-C₁₋₄ alkyl which alkyl is optionally substituted with one 5-6 membered monocyclic heterocycloalkenyl comprising one double bond and one or two O atoms, which heterocycloalkenyl is optionally substituted with one or more independently selected C₁₋₄ alkyl or oxo,
      - -C(=O)-NH-S(=O)₂-C₁₋₄ alkyl,
      - -S(=O)₂NH₂, and
      - 5-6 membered monocyclic heteroaryl comprising one or more heteroatoms independently selected from N, O and S;
   or a pharmaceutically acceptable salt thereof, or a solvate, or the solvate of a salt thereof.
2. The compound or pharmaceutically acceptable salt thereof, according to clause 1, wherein A is C₁₋₄ alkylenyl optionally substituted with one oxo.
3. The compound or pharmaceutically acceptable salt thereof, according to clause 1, wherein A is -CH₂-, -C(=O)-, -CH₂-CH₂-, -CH(CH₃)-, or -C(CH₃)₂-.
4. The compound or pharmaceutically acceptable salt thereof, according to clause 1, wherein A is -CH₂-.
5. The compound or pharmaceutically acceptable salt thereof, according to any one of clauses 1-4, wherein B is C₁₋₄ alkylenyl.
6. The compound or pharmaceutically acceptable salt thereof, according to any one of clauses 1-4, wherein B is -CH₂-, -CH₂-CH₂-, -CH(CH₃)-, or -C(CH₃)₂-.
7. The compound or pharmaceutically acceptable salt thereof, according to any one of clauses 1-4, wherein B is -CH₂- or -CH₂-CH₂-.
8. The compound or pharmaceutically acceptable salt thereof, according to any one of clauses 1-4, wherein B is -CH₂-.
9. The compound or pharmaceutically acceptable salt thereof, according to clause 1, wherein the compound is according to Formula IIa or IIb: 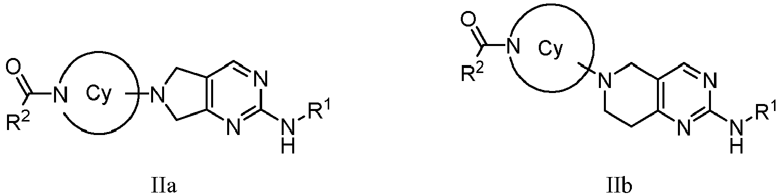
10. The compound or pharmaceutically acceptable salt thereof, according to any one of clauses 1-9, wherein Cy is 4-10 membered monocyclic, bridged bicyclic, or spiro bicyclic heterocycloalkyl comprising one N atom.
11. The compound or pharmaceutically acceptable salt thereof, according to any one of clauses 1-9, wherein Cy is azetidinyl, pyrrolidinyl, piperidinyl, azabicyclo[3.1.1]heptanyl, azaspiro[3.3]heptanyl, or azabicyclo[3.2.1]octanyl.
12. The compound or pharmaceutically acceptable salt thereof, according to any one of clauses 1-9, wherein Cy is azetidinyl, or piperidinyl.
13. The compound or pharmaceutically acceptable salt thereof, according to any one of clauses 1-9, wherein Cy is piperidinyl.
14. The compound or pharmaceutically acceptable salt thereof, according to any one of clauses 1-9, wherein Cy is 4-10 membered monocyclic, bridged bicyclic, or spiro bicyclic heterocycloalkyl comprising one N atom, which heterocycloalkyl is substituted with one or more independently selected R⁴ groups.
15. The compound or pharmaceutically acceptable salt thereof, according to any one of clauses 1-9, wherein Cy is azetidinyl, pyrrolidinyl, piperidinyl, azabicyclo[3.1.1]heptanyl, azaspiro[3.3]heptanyl, or azabicyclo[3.2.1]octanyl, each of which is substituted with one or more independently selected R⁴ groups.
16. The compound or pharmaceutically acceptable salt thereof, according to any one of clauses 1-9, wherein Cy is azetidinyl, or piperidinyl, each of which is substituted with one or more independently selected R⁴ groups.
17. The compound or pharmaceutically acceptable salt thereof, according to any one of clauses 1-9, wherein Cy is piperidinyl substituted with one or more independently selected R⁴ groups.
18. The compound or pharmaceutically acceptable salt thereof, according to any one of clauses 1-9, wherein Cy is 4-10 membered monocyclic, bridged bicyclic, or spiro bicyclic heterocycloalkenyl comprising one double bond and one N atom.
19. The compound or pharmaceutically acceptable salt thereof, according to any one of clauses 1-9, wherein Cy is dihydro-1H-pyrrole or tetrahydropyridinyl.
20. The compound or pharmaceutically acceptable salt thereof, according to any one of clauses 1-9, wherein Cy is 4-10 membered monocyclic, bridged bicyclic, or spiro bicyclic heterocycloalkenyl comprising one double bond and one N atom, which heterocycloalkenyl is substituted with one or more independently selected R⁴ groups.
21. The compound or pharmaceutically acceptable salt thereof, according to any one of clauses 1-9, wherein Cy is dihydro-1H-pyrrole or tetrahydropyridinyl, each of which is substituted with one or more independently selected R⁴ groups.
22. The compound or pharmaceutically acceptable salt thereof, according to any one of clauses 14-17, 20, or 21 wherein one or more independently selected R⁴ is C₁₋₄ alkyl optionally substituted with one or more independently selected halo.
23. The compound or pharmaceutically acceptable salt thereof, according to any one of clauses 14-17, 20, or 21 wherein one or more independently selected R⁴ is -CH₃, -CH₂CH₃, or -CF₃.
24. The compound or pharmaceutically acceptable salt thereof, according to any one of clauses 14-17, 20, or 21 wherein one or more independently selected R⁴ is -CH₃.
25. The compound or pharmaceutically acceptable salt thereof, according to any one of clauses 14-17, 20, or 21 wherein one or more independently selected R⁴ is -C(=O)O-C₁₋₄ alkyl.
26. The compound or pharmaceutically acceptable salt thereof, according to any one of clauses 14-17, 20, or 21 wherein one or more independently selected R⁴ is -C(=O)O-CH₃.
27. The compound or pharmaceutically acceptable salt thereof, according to any one of clauses 14-17, 20, or 21 wherein one or more independently selected R⁴ is halo.
28. The compound or pharmaceutically acceptable salt thereof, according to any one of clauses 14-17, 20, or 21 wherein one or more independently selected R⁴ is F.
29. The compound or pharmaceutically acceptable salt thereof, according to any one of clauses 14-17, 20, or 21 wherein one or more independently selected R⁴ is -CN.
30. The compound or pharmaceutically acceptable salt thereof, according to any one of clauses 14-17, 20, or 21 wherein one or more independently selected R⁴ is -OH.
31. The compound or pharmaceutically acceptable salt thereof, according to any one of clauses 1-9, wherein Cy is: 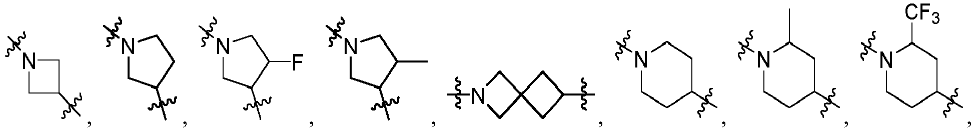 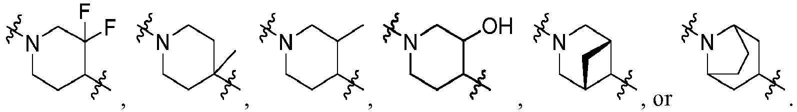
32. The compound or pharmaceutically acceptable salt thereof, according to any one of clauses 1-31, wherein R¹ is 6-10 membered monocyclic or fused bicyclic aryl.
33. The compound or pharmaceutically acceptable salt thereof, according to any one of clauses 1-31, wherein R¹ is phenyl.
34. The compound or pharmaceutically acceptable salt thereof, according to any one of clauses 1-31, wherein R¹ is 6-10 membered monocyclic or fused bicyclic aryl substituted with one or more independently selected R^{3a} groups.
35. The compound or pharmaceutically acceptable salt thereof, according to any one of clauses 1-31, wherein R¹ is phenyl substituted with one or more independently selected R^{3a} groups.
36. The compound or pharmaceutically acceptable salt thereof, according to any one of clauses 1-31, wherein R¹ is phenyl substituted with one or two independently selected R^{3a} groups.
37. The compound or pharmaceutically acceptable salt thereof, according to any one of clauses 34-36, wherein one or more independently selected R^{3a} is halo.
38. The compound or pharmaceutically acceptable salt thereof, according to any one of clauses 34-36, wherein one or more independently selected R^{3a} is F or Cl.
39. The compound or pharmaceutically acceptable salt thereof, according to any one of clauses 34-36, wherein one or more independently selected R^{3a} is F.
40. The compound or pharmaceutically acceptable salt thereof, according to any one of clauses 34-36, wherein one or more independently selected R^{3a} is C₁₋₄ alkyl optionally substituted with one or more independently selected halo.
41. The compound or pharmaceutically acceptable salt thereof, according to any one of clauses 34-36, wherein one or more independently selected R^{3a} is -CH₃, -CH₂CH₃, -CH(CH₃)₂, -CF₃, or -CH₂CF₃.
42. The compound or pharmaceutically acceptable salt thereof, according to any one of clauses 34-36, wherein one or more independently selected R^{3a} is -CH₃.
43. The compound or pharmaceutically acceptable salt thereof, according to any one of clauses 34-36, wherein one or more independently selected R^{3a} is C₁₋₄ alkoxy optionally substituted with one or more independently selected halo.
44. The compound or pharmaceutically acceptable salt thereof, according to any one of clauses 34-36, wherein one or more independently selected R^{3a} is -OCH₃, -OCH₂CH₃, -OCH(CH₃)₂, -OCF₃, or -OCH₂CF₃.
45. The compound or pharmaceutically acceptable salt thereof, according to any one of clauses 34-36, wherein one or more independently selected R^{3a} is -SF₅.
46. The compound or pharmaceutically acceptable salt thereof, according to any one of clauses 34-36, wherein one or more independently selected R^{3a} is -CN.
47. The compound or pharmaceutically acceptable salt thereof, according to any one of clauses 34-36, wherein one or more independently selected R^{3a} is -O-C₃₋₇ cycloalkyl.
48. The compound or pharmaceutically acceptable salt thereof, according to any one of clauses 34-36, wherein one or more independently selected R^{3a} is -O-cyclopropyl.
49. The compound or pharmaceutically acceptable salt thereof, according to any one of clauses 34-36, wherein one or more independently selected R^{3a} is -C(=O)NH₂.
50. The compound or pharmaceutically acceptable salt thereof, according to any one of clauses 1-31, wherein R¹ is5-10 membered monocyclic or fused bicyclic heteroaryl comprising one or more heteroatoms independently selected from N, O and S.
51. The compound or pharmaceutically acceptable salt thereof, according to any one of clauses 1-31, wherein R¹ is pyridinyl, or pyrazolyl.
52. The compound or pharmaceutically acceptable salt thereof, according to any one of clauses 1-31, wherein R¹ is 5-10 membered monocyclic or fused bicyclic heteroaryl comprising one or more heteroatoms independently selected from N, O and S, which heteroaryl is optionally substituted with one or more independently selected R^{3b} groups.
53. The compound or pharmaceutically acceptable salt thereof, according to any one of clauses 1-31, wherein R¹ is pyridinyl, or pyrazolyl, each of which is substituted with one or more independently selected R^{3b} groups.
54. The compound or pharmaceutically acceptable salt thereof, according to any one of clauses 1-31, wherein R¹ is pyridinyl, or pyrazolyl, each of which is substituted with one or two independently selected R^{3b} groups.
55. The compound or pharmaceutically acceptable salt thereof, according to any one of clauses 52-54, wherein one or more independently selected R^{3b} is halo.
56. The compound or pharmaceutically acceptable salt thereof, according to any one of clauses 52-54, wherein one or more independently selected R^{3b} is F or Cl.
57. The compound or pharmaceutically acceptable salt thereof, according to any one of clauses 52-54, wherein one or more independently selected R^{3b} is F.
58. The compound or pharmaceutically acceptable salt thereof, according to any one of clauses 52-54, wherein one or more independently selected R^{3b} is C₁₋₄ alkyl optionally substituted with one or more independently selected halo.
59. The compound or pharmaceutically acceptable salt thereof, according to any one of clauses 52-54, wherein one or more independently selected R^{3b} is -CH₃, -CH₂CH₃, -CH(CH₃)₂, -CF₃, or -CH₂CF₃.
60. The compound or pharmaceutically acceptable salt thereof, according to any one of clauses 52-54, wherein one or more independently selected R^{3b} is -CH₃.
61. The compound or pharmaceutically acceptable salt thereof, according to any one of clauses 52-54, wherein one or more independently selected R^{3b} is C₁₋₄ alkoxy optionally substituted with one or more independently selected halo.
62. The compound or pharmaceutically acceptable salt thereof, according to any one of clauses 52-54, wherein one or more independently selected R^{3b} is -OCH₃, -OCH₂CH₃, -OCH(CH₃)₂, -OCF₃, or -OCH₂CF₃.
63. The compound or pharmaceutically acceptable salt thereof, according to any one of clauses 52-54, wherein one or more independently selected R^{3b} is -SF₅.
64. The compound or pharmaceutically acceptable salt thereof, according to any one of clauses 52-54, wherein one or more independently selected R^{3b} is -CN.
65. The compound or pharmaceutically acceptable salt thereof, according to any one of clauses 52-54, wherein one or more independently selected R^{3b} is -O-C₃₋₇ cycloalkyl.
66. The compound or pharmaceutically acceptable salt thereof, according to any one of clauses 52-54, wherein one or more independently selected R^{3b} is -O-cyclopropyl.
67. The compound or pharmaceutically acceptable salt thereof, according to any one of clauses 52-54, wherein one or more independently selected R^{3b} is -C(=O)NH₂.
68. The compound or pharmaceutically acceptable salt thereof, according to any one of clauses 1-31, wherein R¹ is 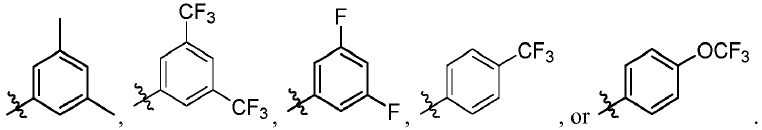
69. The compound or pharmaceutically acceptable salt thereof, according to clause 1, wherein the compound is according to Formula IIIa or IIIb: 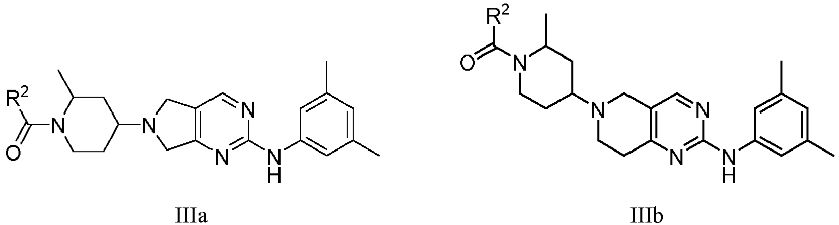
70. The compound or pharmaceutically acceptable salt thereof, according to any one of clauses 1-69, wherein R² is phenyl.
71. The compound or pharmaceutically acceptable salt thereof, according to any one of clauses 1-69, wherein R² is phenyl substituted with one or more independently selected R⁵ groups.
72. The compound or pharmaceutically acceptable salt thereof, according to any one of clauses 1-69, wherein R² is phenyl substituted with one or two independently selected R⁵ groups.
73. The compound or pharmaceutically acceptable salt thereof, according to any one of clauses 1-69, wherein, R² is phenyl substituted with one R⁵ group.
74. The compound or pharmaceutically acceptable salt thereof, according to any one of clauses 1-69, wherein R² is 5-10 membered monocyclic or fused bicyclic heteroaryl comprising one or more heteroatoms independently selected from N, O and S.
75. The compound or pharmaceutically acceptable salt thereof, according to any one of clauses 1-69, wherein R² is indazolyl, benzoimidazolyl, or benzotriazolyl.
76. The compound or pharmaceutically acceptable salt thereof, according to any one of clauses 1-69, wherein R² is benzotriazolyl.
77. The compound or pharmaceutically acceptable salt thereof, according to any one of clauses 1-69, wherein R² is 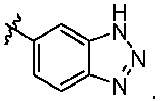
78. The compound or pharmaceutically acceptable salt thereof, according to any one of clauses 1-69, wherein R² is 5-10 membered monocyclic or fused bicyclic heteroaryl comprising one or more heteroatoms independently selected from N, O and S, which heteroaryl is substituted with one or more independently selected R⁵ groups.
79. The compound or pharmaceutically acceptable salt thereof, according to any one of clauses 1-69, wherein R² is pyridinyl, benzotriazolyl, benzoxazolyl, benzothiazolyl, benzoimidazolyl, or indazolyl, each of which is substituted with one or more independently selected R⁵ groups.
80. The compound or pharmaceutically acceptable salt thereof, according to any one of clauses 1-69, wherein R² is pyridinyl, benzotriazolyl, benzoxazolyl, benzothiazolyl, benzoimidazolyl, or indazolyl, each of which is substituted with one or two independently selected R⁵ groups.
81. The compound or pharmaceutically acceptable salt thereof, according to any one of clauses 1-69, wherein R² is pyridinyl, benzotriazolyl, benzoxazolyl, benzothiazolyl, benzoimidazolyl, or indazolyl, each of which is substituted with one R⁵ group.
82. The compound or pharmaceutically acceptable salt thereof, according to any one of clauses 1-69, wherein R² is pyridinyl substituted with one R⁵ group.
83. The compound or pharmaceutically acceptable salt thereof, according to any one of clauses 71-73, or 78-82, wherein one or more independently selected R⁵ is -OH.
84. The compound or pharmaceutically acceptable salt thereof, according to any one of clauses 71-73, or 78-82, wherein one or more independently selected R⁵ is -SH.
85. The compound or pharmaceutically acceptable salt thereof, according to any one of clauses 71-73, or 78-82, wherein one or more independently selected R⁵ is halo.
86. The compound or pharmaceutically acceptable salt thereof, according to any one of clauses 71-73, or 78-82, wherein one or more independently selected R⁵ is F.
87. The compound or pharmaceutically acceptable salt thereof, according to any one of clauses 71-73, or 78-82, wherein one or more independently selected R⁵ is C₁₋₄ alkyl optionally substituted with one or more independently selected -OH, or halo.
88. The compound or pharmaceutically acceptable salt thereof, according to any one of clauses 71-73, or 78-82, wherein one or more independently selected R⁵ is -CH₃, -CH₂CH₃, or -CH(CH₃)₂, each of which is optionally substituted with one or more independently selected -OH, or halo.
89. The compound or pharmaceutically acceptable salt thereof, according to any one of clauses 71-73, or 78-82, wherein one or more independently selected R⁵ is -CH₃, -CF₃, -CH₂CH₃, -CH₂OH, or -C(CH₃)₂OH.
90. The compound or pharmaceutically acceptable salt thereof, according to any one of clauses 71-73, or 78-82, wherein one or more independently selected R⁵ is C₁₋₄ alkoxy optionally substituted with one or more independently selected -OH, or halo.
91. The compound or pharmaceutically acceptable salt thereof, according to any one of clauses 71-73, or 78-82, wherein one or more independently selected R⁵ is -OCH₃, -OCH₂CH₃, or -OCH(CH₃)₂, each of which is optionally substituted with one or more independently selected -OH, or halo.
92. The compound or pharmaceutically acceptable salt thereof, according to any one of clauses 71-73, or 78-82, wherein one or more independently selected R⁵ is -OCH₃, -OCF₃, -OCH₂CH₃.
93. The compound or pharmaceutically acceptable salt thereof, according to any one of clauses 71-73, or 78-82, wherein one or more independently selected R⁵ is -NH-S(=O)₂-C₁₋₄ alkyl, which alkyl is optionally substituted with one or more independently selected halo, or C₁₋₄ alkoxy.
94. The compound or pharmaceutically acceptable salt thereof, according to any one of clauses 71-73, or 78-82, wherein one or more independently selected R⁵ is -NH-S(=O)₂-CH₃, or -NH-S(=O)₂-CH₂CH₃, each of which is optionally substituted with one or more independently selected halo, or C₁₋₄ alkoxy.
95. The compound or pharmaceutically acceptable salt thereof, according to any one of clauses 71-73, or 78-82, wherein one or more independently selected R⁵ is -NH-S(=O)₂-CH₃, or -NH-S(=O)₂-CH₂CH₃, each of which is optionally substituted with one or more independently selected F, -OCH₃ or-OCH₂CH₃.
96. The compound or pharmaceutically acceptable salt thereof, according to any one of clauses 71-73, or 78-82, wherein one or more independently selected R⁵ is -NH-S(=O)₂-CH₃, -NH-S(=O)₂-CH₂CH₃, -NH-S(=O)₂-CH₂CF₃, or -NH-S(=O)₂-CH₂CH₂OCH₃.
97. The compound or pharmaceutically acceptable salt thereof, according to any one of clauses 71-73, or 78-82, wherein one or more independently selected R⁵ is -NH-S(=O)₂-C₃₋₇ cycloalkyl.
98. The compound or pharmaceutically acceptable salt thereof, according to any one of clauses 71-73, or 78-82, wherein one or more independently selected R⁵ is-NH-S(=O)₂-cyclopropyl.
99. The compound or pharmaceutically acceptable salt thereof, according to any one of clauses 71-73, or 78-82, wherein one or more independently selected R⁵ is -NH-C(=O)C₁₋₄ alkyl.
100. The compound or pharmaceutically acceptable salt thereof, according to any one of clauses 71-73, or 78-82, wherein one or more independently selected R⁵ is -NH-C(=O)CH₃, -NH-C(=O)CH₂CH₃ or-NH-C(=O)CH(CH₃)₂.
101. The compound or pharmaceutically acceptable salt thereof, according to any one of clauses 71-73, or 78-82, wherein one or more independently selected R⁵ is -C(=O)OH.
102. The compound or pharmaceutically acceptable salt thereof, according to any one of clauses 71-73, or 78-82, wherein one or more independently selected R⁵ is -C(=O)NH₂.
103. The compound or pharmaceutically acceptable salt thereof, according to any one of clauses 71-73, or 78-82, wherein one or more independently selected R⁵ is -C(=O)O-C₁₋₄ alkyl which alkyl is optionally substituted with one 5-6 membered monocyclic heterocycloalkenyl comprising one double bond and one or two O atoms, which heterocycloalkenyl is optionally substituted with one or more independently selected C₁₋₄ alkyl or oxo.
104. The compound or pharmaceutically acceptable salt thereof, according to any one of clauses 71-73, or 78-82, wherein one or more independently selected R⁵ is -C(=O)O-CH₃, or -C(=O)O-CH₂CH₃, each of which is optionally substituted with one 5-6 membered monocyclic heterocycloalkenyl comprising one double bond and one or two O atoms, which heterocycloalkenyl is optionally substituted with one or more independently selected C₁₋₄ alkyl or oxo.
105. The compound or pharmaceutically acceptable salt thereof, according to any one of clauses 71-73, or 78-82, wherein one or more independently selected R⁵ is -C(=O)O-CH₃, or -C(=O)O-CH₂CH₃, each of which is optionally substituted with 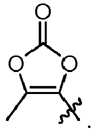
106. The compound or pharmaceutically acceptable salt thereof, according to any one of clauses 71-73, or 78-82, wherein one or more independently selected R⁵ is -C(=O)-NH-S(=O)₂-C₁₋₄ alkyl.
107. The compound or pharmaceutically acceptable salt thereof, according to any one of clauses 71-73, or 78-82, wherein one or more independently selected R⁵ is -C(=O)-NH-S(=O)₂-CH₃, -C(=O)-NH-S(=O)₂-CH₂CH₃ or -C(=O)-NH-S(=O)₂-CH(CH₃)₂.
108. The compound or pharmaceutically acceptable salt thereof, according to any one of clauses 71-73, or 78-82, wherein one or more independently selected R⁵ is -S(=O)₂NH₂.
109. The compound or pharmaceutically acceptable salt thereof, according to any one of clauses 71-73, or 78-82, wherein one or more independently selected R⁵ is 5-6 membered monocyclic heteroaryl comprising one or more heteroatoms independently selected from N, O and S.
110. The compound or pharmaceutically acceptable salt thereof, according to any one of clauses 71-73, or 78-82, wherein one or more independently selected R⁵ is tetrazolyl.
111. A pharmaceutical composition comprising a pharmaceutically acceptable carrier and a pharmaceutically effective amount of a compound or pharmaceutically acceptable salt thereof according to any one of claims 1-110.
112. The pharmaceutical composition according to claim 110, comprising a further therapeutic agent.
113. A compound or pharmaceutically acceptable salt thereof according to any one of claims 1-110, or a pharmaceutical composition according to claim 111 or 112 for use in medicine.
114. A compound or pharmaceutically acceptable salt thereof according to claim any one of claims 1-110, or a pharmaceutical composition according to claim 111 or 112 for use in the prophylaxis and/or treatment of fibrotic diseases, proliferative diseases, inflammatory diseases, autoimmune diseases, respiratory diseases, cardiovascular diseases, neurodegenerative diseases, dermatological disorders, pain, and/or abnormal angiogenesis associated diseases.
115. The pharmaceutical composition according to claim 112, wherein the further therapeutic agent is an agent for the prophylaxis and/or treatment of fibrotic diseases, proliferative diseases, inflammatory diseases, autoimmune diseases, respiratory diseases, cardiovascular diseases, neurodegenerative diseases, dermatological disorders, pain, and/or abnormal angiogenesis associated diseases.

### PHARMACEUTICAL COMPOSITIONS

When employed as a pharmaceutical, a compound of the invention is typically administered in the form of a pharmaceutical composition. Such compositions can be prepared in a manner well known in the pharmaceutical art and comprise at least one active compound of the invention according to Formula I. Generally, a compound of the invention is administered in a pharmaceutically effective amount. The amount of compound of the invention actually administered will typically be determined by a physician, in the light of the relevant circumstances, including the condition to be treated, the chosen route of administration, the actual compound of the invention administered, the age, weight, and response of the individual patient, the severity of the patient's symptoms, and the like.

The pharmaceutical compositions of this invention can be administered by a variety of routes including oral, rectal, transdermal, subcutaneous, intra-articular, intravenous, intramuscular, and intranasal. Depending on the intended route of delivery, a compound of the invention is preferably formulated as either injectable or oral compositions or as salves, as lotions or as patches all for transdermal administration.

The compositions for oral administration can take the form of bulk liquid solutions or suspensions, or bulk powders. More commonly, however, the compositions are presented in unit dosage forms to facilitate accurate dosing. The term 'unit dosage forms' refers to physically discrete units suitable as unitary dosages for human subjects and other mammals, each unit containing a predetermined quantity of active material calculated to produce the desired therapeutic effect, in association with a suitable pharmaceutical excipient, vehicle or carrier. Typical unit dosage forms include prefilled, premeasured ampules or syringes of the liquid compositions or pills, tablets, capsules or the like in the case of solid compositions. In such compositions, the compound of the invention according to Formula I is usually a minor component (from about 0.1 to about 50% by weight or preferably from about 1 to about 40% by weight) with the remainder being various vehicles or carriers and processing aids helpful for forming the desired dosing form.

Liquid forms suitable for oral administration may include a suitable aqueous or non-aqueous vehicle with buffers, suspending and dispensing agents, colorants, flavors and the like. Solid forms may include, for example, any of the following ingredients, or compound of the inventions of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint or orange flavoring.

Injectable compositions are typically based upon injectable sterile saline or phosphate-buffered saline or other injectable carriers known in the art. As before, the active compound of the invention according to Formula I in such compositions is typically a minor component, often being from about 0.05 to 10% by weight with the remainder being the injectable carrier and the like.

Transdermal compositions are typically formulated as a topical ointment or cream containing the active ingredient(s), generally in an amount ranging from about 0.01 to about 20% by weight, preferably from about 0.1 to about 20% by weight, preferably from about 0.1 to about 10% by weight, and more preferably from about 0.5 to about 15% by weight. When formulated as an ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration of stability of the active ingredients or the formulation. All such known transdermal formulations and ingredients are included within the scope of this invention.

A compound of the invention can also be administered by a transdermal device. Accordingly, transdermal administration can be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 of Remington's Pharmaceutical Sciences, 17th edition, 1985, Mack Publishing Company, Easton, Pennsylvania, which is incorporated herein by reference.

A compound of the invention can also be administered in sustained release forms or from sustained release drug delivery systems. A description of representative sustained release materials can be found in Remington's Pharmaceutical Sciences.

The following formulation examples illustrate representative pharmaceutical compositions that may be prepared in accordance with this invention. The present invention, however, is not limited to the following pharmaceutical compositions.

### Formulation 1 - Tablets

A compound of the invention according to Formula I may be admixed as a dry powder with a dry gelatin binder in an approximate 1:2 weight ratio. A minor amount of magnesium stearate may be added as a lubricant. The mixture may be formed into 240-270 mg tablets (80-90 mg of active compound of the invention according to Formula I per tablet) in a tablet press.

### Formulation 2 - Capsules

A compound of the invention according to Formula I may be admixed as a dry powder with a starch diluent in an approximate 1:1 weight ratio. The mixture may be filled into 250 mg capsules (125 mg of active compound of the invention according to Formula I per capsule).

### Formulation 3 - Liquid

A compound of the invention according to Formula I (125 mg), may be admixed with sucrose (1.75 g) and xanthan gum (4 mg) and the resultant mixture may be blended, passed through a No. 10 mesh U.S. sieve, and then mixed with a previously made solution of microcrystalline cellulose and sodium carboxymethyl cellulose (11:89, 50 mg) in water. Sodium benzoate (10 mg), flavor, and color may be diluted with water and added with stirring. Sufficient water may then be added with stirring. Further sufficient water may be then added to produce a total volume of 5 mL.

### Formulation 4 - Tablets

A compound of the invention according to Formula I may be admixed as a dry powder with a dry gelatin binder in an approximate 1:2 weight ratio. A minor amount of magnesium stearate may be added as a lubricant. The mixture may be formed into 450-900 mg tablets (150-300 mg of active compound of the invention according to Formula I) in a tablet press.

### Formulation 5 - Injection

A compound of the invention according to Formula I may be dissolved or suspended in a buffered sterile saline injectable aqueous medium to a concentration of approximately 5 mg/mL.

### Formulation 6 - Topical

Stearyl alcohol (250 g) and a white petrolatum (250 g) may be melted at about 75°C and then a mixture of a compound of the invention according to Formula I (50 g) methylparaben (0.25 g), propylparaben (0.15 g), sodium lauryl sulfate (10 g), and propylene glycol (120 g) dissolved in water (about 370 g) may be added and the resulting mixture may be stirred until it congeals.

### METHODS OF TREATMENT

In one embodiment, the present invention provides compounds of the invention, or pharmaceutical compositions comprising a compound of the invention, for use in medicine. In a particular embodiment, the present invention provides compounds of the invention or pharmaceutical compositions comprising a compound of the invention, for use in the prophylaxis and/or treatment of fibrotic diseases. In a particular embodiment, the fibrotic disease is selected from idiopathic pulmonary fibrosis (IPF), cystic fibrosis, other diffuse parenchymal lung diseases of different etiologies including iatrogenic drug-induced fibrosis, occupational and/or environmental induced fibrosis, granulomatous diseases (sarcoidosis, hypersensitivity pneumonia), collagen vascular disease, alveolar proteinosis, langerhans cell granulomatosis, lymphangioleiomyomatosis, inherited diseases (Hermansky-Pudlak Syndrome, tuberous sclerosis, neurofibromatosis, metabolic storage disorders, familial interstitial lung disease), radiation induced fibrosis, chronic obstructive pulmonary disease (COPD), scleroderma, bleomycin induced pulmonary fibrosis, chronic asthma, silicosis, asbestos induced pulmonary fibrosis, acute respiratory distress syndrome (ARDS), kidney fibrosis, tubulointerstitium fibrosis, glomerular nephritis, focal segmental glomerular sclerosis, IgA nephropathy, hypertension, Alport, gut fibrosis, liver fibrosis, cirrhosis, alcohol induced liver fibrosis, toxic/drug induced liver fibrosis, hemochromatosis, nonalcoholic steatohepatitis (NASH), biliary duct injury, primary biliary cirrhosis, infection induced liver fibrosis, viral induced liver fibrosis, and autoimmune hepatitis, corneal scarring, hypertrophic scarring, Dupuytren disease, keloids, cutaneous fibrosis, cutaneous scleroderma, systemic sclerosis, spinal cord injury/fibrosis, myelofibrosis, vascular restenosis, atherosclerosis, arteriosclerosis, Wegener's granulomatosis, Peyronie's disease, or chronic lymphocytic. More particularly, the fibrotic diseases is idiopathic pulmonary fibrosis (IPF), or systemic sclerosis.

In another embodiment, the present invention provides compounds of the invention, or pharmaceutical compositions comprising a compound of the invention for use in the manufacture of a medicament for use in the prophylaxis and/or treatment of fibrotic diseases. In a particular embodiment, the fibrotic disease is selected from idiopathic pulmonary fibrosis (IPF), cystic fibrosis, other diffuse parenchymal lung diseases of different etiologies including iatrogenic drug-induced fibrosis, occupational and/or environmental induced fibrosis, granulomatous diseases (sarcoidosis, hypersensitivity pneumonia), collagen vascular disease, alveolar proteinosis, langerhans cell granulomatosis, lymphangioleiomyomatosis, inherited diseases (Hermansky-Pudlak Syndrome, tuberous sclerosis, neurofibromatosis, metabolic storage disorders, familial interstitial lung disease), radiation induced fibrosis, chronic obstructive pulmonary disease (COPD), scleroderma, bleomycin induced pulmonary fibrosis, chronic asthma, silicosis, asbestos induced pulmonary fibrosis, acute respiratory distress syndrome (ARDS), kidney fibrosis, tubulointerstitium fibrosis, glomerular nephritis, focal segmental glomerular sclerosis, IgA nephropathy, hypertension, Alport, gut fibrosis, liver fibrosis, cirrhosis, alcohol induced liver fibrosis, toxic/drug induced liver fibrosis, hemochromatosis, nonalcoholic steatohepatitis (NASH), biliary duct injury, primary biliary cirrhosis, infection induced liver fibrosis, viral induced liver fibrosis, and autoimmune hepatitis, corneal scarring, hypertrophic scarring, Dupuytren disease, keloids, cutaneous fibrosis, cutaneous scleroderma, systemic sclerosis, spinal cord injury/fibrosis, myelofibrosis, vascular restenosis, atherosclerosis, arteriosclerosis, Wegener's granulomatosis, Peyronie's disease, or chronic lymphocytic. More particularly, the fibrotic diseases is idiopathic pulmonary fibrosis (IPF), or systemic sclerosis.

In additional method of treatment aspects, this invention provides methods of prophylaxis and/or treatment of a mammal afflicted with fibrotic diseases, which methods comprise the administration of an effective amount of a compound of the invention or one or more of the pharmaceutical compositions herein described for the treatment or prophylaxis of said condition. In a particular embodiment, the fibrotic disease is selected from idiopathic pulmonary fibrosis (IPF), cystic fibrosis, other diffuse parenchymal lung diseases of different etiologies including iatrogenic drug-induced fibrosis, occupational and/or environmental induced fibrosis, granulomatous diseases (sarcoidosis, hypersensitivity pneumonia), collagen vascular disease, alveolar proteinosis, langerhans cell granulomatosis, lymphangioleiomyomatosis, inherited diseases (Hermansky-Pudlak Syndrome, tuberous sclerosis, neurofibromatosis, metabolic storage disorders, familial interstitial lung disease), radiation induced fibrosis, chronic obstructive pulmonary disease (COPD), scleroderma, bleomycin induced pulmonary fibrosis, chronic asthma, silicosis, asbestos induced pulmonary fibrosis, acute respiratory distress syndrome (ARDS), kidney fibrosis, tubulointerstitium fibrosis, glomerular nephritis, focal segmental glomerular sclerosis, IgA nephropathy, hypertension, Alport, gut fibrosis, liver fibrosis, cirrhosis, alcohol induced liver fibrosis, toxic/drug induced liver fibrosis, hemochromatosis, nonalcoholic steatohepatitis (NASH), biliary duct injury, primary biliary cirrhosis, infection induced liver fibrosis, viral induced liver fibrosis, and autoimmune hepatitis, corneal scarring, hypertrophic scarring, Dupuytren disease, keloids, cutaneous fibrosis, cutaneous scleroderma, systemic sclerosis, spinal cord injury/fibrosis, myelofibrosis, vascular restenosis, atherosclerosis, arteriosclerosis, Wegener's granulomatosis, Peyronie's disease, or chronic lymphocytic. More particularly, the fibrotic diseases is idiopathic pulmonary fibrosis (IPF).

In one embodiment, the present invention provides pharmaceutical compositions comprising a compound of the invention, and another therapeutic agent. In a particular embodiment, the other therapeutic agent is a fibrotic diseases treatment agent. In a particular embodiment, the fibrotic disease is selected from idiopathic pulmonary fibrosis (IPF), cystic fibrosis, other diffuse parenchymal lung diseases of different etiologies including iatrogenic drug-induced fibrosis, occupational and/or environmental induced fibrosis, granulomatous diseases (sarcoidosis, hypersensitivity pneumonia), collagen vascular disease, alveolar proteinosis, langerhans cell granulomatosis, lymphangioleiomyomatosis, inherited diseases (Hermansky-Pudlak Syndrome, tuberous sclerosis, neurofibromatosis, metabolic storage disorders, familial interstitial lung disease), radiation induced fibrosis, chronic obstructive pulmonary disease (COPD), scleroderma, bleomycin induced pulmonary fibrosis, chronic asthma, silicosis, asbestos induced pulmonary fibrosis, acute respiratory distress syndrome (ARDS), kidney fibrosis, tubulointerstitium fibrosis, glomerular nephritis, focal segmental glomerular sclerosis, IgA nephropathy, hypertension, Alport, gut fibrosis, liver fibrosis, cirrhosis, alcohol induced liver fibrosis, toxic/drug induced liver fibrosis, hemochromatosis, nonalcoholic steatohepatitis (NASH), biliary duct injury, primary biliary cirrhosis, infection induced liver fibrosis, viral induced liver fibrosis, and autoimmune hepatitis, corneal scarring, hypertrophic scarring, Dupuytren disease, keloids, cutaneous fibrosis, cutaneous scleroderma, systemic sclerosis, spinal cord injury/fibrosis, myelofibrosis, vascular restenosis, atherosclerosis, arteriosclerosis, Wegener's granulomatosis, Peyronie's disease, or chronic lymphocytic. More particularly, the fibrotic diseases is idiopathic pulmonary fibrosis (IPF), or systemic sclerosis.

In one embodiment, the present invention provides compounds of the invention, or pharmaceutical compositions comprising a compound of the invention, for use in medicine. In a particular embodiment, the present invention provides compounds of the invention or pharmaceutical compositions comprising a compound of the invention, for use in the prophylaxis and/or treatment of proliferative diseases. In a particular embodiment, the proliferative disease is selected from cancer, leukemia, multiple myeloma and psoriasis.

In another embodiment, the present invention provides compounds of the invention, or pharmaceutical compositions comprising a compound of the invention for use in the manufacture of a medicament for use in the prophylaxis and/or treatment of proliferative diseases. In a particular embodiment, the proliferative disease is selected from cancer, leukemia, multiple myeloma and psoriasis.

In additional method of treatment aspects, this invention provides methods of prophylaxis and/or treatment of a mammal afflicted with proliferative diseases, which methods comprise the administration of an effective amount of a compound of the invention or one or more of the pharmaceutical compositions herein described for the treatment or prophylaxis of said condition. In a particular embodiment, the proliferative disease is selected from cancer, leukemia, multiple myeloma and psoriasis.

In one embodiment, the present invention provides pharmaceutical compositions comprising a compound of the invention, and another therapeutic agent. In a particular embodiment, the other therapeutic agent is a proliferative diseases treatment agent. In a particular embodiment, the proliferative disease is selected from cancer, leukemia, multiple myeloma and psoriasis.

In one embodiment, the present invention provides compounds of the invention, or pharmaceutical compositions comprising a compound of the invention, for use in medicine. In a particular embodiment, the present invention provides compounds of the invention or pharmaceutical compositions comprising a compound of the invention, for use in the prophylaxis and/or treatment of inflammatory diseases. In a particular embodiment, the inflammatory disease is selected from rheumatoid arthritis, osteoarthritis, allergic airway disease (*e.g*. asthma), chronic obstructive pulmonary disease (COPD) and inflammatory bowel diseases (*e.g*. Crohn's disease and ulcerative colitis). More particularly, the inflammatory disease is selected from rheumatoid arthritis, and chronic obstructive pulmonary disease (COPD).

In another embodiment, the present invention provides compounds of the invention, or pharmaceutical compositions comprising a compound of the invention for use in the manufacture of a medicament for use in the prophylaxis and/or treatment of inflammatory diseases. In a particular embodiment, the inflammatory disease is selected from rheumatoid arthritis, osteoarthritis, allergic airway disease (*e.g.* asthma), chronic obstructive pulmonary disease (COPD) and inflammatory bowel diseases (*e.g.* Crohn's disease and ulcerative colitis). More particularly, the inflammatory disease is selected from rheumatoid arthritis, and chronic obstructive pulmonary disease (COPD).

In additional method of treatment aspects, this invention provides methods of prophylaxis and/or treatment of a mammal afflicted with inflammatory diseases, which methods comprise the administration of an effective amount of a compound of the invention or one or more of the pharmaceutical compositions herein described for the treatment or prophylaxis of said condition. In a particular embodiment, the inflammatory disease is selected from rheumatoid arthritis, osteoarthritis, allergic airway disease (*e.g.* asthma), chronic obstructive pulmonary disease (COPD) and inflammatory bowel diseases (*e.g.* Crohn's disease and ulcerative colitis). More particularly, the inflammatory disease is selected from rheumatoid arthritis, and chronic obstructive pulmonary disease (COPD).

In one embodiment, the present invention provides pharmaceutical compositions comprising a compound of the invention, and another therapeutic agent. In a particular embodiment, the other therapeutic agent is a inflammatory diseases treatment agent. In a particular embodiment, the inflammatory disease is selected from rheumatoid arthritis, osteoarthritis, allergic airway disease (*e.g*. asthma), chronic obstructive pulmonary disease (COPD) and inflammatory bowel diseases (*e.g*. Crohn's disease and ulcerative colitis). More particularly, the inflammatory disease is selected from rheumatoid arthritis, and chronic obstructive pulmonary disease (COPD).

In one embodiment, the present invention provides compounds of the invention, or pharmaceutical compositions comprising a compound of the invention, for use in medicine. In a particular embodiment, the present invention provides compounds of the invention or pharmaceutical compositions comprising a compound of the invention, for use in the prophylaxis and/or treatment of autoimmune diseases. In a particular embodiment, the autoimmune disease is selected from COPD, asthma (e.g intrinsic asthma, extrinsic asthma, dust asthma, infantily asthma) particularly chronic or inveterate asthma (for example late asthma and airway hyperreponsiveness), bronchitis, including bronchial asthma, systemic lupus erythematosus (SLE), cutaneous lupus erythrematosis, lupus nephritis, dermatomyositis, Sjogren's syndrome, multiple sclerosis, psoriasis, dry eye disease, type I diabetes mellitus and complications associated therewith, atopic eczema (atopic dermatitis), thyroiditis (Hashimoto's and autoimmune thyroiditis), contact dermatitis and further eczematous dermatitis, inflammatory bowel disease (*e.g.* Crohn's disease and ulcerative colitis), atherosclerosis and amyotrophic lateral sclerosis. Particularly, the autoimmune disease is selected from COPD, asthma, systemic lupus erythematosis, type I diabetes mellitus and inflammatory bowel disease.

In another embodiment, the present invention provides compounds of the invention, or pharmaceutical compositions comprising a compound of the invention for use in the manufacture of a medicament for use in the prophylaxis and/or treatment of autoimmune diseases. In a particular embodiment, the autoimmune disease is selected from COPD, asthma (e.g intrinsic asthma, extrinsic asthma, dust asthma, infantily asthma) particularly chronic or inveterate asthma (for example late asthma and airway hyperreponsiveness), bronchitis, including bronchial asthma, systemic lupus erythematosus (SLE), cutaneous lupus erythrematosis, lupus nephritis, dermatomyositis, Sjogren's syndrome, multiple sclerosis, psoriasis, dry eye disease, type I diabetes mellitus and complications associated therewith, atopic eczema (atopic dermatitis), thyroiditis (Hashimoto's and autoimmune thyroiditis), contact dermatitis and further eczematous dermatitis, inflammatory bowel disease (*e.g.* Crohn's disease and ulcerative colitis), atherosclerosis and amyotrophic lateral sclerosis. Particularly, the autoimmune disease is selected from COPD, asthma, systemic lupus erythematosis, type I diabetes mellitus and inflammatory bowel disease.

In additional method of treatment aspects, this invention provides methods of prophylaxis and/or treatment of a mammal afflicted with autoimmune diseases, which methods comprise the administration of an effective amount of a compound of the invention or one or more of the pharmaceutical compositions herein described for the treatment or prophylaxis of said condition. In a particular embodiment, the autoimmune disease is selected from COPD, asthma (e.g intrinsic asthma, extrinsic asthma, dust asthma, infantily asthma) particularly chronic or inveterate asthma (for example late asthma and airway hyperreponsiveness), bronchitis, including bronchial asthma, systemic lupus erythematosus (SLE), cutaneous lupus erythrematosis, lupus nephritis, dermatomyositis, Sjogren's syndrome, multiple sclerosis, psoriasis, dry eye disease, type I diabetes mellitus and complications associated therewith, atopic eczema (atopic dermatitis), thyroiditis (Hashimoto's and autoimmune thyroiditis), contact dermatitis and further eczematous dermatitis, inflammatory bowel disease (*e.g.* Crohn's disease and ulcerative colitis), atherosclerosis and amyotrophic lateral sclerosis. Particularly, the autoimmune disease is selected from COPD, asthma, systemic lupus erythematosis, type I diabetes mellitus and inflammatory bowel disease.

In one embodiment, the present invention provides pharmaceutical compositions comprising a compound of the invention, and another therapeutic agent. In a particular embodiment, the other therapeutic agent is a autoimmune diseases treatment agent. In a particular embodiment, the autoimmune disease is selected from COPD, asthma (e.g intrinsic asthma, extrinsic asthma, dust asthma, infantily asthma) particularly chronic or inveterate asthma (for example late asthma and airway hyperreponsiveness), bronchitis, including bronchial asthma, systemic lupus erythematosus (SLE), cutaneous lupus erythrematosis, lupus nephritis, dermatomyositis, Sjogren's syndrome, multiple sclerosis, psoriasis, dry eye disease, type I diabetes mellitus and complications associated therewith, atopic eczema (atopic dermatitis), thyroiditis (Hashimoto's and autoimmune thyroiditis), contact dermatitis and further eczematous dermatitis, inflammatory bowel disease (*e.g.* Crohn's disease and ulcerative colitis), atherosclerosis and amyotrophic lateral sclerosis. Particularly, the autoimmune disease is selected from COPD, asthma, systemic lupus erythematosis, type I diabetes mellitus and inflammatory bowel disease.

In one embodiment, the present invention provides compounds of the invention, or pharmaceutical compositions comprising a compound of the invention, for use in medicine. In a particular embodiment, the present invention provides compounds of the invention or pharmaceutical compositions comprising a compound of the invention, for use in the prophylaxis and/or treatment of respiratory disease. In a particular embodiment, the respiratory disease is selected from asthma, adult respiratory distress syndrome and allergic (extrinsic) asthma, non-allergic (intrinsic) asthma, acute severe asthma, chronic asthma, clinical asthma, nocturnal asthma, allergen-induced asthma, aspirin-sensitive asthma, exercise-induced asthma, isocapnic hyperventilation, child onset asthma, adult-onset asthma, cough-variant asthma, occupational asthma, steroid-resistant asthma, seasonal asthma, seasonal allergic rhinitis, perennial allergic rhinitis, chronic obstructive pulmonary disease, including chronic bronchitis or emphysema, pulmonary hypertension, interstitial lung fibrosis and/or airway inflammation and cystic fibrosis, and hypoxia.

In another embodiment, the present invention provides compounds of the invention, or pharmaceutical compositions comprising a compound of the invention for use in the manufacture of a medicament for use in the prophylaxis and/or treatment of respiratory disease. In a particular embodiment, the respiratory disease is selected from asthma, adult respiratory distress syndrome and allergic (extrinsic) asthma, non-allergic (intrinsic) asthma, acute severe asthma, chronic asthma, clinical asthma, nocturnal asthma, allergen-induced asthma, aspirin-sensitive asthma, exercise-induced asthma, isocapnic hyperventilation, child onset asthma, adult-onset asthma, cough-variant asthma, occupational asthma, steroid-resistant asthma, seasonal asthma, seasonal allergic rhinitis, perennial allergic rhinitis, chronic obstructive pulmonary disease, including chronic bronchitis or emphysema, pulmonary hypertension, interstitial lung fibrosis and/or airway inflammation and cystic fibrosis, and hypoxia.

In additional method of treatment aspects, this invention provides methods of prophylaxis and/or treatment of a mammal afflicted with respiratory disease, which methods comprise the administration of an effective amount of a compound of the invention or one or more of the pharmaceutical compositions herein described for the treatment or prophylaxis of said condition. In a particular embodiment, the respiratory disease is selected from asthma, adult respiratory distress syndrome and allergic (extrinsic) asthma, non-allergic (intrinsic) asthma, acute severe asthma, chronic asthma, clinical asthma, nocturnal asthma, allergen-induced asthma, aspirin-sensitive asthma, exercise-induced asthma, isocapnic hyperventilation, child onset asthma, adult-onset asthma, cough-variant asthma, occupational asthma, steroid-resistant asthma, seasonal asthma, seasonal allergic rhinitis, perennial allergic rhinitis, chronic obstructive pulmonary disease, including chronic bronchitis or emphysema, pulmonary hypertension, interstitial lung fibrosis and/or airway inflammation and cystic fibrosis, and hypoxia.

In one embodiment, the present invention provides pharmaceutical compositions comprising a compound of the invention, and another therapeutic agent. In a particular embodiment, the other therapeutic agent is a respiratory disease treatment agent. In a particular embodiment, the respiratory disease is selected from asthma, adult respiratory distress syndrome and allergic (extrinsic) asthma, non-allergic (intrinsic) asthma, acute severe asthma, chronic asthma, clinical asthma, nocturnal asthma, allergen-induced asthma, aspirin-sensitive asthma, exercise-induced asthma, isocapnic hyperventilation, child onset asthma, adult-onset asthma, cough-variant asthma, occupational asthma, steroid-resistant asthma, seasonal asthma, seasonal allergic rhinitis, perennial allergic rhinitis, chronic obstructive pulmonary disease, including chronic bronchitis or emphysema, pulmonary hypertension, interstitial lung fibrosis and/or airway inflammation and cystic fibrosis, and hypoxia.

In one embodiment, the present invention provides compounds of the invention, or pharmaceutical compositions comprising a compound of the invention, for use in medicine. In a particular embodiment, the present invention provides compounds of the invention or pharmaceutical compositions comprising a compound of the invention, for use in the prophylaxis and/or treatment of cardiovascular disease. In a particular embodiment, the cardiovascular disease is selected from arrhythmia (atrial or ventricular or both), atherosclerosis and its sequelae, angina, cardiac rhythm disturbances, myocardial ischemia, myocardial infarction, cardiac or vascular aneurysm, vasculitis, stroke, peripheral obstructive arteriopathy of a limb, an organ, or a tissue, reperfusion injury following ischemia of the brain, heart, kidney or other organ or tissue, endotoxic, surgical, or traumatic shock, hypertension, valvular heart disease, heart failure, abnormal blood pressure, shock, vasoconstriction (including that associated with migraines), vascular abnormality, inflammation, insufficiency limited to a single organ or tissue.

In another embodiment, the present invention provides compounds of the invention, or pharmaceutical compositions comprising a compound of the invention for use in the manufacture of a medicament for use in the prophylaxis and/or treatment of cardiovascular disease. In a particular embodiment, the cardiovascular disease is selected from arrhythmia (atrial or ventricular or both), atherosclerosis and its sequelae, angina, cardiac rhythm disturbances, myocardial ischemia, myocardial infarction, cardiac or vascular aneurysm, vasculitis, stroke, peripheral obstructive arteriopathy of a limb, an organ, or a tissue, reperfusion injury following ischemia of the brain, heart, kidney or other organ or tissue, endotoxic, surgical, or traumatic shock, hypertension, valvular heart disease, heart failure, abnormal blood pressure, shock, vasoconstriction (including that associated with migraines), vascular abnormality, inflammation, insufficiency limited to a single organ or tissue.

In additional method of treatment aspects, this invention provides methods of prophylaxis and/or treatment of a mammal afflicted with cardiovascular disease, which methods comprise the administration of an effective amount of a compound of the invention or one or more of the pharmaceutical compositions herein described for the treatment or prophylaxis of said condition. In a particular embodiment, the cardiovascular disease is selected from arrhythmia (atrial or ventricular or both), atherosclerosis and its sequelae, angina, cardiac rhythm disturbances, myocardial ischemia, myocardial infarction, cardiac or vascular aneurysm, vasculitis, stroke, peripheral obstructive arteriopathy of a limb, an organ, or a tissue, reperfusion injury following ischemia of the brain, heart, kidney or other organ or tissue, endotoxic, surgical, or traumatic shock, hypertension, valvular heart disease, heart failure, abnormal blood pressure, shock, vasoconstriction (including that associated with migraines), vascular abnormality, inflammation, insufficiency limited to a single organ or tissue.

In one embodiment, the present invention provides pharmaceutical compositions comprising a compound of the invention, and another therapeutic agent. In a particular embodiment, the other therapeutic agent is a cardiovascular disease treatment agent. In a particular embodiment, the cardiovascular disease is selected from arrhythmia (atrial or ventricular or both), atherosclerosis and its sequelae, angina, cardiac rhythm disturbances, myocardial ischemia, myocardial infarction, cardiac or vascular aneurysm, vasculitis, stroke, peripheral obstructive arteriopathy of a limb, an organ, or a tissue, reperfusion injury following ischemia of the brain, heart, kidney or other organ or tissue, endotoxic, surgical, or traumatic shock, hypertension, valvular heart disease, heart failure, abnormal blood pressure, shock, vasoconstriction (including that associated with migraines), vascular abnormality, inflammation, insufficiency limited to a single organ or tissue.

In one embodiment, the present invention provides compounds of the invention, or pharmaceutical compositions comprising a compound of the invention, for use in medicine. In a particular embodiment, the present invention provides compounds of the invention or pharmaceutical compositions comprising a compound of the invention, for use in the prophylaxis and/or treatment of neurodegenerative diseases. In a particular embodiment, the neurodegenerative disease is selected from Alzheimer's disease and other dementias, brain cancer, degenerative nerve diseases, encephalitis, epilepsy, genetic brain disorders, head and brain malformations, hydrocephalus, stroke, Parkinson's disease, multiple sclerosis, amyotrophic lateral sclerosis (ALS or Lou Gehrig's Disease), Huntington's disease, and prion diseases.

In another embodiment, the present invention provides compounds of the invention, or pharmaceutical compositions comprising a compound of the invention for use in the manufacture of a medicament for use in the prophylaxis and/or treatment of neurodegenerative diseases. In a particular embodiment, the neurodegenerative disease is selected from Alzheimer's disease and other dementias, brain cancer, degenerative nerve diseases, encephalitis, epilepsy, genetic brain disorders, head and brain malformations, hydrocephalus, stroke, Parkinson's disease, multiple sclerosis, amyotrophic lateral sclerosis (ALS or Lou Gehrig's Disease), Huntington's disease, and prion diseases.

In additional method of treatment aspects, this invention provides methods of prophylaxis and/or treatment of a mammal afflicted with neurodegenerative diseases, which methods comprise the administration of an effective amount of a compound of the invention or one or more of the pharmaceutical compositions herein described for the treatment or prophylaxis of said condition. In a particular embodiment, the neurodegenerative disease is selected from Alzheimer's disease and other dementias, brain cancer, degenerative nerve diseases, encephalitis, epilepsy, genetic brain disorders, head and brain malformations, hydrocephalus, stroke, Parkinson's disease, multiple sclerosis, amyotrophic lateral sclerosis (ALS or Lou Gehrig's Disease), Huntington's disease, and prion diseases.

In one embodiment, the present invention provides pharmaceutical compositions comprising a compound of the invention, and another therapeutic agent. In a particular embodiment, the other therapeutic agent is a neurodegenerative diseases treatment agent. In a particular embodiment, the neurodegenerative disease is selected from Alzheimer's disease and other dementias, brain cancer, degenerative nerve diseases, encephalitis, epilepsy, genetic brain disorders, head and brain malformations, hydrocephalus, stroke, Parkinson's disease, multiple sclerosis, amyotrophic lateral sclerosis (ALS or Lou Gehrig's Disease), Huntington's disease, and prion diseases.

In one embodiment, the present invention provides compounds of the invention, or pharmaceutical compositions comprising a compound of the invention, for use in medicine. In a particular embodiment, the present invention provides compounds of the invention or pharmaceutical compositions comprising a compound of the invention, for use in the prophylaxis and/or treatment of dermatological disorders. In a particular embodiment, the dermatological disease is selected from atopic dermatitis, bullous disorders, collagenoses, psoriasis, psoriatic lesions, dermatitis, contact dermatitis, eczema, pruritus, urticaria, rosacea, scleroderma, wound healing, scarring, hypertrophic scarring, keloids, Kawasaki disease, or Sjögren-Larsson syndrome.

In another embodiment, the present invention provides compounds of the invention, or pharmaceutical compositions comprising a compound of the invention for use in the manufacture of a medicament for use in the prophylaxis and/or treatment of dermatological disorders. In a particular embodiment, the dermatological disease is selected from atopic dermatitis, bullous disorders, collagenoses, psoriasis, psoriatic lesions, dermatitis, contact dermatitis, eczema, pruritus, urticaria, rosacea, scleroderma, wound healing, scarring, hypertrophic scarring, keloids, Kawasaki disease, or Sjögren-Larsson syndrome.

In additional method of treatment aspects, this invention provides methods of prophylaxis and/or treatment of a mammal afflicted with dermatological disorders, which methods comprise the administration of an effective amount of a compound of the invention or one or more of the pharmaceutical compositions herein described for the treatment or prophylaxis of said condition. In a particular embodiment, the dermatological disease is selected from atopic dermatitis, bullous disorders, collagenoses, psoriasis, psoriatic lesions, dermatitis, contact dermatitis, eczema, pruritus, urticaria, rosacea, scleroderma, wound healing, scarring, hypertrophic scarring, keloids, Kawasaki disease, or Sjögren-Larsson syndrome.

In one embodiment, the present invention provides pharmaceutical compositions comprising a compound of the invention, and another therapeutic agent. In a particular embodiment, the other therapeutic agent is a dermatological disorders treatment agent. In a particular embodiment, the dermatological disease is selected from atopic dermatitis, bullous disorders, collagenoses, psoriasis, psoriatic lesions, dermatitis, contact dermatitis, eczema, pruritus, urticaria, rosacea, scleroderma, wound healing, scarring, hypertrophic scarring, keloids, Kawasaki disease, or Sjögren-Larsson syndrome.

In a particular embodiment, the present invention provides compounds of the invention or pharmaceutical compositions comprising a compound of the invention, for use in the prophylaxis and/or treatment of pain. In particular, the term refers to neuropathic pain.

In another embodiment, the present invention provides the use of compounds of the invention or pharmaceutical compositions comprising a compound of the invention in the manufacture of a medicament for the prophylaxis and/or treatment of pain. In particular, the term refers to neuropathic pain.

In additional method of treatment aspects, this invention provides methods of prophylaxis and/or treatment of a mammal afflicted with pain, which methods comprise the administration of an effective amount of a compound of the invention or one or more of the pharmaceutical compositions herein described for the treatment or prophylaxis of said condition. In particular, the term refers to neuropathic pain.

In one embodiment, the present invention provides pharmaceutical compositions comprising a compound of the invention, and another therapeutic agent. In a particular embodiment, the other therapeutic agent is a pain treatment agent. In particular, the term refers to neuropathic pain.

In one embodiment, the present invention provides compounds of the invention, or pharmaceutical compositions comprising a compound of the invention, for use in medicine. In a particular embodiment, the present invention provides compounds of the invention or pharmaceutical compositions comprising a compound of the invention, for use in the prophylaxis and/or treatment of abnormal angiogenesis associated diseases. In a particular embodiment, the abnormal angiogenesis associated disease is selected from atherosclerosis, hypertension, tumor growth, inflammation, rheumatoid arthritis, wet-form macular degeneration, choroidal neovascularization, retinal neovascularization, diabetic retinopathy, and glioblastoma multiforma.

In another embodiment, the present invention provides compounds of the invention, or pharmaceutical compositions comprising a compound of the invention for use in the manufacture of a medicament for use in the prophylaxis and/or treatment of abnormal angiogenesis associated diseases. In a particular embodiment, the abnormal angiogenesis associated disease is selected from atherosclerosis, hypertension, tumor growth, inflammation, rheumatoid arthritis, wet-form macular degeneration, choroidal neovascularization, retinal neovascularization, diabetic retinopathy, and glioblastoma multiforma.

In additional method of treatment aspects, this invention provides methods of prophylaxis and/or treatment of a mammal afflicted with abnormal angiogenesis associated diseases, which methods comprise the administration of an effective amount of a compound of the invention or one or more of the pharmaceutical compositions herein described for the treatment or prophylaxis of said condition. In a particular embodiment, the abnormal angiogenesis associated disease is selected from atherosclerosis, hypertension, tumor growth, inflammation, rheumatoid arthritis, wet-form macular degeneration, choroidal neovascularization, retinal neovascularization, diabetic retinopathy, and glioblastoma multiforma.

In one embodiment, the present invention provides pharmaceutical compositions comprising a compound of the invention, and another therapeutic agent. In a particular embodiment, the other therapeutic agent is an abnormal angiogenesis associated diseases treatment agent. In a particular embodiment, the abnormal angiogenesis associated disease is selected from atherosclerosis, hypertension, tumor growth, inflammation, rheumatoid arthritis, wet-form macular degeneration, choroidal neovascularization, retinal neovascularization, diabetic retinopathy, and glioblastoma multiforma.

For the prophylaxis and/or treatment of long-term conditions, such as degenerative conditions, the regimen for treatment usually stretches over many months or years so oral dosing is preferred for patient convenience and tolerance. With oral dosing, one to four (1-4) regular doses daily, especially one to three (1-3) regular doses daily, typically one to two (1-2) regular doses daily, and most typically one (1) regular dose daily are representative regimens. Alternatively for long lasting effect drugs, with oral dosing, once every other week, once weekly, and once a day are representative regimens. In particular, dosage regimen can be every 1-14 days, more particularly 1-10 days, even more particularly 1-7 days, and most particularly 1-3 days.

Using these dosing patterns, each dose provides from about 1 to about 1000 mg of a compound of the invention, with particular doses each providing from about 10 to about 500 mg and especially about 30 to about 250 mg.

Transdermal doses are generally selected to provide similar or lower blood levels than are achieved using injection doses. For example, injection dose levels range from about 0.1 mg/kg/h to at least 10 mg/kg/h, all for from about 1 to about 120 h and especially 24 to 96 h. A preloading bolus of from about 0.1 mg/kg to about 10 mg/kg or more may also be administered to achieve adequate steady state levels. The maximum total dose is not expected to exceed about 1 g/day for a 40 to 80 kg human patient.

When used to prevent the onset of a condition, a compound of the invention will be administered to a patient at risk for developing the condition, typically on the advice and under the supervision of a physician, at the dosage levels described above. Patients at risk for developing a particular condition generally include those that have a family history of the condition, or those who have been identified by genetic testing or screening to be particularly susceptible to developing the condition.

A compound of the invention can be administered as the sole active agent or it can be administered in combination with other therapeutic agents, including other compound of the inventions that demonstrate the same or a similar therapeutic activity and that are determined to be safe and efficacious for such combined administration. In a specific embodiment, co-administration of two (or more) agents allows for significantly lower doses of each to be used, thereby reducing the side effects seen.

In one embodiment, a compound of the invention or a pharmaceutical composition comprising a compound of the invention is administered as a medicament. In a specific embodiment, said pharmaceutical composition additionally comprises a further active ingredient.

In one embodiment, a compound of the invention is co-administered with one or more further therapeutic agents for the treatment and/or prophylaxis of a fibrotic disease. In a particular embodiment, a compound of the invention is co-administered with one or two further therapeutic agents for the treatment and/or prophylaxis of a fibrotic disease. In a more particular embodiment, a compound of the invention is co-administered with one further therapeutic agent for the treatment and/or prophylaxis of a fibrotic disease.

In one embodiment, the further therapeutic agent for the treatment and/or prophylaxis of a fibrotic disease include, but are not limited to 5-methyl-1-phenyl-2-(1H)-pyridone (Pirfenidone ®); Nintedanib (Ofev® or Vargatef®); STX-100 (ClinicalTrials.gov Identifier NCT01371305), FG-3019 (ClinicalTrials.gov Identifier NCT01890265), Lebrikizumab (CAS n# 953400-68-5); Tralokinumab (CAS n# 1044515-88-9), PRM-151 (ClinicalTrials.gov Identifier NCT02550873) and PBI-4050 (ClinicalTrials.gov Identifier NCT02538536). In another particular embodiment, the further therapeutic agent for the treatment and/or prophylaxis of a fibrotic disease is an autotaxin (or ectonucleotide pyrophosphatase/phosphodiesterase 2 or NPP2 or ENPP2) inhibitor, examples of which are described in WO 2014/139882. In another particular embodiment, the further therapeutic agent for the treatment and/or prophylaxis of a fibrotic disease is GLPG1690.

In one embodiment, a compound of the invention is co-administered with another therapeutic agent for the treatment and/or prophylaxis of a disease involving inflammation, particular agents include, but are not limited to, immunoregulatory agents *e.g.* azathioprine, corticosteroids (*e.g.* prednisolone or dexamethasone), cyclophosphamide, cyclosporin A, tacrolimus, mycophenolate, mofetil, muromonab-CD3 (OKT3, *e.g*. Orthocolone®), ATG, aspirin, acetaminophen, ibuprofen, naproxen, and piroxicam.

In one embodiment, a compound of the invention is co-administered with another therapeutic agent for the treatment and/or prophylaxis of arthritis (*e.g.* rheumatoid arthritis), particular agents include but are not limited to analgesics, non-steroidal anti-inflammatory drugs (NSAIDS), steroids, synthetic DMARDS (for example but without limitation methotrexate, leflunomide, sulfasalazine, auranofin, sodium aurothiomalate, penicillamine, chloroquine, hydroxychloroquine, azathioprine, tofacitinib, baricitinib, fostamatinib, and cyclosporin), and biological DMARDS (for example but without limitation infliximab, etanercept, adalimumab, rituximab, and abatacept).

In one embodiment, a compound of the invention is co-administered with another therapeutic agent for the treatment and/or prophylaxis of proliferative disorders, particular agents include but are not limited to: methotrexate, leukovorin, adriamycin, prednisone, bleomycin, cyclophosphamide, 5-fluorouracil, paclitaxel, docetaxel, vincristine, vinblastine, vinorelbine, doxorubicin, tamoxifen, toremifene, megestrol acetate, anastrozole, goserelin, anti-HER2 monoclonal antibody (*e*.*g.* Herceptin™), capecitabine, raloxifene hydrochloride, EGFR inhibitors (*e.g.* lressa®, Tarceva™, Erbitux™), VEGF inhibitors (*e.g.* Avastin™), proteasome inhibitors (*e.g.* Velcade™), Glivec® and hsp90 inhibitors (*e.g.* 17-AAG). Additionally, the compound of the invention according to Formula I may be administered in combination with other therapies including, but not limited to, radiotherapy or surgery. In a specific embodiment the proliferative disorder is selected from cancer, myeloproliferative disease or leukaemia.

In one embodiment, a compound of the invention is co-administered with another therapeutic agent for the treatment and/or prophylaxis of autoimmune diseases, particular agents include but are not limited to: glucocorticoids, cytostatic agents (*e.g*. purine analogs), alkylating agents, (e.g nitrogen mustards (cyclophosphamide), nitrosoureas, platinum compound of the inventions, and others), antimetabolites (*e.g*. methotrexate, azathioprine and mercaptopurine), cytotoxic antibiotics (*e.g.* dactinomycin anthracyclines, mitomycin C, bleomycin, and mithramycin), antibodies (*e.g.* anti-CD20, anti-CD25 or anti-CD3 (OTK3) monoclonal antibodies, Atgam® and Thymoglobuline®), cyclosporin, tacrolimus, rapamycin (sirolimus), interferons (*e.g.* IFN-β), TNF binding proteins (*e.g.* infliximab, etanercept, or adalimumab), mycophenolate, fingolimod and myriocin..

In one embodiment, a compound of the invention is co-administered with another therapeutic agent for the treatment and/or prophylaxis of transplant rejection, particular agents include but are not limited to: calcineurin inhibitors (*e.g.* cyclosporin or tacrolimus (FK506)), mTOR inhibitors (*e.g.* sirolimus, everolimus), anti-proliferatives (*e.g.* azathioprine, mycophenolic acid), corticosteroids (*e.g.* prednisolone, hydrocortisone), antibodies (*e.g*. monoclonal anti-IL-2Rα, receptor antibodies, basiliximab, daclizumab), polyclonal anti-T-cell antibodies (*e.g*. anti-thymocyte globulin (ATG), anti-lymphocyte globulin (ALG)).

In one embodiment, a compound of the invention is co-administered with another therapeutic agent for the treatment and/or prophylaxis of asthma and/or rhinitis and/or COPD, particular agents include but are not limited to: beta2-adrenoceptor agonists (*e.g*. salbutamol, levalbuterol, terbutaline and bitolterol), epinephrine (inhaled or tablets), anticholinergics (*e.g*. ipratropium bromide), glucocorticoids (oral or inhaled). Long-acting β2-agonists (*e.g.* salmeterol, formoterol, bambuterol, and sustained-release oral albuterol), combinations of inhaled steroids and long-acting bronchodilators (*e.g*. fluticasone/salmeterol, budesonide/formoterol), leukotriene antagonists and synthesis inhibitors (*e.g*. montelukast, zafirlukast and zileuton), inhibitors of mediator release (*e.g*. cromoglycate and ketotifen), biological regulators of IgE response (*e.g.* omalizumab), antihistamines (*e.g.* ceterizine, cinnarizine, fexofenadine) and vasoconstrictors (*e.g.* oxymethazoline, xylomethazoline, nafazoline and tramazoline).

Additionally, a compound of the invention may be administered in combination with emergency therapies for asthma and/or COPD, such therapies include oxygen or heliox administration, nebulized salbutamol or terbutaline (optionally combined with an anticholinergic (*e.g*. ipratropium), systemic steroids (oral or intravenous, *e.g.* prednisone, prednisolone, methylprednisolone, dexamethasone, or hydrocortisone), intravenous salbutamol, non-specific beta-agonists, injected or inhaled (*e.g*. epinephrine, isoetharine, isoproterenol, metaproterenol), anticholinergics (IV or nebulized, *e.g.* glycopyrrolate, atropine, ipratropium), methylxanthines (theophylline, aminophylline, bamiphylline), inhalation anesthetics that have a bronchodilatory effect (*e.g.* isoflurane, halothane, enflurane), ketamine and intravenous magnesium sulfate.

In one embodiment, a compound of the invention is co-administered with another therapeutic agent for the treatment and/or prophylaxis of inflammatory bowel disease (IBD), particular agents include but are not limited to: glucocorticoids (*e.g.* prednisone, budesonide) synthetic disease modifying, immunomodulatory agents (*e.g.* methotrexate, leflunomide, sulfasalazine, mesalazine, azathioprine, 6-mercaptopurine and cyclosporin) and biological disease modifying, immunomodulatory agents (infliximab, adalimumab, rituximab, and abatacept).

In one embodiment, a compound of the invention is co-administered with another therapeutic agent for the treatment and/or prophylaxis of SLE, particular agents include but are not limited to: human monoclonal antibodies (belimumab (Benlysta)), Disease-modifying antirheumatic drugs (DMARDs) such as antimalarials (*e.g.* plaquenil, hydroxychloroquine), immunosuppressants (*e.g.* methotrexate and azathioprine), cyclophosphamide and mycophenolic acid, immunosuppressive drugs and analgesics, such as nonsteroidal anti-inflammatory drugs, opiates (*e.g.* dextropropoxyphene and co-codamol), opioids (*e.g.* hydrocodone, oxycodone, MS Contin, or methadone) and the fentanyl duragesic transdermal patch.

In one embodiment, a compound of the invention is co-administered with another therapeutic agent for the treatment and/or prophylaxis of psoriasis, particular agents include but are not limited to: topical treatments such as bath solutions, moisturizers, medicated creams and ointments containing coal tar, dithranol (anthralin), corticosteroids like desoximetasone (Topicort™), fluocinonide, vitamin D3 analogues (for example, calcipotriol), argan oil and retinoids (etretinate, acitretin, tazarotene), systemic treatments such as methotrexate, cyclosporine, retinoids, tioguanine, hydroxyurea, sulfasalazine, mycophenolate mofetil, azathioprine, tacrolimus, fumaric acid esters or biologics such as Amevive™, Enbrel™, Humira™, Remicade™, Raptiva™ and ustekinumab (a IL-12 and IL-23 blocker). Additionally, a compound of the invention may be administered in combination with other therapies including, but not limited to phototherapy, or photochemotherapy (*e.g.* psoralen and ultraviolet A phototherapy (PUVA)).

In one embodiment, a compound of the invention is co-administered with another therapeutic agent for the treatment and/or prophylaxis of allergic reaction, particular agents include but are not limited to: antihistamines (*e.g.* cetirizine, diphenhydramine, fexofenadine, levocetirizine), glucocorticoids (*e.g.* prednisone, betamethasone, beclomethasone, dexamethasone), epinephrine, theophylline or anti-leukotrienes (*e.g.* montelukast or zafirlukast), anti-cholinergics and decongestants.

By co-administration is included any means of delivering two or more therapeutic agents to the patient as part of the same treatment regime, as will be apparent to the skilled person. Whilst the two or more agents may be administered simultaneously in a single formulation, *i.e.* as a single pharmaceutical composition, this is not essential. The agents may be administered in different formulations and at different times.

### CHEMICAL SYNTHETIC PROCEDURES

### General

The compound of the invention can be prepared from readily available starting materials using the following general methods and procedures. It will be appreciated that where typical or preferred process conditions (*i.e.* reaction temperatures, times, mole ratios of reactants, solvents, pressures, etc.) are given, other process conditions can also be used unless otherwise stated. Optimum reaction conditions may vary with the particular reactants or solvent used, but such conditions can be determined by one skilled in the art by routine optimization procedures.

Additionally, as will be apparent to those skilled in the art, conventional protecting groups may be necessary to prevent certain functional groups from undergoing undesired reactions. The choice of a suitable protecting group for a particular functional group as well as suitable conditions for protection and deprotection are well known in the art (Wuts & Greene 2014).

The following methods are presented with details as to the preparation of a compound of the invention as defined hereinabove and the comparative examples. A compound of the invention may be prepared from known or commercially available starting materials and reagents by one skilled in the art of organic synthesis.

All reagents are of commercial grade and are used as received without further purification, unless otherwise stated. Commercially available anhydrous solvents are used for reactions conducted under inert atmosphere. Reagent grade solvents are used in all other cases, unless otherwise specified. Column chromatography is performed on silica gel 60 (35-70 µm). Thin layer chromatography is carried out using pre-coated silica gel F-254 plates (thickness 0.25 mm). Chemical shifts (δ) for ¹H NMR spectra are reported in parts per million (ppm) relative to tetramethylsilane (δ 0.00) or the appropriate residual solvent peak, i.e. CHCl₃ (δ 7.27), as internal reference. Multiplicities are given as singlet (s), doublet (d), triplet (t), quartet (q), quintet (quin), multiplet (m) and broad (br). Electrospray MS spectra are obtained on a Waters platform LC/MS spectrometer or with a Waters Acquity UPLC with Waters Acquity PDA detector and SQD mass spectrometer. Columns used: UPLC BEH C18 1.7 µm, 2.1 × 5 mm VanGuard pre-column with Acquity UPLC BEH C18 1.7 µm, 2.1 × 30 mm column or Acquity UPLC BEH C18 1.7 µm, 2.1 × 50 mm column. All the methods are using MeCN/H₂O gradients. MeCN and H₂O contain either 0.1% formic acid or 0.05% NH₃. Preparative LCMS: columns used, Waters XBridge Prep C18 5 µm ODB 30 × 100 mm (preparative column) and Waters XBridge C18 5 µm, 4.6 mm × 100 mm (analytical column). All the methods are using MeCN/H₂O gradients. MeCN and H₂O contain either 0.1% formic acid or 0.1% diethylamine. Microwave heating is performed with a Biotage Initiator.

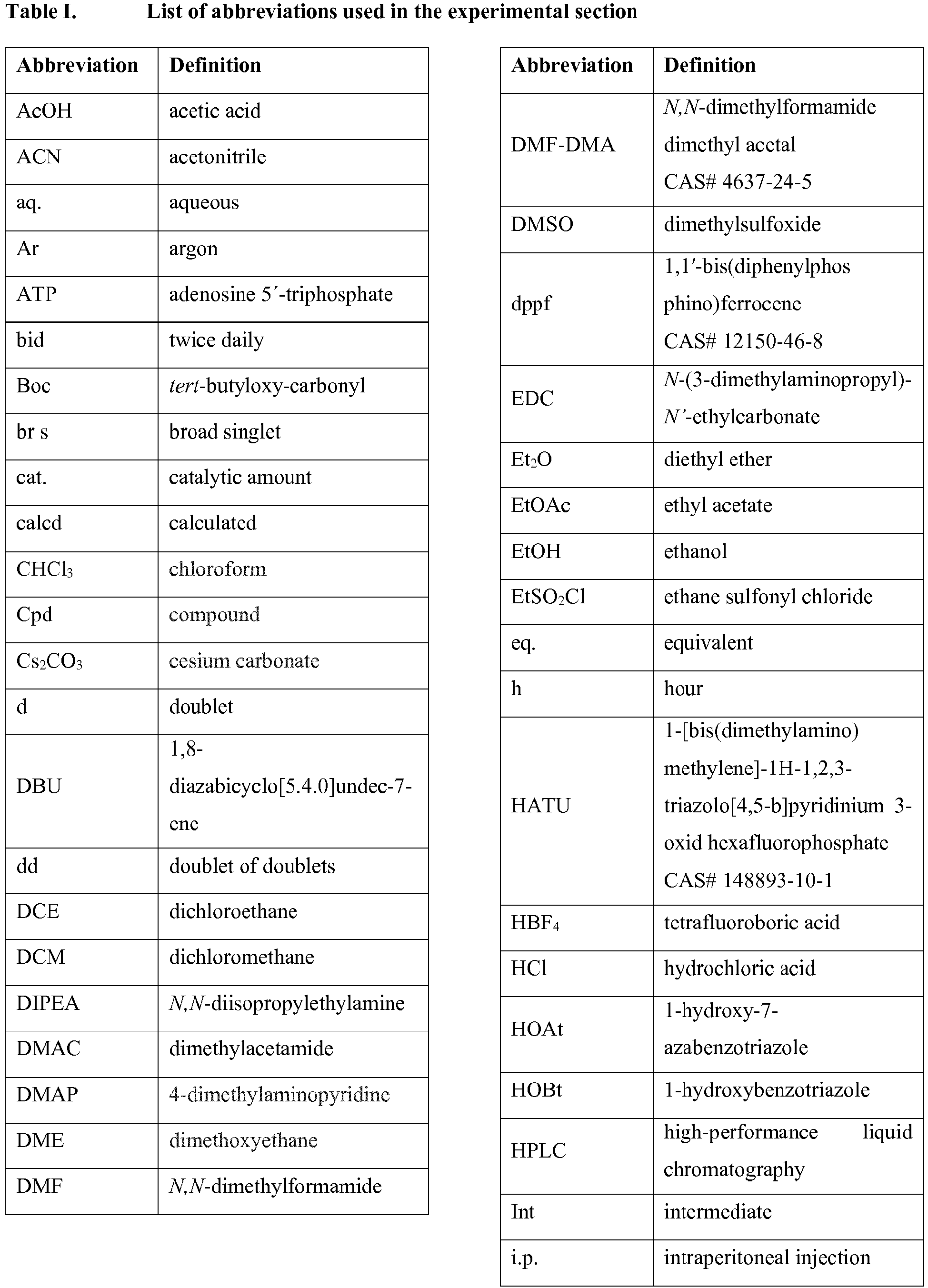

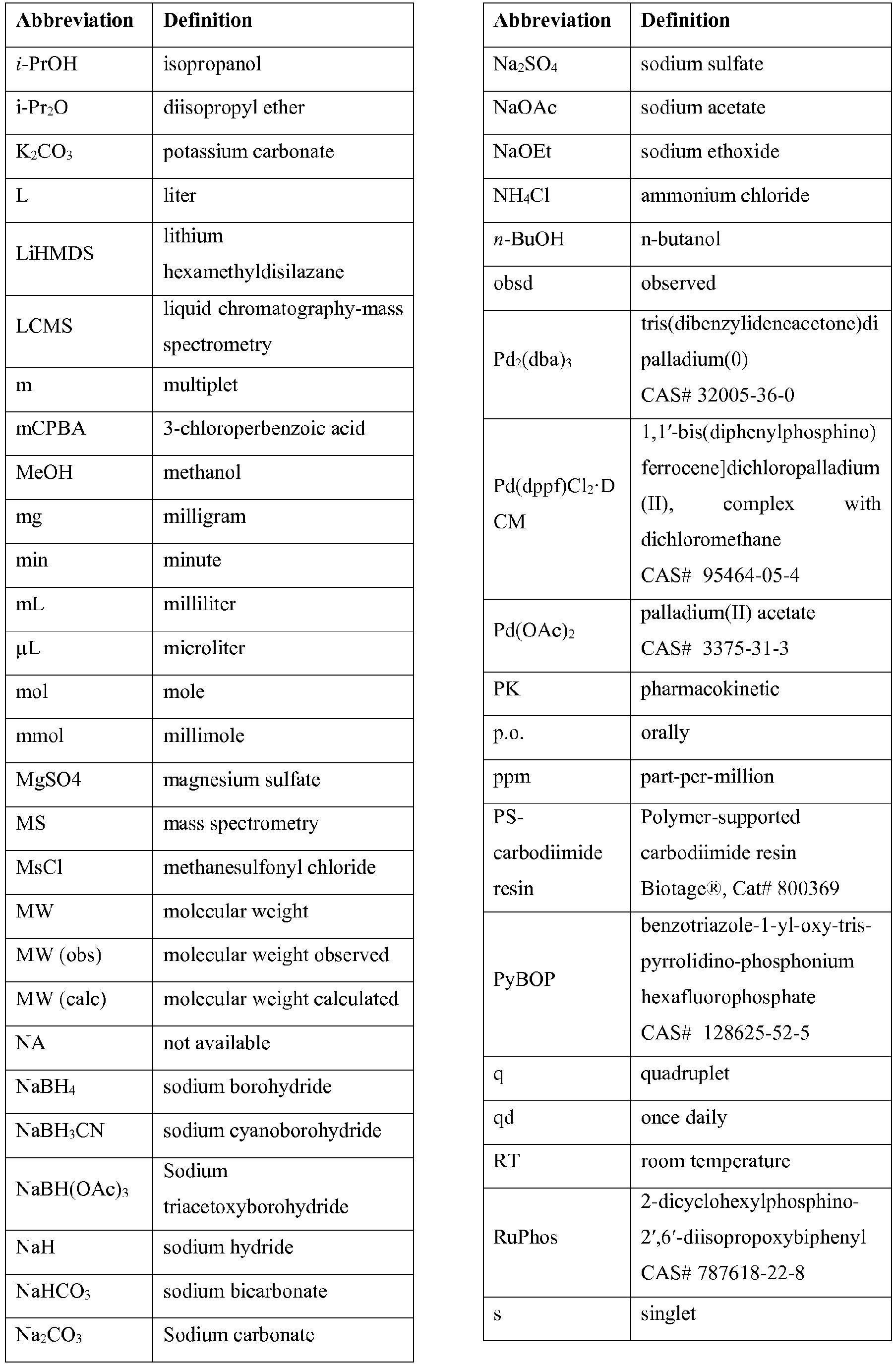

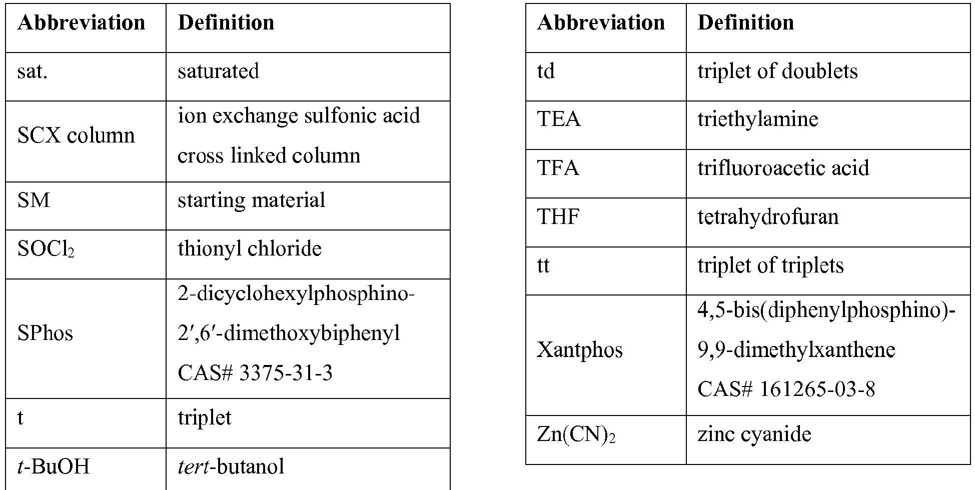

### SYNTHETIC PREPARATION OF THE COMPOUNDS OF THE INVENTION

### Example 1. General synthetic methods

### 1.1. Method A

### 1.1.1. General method A: amide coupling

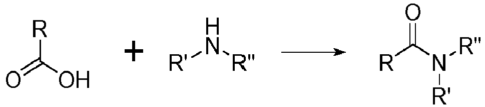

The acid (1 eq.), a base, typically tetramethyl piperidine (3 eq. in the case of salt free amines, up to 5.0 eq. in the case of HCl or TFA salt form amines), DIPEA (up to 2.5 eq. in the case of salt free amines, up to 5.0 eq. in the case of HCl or TFA salt form amines) or TEA (up to 3.0 eq. in the case of salt free amines, up to 10 eq. in the case of HCl or TFA salt form amines) and a coupling agent, typically HATU (1 to 1.5 eq.) or EDC.HCl/HOBt (1.5 eq. and 0.15 to 1.0 eq.) or PyBOP (1.1 eq.) or PS-carbodiimide resin (Biotage, LLC, Cat# 800369; 1.24 to 1.60 mmol.g-1, 1.1 to 1.5 eq.) and HOBt hydrate or HOAT hydrate (0.7 to 1.3 eq.) are mixed in an organic solvent, typically DMF, THF, DCM or a mixture of those at a temperature between 0°C and RT. The amine as a free base or as its salt, HCl or TFA for example, (0.8 to 1.5 eq) is added and the mixture is stirred at RT for 0.5 to 120 h or heated under microwave irradiation between 70 and 75°C for 10 min to 1.25 h.

If appropriate, the reaction medium is filtered and is then concentrated to dryness or not. The residue is then diluted with an organic solvent and the resulting mixture undergoes an aqueous work-up. The organic phase is concentrated to afford the desired product, which may be further purified by flash column chromatography and/or preparative HPLC. If needed, the obtained compound is triturated in a DCM/cyclohexane mixture.

### Alternative work-up 1:

The reaction mixture is diluted with water and extracted with an organic solvent (typically DCM, EtOAc). The combined organic layers are passed through a phase separator, concentrated and the residue is purified by flash column chromatography or recrystallized in ACN.

Alternative work-up 3: The reaction mixture is diluted with water and extracted with DCM. The combined organic layers are separated, dried over Na₂SO₄. The volatiles are removed under reduced pressure. The residue is triturated in acetone. The product is collected by filtration and dried under reduced pressure.

### Alternative work-up 2:

The reaction medium is diluted with water and/or a sat. NaHCO₃ aq. solution. The precipitate is filtered and purified by flash column chromatography.

Alternative work-up 5: The reaction medium is filtered on a phase separator that is washed with DCM/MeOH. The filtrate is diluted with a sat. NaHCO₃ aq. solution and extracted with an organic solvent. The combined organic phases are concentrated and purified by flash column chromatography.

### Alternative work-up 3:

a NaOH aq. solution (0.5 to 2N) and EtOAc are added to the reaction medium. A small amount of solid NaCl is added to the aqueous phase that is then concentrated to dryness. The residue is taken up in DCM and washed with water. The aqueous phase is then extracted with EtOAc/n-BuOH or not. The combined organic phases are washed with brine, dried over Na₂SO₄, filtered and concentrated.

### 1.1.2. Illustrative example of method A: Synthesis of 6-[3-[2-(3,S-dimethylanilino)-7,8-dihydro-5H-pyrido[4,3-d]pyrimidin-6-yl]azetidine-1-carbonyl]-3H-1,3-benzoxazol-2-one (Cpd 1)

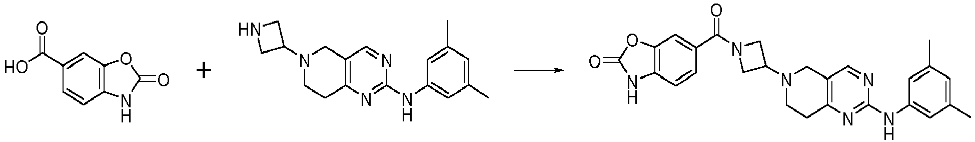

2-oxo-3H-1,3-benzoxazole-6-carboxylic acid (CAS# 54903-16-1; 20 mg, 0.11 mmol, 1.0 eq.) was added to a suspension of PS-carbodiimide resin (107.0 mg, 1.33 mmol/g, 1.3 eq.) and HOBT hydrate (CAS# 123333-53-9; 12.0 mg, 0.076 mmol, 0.7 eq.) in a 10/1 mixture of anhydrous DMF/DCM (3.3 mL). The mixture was stirred at RT for 5 min. 6-(azetidin-3-yl)-N-(3,5-dimethylphenyl)-7,8-dihydro-5H-pyrido[4,3-d]pyrimidin-2-amine (Int 1, 35.0 mg, 0.11 mmol, 1.0 eq.) was added to the suspension and then heated at 75°C under microwave irradiation for 20 min. The mixture was cooled to RT. 5 mL of MeOH were added to the suspension. After filtration through a pad of cotton rinsed with MeOH (10 mL), the filtrate was concentrated under reduced pressure. The crude product was purified by flash liquid chromatography on silica gel (eluted with heptane/EtOAc) to afford Cpd 1.

### 1.2. Method B

### 1.2.1. General method B: Boc deprotection on protected nitrogen

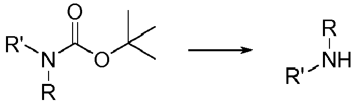

The Boc-protected amine (1.0 eq.) is stirred a RT in an acidic medium (typically 4:1 DCM/TFA, or 1:1 ACN/4 M HCl in 1,4-dioxane, or 15 to 35 eq. HCl (4M in 1,4-dioxane) in 1,4-dioxane, or 10:1 MeOH/acetyl chloride) for 1 to 16 h. The reaction mixture is concentrated to afford the desired compound, or undergoes one or multiple purification steps, such as aq. work-up, flash column chromatography, SCX resin exchange or preparative HPLC to afford the desired product.

### 1.2.2. Illustrative example of method B: synthesis of N-(3,5-dimethylphenyl)-5,6,7,8-tetrahydropyrido[4,3-d]pyrimidin-2-amine (Int 2001)

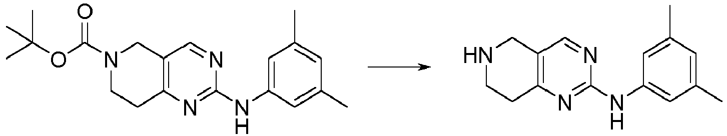

To a solution of Int 3001 (3.7 g, 9.60 mmol, 1.0 eq.) in 170 mL of anhydrous DCM at RT was added TFA (14.7 mL, 192 mmol, 20.0 eq.). The reaction was stirred at RT for 5 h. 10% K₂CO₃ aq. solution (400 mL) was then added at 0°C. After extraction with DCM, the combined organic layers were dried over MgSO₄, filtered and concentrated under reduced pressure to afford the title compound Int 2001.

### 1.2.3. Illustrative example of method B: Synthesis of N-(3,5-dimethylphenyl)-6,7-dihydro-5H-pyrrolo[3,4-d]pyrimidin-2-amine, Int 2011

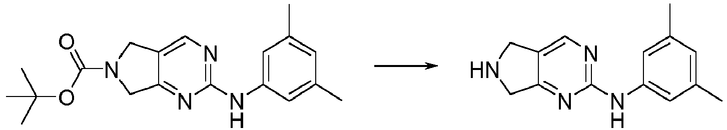

Int 3011 (39.8 g, 1151.7 mmol, 1.0 eq.) is suspended in a solution of 4N HCl in 1,4-dioxane (400 mL). The mixture is stirred at RT for 3 h and is then concentrated to dryness. The resulting solid is dissolved in water, DCM and NaOH 2N are added. The layers are separated and the aqueous layer is back-extracted twice with DCM. The organic layers are evaporated to dryness to obtain Int 2011.

### 1.2.4. Illustrative example of method B: Synthesis of N-(3,5-dimethylphenyl)-6-[(2R,4R)-2-methyl-4-piperidyl]-5,7-dihydropyrrolo[3,4-d]pyrimidin-2-amine Int 39

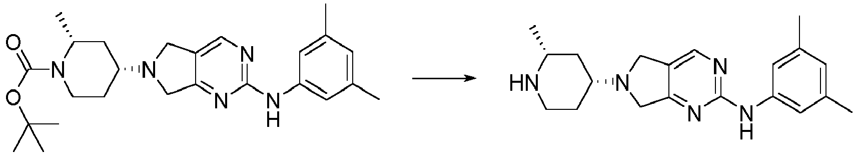

Int 1039 (15.4 g, 35.2 mmol, 1.0 eq.) was suspended in HCl (4N in 1,4-dioxane, 250 mL) and the mixture was stirred at RT for 2 h. The medium was concentrated to dryness and the residue was taken up DCM. Water was added and the pH of the aqueous phase was adjusted to 10 using a 40% NaOH aq. solution. The layers were separated and the aqueous layer was extracted with DCM. The combined organic phases were concentrated to afford Int 39.

### 1.3. Method C

### 1.3.1. General method C: Reductive amination of ketones

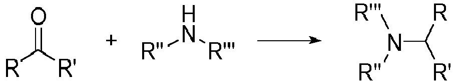

The amine derivative (1.0 eq.) and the ketone (0.66 to 1.5 eq.) are placed in an appropriate solvent (MeOH, DCE, DCM, DME) potentially with addition of 4 Å molecular sieves as desiccant. In some cases AcOH is also added (0.1 to 4.0 eq.). Immediately or after up to 2 h stirring the reducing agent (NaBH₄, NaBH(OAc)₃, NaBH₃CN, up to 10.0 eq.) is added and the mixture is stirred at RT for 1 h to 72 h. In some cases more reducing agent is added during stirring (up to 3.0 eq.).

The reaction medium is diluted with an organic solvent and undergoes aqueous work-up, typically with water, a sat. NaHCO₃ aq. solution or a NaOH solution (2N). The combined organic layers are filtered through a phase separator and the filtrate is concentrated, or are dried over a desiccant, filtered and evaporated. The residue is used as such, or is triturated in EtOAc, or is purified by column chromatography on silica gel, or is purified by preparative HPLC to afford the desired compound.

### Alternative work-up 1:

The reaction medium is diluted or not with MeOH and then is poured into a sat. NaOAc solution before being extracted with DCM or EtOAc. The organic phase is concentrated and the residue is purified by column chromatography on silica gel or by preparative HPLC or a combination of both techniques to afford the desired compound.

### Alternative work-up 2 :

The reaction mixture is quenched or not with water, the solvents are concentrated to dryness. The residue is purified by column chromatography on silica gel or by preparative HPLC or a combination of both techniques to afford the desired compound that is triturated or not in a DCM/heptane mixture.

### Alternative work-up 3:

The reaction medium is directly loaded for purification by flash chromatography on silica gel and the fractions containing the product are concentrated. The residue is taken up in acetone and filtered, or taken up in acetone, filtered and purified by preparative HPLC, to afford the desired compound. Illustrative example of method C

### 1.3.2. Synthesis of tert-butyl (2R,4R)-4-[2-(3,5-dimethylanilino)-5,7-dihydropyrrolo[3,4-d]pyrimidin-6-yl]-2-methyl-piperidine-1-carboxylate, Int 1039

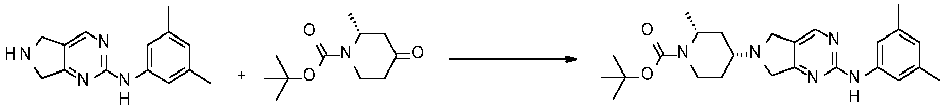

Int 2011 (27 g, 112.4 mmol, 1.0 eq.) and tert-butyl (2R)-2-methyl-4-oxo-piperidine-1-carboxylate (CAS# 790667-43-5; 25.2 g, 118.2 mmol, 1.05 eq.) were stirred in DCM (350 mL) and AcOH (0.7 ml) for 30 min under inert atmosphere. NaBH(OAc)₃ (35.6 g, 168.0 mmol, 1.5 eq.) was added and the mixture was stirred at RT for 1 h. The mixture was poured in a NaOAc solution, the aqueous phase was extracted with DCM, dried over Na₂SO₄ and concentrated to dryness. The crude product was purified by flash column chromatography on silica gel (eluted with heptane/EtOAc) to afford the title product Int 1039.

### 1.4. General method D

### 1.4.1. General method D: Buchwald coupling with aryl halides

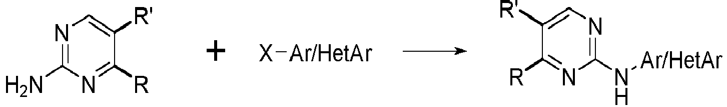

A pressure reactor or an open round bottom flask equipped with a condenser is charged with heteroarylbromide or iodide derivative (0.8 to 2.0 eq.), aminopyrimidine derivative (1.0 eq.), a base (sodium tert-butoxide, Cs₂CO₃, K₂CO₃, 1.3 to 3.0 eq.) and an appropriate solvent (1,4-dioxane, toluene). The mixture is degassed with N₂ or Ar at RT in the presence of a ligand if required (Xantphos, PPh₃, SPhos, 0.05 to 0.2 eq.) before the addition of Pd catalyst (Pd₂(dba)₃ or Pd(dppf)Cl_{2·}DCM adduct; or Pd(OAc)₂, 0.07 to 0.2 eq.). The mixture is stirred at reflux or heated at 100-115°C for 5 min to 18 h.

The reaction mixture is concentrated in vacuo or not, and diluted in EtOAc or DCM and water or a sat. NaHCO₃ solution or brine. The resulting mixture is filtered over Celite® or not. The mixture is then extracted with EtOAc or DCM. The combined organic layers are optionally washed with brine, dried over anhydrous Na₂SO₄ or MgSO₄, filtered, or passed through a phase separator. The filtrate is concentrated in vacuo. The residue is either purified by flash chromatography on silica gel followed or not by trituration in MeOH, or triturated in EtOAc to afford the expected compound.

### Alternative work-up 1:

The reaction mixture is mixed or not with Dicalite®, filtered on Celite®, washed with EtOAc and concentrated in vacuo. The residue is used as such, or directly purified by flash chromatography on silica gel, or triturated in cold Et₂O to afford the expected product.

### Alternative work-up 2 :

The reaction mixture is filtered on Clarcel® and the Clarcel® pad is washed with DCM. The filtrate is concentrated and optionally taken up in DCM and filtered. The residue is purified by flash chromatography on silica gel and triturated in Et₂O or iPr₂O to afford the expected product.

Alternative work-up 3: the reaction mixture is filtered on Dicalite® or on a Whatman® filter. The filtrate is diluted with water or brine and is extracted with EtOAc. The combined organic layers are dried over anhydrous Na₂SO₄ or MgSO₄, filtered and concentrated to be either used as such or purified by flash chromatography on silica gel.

### 1.4.2. Illustrative example of method D: Synthesis of tert-butyl 2-(3,5-dimethylanilino)-7,8-dihydro-5H-pyrido[4,3-d]pyrimidine-6-carboxylate (Int 3001)

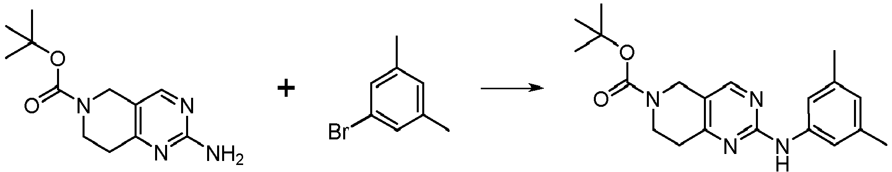

The mixture of Int 4001 (251 g, 1 mol, 1eq.), 1-bromo-3,5-dimethyl-benzene (CAS# 556-96-7; 143.1 mL, 1.05 mol, 1.05 eq) in dry 1,4-dioxane (3500 mL) was purged with N₂ before XantPhos (100 g, 210.8 mmol, 0.2 eq), Pd₂(dba)₃ (91.9 g, 0.1 mol, 0.1 eq), and sodium tert-butoxide (289.2 g, 3.12 mol, 3.0 eq) were added. The reaction mixture was then stirred at reflux for 3 h. The reaction mixture was cooled to RT and diluted with water and EtOAc. The aqueous layer was then extracted several times with EtOAc. The combined organic layers were washed with brine, filtered through Celite® and concentrated under reduced pressure to afford a crude product that was purified by chromatography on silica gel (eluting with heptane/EtOAc). The fractions of interest were pooled and concentrated under reduced pressure to afford Int 3001.

### 1.5. Method E

### 1.5.1. General method E: Cyclization of dimethylaminomethylidene into aminopyrimidine

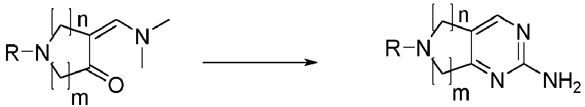

A pressure reactor or an open round bottom flask equipped with a condenser is charged with an cyclic oxodimethylaminomethylidine derivative (1.0 eq.), a guanidine derivative (guanidine free base, guanidine.HCl or guanidine carbonate, for example), a base (NaOAc, K₂CO₃, 1.0 to 10.0 eq.) and an appropriate solvent (typically EtOH). The mixture is stirred at reflux or heated at 100°C for 5 min to 18 h. The reaction mixture is concentrated in vacuo. The residue is diluted in EtOAc or CHCl₃ and water or brine. The mixture is then extracted with EtOAc, a EtOAc/i-PrOH mixture or CHCl₃. The combined organic layers are optionally washed with brine, dried over anhydrous Na₂SO₄ or MgSO₄, filtered and concentrated in vacuo. The residue is used as such or triturated in and organic solvent (typically Et₂O or MeOH) to afford the expected compound.

Alternative work-up 1: the mixture is cooled to RT and filtered. The filtrate is concentrated and the residue undergoes an aqueous work-up with water and DCM. The combined organic phases are evaporated to afford the expected product.

### 1.5.2. Illustrative example of method E: Synthesis of tert-butyl 2-amino-7,8-dihydro-5H-pyrido[4,3-d]pyrimidine-6-carboxylate, Int 4001

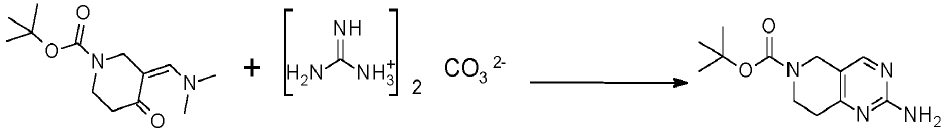

To a solution of Int 5001 (255 g, 1004 mmol, 1.0 eq.) in EtOH (5.5 L) at RT were added guanidine carbonate (795 g, 4016 mmol, 4.4 eq.) and NaOAc (741 g, 9036 mmol, 9.0 eq.) under N₂. The reaction mixture was heated to reflux for 16 h, then cooled down to RT and filtered. The precipitate was discarded and the filtrate evaporated under reduced pressure. DCM (1.0 L) and water (1.0 L) were added. The aqueous layer was back extracted with DCM (2 × 1.0 L). The organic layers were combined and the solvent evaporated to dryness giving Int 4001.

### 1.6. Method F

### 1.6.1. General method F: dimethylaminomethylidene on cyclic ketones

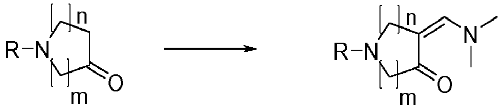

A pressure reactor or an open round bottom flask equipped with a condenser is charged with the ketone derivative (1.0 eq.), dissolved or not in toluene, and DMF-DMA (3.0 to 10.0 eq.). The mixture is heated at the appropriate temperature (60-120 °C) for complete conversion in 0.5-18 h. The mixture is concentrated under reduced pressure. The residue is used as such, or purified by column chromatography on silica gel, or suspended in n-hexane, stirred at RT for 2 to 15 h, filtered and dried to afford the expected product.

Alternative work-up 2: The reaction mixture is concentrated until precipitation occurs. The precipitate is filtered, washed with n-hexane and dried to afford the expected product.

### 1.6.2. Illustrative examples of method F: Synthesis of tert-butyl (3Z)-3-(dimethylaminomethylene)-4-oxo-piperidine-1-carboxylate Int 5001

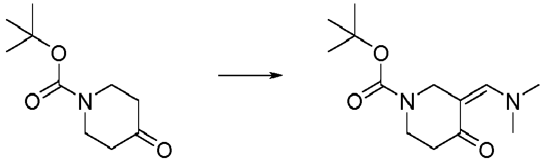

To a solution of t-butyl-4-oxopiperidine carboxylate (CAS# 79099-07-3; 200 g, 1003 mmol, 1.0 eq.) in toluene (800 mL), DMF-DMA (772 mL, 5015 mmol, 5.0 eq.) was added under N₂ and then heated to 110 °C. The reaction mixture was stirred overnight at 110 °C. The solvent was then removed under reduced pressure. The resulting Int 5001 was used in the next step without further purification.

### 1.6.3. Illustrative examples of method F: Synthesis of tert-butyl (3Z)-3-(dimethylaminomethylene)-4-oxo-pyrrolidine-1-carboxylate Int 5011

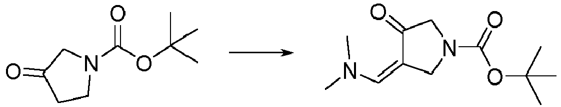

A solution of tert-butyl 3-oxopyrrolidine-1-carboxylate (1 kg, 2.7 mol, 1.0 eq.) in DMF-DMA (5 L, 18.9 mol, 7.0 eq.) was stirred at reflux for 2 h. Then the volatiles were concentrated until precipitation occurs. The precipitate if filtered, washed with n-hexanes (1 L) and dried in a vacuum oven to afford Int 5011.

### 1.7. Method G

### 1.7.1. General Method G: saponification of methyl and ethyl esters

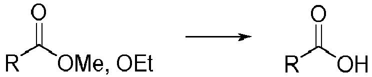

To the ester (1.0 eq.) in an organic solvent (typically MeOH or EtOH) is added NaOH (1N or 2N aq. solution, 2.0 to 30.0 eq.) and the mixture is stirred at the appropriate temperature (RT to 60 °C) for 30 min to 72 h. Additional NaOH (1N or 2N aq. solution, up to 10.0 eq.) can be added during stirring. Then the organic solvent is evaporated or not, and the medium is washed or not with DCM or EtOAc. The aqueous phase is acidified to pH 2 to 6 with HCl (1N or 2N aq. solution) and is extracted with EtOAc or EtOAc/i-PrOH. The combined organic phases are concentrated to dryness to afford the expected product. If at any stage of the work-up a solid precipitates, it is filtered and dried to afford the expected product.

### Alternative work-up 2 :

To the reaction mixture is added activated Dowex® acidic resin (CAS# 69011-20-7) to reach pH 4 to 5, the mixture is stirred at RT before being filtered. The filtrate is concentrated to afford the expected product.

### Alternative work-up 3:

HCl (1N or 2N aq. solution) is added to the reaction mixture and the solvents are evaporated. The residue is purified by preparative HPLC to afford the expected product.

### 1.7.2. Illustrative example of method G: Cpd 310

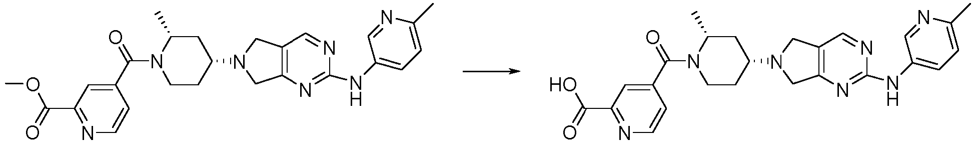

To a solution of Cpd 308 (130 mg, 0.27 mmol, 1.0 eq.) in MeOH (5 mL) was added NaOH (2N in water, 0.5 mL, 1.0 mmol, 3.7 eq.) and the mixture was stirred at RT for 2 h. Activated acidic Dowex® resin (CAS# 69011-20-7) was added to reach pH 5, the mixture was agitated and the medium filtered. The resin was washed with MeOH and the filtrate was concentrated to afford Cpd 310.

### 1.8. Int 53

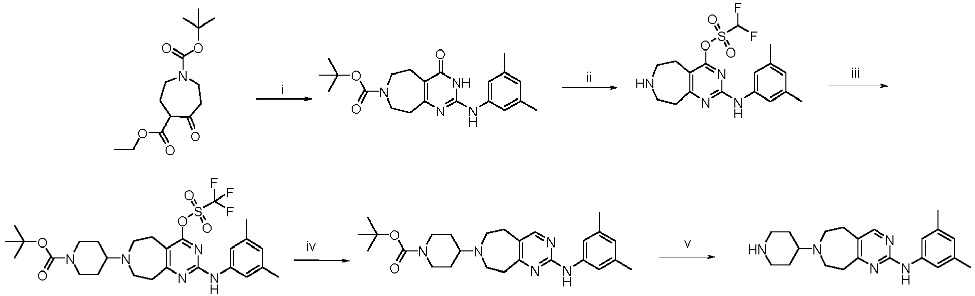

### 1.8.1. Step i

To a solution of NaOEt (21% in EtOH, 2.36 mL, 12.2 mmol, 3.0 eq.) in EtOH (12 mL) were added 1-Tert-butyl 4-ethyl 5-oxoazepane-1,4-dicarboxylate (CAS# 141642-82-2; 800 mg, 2.80 mmol, 1.0 eq.) and Int 141 (634 mg, 2.80 mmol, 1.0 eq.). The mixture was stirred at RT for 16 h and was then hydrolysed with water. The aqueous phase was extracted with EtOAc and the organic layer was dried over Na₂SO₄, filtered and concentrated to dryness. The residue was purified by flash chromatography on silica gel (eluting with DCM/EtOAc) to afford tert-butyl 2-(3,5-dimethylanilino)-4-oxo-5,6,8,9-tetrahydro-3H-pyrimido[4,5-d] azepine-7-carboxylate.
LCMS: MW (calcd): 384.5; m/z MW (obsd): 385.5 (M+H)

### 1.8.2. Step ii

To a solution of tert-butyl 2-(3,5-dimethylanilino)-4-oxo-5,6,8,9-tetrahydro-3H-pyrimido[4,5-d]azepine-7-carboxylate (271 mg, 0.70 mmol, 1.0 eq.) in DCM (4 mL) at 0 °C were added TEA (84 µL, 0.78 mmol, 1.1 eq.) and dropwise triflic anhydride (130 µL, 0.78 mmol, 1.1 eq.). The reaction mixture was stirred at 0 °C for 2 h. The mixture was diluted with water and the phases were separated using a phase separator. The organic phase was concentrated to dryness and the residue was purified by flash chromatography on silica gel (eluting with DCM/MeOH) to obtain [2-(3,5-dimethylanilino)-6,7,8,9-tetrahydro-5H-pyrimido[4,5-d]azepin-4-yl] trifluoromethanesulfonate.
LCMS: MW (calcd): 416.4; m/z MW (obsd): 417.4 (M+H)

### 1.8.3. Step iii

Tert-butyl 4-[2-(3,5-dimethylanilino)-4-(trifluoromethylsulfonyloxy)-5,6,8,9-tetrahydropyrimido[4,5-d]azepin-7-yl]piperidine-1-carboxylate was obtained from [2-(3,5-dimethylanilino)-6,7,8,9-tetrahydro-5H-pyrimido[4,5-d]azepin-4-yl] trifluoromethanesulfonate following general method C with tert-butyl 4-oxopiperidine-1-carboxylate (CAS# 79099-07-3).
LCMS: MW (calcd): 599.7; m/z MW (obsd): 600.6 (M+H)

### 1.8.4. Step iv

To a solution of tert-butyl 4-[2-(3,5-dimethylanilino)-4-(trifluoromethylsulfonyloxy)-5,6,8,9-tetrahydropyrimido[4,5-d]azepin-7-yl]piperidine-1-carboxylate (163 mg, 0.27 mmol, 1.0 eq.) in DMF (2 mL) were added Pd(OAc)₂ (1.5 mg, 0.005 mmol, 0.02 eq.), dppf (6 mg, 0.01 mmol, 0.04 eq.), TEA (151 µL, 1.09 mmol, 3.0 eq.) and formic acid (20 µL, 0.54 mmol, 2.0 eq.). The reaction mixture was stirred at 50 °C for 2 h. DMF was concentrated, the residue was taken up in a sat. NaHCO₃ aq. solution and extracted with EtOAc. The combined organic layers were dried over Na₂SO₄, filtered and concentrated to dryness. The residue was purified by flash chromatography on silica gel (eluting with DCM/MeOH) to afford tert-butyl 4-[2-(3,5-dimethylanilino)-5,6,8,9-tetrahydropyrimido[4,5-d]azepin-7-yl]piperidine-1-carboxylate.
LCMS: MW (calcd): 451.6; m/z MW (obsd): 452.6 (M+H)

### 1.8.5. Step v

Int 53 is obtained from tert-butyl 4-[2-(3,5-dimethylanilino)-5,6,8,9-tetrahydropyrimido[4,5-d]azepin-7-yl]piperidine-1-carboxylate following general method B.

### 1.9. Int 57

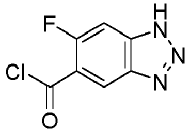

To 6-fluoro-1H-benzotriazole-5-carboxylic acid (CAS# 1427081-62-6; 200 mg, 1.1 mmol, 1.0 eq.) in THF (4 mL) was added DMF (9 µL, 0.11 mmol, 0.1 eq.) and the solution was cooled down to 0 °C. Then oxalyl chloride (284 µL, 3.31 mmol, 3.0 eq.) was slowly added and the mixture was stirred at RT for 3 h. The solvents were evaporated to afford Int 57.

### 1.10. Int 58

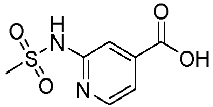

To a solution of methyl 2-aminopyridine-4-carboxylate (CAS# 6937-03-7; 250 mg, 1.6 mmol, 1.0 eq.) in DCM (13 mL) and TEA (251 µL, 1.8 mmol, 1.1 eq.) cooled down to 0 °C was added MsCl (140 µL, 1.8 mmol, 1.1 eq.) dropwise. The reaction mixture was stirred at RT for 2 h before being concentrated in vacuo. The residue was taken up in MeOH (6 mL) and a NaOH aq. solution (1N, 8 mL), the mixture was stirred at RT for 72 h and was then quenched with an HCl aq. solution (1N, 8 mL). MeOH was removed in vacuo and the residue was taken up in DCM. The phases were separated and the aqueous layer was basified with a NaOH aq. solution (IN). The basic aqueous phase was washed with DCM and was then acidified to pH 1 with concentrated HCl. The aqueous phase was extracted with EtOAc. The combined EtOAc layers were dried over Na₂SO₄, filtered and concentrated to dryness to afford Int 58.

### 1.11. Int 59

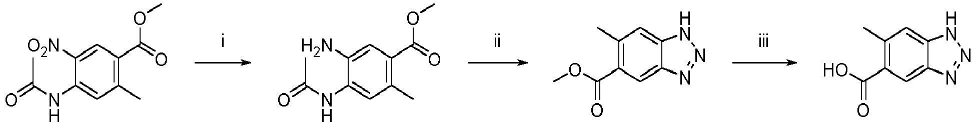

### 1.11.1. Step i

To a solution of Int 137 (2.52 g, 10 mmol, 1.0 eq.) in a MeOH/DCM mixture (3/1, 145 mL) was added Pd/C (10%, 1.26 g) and the reaction medium was placed under H₂ atmosphere for 18 h. The mixture was filtered on a Whatman® filter, the solid was washed with EtOH and the filtrate was evaporated to afford methyl 4-acetamido-5-amino-2-methyl-benzoate.
LCMS: MW (calcd): 222.3; m/z MW (obsd): 223.3 (M+H)

### 1.11.2. Step ii

To a solution of methyl 4-acetamido-5-amino-2-methyl-benzoate (2.2 g, 9.9 mmol, 1.0 eq.) in EtOH (94 mL) at 0°C was added dropwise isoamylnitrite (1.4 mL, 10.4 mmol, 1.05 eq.) and HBF₄ (54% solution in water, 1.4 mL, 10.4 mmol, 1.05 eq.) and the mixture was stirred at 0°C for 2 h. The solvents were removed in vacuo and the residue was taken up in EtOAc and a sat. aq. NaHCO₃ solution. Phases were separated and the aqueous phase was extracted with EtOAc. The combined organic layers were dried over Na₂SO₄, filtered and concentrated to dryness. The residue was triturated in pentane to afford methyl 6-methyl-1H-benzotriazole-5-carboxylate.
LCMS: MW (calcd): 191.2; m/z MW (obsd): 192.0 (M+H)

### 1.11.3. Step iii

Int 59 is synthesized from methyl 6-methyl-1H-benzotriazole-5-carboxylate following general method G.

### 1.12. Int 60

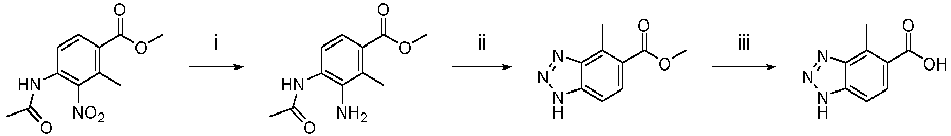

### 1.12.1. Step i

To a solution of Int 138 (1.83 g, 7.3 mmol, 1.0 eq.) in a MeOH/DCM mixture (3/1, 105 mL) was added Pd/C (10%, 915 mg) and the reaction medium was placed under H₂ atmosphere for 18 h. The mixture was filtered on a Whatman® filter, the solid was washed with EtOH and the filtrate was evaporated. The residue was triturated in pentane and diisopropylether and was then purified by flash chromatography on silica gel (eluting with DCM/MeOH) to afford methyl 4-acetamido-3-amino-2-methyl-benzoate.
LCMS: MW (calcd): 222.3; m/z MW (obsd): 223.6 (M+H)

### 1.12.2. Step ii

To a solution of methyl 4-acetamido-3-amino-2-methyl-benzoate (0.77 g, 3.5 mmol, 1.0 eq.) in EtOH (94 mL) at 0°C was added dropwise isoamylnitrite (489 µL, 3.6 mmol, 1.05 eq.) and HBF₄ (54% in water, 501 µL, 3.6 mmol, 1.05 eq.) and the mixture was stirred at 0°C for 2 h. The solvents were removed in vacuo and the residue was taken up in EtOAc and a sat. aq. NaHCO₃ solution. The phases were separated and the aqueous phase was extracted with EtOAc. The combined organic layers were dried over Na₂SO₄, filtered and concentrated to dryness. The residue was purified by flash chromatography on silica gel (eluting with DCM/MeOH) to afford methyl 4-methyl-1H-benzotriazole-5-carboxylate.
LCMS: MW (calcd): 191.2; m/z MW (obsd): 192.3 (M+H)

### 1.12.3. Step iii

Int 60 is synthesized from methyl 4-methyl-1H-benzotriazole-5-carboxylate following general method G.

### 1.13. Int 139

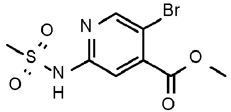

To a solution of methyl 2-amino-5-bromo-pyridine-4-carboxylate (CAS# 882499-87-8; 1 g, 4.32 mmol, 1.0 eq.) in pyridine (10 mL) at 0°C was added MsCl (401 µL, 5.2 mmol, 1.2 eq.) and the mixture was stirred at RT for 72 h. The reaction mixture was quenched with water. The precipitate was filtered, rinsed with water and dried to afford Int 139.

### 1.14. Int 61

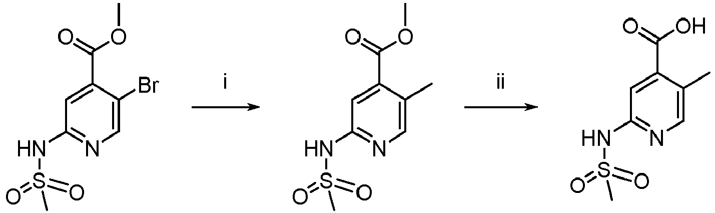

### 1.14.1. Step i

To a solution of Int 139 (200 mg, 0.65 mmol, 1.0 eq.) in 1,4-dioxane (2 mL) was added dimethylzinc (1.2 M in toluene, 646 µL, 0.77 mmol, 1.2 eq.). When the gas release was over, Pd(dppf)Cl₂ (CAS# 72287-26-4; 5 mg, 0.006 mmol, 0.01 eq.) was added, the reaction mixture was degassed by N₂ bubbling and heated to 60 °C in a sealed vial for 18 h. Then more dimethylzinc (2 M in toluene, 0.98 mL, 1.95 mmol, 3.0 eq.) was added and the mixture was heated to 60 °C for 18 h. A sat. aq. NaHCO₃ solution was added and the medium was extracted with EtOAc. The combined organic layers were dried over Na₂SO₄, filtered and concentrated to dryness. The residue was purified by flash chromatography on silica gel (eluting with heptane/EtOAc) to afford methyl 2-(methanesulfonamido)-5-methyl-pyridine-4-carboxylate.
LCMS: MW (calcd): 244.3; m/z MW (obsd): 245.1 (M+H)

### 1.14.2. Step ii

Int 61 is synthesized from methyl 2-(methanesulfonamido)-5-methyl-pyridine-4-carboxylate following general method G.

### 1.15. Int 63

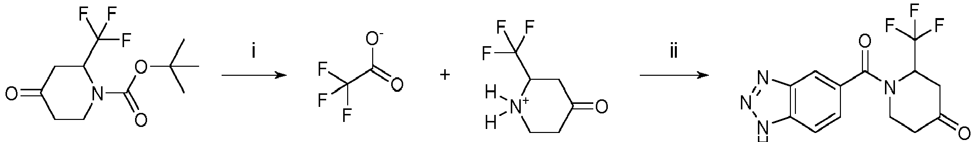

### 1.15.1. Step i

To a solution of tert-butyl 4-oxo-2-(trifluoromethyl)piperidine-1-carboxylate (CAS# 1245648-32-1; 200 mg, 0.75 mmol, 1.0 eq.) in DCM (6 mL) at 0°C was added TFA (1.5 mL, 14.97 mmol, 10 eq.) and the mixture was stirred at RT for 1.5 h. The reaction medium was concentrated to dryness to afford 2-(trifluoromethyl)piperidin-4-one trifluoroaceate salt.

### 1.15.2. Step ii

To a solution of 2-(trifluoromethyl)piperidin-4-one trifluoroaceate salt (125 mg, 0.75 mmol, 1.0 eq.) in DMF (15 mL) were added TEA (728 µL, 5.24 mmol, 7.0 eq.), DMAP (2 mg, 0.015 mmol, 0.02 eq.) and 1H-benzotriazole-5-carbonyl chloride (CAS# 46053-85-4; 543 mg, 2.99 mmol, 4.0 eq.) and the mixture was stirred at RT for 3 days. The reaction medium was hydrolysed with a sat. NaHCO₃ aq. solution and a precipitate was filtered off. The filtrate was extracted with EtOAc and the combined organic layers were dried on Na₂SO₄, filtered and concentrated to dryness. The residue was purified by flash chromatography on silica gel (eluting with DCM/EtOAc) to afford Int 63.

### 1.16. Int 64

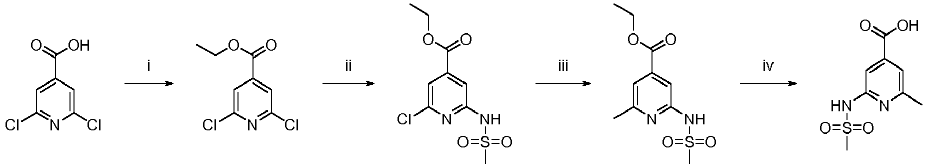

### 1.16.1. Step i

To a solution of 2,6-dichloropyridine-4-carboxylic acid (CAS# 5398-44-7; 1 g, 5.2 mmol, 1.0 eq.) in EtOH (10 mL) at 0°C was slowly added SOCl₂ (1.1 mL, 15.6 mmol, 3.0 eq.) and the mixture was stirred at RT for 18 h. Solvents were concentrated and the residue was purified by flash chromatography on silica gel (eluting with heptane/EtOAc) to afford ethyl 2,6-dichloropyridine-4-carboxylate.
LCMS: MW (calcd): 220.1; m/z MW (obsd): 220.1-222.1 (M+H)

### 1.16.2. Step ii

To a solution of ethyl 2,6-dichloropyridine-4-carboxylate (1 g, 5.2 mmol, 1.0 eq.) in 1,4-dioxane were added methanesulfonamide (743 mg, 7.8 mmol, 1.5 eq.), K₃PO₄ (1.7 g, 7.8 mmol, 1.5 eq.), Pd₂(dba)₃ (238 mg, 0.26 mmol, 0.05 eq.) and Xantphos (270 mg, 0.52 mmol, 0.1 eq.). The reaction medium was degassed by N₂ bubbling and heated at 90°C for 18 h. The mixture was quenched with water and extracted with EtOAc. The combined organic layers were dried over Na₂SO₄, filtered and concentrated to dryness. The residue was purified by flash chromatography on silica gel (eluting with heptanes/EtOAc) to afford ethyl 2-chloro-6-(methanesulfonamido)pyridine-4-carboxylate.
LCMS: MW (calcd): 278.7; m/z MW (obsd): 279.1-281.1 (M+H)

### 1.16.3. Step iii

To a solution of ethyl 2-chloro-6-(methanesulfonamido)pyridine-4-carboxylate (200 mg, 0.72 mmol, 1.0 eq.) in 1,4-dioxane (2 mL) was added dimethylzinc (2 M in toluene, 1 mL, 3.0 eq.). When the gas release was over, PdCl₂(dppf) (5 mg, 0.006 mmol, 0.01 eq.) was added, the reaction mixture was degassed by N₂ bubbling and heated to 60°C in a sealed vial for 18 h. The reaction medium was poured on a mixture of ice and sat. aq. NaHCO₃ solution before being extracted with EtOAc. The combined organic layers were dried over Na₂SO₄, filtered and concentrated to dryness. The residue was purified by flash chromatography on silica gel (eluting with heptanes/EtOAc) to afford ethyl 2-(methanesulfonamido)-6-methyl-pyridine-4-carboxylate.
LCMS: MW (calcd): 258.3; m/z MW (obsd): 259.2 (M+H)

### 1.16.4. Step iii

Int 64 is synthesized from ethyl 2-(methanesulfonamido)-6-methyl-pyridine-4-carboxylate following general method G.

### 1.17. Int 65

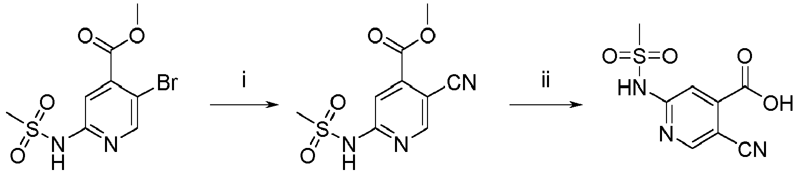

### 1.17.1. Step i

To a solution of Int 139 (100 mg, 0.32 mmol, 1.0 eq.) in DMAC (3 mL) were added Zn(CN)₂ (23 mg, 0.19 mmol, 0.6 eq.), zinc powder (3 mg, 0.04 mmol, 0.12 mmol), dppf (34 mg, 0.06 mmol, 0.2 eq.) and Pd₂(dba)₃ (30 mg, 0.03 mmol, 0.1 eq.) and the mixture was heated under microwave irradiation at 120°C for 25 min. The reaction mixture was quenched with water and a precipitate was filtered. The filtrate was diluted with a sat. aq. NaHCO₃ solution that was then acidified with a 1N aq. HCl solution until pH 2. The aqueous phase was extracted with EtOAc, the combined organic layers were dried over Na₂SO₄, filtered and concentrated to dryness. The residue was purified by flash chromatography on silica gel (eluting with DCM/MeOH) to afford methyl 5-cyano-2-(methanesulfonamido)pyridine-4-carboxylate.
LCMS: MW (calcd): 255.3; m/z MW (obsd): 256.1 (M+H)

### 1.17.2. Step ii

To a solution of methyl 5-cyano-2-(methanesulfonamido)pyridine-4-carboxylate (50 mg, 0.20 mmol, 1.0 eq.) in a 1,4-dioxane/water mixture (4 mL/800 µL) was added LiOH monohydrate (41 mg, 0.98 mmol, 5.0 eq.) and the mixture was stirred at RT for 18 h. The reaction medium was diluted with water, acidified to pH 2 with a 1N aq. HCl solution and extracted with EtOAc. The combined organic layers were dried over Na₂SO₄, filtered and concentrated to dryness to afford Int 65.

### 1.18. Int 66

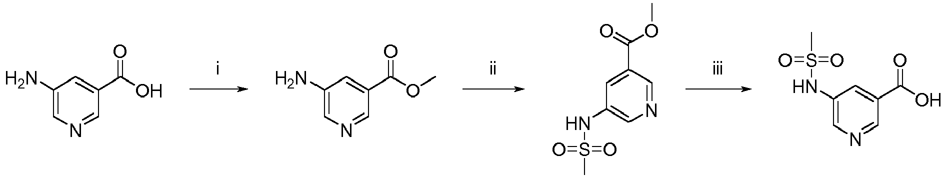

### 1.18.1. Step i

To a solution of 5-aminopyridine-3-carboxylic acid (CAS# 24242-19-1; 722 mg, 5.23 mmol, 1.0 eq.) in MeOH (10 mL) was added SOCl₂ (455 µL, 6.27 mmol, 1.2 eq.) dropwise at 0°C and the mixture was heated to reflux until completion. The solvents were concentrated and the residue was taken up in water and a sat. aq. NaHCO₃ solution. The aqueous phase was extracted with DCM and the combined organic layers were concentrated to afford methyl 5-aminopyridine-3-carboxylate.

### 1.18.2. Step ii

To a solution of methyl 5-aminopyridine-3-carboxylate (570 mg, 3.75 mmol, 1.0 eq.) in DCM (10 mL) were added pyridine (908 µL, 11.24 mmol, 3.0 eq.) and MsCl (348 µL, 4.50 mmol, 1.2 eq.) at 0°C and the mixture was stirred at this temperature for 1 h followed by 30 min at RT. Water was added and the mixture was extracted with DCM. The combined organic layers were dried over Na₂SO₄, filtered and concentrated to dryness. The residue was taken up in DCM and the solid was filtered to obtain methyl 5-(methanesulfonamido)pyridine- 3 -carboxylate.
LCMS: MW (calcd): 230.2; m/z MW (obsd): 231.1 (M+H)

### 1.18.3. Step iii

Int 66 is synthesized from 5-(methanesulfonamido)pyridine-3-carboxylate following general method G.

### 1.19. Int 67

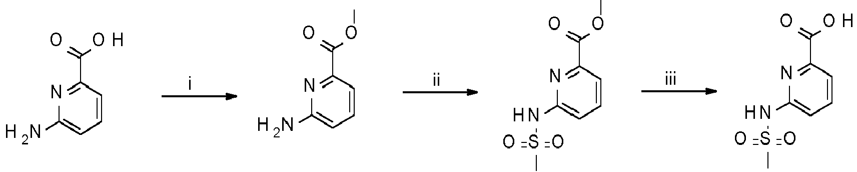

### 1.19.1. Step i

To a solution of 6-aminopyridine-2-carboxylic acid (CAS# 23628-31-1; 734 mg, 5.31 mmol, 1.0 eq.) in MeOH (10 mL) was added SOCl₂ (462 µL, 6.37 mmol, 1.2 eq.) dropwise at 0°C and the mixture was heated to reflux. The solvents were concentrated and the residue was taken up in water and a sat. aq. NaHCO₃ solution. The aqueous phase was extracted with EtOAc and the combined organic layers were concentrated to afford methyl 6-aminopyridine-2-carboxylate.

### 1.19.2. Step ii

To a solution of methyl 6-aminopyridine-2-carboxylate (470 mg, 3.10 mmol, 1.0 eq.) in DCM (10 mL) were added pyridine (748 µL, 9.27 mmol, 3.0 eq.) and MsCl (287 µL, 3.71 mmol, 1.2 eq.) at 0°C and the mixture was stirred at this temperature for 1 h followed by 30 min at RT. Water was added and the mixture was extracted with DCM. The combined organic layers were dried over Na₂SO₄, filtered and concentrated to dryness. The residue was purified by flash chromatography on silica gel (eluting with DCM/MeOH) to afford methyl 6-(methanesulfonamido)pyridine-2-carboxylate.
LCMS: MW (calcd): 230.2; m/z MW (obsd): 231.1 (M+H)

### 1.19.3. Step iii

Int 67 is synthesized from methyl 6-(methanesulfonamido)pyridine-2-carboxylate following general method G.

### 1.20. Int 68

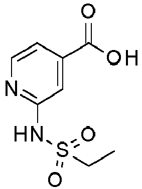

To a solution of methyl 2-aminopyridine-4-carboxylate (CAS# 6937-03-7; 300 mg, 1.97 mmol, 1.0 eq.) in DCM (10 mL) were added TEA (0.55 mL, 3.94 mmol, 2.0 eq.) and ethanesulfonyl chloride (280 µL, 2.96 mmol, 1.5 eq.) and the mixture was stirred at RT for 4 h. The reaction mixture was quenched with a sat. aq. NH₄Cl solution and the phases were separated. The organic phase was washed with a 0.1N aq. HCl solution, dried over Na₂SO₄, filtered and concentrated to dryness. The residue was then taken up in MeOH (10 mL). A 1N aq. NaOH solution was added (4 mL, 3.94 mmol, 2.0 eq.) and the solution was stirred at RT for 18 h. MeOH was concentrated, the obtained aqueous phase was acidified with a 1N aq. HCl solution (4 mL) and the solution was stirred 2 h at RT. Precipitation occurs, the solid was filtered and dried to afford Int 68.

### 1.21. Int 69

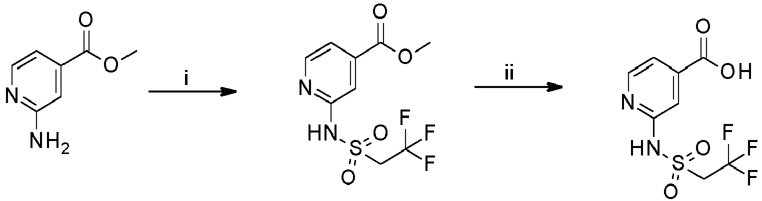

### 1.21.1. Step i

To a solution of methyl 2-aminopyridine-4-carboxylate (CAS# 6937-03-7; 300 mg, 1.97 mmol, 1.0 eq.) in pyridine (7 mL) was added 2,2,2-trifluoroethylsulfonyl chloride (240 µL, 2.17 mmol, 1.1 eq.) and the mixture was stirred at RT for 6 h. The solvents were concentrated and the residue was taken up in water, the obtained precipitate was filtered. The solid was dissolved in DCM, the organic layer was washed with water before being concentrated to afford methyl 2-(2,2,2-trifluoroethylsulfonylamino)pyridine-4-carboxylate.
LCMS: MW (calcd): 298.2; m/z MW (obsd): 299.2 (M+H)

### 1.21.2. Step ii

Int 69 is synthesized from methyl 2-(2,2,2-trifluoroethylsulfonylamino)pyridine-4-carboxylate following general method G.

### 1.22. Int 70

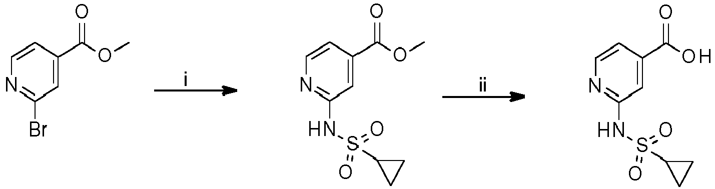

### 1.22.1. Step i

To a solution of methyl 2-bromopyridine-4-carboxylate (CAS# 26156-48-9; 300 mg, 1.39 mmol, 1.0 eq.) in 1,4-dioxane (5 mL) were added Pd₂(dba)₃ (64 mg, 0.07 mmol, 0.05 eq.), Xantphos (81 mg, 0.14 mmol, 0.1 eq.), K₃PO₄ (305 mg, 2.21 mmol, 1.5 eq.) and cyclopropyl sulfonamide (211 mg, 1.74 mmol, 1.3 eq.) and the mixture was heated to reflux for 18 h. After dilution with EtOAc the reaction medium was washed with water. The organic layer was concentrated and the residue was purified by flash chromatography on silica gel (eluting with DCM/MeOH 98/2) to afford methyl 2-(cyclopropylsulfonylamino)pyridine-4-carboxylate.
LCMS: MW (calcd): 256.3; m/z MW (obsd): 257.14 (M+H)

### 1.22.2. Step ii

Int 70 is synthesized from methyl 2-(cyclopropylsulfonylamino)pyridine-4-carboxylate following general method G.

### 1.23. Int 71

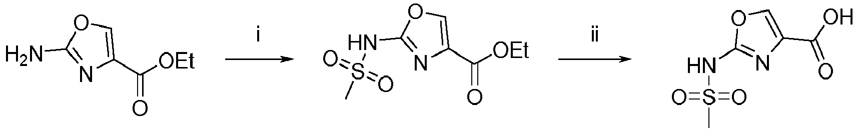

### 1.23.1. Step i

To a solution of ethyl 2-aminooxazole-4-carboxylate (CAS# 177760-52-0; 100 mg, 0.64 mmol, 1.0 eq.) in THF (5 mL) at 0°C was added NaH (60% in mineral oil, 31 mg, 0.77 mmol, 1.2 eq.) and the suspension was stirred at 0°C for 10 min. Then MsCl was added and the mixture was stirred at RT for 2 h. The volatiles were concentrated, the residue was dissolved in EtOAc and washed with a sat. aq. NaHCO₃ solution. The organic layer was dried over Na₂SO₄, filtered and concentrated to dryness to afford ethyl 2-(methanesulfonamido)oxazole-4-carboxylate.
LCMS: MW (calcd): 234.2; m/z MW (obsd): 235.1 (M+H)

### 1.23.2. Step ii

Int 71 is synthesized from ethyl 2-(methanesulfonamido)oxazole-4-carboxylate following general method G.

### 1.24. Int 72

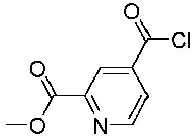

2-(Methoxycarbonyl)isonicotinic acid (CAS# 24195-10-6; 10 g, 55.2 mmol, 1.0 eq.) was suspended in SOCl₂ (100 mL) and the mixture was refluxed for 1 h. The medium was concentrated to dryness to afford Int 72.

### 1.25. Int 76

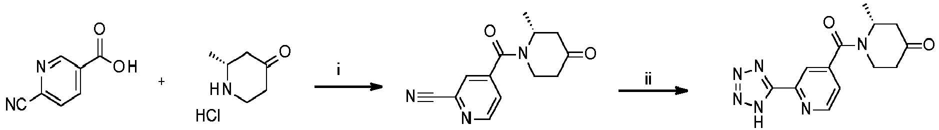

### 1.25.1. Step i

4-[(2R)-2-methyl-4-oxo-piperidine-1-carbonyl]pyridine-2-carbonitrile is synthesized from 6-cyanopyridine-3-carboxylic acid (CAS# 70165-31-0) and (2R)-2-methylpiperidin-4-one HCl salt (CAS# 1434126-97-2) following general method A.

### 1.25.2. Step ii

To a solution of 4-[(2R)-2-methyl-4-oxo-piperidine-1-carbonyl]pyridine-2-carbonitrile (690 mg, 2.84 mmol, 1.0 eq.) in 1,4-dioxane (10 mL) was added tri-n-butylstannyl azide (CAS# 17846-68-3; 1.9 g, 5.72 mmol, 2.0 eq.) and the mixture was stirred at reflux for 5 h. The reaction medium was concentrated to dryness, the residue was taken up in Et₂O and a 2N HCl aq. solution and the mixture was stirred at RT for 2 h. Et₂O was removed in vacuo, pentane was added and the mixture was filtered. The obtained crude mixture was purified by flash chromatography on silica gel (eluting with DCM/MeOH) to afford Int 76.

### 1.26. Int 78

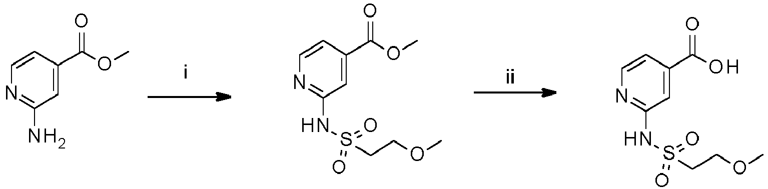

### 1.26.1. Step i

To a solution of methyl 2-aminopyridine-4-carboxylate (CAS# 6937-03-7; 200 mg, 1.31 mmol, 1.0 eq.) in pyridine (5 mL) was added 2-methoxyethane sulfonyl chloride (185 µL, 1.58 mmol, 1.2 eq.) and the mixture was stirred at RT for 18 h. The solvents were concentrated, the residue was dissolved in EtOAc and washed with a sat. aq. NaHCO₃ solution. The organic layer was concentrated and the residue was purified by flash chromatography on silica gel (eluting with DCM/MeOH) to afford methyl 2-(2-methoxyethylsulfonylamino)pyridine-4-carboxylate.

### 1.26.2. Step ii

Int 78 is synthesized from methyl 2-(2-methoxyethylsulfonylamino)pyridine-4-carboxylate following general method G.

### 1.27. Int 103

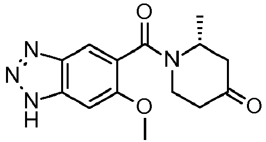

To (2R)-2-methylpiperidin-4-one HCl salt (CAS# 1434126-97-2; 73 mg, 0.5 mmol, 1.2 eq.) and 6-methoxy-1H-benzotriazole-5-carboxylic acid (CAS# 59338-92-0; 80 mg, 0.42 mmol, 1.0 eq.) in DMF (3 mL) were added HATU (223 mg, 0.59 mmol, 1.4 eq.) and TEA (234 µL, 1.68 mmol, 4.0 eq.) and the mixture was stirred at RT overnight. The reaction medium was concentrated to dryness, the residue was partitioned between a sat. NH₄Cl aq. solution and DCM and the phases were separated. The aqueous phase was then extracted with a 1/1 mixture of EtOAc and n-BuOH. The combined organic layers were dried over Na₂SO₄, filtered and concentrated to dryness. The residue was purified by flash chromatography on silica gel (eluting with DCM/MeOH) to afford Int 103.

### 1.28. Int 104

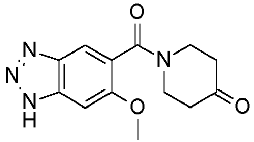

To piperidin-4-one HCl salt (73 mg, 0.5 mmol, 1.2 eq.) and 6-methoxy-1H-benzotriazole-5-carboxylic acid (CAS# 59338-92-0; 80 mg, 0.42 mmol, 1.0 eq.) in DMF (3 mL) were added HATU (223 mg, 0.59 mmol, 1.4 eq.) and TEA (234 µL, 1.68 mmol, 4.0 eq.) and the mixture was stirred at RT overnight. The reaction medium was concentrated to dryness. The residue was partitioned between a sat. NH₄Cl aq. solution and DCM and the phases were separated. The aqueous phase was then extracted with a 1/1 mixture of EtOAc and n-BuOH. The combined organic layers were dried over Na₂SO₄, filtered and concentrated to dryness. The residue was purified by flash chromatography on silica gel (eluting with DCM/MeOH) to afford Int 104.

### 1.29. Int 105

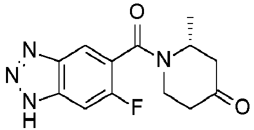

To (2R)-2-methylpiperidin-4-one HCl salt (CAS# 1434126-97-2; 97 mg, 0.66 mmol, 1.2 eq.) and 6-fluoro-1H-benzotriazole-5-carboxylic acid (CAS# 1427081-62-6; 100 mg, 0.55 mmol, 1.0 eq.) in DMF (3 mL) were added HATU (293 mg, 0.77 mmol, 1.4 eq.) and TEA (306 µL, 2.2 mmol, 4.0 eq.) and the mixture was stirred at RT for 3 h. The reaction medium was concentrated to dryness, the residue was partitioned between a sat. NH₄Cl aq. solution and a 9/1 mixture of DCM and i-PrOH. The layers were separated and the organic layer was dried over Na₂SO₄, filtered and concentrated to dryness. The residue was purified by flash chromatography on silica gel (eluting with DCM/MeOH) to afford Int 105.

### 1.30. Int 106

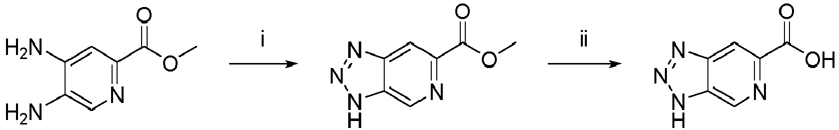

### 1.30.1. Step i

To a solution of methyl 4,5-diaminopyridine-2-carboxylate (CAS# 850689-13-3; 153 mg, 0.92 mmol, 1.0 eq.) in AcOH/H₂O (2/1, 3 mL) was added NaNO₂ (70 mg, 0.95 mmol, 1.03 eq.) and the mixture was stirred at RT for 18 h. The obtained precipitate was filtered and dried to afford methyl 3H-triazolo[4,5-c]pyridine-6-carboxylate.
LCMS: MW (calcd): 178.2; m/z MW (obsd): 179.2 (M+H)

### 1.30.2. Step ii

Int 106 iss synthesized from methyl 3H-triazolo[4,5-c]pyridine-6-carboxylate following general method G.

### 1.31. Int 108

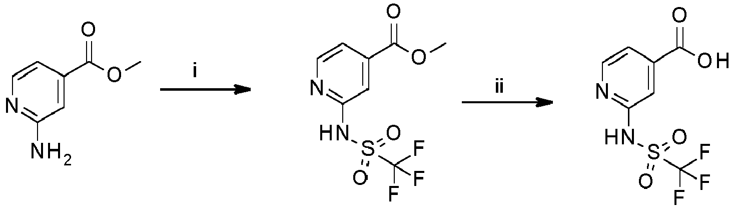

### 1.31.1. Step i

To a solution of methyl 2-aminopyridine-4-carboxylate (CAS# 6937-03-7; 300 mg, 1.97 mmol, 1.0 eq.) in DCM (10 mL) and TEA (548 µL, 3.94 mmol, 2.0 eq.) was added triflic anhydride (500 µL, 2.96 mmol, 1.5 eq.) and the mixture was stirred at RT for 5 h. The volatiles were evaporated in vacuo to afford methyl 2-(trifluoromethylsulfonylamino)pyridine-4-carboxylate.

### 1.31.2. Step ii

Int 108 is synthesized from methyl 2-(trifluoromethylsulfonylamino)pyridine-4-carboxylate following general method G.

### 1.32. Int 111 :

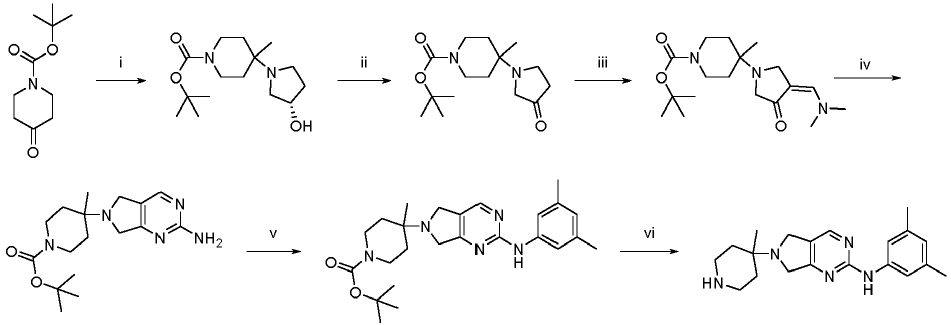

### 1.32.1. Step i

To a solution of (3S)-pyrrolidin-3-ol (4 g, 45.9 mmol, 1.0 eq.) in toluene (46 mL) were added 1H-triazole (3.56 g, 51.6 mmol, 1.1 eq.) and tert-butyl 4-oxopiperidine-1-carboxylate (CAS# 79099-07-3; 8.69 g, 43.6 mmol, 0.95 eq.) and the mixture was stirred at reflux for 4 h using a Dean-Stark apparatus to remove water from the reaction medium. After cooling down to RT a MeMgBr solution (3 M in Et₂O, 58.2 mL, 3.8 eq.) was added dropwise to the reaction mixture, maintaining the temperature below 24 °C with an ice bath. At the end of the addition the bath was removed and the mixture was stirred at RT for 4 h. The reaction medium was diluted with water and extracted with EtOAc. The combined organic layers were dried over Na₂SO₄, filtered and concentrated to dryness. The residue was purified by flash chromatography on silica gel (eluting with DCM/MeOH) to afford tert-butyl 4-[(3S)-3-hydroxypyrrolidin-1-yl]-4-methyl-piperidine-1 -carboxylate.
LCMS: MW (calcd): 284.4; m/z MW (obsd): 285.4 (M+H)

### 1.32.2. Step ii

To a solution of oxalyl chloride (2 mL, 23.8 mmol, 1.5 eq.) in DCM (30 mL) at -78°C was added a cooled solution (at -78°C) of DMSO (3.34 mL, 47.0 mmol, 3.0 eq.) in DCM (15 mL). The mixture was stirred at -78°C for 10 min and a solution of tert-butyl 4-[(3S)-3-hydroxypyrrolidin-1-yl]-4-methylpiperidine-1-carboxylate (4.45 g, 15.7 mmol, 1.0 eq.) in DCM (15 mL) followed by TEA (8.74 mL, 62.7 mmol, 4.0 eq.) were added. The reaction mixture was then stirred for another 20 min at -78°C and then for 1 h at RT. The reaction was quenched by the addition of water. After extraction with DCM the combined organic layers were washed with brine, dried over Na₂SO₄, filtered and concentrated to dryness to afford tert-butyl 4-methyl-4-(3-oxopyrrolidin-1-yl)piperidine-1-carboxylate.

### 1.32.3. Step iii

Tert-butyl 4-[(3Z)-3-(dimethylaminomethylene)-4-oxo-pyrrolidin-1-yl]-4-methyl-piperidine-1-carboxylate is synthesized from tert-butyl 4-[(3Z)-3-(dimethylaminomethylene)-4-oxo-pyrrolidin-1-yl]-4-methyl-piperidine-1-carboxylate following general method F.

### 1.32.4. Step iv

tert-butyl 4-(2-amino-5,7-dihydropyrrolo[3,4-d]pyrimidin-6-yl)-4-methyl-piperidine-1-carboxylate is synthesized from tert-butyl 4-[(3Z)-3-(dimethylaminomethylene)-4-oxo-pyrrolidin-1-yl]-4-methyl-piperidine-1-carboxylate following general method E.

### 1.32.5. Step v

Tert-butyl 4-[2-(3,5-dimethylanilino)-5,7-dihydropyrrolo[3,4-d]pyrimidin-6-yl]-4-methylpiperidine-1-carboxylate is obtained from tert-butyl 4-[(3Z)-3-(dimethylaminomethylene)-4-oxopyrrolidin-1-yl]-4-methyl-piperidine-1-carboxylate following general method D with 3,5-dimethylbromobenzene (CAS# 556-96-7).
LCMS: MW (calcd): 437.6; m/z MW (obsd): 438.5 (M+H)

### 1.32.6. Step vi

Int 111 is obtained from tert-butyl 4-[2-(3,5-dimethylanilino)-5,7-dihydropyrrolo[3,4-d]pyrimidin-6-yl]-4-methyl-piperidine-1-carboxylate following general method B.

### 1.33. Int 130

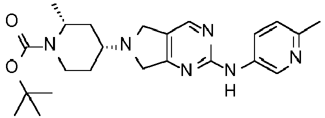

To a solution of Int 2056 (500 mg, 1.76 mmol, 1.0 eq.) in DCM (30 mL) were added activated 4 Å molecular sieves (800 mg), tert-butyl (2R)-2-methyl-4-oxo-piperidine-1-carboxylate (CAS# 790667-43-5; 412 mg, 1.94 mmol, 1.1 eq.), AcOH (59 µL, 0.88 mmol, 0.5 eq.) and NaBH(OAc)₃ (522 mg, 2.46 mmol, 1.4 eq.) and the mixture was stirred at RT for 3 h. The reaction medium was diluted with water and was extracted with DCM. The combined organic layers were dried over Na₂SO₄, filtered and concentrated to dryness. The crude mixture was purified by flash chromatography on silica gel (eluting with DCM/MeOH) to afford a residue that was dissolved in DCM and treated with PhosphonicS SPM32 metal scavenging resin (Strem, Catalog #16-1706). Filtration of the resin and concentration of the filtrate affords Int 130.

### 1.34. Int 131

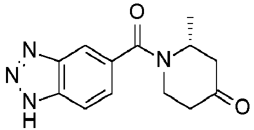

To a solution of (2R)-2-methylpiperidin-4-one HCl salt (CAS# 1434126-97-2; 10 g, 66 mmol, 1.0 eq.) and 1H-benzotriazole-5-carboxylic acid (12.95 g, 79 mmol, 1.2 eq.) in DMF (150 mL) were added HATU (30.2 g, 79 mmol, 1.4 eq.) and TEA (26.8 g, 265 mmol, 4.0 eq.) and the mixture was stirred at RT overnight. The reaction medium was filtered and the filtrate was concentrated to dryness. The residue was partitioned between a sat. NH₄Cl aq. solution and DCM, the layers were separated and the organic layer was washed with water, dried over Na₂SO₄, filtered and concentrated to dryness. The residue was triturated in DCM and the precipitate was filtered off. The filtrate was concentrated and the obtained crude mixture was purified by flash chromatography on silica gel (eluting with DCM/EtOAc) to afford Int 131.

### 1.35. Int 134

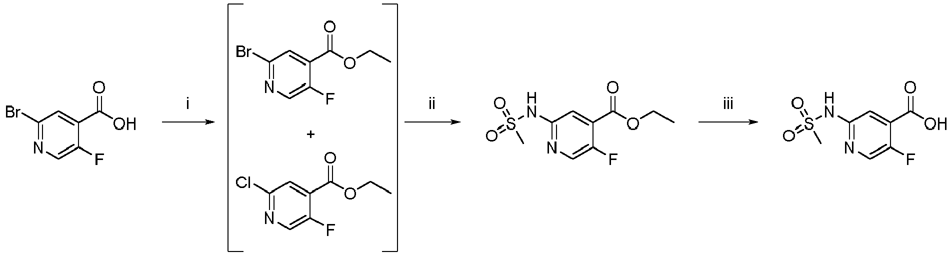

### 1.35.1. Step i

To a solution of 2-bromo-5-fluoro-pyridine-4-carboxylic acid (1 g, 4.54 mmol, 1.0 eq.) in EtOH (10 mL) at 0°C was added dropwise SOCl₂ and the reaction mixture was heated to reflux. Solvents were concentrated, the residue was taken up in water and a sat. NaHCO₃ aq. solution, and was extracted with DCM. The combined organic phases were concentrated to afford a mixture of ethyl 2-bromo-5-fluoro-pyridine-4-carboxylate and ethyl 2-chloro-5-fluoro-pyridine-4-carboxylate.

### 1.35.2. Step ii

A mixture of ethyl 2-bromo-5-fluoro-pyridine-4-carboxylate and ethyl 2-chloro-5-fluoro-pyridine-4-carboxylate (938 mg, 3.78 mmol, 1.0 eq.), methanesulfonamide (540 mg, 5.87 mmol, 1.5 eq.), K₃PO₄ (2.01 g, 9.45 mmol, 2.5 eq.), Pd₂(dba)₃ (173 mg, 0.19 mmol, 0.05 eq.), and Xantphos (392 mg, 0.76 mmol, 0.2 eq.) were placed in 1,4-dioxane (15 mL) and heated to reflux for 7 h. The solvents were evaporated and the residue was purified by flash chromatography on silica gel (eluting with DCM/ MeOH) to afford ethyl 5-fluoro-2-(methanesulfonamido)pyridine-4-carboxylate.
LCMS: MW (calcd): 262.3; m/z MW (obsd): 263.1 (M+H)

### 1.35.3. Step iii

To a solution of ethyl 5-fluoro-2-(methanesulfonamido)pyridine-4-carboxylate (170 mg, 0.65 mmol, 1.0 eq.) in MeOH (3.5 mL) was added was large excess of NaOH (2N aq. solution) and the mixture was stirred at 40°C. The medium was concentrated to dryness, the residue was taken up in water and the pH s adjusted to 2 with a 1N HCl aq. solution. The mixture was extracted with EtOAc and the combined organic phases were concentrated to afford Int 134.

### 1.36. Int 135

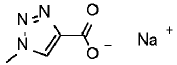

To methyl 1-methyltriazole-4-carboxylate (210 mg, 1.49 mmol, 1.0 eq.) in water (2.5 mL) was added solid NaOH (131 mg, 3.29 mmol, 2.2 eq.), the mixture was refluxed 2 h and stirred at RT for 15 h. The reaction mixture was then concentrated to afford Int 135.

### 1.37. Int 137 and Int 138

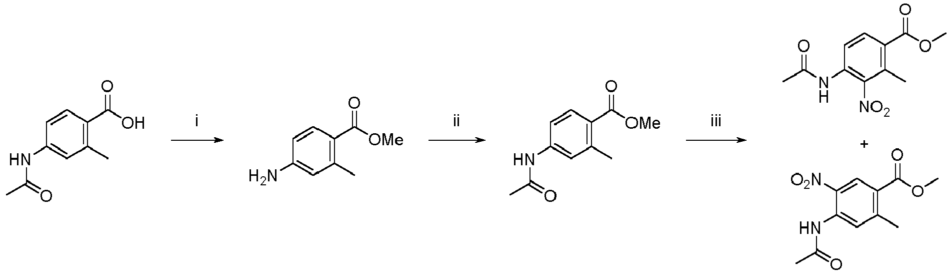

### 1.37.1. Step i : methyl 4-amino-2-methyl-benzoate

To a suspension of 4-acetamido-2-methyl-benzoic acid (CAS# 103204-69-9; 3.95 g, 20.4 mmol, 1.0 eq.) in MeOH (82 mL) was added dropwise sulfuric acid (98%, catalytic amount) at RT and the mixture was heated to reflux for 18 h. The solvents were concentrated, the residue was taken up in a sat. aq. NaHCO₃ solution and the aqueous phase was extracted with EtOAc. The combined organic layers were washed with a sat. aq. NaHCO₃ solution and with brine, were then dried over MgSO₄, filtered and concentrated to dryness to obtain methyl 4-amino-2-methyl-benzoate.

### 1.37.2. Step ii: methyl 4-acetamido-2-methyl-benzoate

To a solution of 4-amino-2-methyl-benzoate (3.43 g, 20.8 mmol, 1.0 eq.) in acetone (35 mL) was added dropwise acetic anhydride (4 mL, 42.6 mmol, 2.05 eq.) at 0°C and the reaction mixture was then stirred at RT for 19 h. The solvents were concentrated and the crude residue was partitioned between a sat. aq. Na₂CO₃ solution and EtOAc. The layers were separated and the aqueous phase was extracted with EtOAc. The combined organic phases were washed with a sat. aq. Na₂CO₃ solution, dried over MgSO₄, filtered and concentrated to dryness to obtain methyl 4-acetamido-2-methyl-benzoate.
LCMS: MW (calcd): 207.3; m/z MW (obsd): 208.3 (M+H)

### 1.37.3. Step iii : Int 137 and Int 138

To a solution of methyl 4-acetamido-2-methyl-benzoate (4.47 g, 21.3 mmol, 1.0 eq.) in acetic anhydride (43 mL) at -10°C was added dropwise nitric acid (concentrated, 9.05 mL, 215.7 mmol, 10 eq.) over 25 min. The reaction mixture was then stirred at -10°C for 30 min. The reaction medium was slowly poured on ice and the aqueous phase was extracted with EtOAc. The combined organic layers were washed with brine, dried over Na₂SO₄, filtered and concentrated to dryness. The residue was purified by flash chromatography on silica gel (eluting with heptanes/EtOAc) to afford 2 crude products. Each crude was triturated in Et₂O and pentane to afford Int 137 (first eluted) and Int 138 (second eluted) respectively.

### 1.38. Int 141

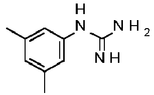

To a solution of 3,5-dimethylaniline (2 g, 16.50 mmol, 1.0 eq.) in EtOH (15 mL) at 0°C was slowly added nitric acid (concentrated, 1.5 mL, 16.5 mmol, 1.0 eq.) and cyanimide (50% in water, 2.78 mL, 33.2 mmol, 2.0 eq.) and the mixture was refluxed for 18 h. The reaction medium was diluted with Et₂O and the mixture was placed in the fridge for 15 h. The obtained precipitate was filtered and the solid dried to afford Int 141.

### 1.39. Int 1011

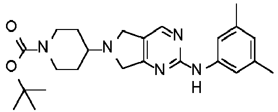

To a solution of Int 2011 (3.22 g, 13.40 mmol, 1.0 eq.) in DCE (220 mL) were added activated 4 Å molecular sieves and tert-butyl4-oxopiperidine-1-carboxylate (2.99 g, 14.74 mmol, 1.1 eq.) and the mixture was stirred at RT for 1 h. Then NaBH(OAc)₃ was added and the reaction was stirred at RT for 16 h. The reaction medium was diluted with a sat. NaHCO₃ aq. solution and water and was extracted with DCM. The phases were separated using a phase separator, the organic solvent was evaporated and the residue was suspended in a cyclohexane/EtOAc mixture (3/1). The precipitate was filtered and dried to afford Int 1011.

### 1.40. Int 1043

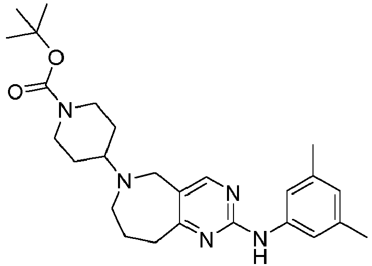

To a suspension of Int 2043 (176 mg, 0.66 mmol, 1.0 eq.) and activated 4 Å molecular sieves in DCE (3.9 mL) was added tert-butyl 4-oxopiperidine-1-carboxylate (133 mg, 0.66 mmol, 1.0 eq.) and the mixture was stirred at RT for 15 min. Then NaBH(OAc)₃ (205 mg, 0.92 mmol, 1.4 eq.) was added and the resulting mixture was left to stir at RT overnight. More DCE (2 mL) was added and the reaction mixture was stirred for 24 h. Then, more NaBH(OAc)₃ (102 mg, 0.46 mmol, 0.7 eq.) and more DCE (2 mL) were added and the suspension was stirred for another 24 h. The reaction medium was diluted with a sat. NaHCO₃ aq. solution and was extracted with DCM. The combined organic layers were dried over Na₂SO₄, filtered and concentrated to dryness. The residue was purified by flash chromatography on silica gel (eluting with DCM/MeOH) to afford Int 1043.

### 1.41. Int 1044

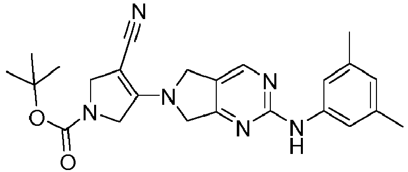

To a solution of Int 2011 (50 mg, 0.21 mmol, 1.0 eq.) and activated 4 Å molecular sieves in DCE (1.25 mL) was added tert-butyl 3-cyano-4-oxo-pyrrolidine-1-carboxylate (CAS# 175463-32-8; 44 mg, 0.21 mmol, 1.0 eq.) and NaBH(OAc)₃ (65 mg, 0.29 mmol, 1.4 eq.) and the mixture was stirred at RT for 18 h. The reaction medium was diluted with a sat. NaHCO₃ aq. solution and was extracted with DCM. The combined organic layers were dried over Na₂SO₄, filtered and concentrated to dryness. The residue was triturated in MeOH, the solid was filtered and dried to afford Int 1044.

### 1.42. Int 3007

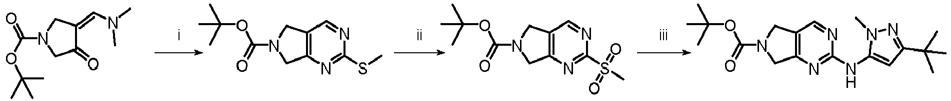

### 1.42.1. Step i

To a solution of S-methylisothiourea hemisulfate (CAS# 867-44-7; 8.51 g, 29.66 mmol, 1.5 eq.) in EtOH at 0 °C was added NaOEt (4.25 g, 59.30 mmol, 3.0 eq.) and the mixture was stirred for 15 min. Then Int 5011 (5 g, 19.77 mmol, 1.0 eq.) was added and the suspension was refluxed for 60 h. The reaction mixture was concentrated to dryness, the residue was taken up in water and extracted with EtOAc. The combined organic layers were dried over Na₂SO₄, filtered and concentrated to dryness. The residue was purified by flash chromatography on silica gel (eluting with cyclohexane/EtOAc) to afford tert-butyl 2-methylsulfanyl-5,7 -dihydropyrrolo[3,4-d]pyrimidine-6-carboxylate.
LCMS: MW (calcd): 267.4; m/z MW (obsd): 268.2 (M+H)

### 1.42.2. Step ii

To a solution of tert-butyl 2-methylsulfanyl-5,7-dihydropyrrolo[3,4-d]pyrimidine-6-carboxylate (2.5 g, 9.16 mmol, 1.0 eq.) in DCM (300 mL) at -5° C was added mCPBA (75% purity, 4.64 g, 20.16 mmol, 2.2 eq.) and the mixture was stirred at RT for 20 h. The reaction medium was diluted with a 0.2N NaOH aq. solution and extracted with DCM. The phases were separated using a phase separator and the filtrate was evaporated to afford tert-butyl 2-methylsulfonyl-5,7-dihydropyrrolo[3,4-d]pyrimidine-6-carboxylate.
LCMS: MW (calcd): 299.4; m/z MW (obsd): 300.1 (M+H)

### 1.42.3. Step iii

To a solution of tert-butyl 2-methylsulfonyl-5,7-dihydropyrrolo[3,4-d]pyrimidine-6-carboxylate (300 mg, 0.95 mmol, 1.0 eq.) and 5-tert-butyl-2-methyl-pyrazol-3-amine (CAS# 118430-73-2; 372 mg, 2.38 mmol, 2.5 eq.) in DMF (5 mL) at -78°C was added dropwise LiHMDS (1 M in THF, 2.39 mL, 2.39 mmol, 2.5 eq.) and the mixture was stirred between -60°C and -40°C for 2 h. The reaction medium was then poured into brine and extracted with EtOAc. The combined organic layers were dried over Na₂SO₄, filtered and concentrated to dryness. The residue was purified by flash chromatography on silica gel (eluting with cyclohexane/EtOAc) to afford Int 3007.

### 1.43. Int 3037

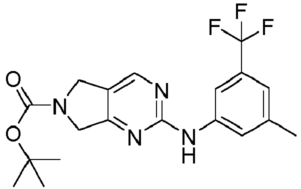

A mixture of Int 4011 (300 mg, 1.27 mmol, 1.0 eq.) and 1-bromo-3-methyl-5-(trifluoromethyl)benzene (CAS# 86845-28-5; 352 mg, 1.33 mmol, 1.05 eq.) in 1,4-dioxane (4.8 mL) was degassed with Ar before Xantphos (150 mg, 0.25 mmol, 0.2 eq.), Pd₂(dba)₃ (116 mg, 0.13 mmol, 0.1 eq.), and NaOtBu (366 mg, 3.81 mmol, 3.0 eq.) were added. The mixture was stirred at 100°C overnight, the medium was diluted with water and extracted with DCM. The pH of the aqueous layer was adjusted to 12 and the obtained basic phase was extracted with DCM. The combined organic layers were dried over Na₂SO₄, filtered and concentrated. The residue was purified by flash chromatography on silica gel (eluting with hexane/EtOAc) to afford Int 3037.

### 1.44. Int 2074

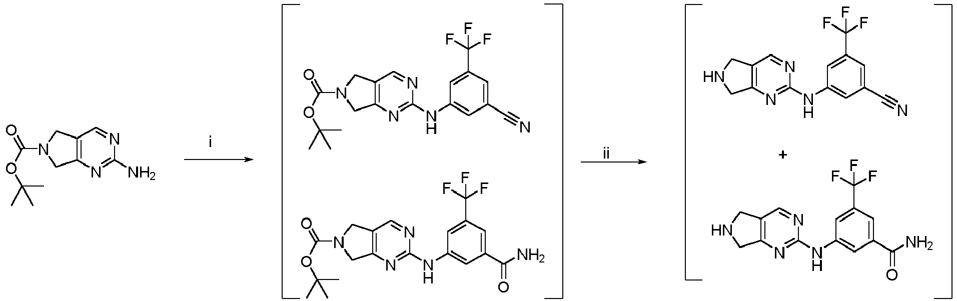

### 1.44.1. Step i

Int 4011 (500 mg, 2.12 mmol, 1.0 eq.), 3-bromo-5-(trifluoromethyl)benzonitrile (CAS# 691877-03-9; 582 mg, 2.33 mmol, 1.1 eq.), Xantphos (122 mg, 0.21 mmol, 0.1 eq.), Pd₂(dba)₃ (97 mg, 0.11 mmol, 0.05 eq.) and NaOtBu (610 mg, 6.35 mmol, 3.0 eq.) were placed in 1,4-dioxane (8.5 mL) and the mixture was degassed by N₂ bubbling. Then the reaction mixture was heated at 100°C for 18 h. More Xantphos (122 mg, 0.21 mmol, 0.1 eq.) and Pd₂(dba)₃ (97 mg, 0.11 mmol, 0.05 eq.) were added and the reaction was stirred at 100°C for 2 h. Celpure® P65 was added to the reaction medium that was stirred at RT for 30 min before being filtered on Celpure® P65. The cake was washed with EtOAc/MeOH (9/1) and the filtrate was concentrated. Water was added to the residue, the gum was triturated, filtered and washed with water and Et₂O to afford a mixture of tert-butyl 2-[3-cyano-5-(trifluoromethyl)anilino]-5,7-dihydropyrrolo[3,4-d]pyrimidine-6-carboxylate and tert-butyl 2-[3-carbamoyl-5-(trifluoromethyl)anilino]-5,7-dihydropyrrolo[3,4-d]pyrimidine-6-carboxylate.
LCMS: MW (calcd): 405.4 and 423.4; m/z MW (obsd): 406.4 (M+H) and 424.5 (M+H)

### 1.44.2. Step ii

To a solution of tert-butyl 2-[3-cyano-5-(trifluoromethyl)anilino]-5,7-dihydropyrrolo[3,4-d]pyrimidine-6-carboxylate and tert-butyl 2-[3-carbamoyl-5-(trifluoromethyl)anilino]-5,7-dihydropyrrolo[3,4-d]pyrimidine-6-carboxylate (860 mg, 2.12 mmol, 1.0 eq.) in 1,4-dioxane (10 mL) was added HCl (4N in 1,4-dioxane, 10 mL) and the mixture was stirred at RT for 18 h. Dioxane and water were added to the medium and the solvents were removed in vacuo. The residue was triturated in Et₂O and the solvent was evaporated. The resulting crude was taken up in water and the pH was adjusted to 10 with a 2N NaOH aq. solution. The resulting solid was filtered, washed with water and Et₂O, and dried to afford Int 2074 as a mixture of 3-(6,7-dihydro-5H-pyrrolo[3,4-d]pyrimidin-2-ylamino)-5-(trifluoromethyl)benzonitrile and 3-(6,7-dihydro-5H-pyrrolo[3,4-d]pyrimidin-2-ylamino)-5-(trifluoromethyl)benzamide.

### 1.45. Int 3011

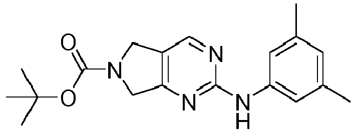

Int 4011 (11 g, 46.6 mmol, 1.0 eq.), 1-bromo-3,5-dimethyl-benzene (9 g, 48.9 mmol, 1.05 eq.), Xantphos (5.49 g, 9.31 mmol, 0.2 eq.), NaOtBu (13.8 g, 139.7 mmol, 3.0 eq.) and Pd₂(dba)₃ (4.35 g, 4.7 mmol, 0.1 eq.) were placed in 1,4-dioxane (150 mL) under Ar atmosphere and the mixture was vigorously stirred at 110°C for 3 h and at 80°C overnight. The reaction medium was poured into a well-stirred mixture of NaCl (250 g) in EtOAc/water (2/1, 1.5 L). The layers were separated and the aqueous phase was extracted with EtOAc. The combined organic layers were filtered over Celite®, dried over Na₂SO₄, filtered and concentrated. The residue was suspended in a EtOAc/cyclohexane mixture (1/1, 200 mL), the resulting precipitate was filtered and washed with a EtOAc/cyclohexane mixture (1/1). The solid was dried to afford Int 3011.

### 1.46. Int 3086

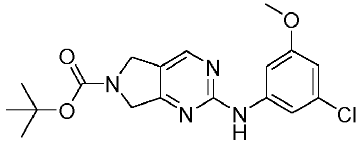

Int 4011 (2 g, 8.5 mmol, 1.0 eq.), 1-bromo-3-chloro-5-methoxy-benzene (CAS# 174913-12-3; 2.8 g, 12.7 mmol, 1.5 eq.), Xantphos (492 mg, 0.85 mmol, 0.1 eq.), Pd₂(dba)₃ (390 mg, 0.43 mmol, 0.05 eq.) and NaOtBu (2.5 g, 25.5 mmol, 3.0 eq.) were place in 1,4-dioxane (70 mL) and the mixture was degassed by N2 bubbling. Then the reaction mixture was heated to 100°C for 2 h. The solvents were removed in vacuo and the residue was taken up in EtOAc. The suspension was filtered on Celite®, the filtrate was concentrated and the residue was taken up in Et₂O. The precipitate was filtered, washed with Et₂O and dried to afford Int 3086.

### 1.47. Int 3087

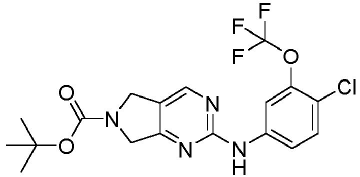

Int 4011 (2 g, 8.5 mmol, 1.0 eq.), 4-bromo-1-chloro-2-(trifluoromethoxy)benzene (CAS# 406232-79-9; 3.5 g, 12.7 mmol, 1.5 eq.), Xantphos (492 mg, 0.85 mmol, 0.1 eq.), Pd₂(dba)₃ (390 mg, 0.43 mmol, 0.05 eq.) and NaOtBu (2.5 g, 25.5 mmol, 3.0 eq.) were place in 1,4-dioxane (70 mL) and the mixture was degassed by N₂ bubbling. Then the reaction mixture was heated to 100°C for 2 h. The solvents were removed in vacuo and the residue was taken up in EtOAc. The suspension was filtered on Celite®, the filtrate was concentrated and the residue was taken up in Et₂O. The precipitate was filtered, washed with Et₂O and dried to afford Int 3087.

### 1.48. Int 4011

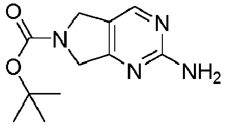

To a solution of Int 5011 (414 g, 1.73 mol, 1.0 eq.) in EtOH (8 L) was added guanidine carbonate (1240 g, 6.92 mol, 4.0 eq.) and NaOAc (942 g, 13.84 mol, 8.0 eq.) and the resulting mixture was stirred at reflux for 42 h. The reaction medium was cooled down to 5°C, the precipitate was filtered and washed with EtOH. The solid was collected and placed in a 2/1 water/CHCl₃ mixture. A precipitate was filtered off, the solid was triturated in water and CHCl₃ then dried to afford a first fraction of the expected product.

All the filtrates were combined, phases were separated and the aqueous phase was extracted with CHCl₃. The combined organic layers were washed with brine, concentrated and triturated in MeOH to afford a second fraction of the expected product. The 2 fractions of the expected product were gathered to afford Int 4011.

### 1.49. Cpd 3

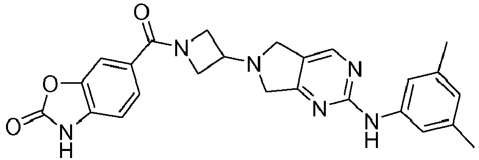

To a suspension of PS-carbodiimide resin (loading 1.33 mmol/g, 2.81 g, 3.74 mmol, 1.3 eq.) and HOBt hydrate (0.32 g, 2.01 mmol, 0.7 eq.) in DCM (85 mL) and DMF (8.5 mL) was added 2-oxo-3H-1,3-benzoxazole-6-carboxylic acid (CAS# 54903-16-1; 2.54 g, 2.88 mmol, 1.0 eq.) and the mixture was stirred at RT for 5 min. Then Int 13 (0.85 g, 2.88 mmol, 1.0 eq.) was added and the suspension was stirred at 70°C overnight. The reaction mixture was filtered through a phase separator, washed with DCM, MeOH and a 1/1 DCM/MeOH mixture. The filtrate was concentrated and the residue was taken up in a sat. NaHCO₃ aq. solution and extracted with DCM. The combined organic layers were dried over Na₂SO₄, filtered and concentrated. The residue was purified by flash chromatography on silica gel (eluting with DCM/MeOH) to afford a solid that was triturated in ACN to afford Cpd 3.

### 1.50. Cpd 25

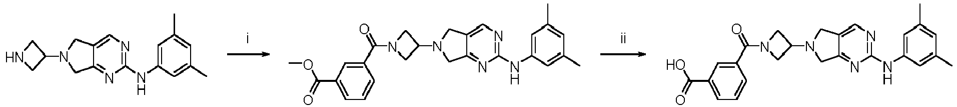

### 1.50.1. Step i

Methyl 3-[3-[2-(3,5-dimethylanilino)-5,7-dihydropyrrolo[3,4-d]pyrimidin-6-yl]azetidine-1-carbonyl]benzoate is obtained from Int 13 following general method A with 3-methoxycarbonylbenzoic acid.
LCMS: MW (calcd): 457.5; m/z MW (obsd): 458.4 (M+H)

### 1.50.2. Step ii

To a solution of methyl 3-[3-[2-(3,5-dimethylanilino)-5,7-dihydropyrrolo[3,4-d]pyrimidin-6-yl]azetidine-1-carbonyl]benzoate (60 mg, 0.13 mmol, 1.0 eq.) in t-BuOH (2.6 mL) was added NaOH (0.1N in water, 2.6 mL, 0.26 mmol, 2.0 eq.) and the mixture was stirred at 40°C for 3 h. The solvents were concentrated and the residue was taken up in water. The pH was adjusted to 6 with a 1N HCl aq. solution and the resulting solid was filtered, washed with water and dried. The solid was collected and triturated in DCM to afford Cpd 25.

### 1.51. Cpd 60

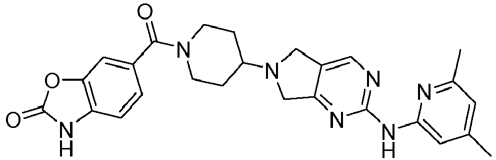

To PS-carbodiimide resin (loading 1.24 mmol/g, 162 mg, 0.2 mmol, 1.3 eq.) were added 2-oxo-3H-1,3-benzoxazole-6-carboxylic acid (29 mg, 0.15 mmol, 1.0 eq.) and HOBt hydrate (17 mg, 0.11 mmol, 0.7 eq.) in DCM (2.5 mL) and DMF (0.5 mL). The mixture was stirred at RT for 5 min. Then Int 38 (29 mg, 0.15 mmol, 1.0 eq.) in DCM (2.5 mL) was added, the suspension was stirred at RT for 15 h and at 40°C for 15 h. The reaction mixture was filtered through a phase separator, washed with DCM, MeOH and a 1/1 DCM/MeOH mixture. The filtrate was concentrated and the residue was taken up in a sat. NaHCO₃ aq. solution and extracted with DCM. The combined organic layers were dried over Na₂SO₄, filtered and concentrated. The residue was purified by preparative HPLC to afford Cpd 60.

### 1.52. Cpd 66

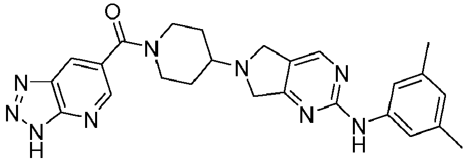

To PS-carbodiimide resin (loading 1.24 mmol/g, 162 mg, 0.2 mmol, 1.3 eq.) were added 3H-triazolo[4,5-b]pyridine-6-carboxylic acid (25 mg, 0.15 mmol, 1.0 eq.) and HOBt hydrate (17 mg, 0.11 mmol, 0.7 eq.) in DCM (2.5 mL) and DMF (0.5 mL). The mixture was stirred at RT for 5 min. Then Int 11 (50 mg, 0.15 mmol, 1.0 eq.) in DCM (2.5 mL) was added and the suspension was stirred at RT for 15 h. The reaction mixture was filtered and the resin was washed with DCM, MeOH and a 1/1 DCM/MeOH mixture. The filtrate was concentrated and the residue was taken up in a sat. NaHCO₃ aq. solution and extracted with DCM. The pH of the aqueous layer was adjusted to 7 with a 6N HCl aq. solution and extracted again with EtOAc. The combined organic layers were dried over Na₂SO₄, filtered and concentrated. The residue was purified by flash chromatography on silica gel (eluting with DCM/MeOH) to afford Cpd 66.

### 1.53. Cpd 67

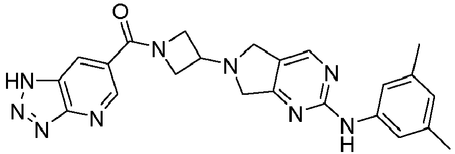

To PS-carbodiimide resin (loading 1.24 mmol/g, 177 mg, 0.22 mmol, 1.3 eq.) were added 3H-triazolo[4,5-b]pyridine-6-carboxylic acid (28 mg, 0.17 mmol, 1.0 eq.) and HOBt hydrate (18 mg, 0.12 mmol, 0.7 eq.) in DCM (2.5 mL) and DMF (0.5 mL). The mixture was stirred at RT for 5 min. Then Int 13 (50 mg, 0.17 mmol, 1.0 eq.) in DCM (2.5 mL) was added and the suspension was stirred at RT for 15 h. The reaction mixture was filtered and the resin was washed with DCM, MeOH and a 1/1 DCM/MeOH mixture. The filtrate was concentrated and the residue was taken up in a sat. NaHCO₃ aq. solution and extracted with DCM. The pH of the aqueous layer was adjusted to 7 with a 6N HCl aq. solution and extracted again with DCM. The combined organic layers were dried over Na₂SO₄, filtered and concentrated. The residue was purified by flash chromatography on silica gel (eluting with DCM/MeOH) to afford Cpd 67.

### 1.54. Cpd 76

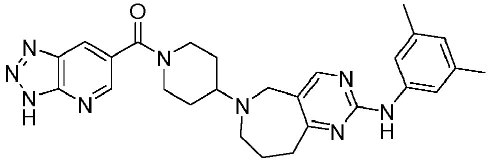

To PS-carbodiimide resin (loading 1.6 mmol/g, 132 mg, 0.19 mmol, 1.3 eq.) were added 3H-triazolo[4,5-b]pyridine-6-carboxylic acid (33 mg, 0.20 mmol, 1.0 eq.) and HOBt hydrate (21 mg, 0.14 mmol, 0.7 eq.) in DCM (2.5 mL) and DMF (0.5 mL) and the mixture was stirred at RT for 5 min. Then Int 43 (70 mg, 0.2 mmol, 1.0 eq.) in DCM (3.2 mL) and DMF (0.32 mL) was added and the suspension was stirred at RT for 15 h. The reaction mixture was filtered and the resin was washed with DCM, MeOH and a 1/1 DCM/MeOH mixture. The filtrate was concentrated and the residue was taken up in a sat. NaHCO₃ aq. solution and extracted with EtOAc. The pH of the aqueous layer was adjusted to 7 with a 6N HCl aq. solution and extracted again with DCM. The combined organic layers were washed with brine dried over Na₂SO₄, filtered and concentrated to afford Cpd 76.

### 1.55. Cpd 86

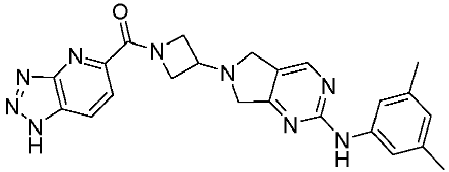

To PS-carbodiimide resin (loading 1.21 mmol/g, 239 mg, 0.29 mmol, 1.3 eq.) were added 3H-triazolo[4,5-b]pyridine-5-carboxylic acid (37 mg, 0.22 mmol, 1.1 eq.) and HOBt hydrate (24 mg, 0.16 mmol, 0.7 eq.) in DCM (3.25 mL) and DMF (0.325 mL) and the mixture was stirred at RT for 5 min. Then Int 13 (66 mg, 0.22 mmol, 1.0 eq.) in DCM (3.25 mL) and DMF (0.325 mL) was added and the suspension was stirred at RT for 4 days and at 40°C for 3 days. The reaction mixture was filtered and the resin was washed with DCM, MeOH and a 1/1 DCM/MeOH mixture. The filtrate was concentrated and the residue was taken up in a sat. NaHCO₃ aq. solution and extracted with DCM. The combined organic layers were washed with brine, dried over Na₂SO₄, filtered and concentrated. The residue was purified by flash chromatography on silica gel (eluting with DCM/MeOH) to afford Cpd 86.

### 1.56. Cpd 96

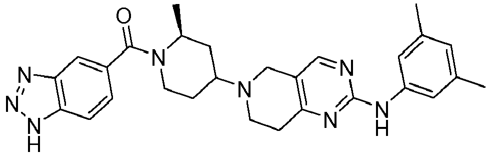

Int 62 (197 mg, 0.76 mmol, 1.1 eq.) and Int 2001 (176 mg, 0.69 mmol, 1.0 eq.) were placed in DCM (11 mL) and AcOH (21 µL, 0.35 mmol, 0.5 eq.) and activated 4 Å molecular sieves (373 mg) were added. The mixture was stirred at RT for 1 h, then NaBH(OAc)₃ (205 mg, 0.97 mmol, 1.4 eq.) was added and the reaction was stirred at RT overnight. The medium was hydrolysed with a sat. NaHCO₃ aq. solution and was extracted with DCM. The combined organic layers were dried over Na₂SO₄, filtered and concentrated to dryness. The residue was taken up in DCM and washed with 1N HCl aq. solution. The aqueous phase was basified with a 2N NaOH aq. solution, was washed with EtOAc and extracted with n-BuOH. The combined n-BuOH layers were dried over Na₂SO₄, filtered and concentrated to dryness. The residue was purified by flash chromatography on silica gel (eluting with DCM/MeOH) to afford Cpd 96.

### 1.57. Cpd 97

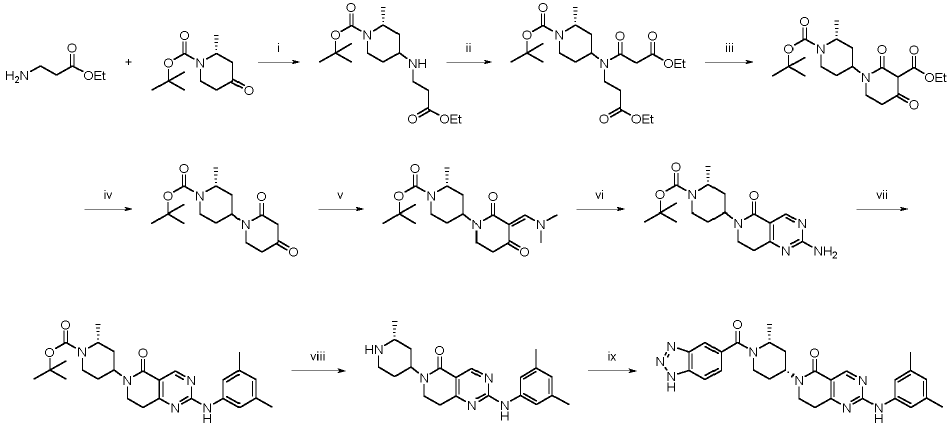

### 1.57.1. Step i

To a solution of tert-butyl (2R)-2-methyl-4-oxo-piperidine-1-carboxylate (4.0 g, 18.76 mmol, 1.0 eq.) in DCM (80 mL) were added activated 4 Å molecular sieves (7 g), ethyl 3-aminopropanoate (3.17 g, 20.63 mmol, 1.1 eq.), NaOAc (1.77 g, 21.57 mmol, 1.2 eq.) and NaBH(OAc)₃ portionwise (5.96 g, 28.14 mmol, 1.5 eq.). The suspension was stirred at RT for 20 h before being filtered and the solid washed with DCM. The filtrate was concentrated to dryness. The residue was purified by flash chromatography on silica gel (eluting with DCM/MeOH) to afford the desired product.
LCMS: MW (calcd): 314.4; m/z MW (obsd): 315.5 (M+H)

### 1.57.2. Step ii

To a solution of tert-butyl (2R)-4-[(3-ethoxy-3-oxo-propyl)amino]-2-methyl-piperidine-1-carboxylate (3.4 g, 10.81 mmol, 1.0 eq.) in DCM (40 mL) at 0°C were added ethyl 3-chloro-3-oxo-propanoate (1.77 g, 12.98 mmol, 1.2 eq.) and TEA (1.8 mL, 12.98 mmol, 1.2 eq.) and the mixture was stirred at RT for 1 h 45. The reaction medium was then diluted with DCM and washed with water. The organic layer was dried over MgSO₄, filtered and concentrated to dryness. The residue was purified by flash chromatography on silica gel (eluting with heptane/EtOAc) to afford tert-butyl (2R)-4-[(3-ethoxy-3-oxo-propanoyl)-(3-ethoxy-3-oxo-propyl)amino]-2-methyl-piperidine-1-carboxylate.
LCMS: MW (calcd): 428.5; m/z MW (obsd): 429.6 (M+H)

### 1.57.3. Step iii

A solution of NaOEt in EtOH was freshly prepared by dissolution of 1.45 g of sodium in 150 mL of EtOH. Tert-butyl (2R)-4-[(3-ethoxy-3-oxo-propanoyl)-(3-ethoxy-3-oxo-propyl)amino]-2-methylpiperidine-1-carboxylate (3.8 g, 8.87 mmol, 1.0 eq.) was dissolved in 22 mL of the above NaOEt solution in EtOH (1.05 eq.) and the mixture was refluxed for 30 min. The reaction medium was then diluted with DCM and washed with a 0.5 M HCl aq. solution and with water. The organic layer was dried over MgSO₄, filtered and concentrated to dryness to afford ethyl 1-[(2R)-1-tert-butoxycarbonyl-2-methyl-4-piperidyl]-2,4-dioxo-piperidine-3-carboxylate.
LCMS: MW (calcd): 382.3; m/z MW (obsd): 383.6 (M+H)

### 1.57.4. Step iv

To a solution of ethyl 1-[(2R)-1-tert-butoxycarbonyl-2-methyl-4-piperidyl]-2,4-dioxo-piperidine-3-carboxylate (1 g, 2.62 mmol, 1.0 eq.) in ACN (150 mL) was added water (150 mL) and the mixture was refluxed for 1 h. The reaction mixture was concentrated to dryness and the residue was purified by flash chromatography on silica gel (eluting with heptane/EtOAc) to afford tert-butyl (2R)-4-(2,4-dioxo-1-piperidyl)-2-methyl-piperidine-1 -carboxylate.
LCMS: MW (calcd): 310.4; m/z MW (obsd): 311.5 (M+H)

### 1.57.5. Step v

Tert-butyl (2R)-4-[(3E)-3-(dimethylaminomethylene)-2,4-dioxo-1-piperidyl]-2-methylpiperidine-1-carboxylate is obtained from tert-butyl (2R)-4-(2,4-dioxo-1-piperidyl)-2-methyl-piperidine-1-carboxylate following general method F.
LCMS: MW (calcd): 365.5; m/z MW (obsd): 366.5 (M+H)

### 1.57.6. Step vi

Tert-butyl (2R)-4-(2-amino-5-oxo-7,8-dihydropyrido[4,3-d]pyrimidin-6-yl)-2-methyl-piperidine-1-carboxylate is obtained from tert-butyl (2R)-4-[(3E)-3-(dimethylaminomethylene)-2,4-dioxo-1-piperidyl]-2-methyl-piperidine-1-carboxylate following general method E.
LCMS: MW (calcd): 361.5; m/z MW (obsd): 362.5 (M+H)

### 1.57.7. Step vii

Tert-butyl (2R)-4-[2-(3,5-dimethylanilino)-5-oxo-7,8-dihydropyrido[4,3-d]pyrimidin-6-yl]-2-methyl-piperidine-1-carboxylate is obtained from tert-butyl (2R)-4-(2-amino-5-oxo-7,8-dihydropyrido[4,3-d]pyrimidin-6-yl)-2-methyl-piperidine-1-carboxylate following general method D with 1-bromo-3,5-dimethyl-benzene.
LCMS: MW (calcd): 465.6; m/z MW (obsd): 466.6 (M+H)

### 1.57.8. Step viii

2-(3,5-dimethylanilino)-6-[(2R)-2-methyl-4-piperidyl]-7,8-dihydropyrido[4,3-d]pyrimidin-5-one is obtained from tert-butyl (2R)-4-(2-amino-5-oxo-7,8-dihydropyrido[4,3-d]pyrimidin-6-yl)-2-methylpiperidine-1-carboxylate following general method B.
LCMS: MW (calcd): 365.5; m/z MW (obsd): 366.5 (M+H)

### 1.57.9. Step ix

Cpd 97 is obtained from 2-(3,5-dimethylanilino)-6-[(2R)-2-methyl-4-piperidyl]-7,8-dihydropyrido[4,3-d]pyrimidin-5-one and 1H-benzotriazole-5-carboxylic acid following general method A.

### 1.58. Cpd 100

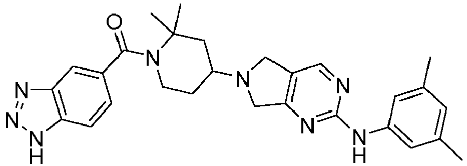

To a solution of Int 52 (50 mg, 0.14 mmol, 1.0 eq.) in DMF (3 mL) were added TEA (138 µL, 0.99 mmol, 7.0 eq.), DMAP (1 mg, 0.003 mmol, 0.02 eq.) and 1H-benzotriazole-5-carbonyl chloride (CAS# 46053-85-4; 65 mg, 0.36 mmol, 2.5 eq.) and the mixture was stirred at RT for 3 days. Then 1H-benzotriazole-5-carbonyl chloride, TEA and DMAP were added in the same amounts as previously and the mixture was stirred at RT overnight. The reaction medium was diluted with a sat. NaHCO₃ aq. solution and extracted with EtOAc. The combined organic layers were dried over Na₂SO₄, filtered and concentrated to dryness. The residue was purified by flash chromatography on silica gel (eluting with heptane/EtOAc) to afford Cpd 100.

### 1.59. Cpd 101

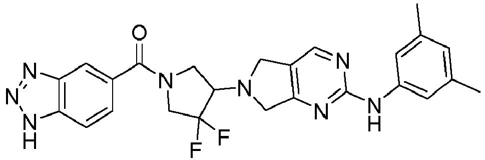

Int 85 (62 mg, 0.18 mmol, 1.0 eq.), 1H-benzotriazole-5-carboxylic acid (36 mg, 0.22 mmol, 1.25 eq.), HATU (85 mg, 0.22 mmol, 1.25 eq.) and TEA (175 µL, 1.25 mmol, 7.0 eq.) were placed in DMF (2 mL) and the mixture was stirred at RT overnight. The reaction medium was diluted with a sat. NaHCO₃ aq. solution and extracted with EtOAc. The combined organic layers were dried over Na₂SO₄, filtered and concentrated to dryness. The residue was purified by flash chromatography on silica gel (eluting with DCM/MeOH) to afford a residue that was taken up in EtOAc and washed several times with a sat. NaHCO₃ aq. solution. The combined organic layers were dried over MgSO₄, filtered and concentrated to dryness to afford Cpd 101.

### 1.60. Cpd 104

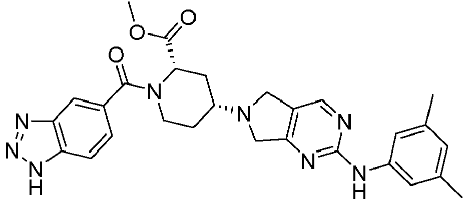

Int 88 (301 mg, 0.72 mmol, 1.0 eq.), 1H-benzotriazole-5-carboxylic acid (147 mg, 0.90 mmol, 1.25 eq.), HATU (342 mg, 0.90 mmol, 1.25 eq.) and TEA (301 µL, 2.16 mmol, 3.0 eq.) were placed in DMF (3 mL) and the mixture was stirred at RT overnight. The reaction medium was diluted with a sat. NaHCO₃ aq. solution and extracted with EtOAc. The combined organic layers were dried over Na₂SO₄, filtered and concentrated to dryness. The residue was purified by flash chromatography on silica gel (eluting with DCM/MeOH) to afford a residue that was taken up in EtOAc and washed several times with a sat. NaHCO₃ aq. solution. The combined organic layers were dried over MgSO₄, filtered and concentrated to dryness to afford Cpd 104.

### 1.61. Cpd 108

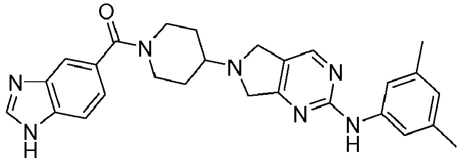

A solution of Int 73 (0.37 g, 1.52 mmol, 1.0 eq.) and Int 2011 (0.46 g, 1.9 mmol, 1.25 eq.) in DME (8 mL) and 3 drops of AcOH was stirred at RT overnight. Then NaBH(OAc)₃ (805 mg, 3.8 mmol, 2.5 eq.) was added and the mixture was stirred at RT for 2 h. The solvents were concentrated, the residue was taken up in DCM and washed with water. The organic phase was dried over Na₂SO₄, filtered and concentrated. The residue was purified by flash chromatography on silica gel (eluting with DCM/MeOH) to afford Cpd 108.

### 1.62. Cpd 110 and Cpd 111

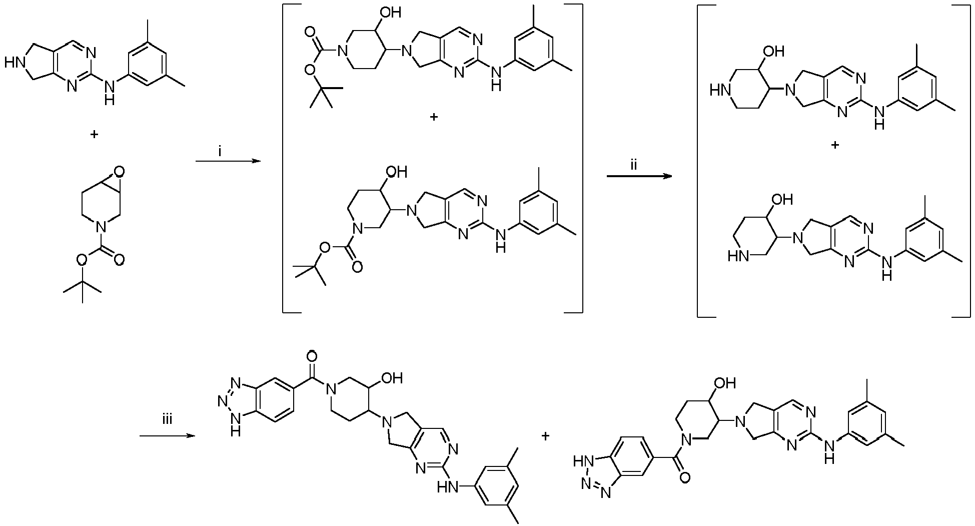

### 1.62.1. Step i

Tert-butyl 7-oxa-3-azabicyclo[4.1.0]heptane-3-carboxylate (CAS# 161157-50-2; 400 mg, 2.01 mmol, 0.95 eq.) and Int 2011 (508 mg, 2.12 mmol, 1.0 eq.) were dissolved in ACN (21 mL) and Libr (368 mg, 4.23 mmol, 2.0 eq.) was added. The mixture was stirred at 100°C for 96 h and the medium was filtered on Celite®. The filtrate was concentrated and the residue was purified by flash chromatography on silica gel (eluting with DCM/MeOH). The fractions containing the expected product were treated with activated charcoal, filtered on Celite® and concentrated to afford a mixture of tert-butyl 4-[2-(3,5-dimethylanilino)-5,7-dihydropyrrolo[3,4-d]pyrimidin-6-yl]-3-hydroxy-piperidine-1-carboxylate and tert-butyl 3-[2-(3,5-dimethylanilino)-5,7-dihydropyrrolo[3,4-d]pyrimidin-6-yl]-4-hydroxy-piperidine-1-carboxylate (regioisomers).
LCMS: MW (calcd): 439.6; m/z MW (obsd): 440.5 (M+H) under 2 peaks

### 1.62.2. Step ii

To a suspension of tert-butyl 4-[2-(3,5-dimethylanilino)-5,7-dihydropyrrolo[3,4-d]pyrimidin-6-yl]-3-hydroxy-piperidine-1-carboxylate and tert-butyl 3-[2-(3,5-dimethylanilino)-5,7-dihydropyrrolo[3,4-d]pyrimidin-6-yl]-4-hydroxy-piperidine-1-carboxylate (38 mg, 0.09 mmol, 1.0 eq.) in 1,4-dioxane (0.2 mL) was added HCl (4N in 1,4-dioxane, 0.3 mL, 0.23 mmol, 15 eq.) and the mixture was stirred at RT for 20 min. The reaction medium was concentrated to dryness and the residue was triturated in Et₂O. Et₂O was evaporated to afford a mixture of 4-[2-(3,5-dimethylanilino)-5,7-dihydropyrrolo[3,4-d]pyrimidin-6-yl]piperidin-3-ol and 3-[2-(3,5-dimethylanilino)-5,7-dihydropyrrolo[3,4-d]pyrimidin-6-yl]piperidin-4-ol as bis HCl salts

### 1.62.3. Step iii

A mixture of 4-[2-(3,5-dimethylanilino)-5,7-dihydropyrrolo[3,4-d]pyrimidin-6-yl]piperidin-3-ol, bis HCl salt and 3-[2-(3,5-dimethylanilino)-5,7-dihydropyrrolo[3,4-d]pyrimidin-6-yl]piperidin-4-ol, bis HCl salt (36 mg, 0.086 mmol, 1.0 eq.) and 1H-benzotriazole-5-carboxylic acid (18 mg, 0.11 mmol, 1.25 eq.) were placed in DMF (1.1 mL). TEA (60 µL, 0.43 mmol, 5.0 eq.) and HATU (41 mg, 0.11 mmol, 1.25 eq.) were added. The mixture was stirred at RT for 1 h and the medium was concentrated to dryness. The residue was taken up in EtOAc, the precipitate was filtered and washed with DCM. The filtrates were concentrated and the crude mixture was purified by flash chromatography on silica gel (eluting with DCM/MeOH). The obtain residue was then purified by preparative HPLC to afford Cpd 110 (first eluting) and Cpd 111 (second eluting).

### 1.63. Cpd 115

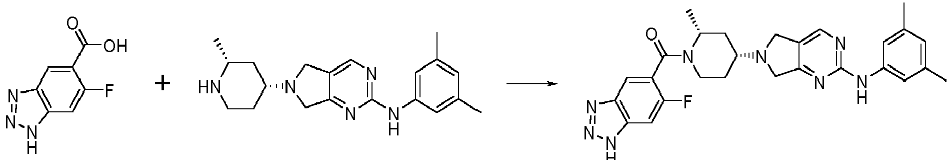

6-fluoro-1H-benzotriazole-5-carboxylic acid (CAS# 1427081-62-6; 5.4 g, 29.8 mmol, 1.0 eq.), HATU (11.4 g, 29.8 mmol, 1.0 eq.), HOAt (2.0 g, 14.9 mmol, 0.5 eq.) and tetramethyl piperidine (10.5 g, 74.5 mmol, 2.5 eq.) were dissolved in DMF (100 mL). The reaction mixture was stirred at RT for 5 min. Int 39 (10.1 g, 30.0 mmol, 1.03 eq.) was then added and the mixture stirred at RT overnight. The reaction mixture was poured on a sat. NH₄Cl aq. solution (400 mL) and stirred for 40 min in an ice bath. The precipitate was filtered off, rinsed with water, dried under suction for 1 h and under vacuum at 40 °C to yield a crude material. The crude product was purified by chromatography on silica gel (eluting with DCM:MeOH:NH₄OH=90:9:1.5). The fractions of interest were pooled and concentrated under reduced pressure. The solid material obtained was triturated in acetone (20 Vol.) at 0 °C for 40 min, filtered, dried under vacuum for 4 h to afford Cpd 115.

### 1.64. Cpd 117

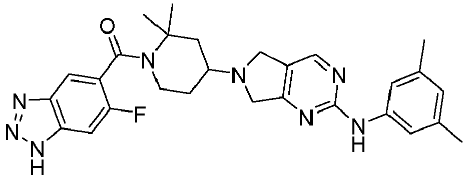

To a solution of Int 52 (100 mg, 0.28 mmol, 1.0 eq.) and Int 57 (220 mg, 1.10 mmol, 3.8 eq.) in DMF were added DMAP (0.6 mg, 0.006 mmol, 0.02 eq.) and TEA (276 µL, 1.99 mmol, 7.0 eq.) and the mixture was stirred at RT for 48 h. The reaction medium was quenched with a mixture of sat. NaHCO₃ aq. solution and brine and was then extracted with EtOAc. The combined organic layers were dried over Na₂SO₄, filtered and concentrated to dryness. The residue was purified by flash chromatography on silica gel (eluting with DCM/EtOAc) to afford a solid that was triturated in DCM to afford Cpd 117.

### 1.65. Cpd 118

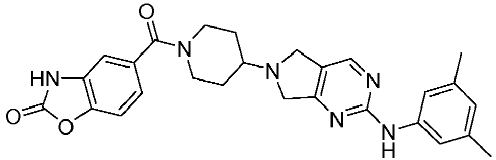

To a solution of Int 81 (81 mg, 0.31 mmol, 1.0 eq.) and Int 2011 (75 mg, 0.31 mmol, 1.0 eq.) in DCM (3 mL) were added activated 4 Å molecular sieves (113 mg), AcOH (75 mg, 1.25 mmol, 4.0 eq.) and NaBH(OAc)₃ (93 mg, 0.44 mmol, 1.4 eq.). The mixture was stirred at RT overnight before being filtered and quenched with water. The medium was then extracted with DCM, the combined organic layers were washed with brine, dried over Na₂SO₄, filtered and concentrated to dryness. The crude mixture was purified by flash chromatography on silica gel (eluting with DCM/MeOH) followed by a preparative HPLC to afford a residue that was then taken up in EtOH and concentrated several times. The obtained solid was then triturated in minimum DCM to afford Cpd 118.

### 1.66. Cpd 121

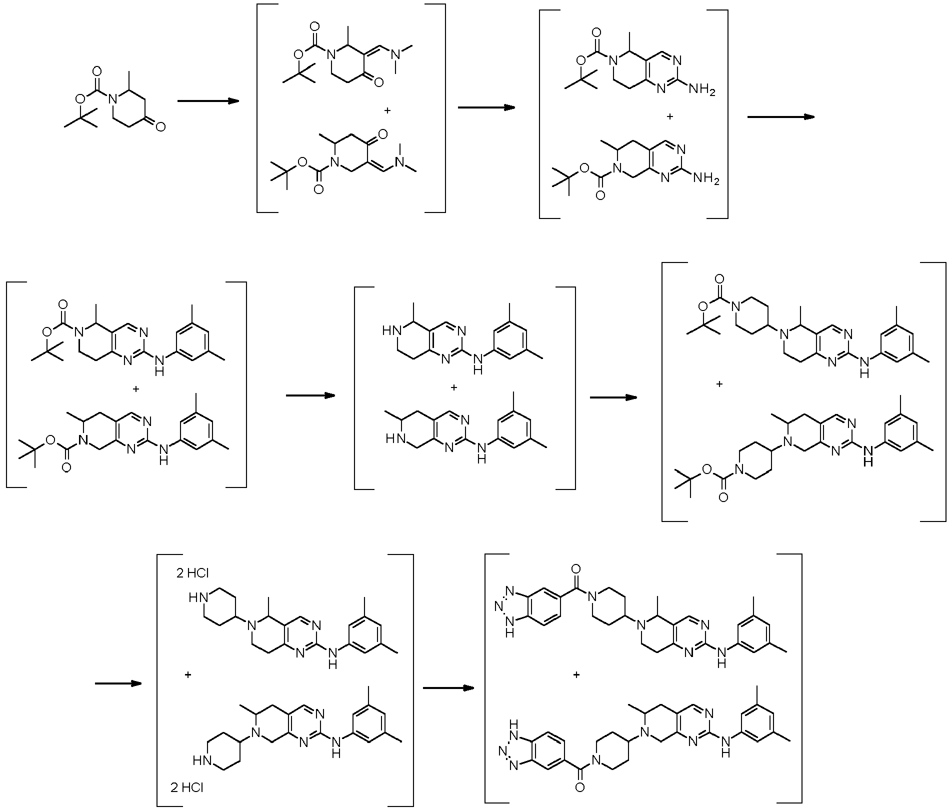

### 1.66.1. Step i

A mixture of tert-butyl (3Z)-3-(dimethylaminomethylene)-2-methyl-4-oxo-piperidine-1-carboxylate and tert-butyl (5Z)-5-(dimethylaminomethylene)-2-methyl-4-oxo-piperidine-1-carboxylate is obtained from tert-butyl 2-methyl-4-oxo-piperidine-1-carboxylate following general method F with 2 purifications by flash chromatography on silica gel.
LCMS: MW (calcd): 268.4; m/z MW (obsd): 269.4 (M+H)

### 1.66.2. Step ii

A mixture of tert-butyl (3Z)-3-(dimethylaminomethylene)-2-methyl-4-oxo-piperidine-1-carboxylate and tert-butyl (5Z)-5-(dimethylaminomethylene)-2-methyl-4-oxo-piperidine-1-carboxylate (2.1 g, 7.63 mmol, 1.0 eq.) was dissolved in EtOH (30 mL) and guanidine carbonate (2.9 g, 32.05 mmol, 4.2 eq.) and NaOAc (5.9 g, 72.50 mmol, 9.5 eq.) were added. The mixture was refluxed for 1 h, the reaction medium was filtered through Celite® and the Celite® pad was rinsed with EtOAc. The filtrate was concentrated to dryness, the residue was taken up in EtOAc and washed with brine. The combined organic layers were washed with brine, dried over MgSO₄, filtered and concentrated to dryness to afford a mixture of tert-butyl 2-amino-5-methyl-7,8-dihydro-5H-pyrido[4,3-d]pyrimidine-6-carboxylate and tert-butyl 2-amino-6-methyl-6,8-dihydro-5H-pyrido[3,4-d]pyrimidine-7-carboxylate.
LCMS: MW (calcd): 264.3; m/z MW (obsd): 265.1 (M+H)

### 1.66.3. Step iii

A mixture of of tert-butyl 2-(3,5-dimethylanilino)-5-methyl-7,8-dihydro-5H-pyrido[4,3-d]pyrimidine-6-carboxylate and tert-butyl 2-(3,5-dimethylanilino)-6-methyl-6,8-dihydro-5H-pyrido[3,4-d]pyrimidine-7-carboxylate was obtained from a mixture of tert-butyl 2-amino-5-methyl-7,8-dihydro-5H-pyrido[4,3-d]pyrimidine-6-carboxylate and tert-butyl 2-amino-6-methyl-6,8-dihydro-5H-pyrido[3,4-d]pyrimidine-7-carboxylate following general method D with 3,5-dimethylbromobenzene .
LCMS: MW (calcd): 368.5; m/z MW (obsd): 369.2 (M+H)

### 1.66.4. Step iv

A mixture of tert-butyl 2-(3,5-dimethylanilino)-5-methyl-7,8-dihydro-5H-pyrido[4,3-d]pyrimidine-6-carboxylate and tert-butyl 2-(3,5-dimethylanilino)-6-methyl-6,8-dihydro-5H-pyrido[3,4-d]pyrimidine-7-carboxylate (822 mg, 2.23 mmol, 1.0 eq.) was dissolved in 1,4-dioxane (4 mL) and HCl (4N in 1,4-dioxane, 8.4 mL, 33.46 mmol, 15.0 eq.) was added. The mixture was stirred at RT for 1h45 and the reaction medium was concentrated to dryness. The residue was taken up in Et₂O and concentrated to dryness. The crude mixture was taken up in water, basified to pH 9 using a 2N NaOH aq. solution and the precipitate was filtered. The obtained solid was dissolved in MeOH, solvent was removed and the crude was taken up in a DCM/pentane 3/7 mixture. The medium was concentrated to dryness to afford a mixture of N-(3,5-dimethylphenyl)-5-methyl-5,6,7,8-tetrahydropyrido[4,3-d]pyrimidin-2-amine and N-(3,5-dimethylphenyl)-6-methyl-5,6,7,8-tetrahydropyrido[3,4-d]pyrimidin-2-amine as free bases.
LCMS: MW (calcd): 268.4; m/z MW (obsd): 269.0 (M+H)

### 1.66.5. Step v

A mixture ofN-(3,5-dimethylphenyl)-5-methyl-5,6,7,8-tetrahydropyrido[4,3-d]pyrimidin-2-amine and N-(3,5-dimethylphenyl)-6-methyl-5,6,7,8-tetrahydropyrido[3,4-d]pyrimidin-2-amine as free bases (80 mg, 0.30 mmol) was placed in DCM (3 mL) with tert-butyl 4-oxopiperidine-1-carboxylate (65 mg, 0.33 mmol, 1.1 eq.). Then activated 4 Å molecular sieves (200 mg), AcOH (0.1 eq.) and NaBH(OAc)₃ (126 mg, 0.60 mmol, 2.0 eq.) were added and the suspension was stirred at RT for 20 h. Then more tert-butyl 4-oxopiperidine-1-carboxylate (30 mg, 0.15 mmol, 0.5 eq.) and NaBH(OAc)₃ (63 mg, 0.30 mmol, 1.0 eq.) were added and the mixture was stirred for another 72 h. The reaction medium was filtered over Celite® that was rinsed with EtOAc. The filtrate was washed with brine, a sat. NaHCO₃ aq. solution and water. The organic layer was dried over MgSO₄, filtered and concentrated to dryness. The crude mixture was purified by flash chromatography on silica gel (eluting with heptaneheptane/EtOAc) to afford a mixture of tert-butyl 4-[2-(3,5-dimethylanilino)-5-methyl-7,8-dihydro-5H-pyrido[4,3-d]pyrimidin-6-yl]piperidine-1-carboxylate and tert-butyl 4-[2-(3,5-dimethylanilino)-6-methyl-6,8-dihydro-5H-pyrido[3,4-d]pyrimidin-7-yl]piperidine-1-carboxylate.
LCMS: MW (calcd): 451.6; m/z MW (obsd): 452.6 (M+H)

### 1.66.6. Step vi

A mixture of N-(3,5-dimethylphenyl)-5-methyl-6-(4-piperidyl)-7,8-dihydro-5H-pyrido[4,3-d]pyrimidin-2-amine and N-(3,5-dimethylphenyl)-6-methyl-7-(4-piperidyl)-6,8-dihydro-5H-pyrido[3,4-d]pyrimidin-2-amine as bis HCl salts is obtained from a mixture of tert-butyl 4-[2-(3,5-dimethylanilino)-5-methyl-7,8-dihydro-5H-pyrido[4,3-d]pyrimidin-6-yl]piperidine-1-carboxylate and tert-butyl 4-[2-(3,5-dimethylanilino)-6-methyl-6,8-dihydro-5H-pyrido[3,4-d]pyrimidin-7-yl]piperidine-1-carboxylate following general method B.

### 1.66.7. Step vii

A mixture of N-(3,5-dimethylphenyl)-5-methyl-6-(4-piperidyl)-7,8-dihydro-5H-pyrido[4,3-d]pyrimidin-2-amine and N-(3,5-dimethylphenyl)-6-methyl-7-(4-piperidyl)-6,8-dihydro-5H-pyrido[3,4-d]pyrimidin-2-amine (74 mg, 0.16 mmol, 1.0 eq.) was placed in DMF (2 mL) with 1H-benzotriazole-5-carboxylic acid (33 mg, 0.21 mmol, 1.25 eq.), TEA (114 µL, 0.82 mmol, 5.0 eq.) and HATU (78 mg, 0.21 mmol, 1.25 eq.). The mixture was stirred at RT for 12 h then NaOH (2 M in water, 200 µL) was added and the reaction medium was dry loaded on a silica gel column for flash chromatography purification (eluting with DCM/MeOH). The obtained residue was purified by preparative HPLC to afford Cpd 121 as a mixture of regioisomers.

### 1.67. Cpd 122

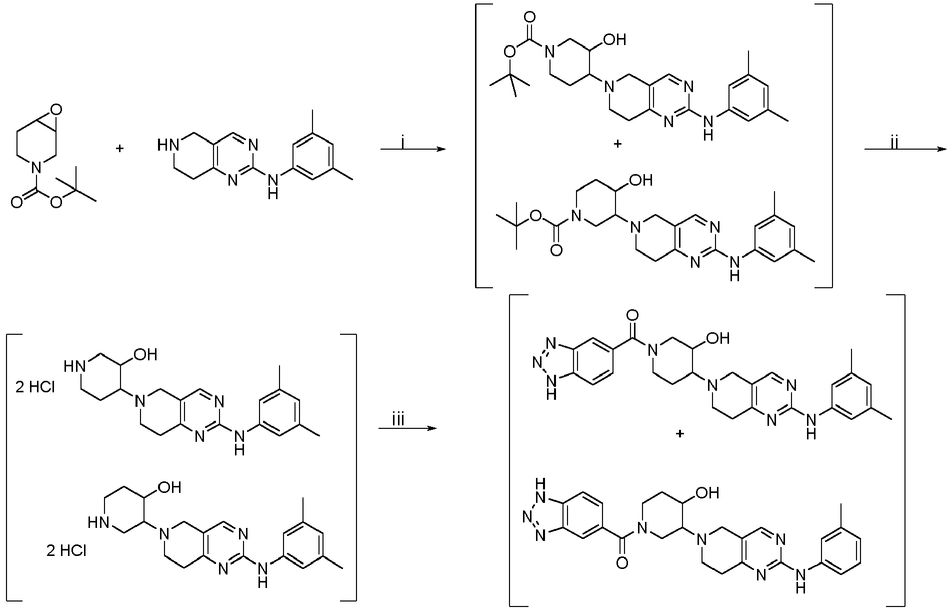

### 1.67.1. Step i

Tert-butyl 7-oxa-3-azabicyclo[4.1.0]heptane-3-carboxylate (CAS# 161157-50-2; 200 mg, 1.00 mmol, 1.5 eq.) and Int 2001 (170 mg, 0.67 mmol, 1.0 eq.) were dissolved in ACN (6.7 mL) and Libr (174 mg, 2.01 mmol, 2.0 eq.) was added. The mixture was stirred at 100°C for 96 h and the medium was filtered on Celite®. The filtrate was concentrated and the residue was purified by flash chromatography on silica gel (eluting with heptaneheptane/EtOAc) to afford a mixture of regioisomers tert-butyl 4-[2-(3,5-dimethylanilino)-7,8-dihydro-5H-pyrido[4,3-d]pyrimidin-6-yl]-3-hydroxy-piperidine-1-carboxylate and tert-butyl 3-[2-(3,5-dimethylanilino)-7,8-dihydro-5H-pyrido[4,3-d]pyrimidin-6-yl]-4-hydroxy-piperidine-1-carboxylate.
LCMS: MW (calcd): 453.6; m/z MW (obsd): 454.6 (M+H) under 2 peaks

### 1.67.2. Step ii

A mixture of 4-[2-(3,5-dimethylanilino)-7,8-dihydro-5H-pyrido[4,3-d]pyrimidin-6-yl]piperidin-3-ol and 3-[2-(3,5-dimethylanilino)-7,8-dihydro-5H-pyrido[4,3-d]pyrimidin-6-yl]piperidin-4-ol, bis HCl salts is obtained from a mixture of tert-butyl 4-[2-(3,5-dimethylanilino)-7,8-dihydro-5H-pyrido[4,3-d]pyrimidin-6-yl]-3-hydroxy-piperidine-1-carboxylate and tert-butyl 3-[2-(3,5-dimethylanilino)-7,8-dihydro-5H-pyrido[4,3-d]pyrimidin-6-yl]-4-hydroxy-piperidine-1-carboxylate following general method B.
LCMS: MW (calcd): 353.5; m/z MW (obsd): 354.5 (M+H) under 2 peaks

### 1.67.3. Step iii

4-[2-(3,5-dimethylanilino)-7,8-dihydro-5H-pyrido[4,3-d]pyrimidin-6-yl]piperidin-3-ol and 3-[2-(3,5-dimethylanilino)-7,8-dihydro-5H-pyrido[4,3-d]pyrimidin-6-yl]piperidin-4-ol, bis HCl salts (155 mg, 0.36 mmol, 1.0 eq.) were placed in DMF (4.6 mL) with 1H-benzotriazole-5-carboxylic acid (74 mg, 0.46 mmol, 1.25 eq.), TEA (254 µL, 1.82 mmol, 5.0 eq.) and HATU (173 mg, 0.46 mmol, 1.25 eq.). The mixture was stirred at RT for 12 h then NaOH (2 M in water, 700 µL) was added and the reaction medium was concentrated to dryness. The crude mixture was purified by flash chromatography on silica gel (eluting with DCM/MeOH) followed by preparative HPLC to afford Cpd 122 as a mixture of regioisomers.

### 1.68. Cpd 124

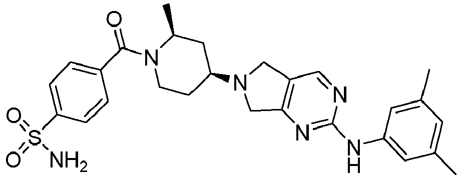

To a solution of 4-sulfamoylbenzoic acid (CAS# 138-41-0; 298 mg, 1.48 mmol, 1.25 eq.) in DMF (28 mL) were added TEA (1.15 mL, 8.29 mmol, 7.0 eq.), HATU (563 mg, 1.48 mmol, 1.25 eq.) and Int 40 (400 mg, 1.18 mmol, 1.0 eq.) and the mixture was stirred at RT for 3 h. The reaction medium was quenched with a sat. NaHCO₃ aq. solution and precipitation occurs. The precipitate was filtered and the filtrate was extracted with EtOAc. The combined organic layers were dried over Na₂SO₄, filtered and concentrated to dryness. The crude mixture was purified by flash chromatography on silica gel (eluting with DCM/EtOAc) to afford Cpd 124.

### 1.69. Cpd 125

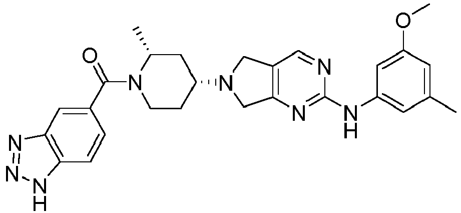

To a solution of Int 2026 (100 mg, 0.39 mmol, 1.0 eq.) and Int 131 (101 mg, 0.39 mmol, 1.0 eq.) in DCM (1.3 mL) were added AcOH (1 drop) and activated 4 Å molecular sieves (200 mg). The mixture was stirred at RT for 30 min and NaBH(OAc)₃ was added in one portion (115 mg, 0.55 mmol, 1.4 eq.). The reaction mixture was stirred at RT overnight before being filtered. The fritt was washed with a DCM/MeOH mixture (9/1) and the filtrate was evaporated. The residue was taken up in EtOAc and washed with water. The aqueous phase was extracted with EtOAc and the combined organic layers were washed with brine, dried over MgSO₄, filtered and concentrated to dryness. The residue was purified by preparative HPLC to afford Cpd 125.

### 1.70. Cpd 130

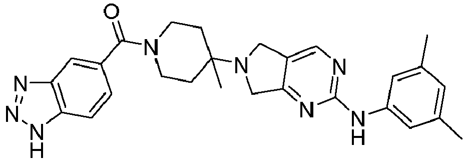

To a solution of 1H-benzotriazole-5-carboxylic acid (25 mg, 0.15 mmol, 1.0 eq.) in DMF (3 mL) were added Int 111 (52 mg, 0.15 mmol, 1.0 eq.), TEA (62 mg, 0.61 mmol, 4.0 eq.) and HATU (70 mg, 0.18 mmol, 1.2 eq.) and the mixture was stirred at RT overnight. The reaction medium was concentrated to dryness and the residue was purified twice by flash chromatography on silica gel (eluting with DCM/ MeOH, then DCM/EtOAc) to afford a solid that was triturated in DCM/heptane to afford Cpd 130.

### 1.71. Cpd 131

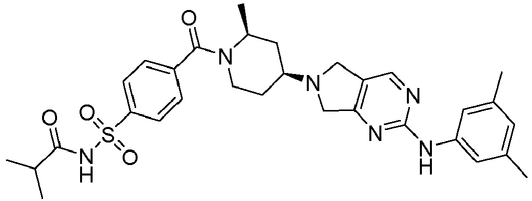

To a solution of Cpd 124 (60 mg, 0.12 mmol, 1.0 eq.) in DMF (1 mL) were added 2-methylpropanoic acid (13 µL, 0.14 mmol, 1.2 eq.), EDC.HCl (28 mg, 0.15 mmol, 1.3 eq.) and DMAP (19 mg, 0.15 mmol, 1.3 eq.) and the mixture was stirred at RT for 48 h. The reaction mixture was quenched with water, extracted with EtOAc and the combined organic layers were dried over MgSO₄, filtered and concentrated to dryness. The residue was purified by flash chromatography on silica gel (eluting with DCM/EtOAc then DCM/MeOH) to afford Cpd 131.

### 1.72. Cpd 132

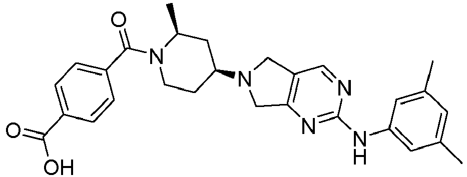

To a solution of terephthalic acid (CAS# 100-21-0; 40 mg, 0.24 mmol, 1.0 eq.) in DMF (3 mL) were added TEA (131 µL, 0.95 mmol, 4.0 eq.) and HATU (90 mg, 0.24 mmol, 1.0 eq.), the mixture was stirred at RT for 30 min and Int 40 (80 mg, 0.24 mmol, 1.0 eq.) was added. The reaction mixture was stirred at RT for 2 h. The reaction medium was concentrated to dryness, the residue was taken up in water and the pH was adjusted to 4 with a 2N HCl aq. solution. The aqueous phase was extracted with EtOAc and the combined organic layers were dried over Na₂SO₄, filtered and concentrated to dryness. The residue was purified by preparative HPLC to afford Cpd 132.

### 1.73. Cpd 133

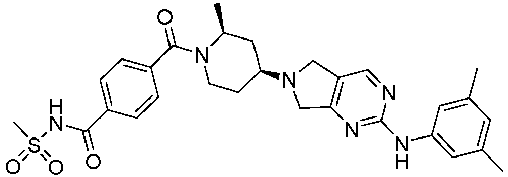

To a solution of Cpd 132 (96 mg, 0.20 mmol, 1.0 eq.) in DCM (2.5 mL) was added methane sulfonamide (25 mg, 0.26 mmol, 1.3 eq.), EDC.HCl (50 mg, 0.26 mmol, 1.3 eq.) and DMAP (24 mg, 0.2 mmol, 1.0 eq.) and the mixture was stirred at RT for 18 h. The reaction medium was quenched with a sat. NaHCO₃ aq. solution, extracted with DCM and filtered on a phase separator. The filtrate was concentrated to dryness and the residue was purified by flash chromatography on silica gel (eluting with DCM/MeOH) and then by preparative HPLC to afford Cpd 133.

### 1.74. Cpd 136 and Cpd 137

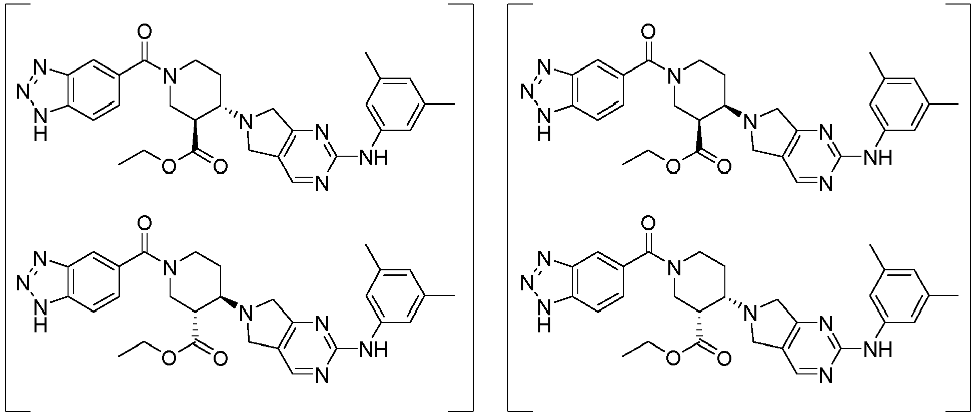

Int 2011 (417 mg, 1.73 mmol, 1.0 eq.) and Int 77 (1.05 g, 3.50 mmol, 2.0 eq.) were placed in DCM (25 mL) and activated 4 Å molecular sieves (1 g) followed by NaBH(OAc)₃ were added. The suspension was stirred at RT for 20 h and more Int 77 (1.05 g, 3.50 mmol, 2.0 eq.) and Int 2011 (834 mg, 3.50 mmol, 2.0 eq.)were added. The mixture was stirred for 15 h before being quenched with a sat. NaHCO₃ aq. solution and extracted with a CHCl₃/n-BuOH (9/1) mixture. The combined organic layers were dried over MgSO₄, filtered and concentrated to dryness. The residue was purified by flash chromatography on silica gel (eluting with DCM/MeOH) and by preparative HPLC to afford Cpd 136 (racemic mixture of TRANS isomers, first eluting) and Cpd 137 (racemic mixture of CIS isomers, second eluting).

### 1.75. Cpd 138 and Cpd 139

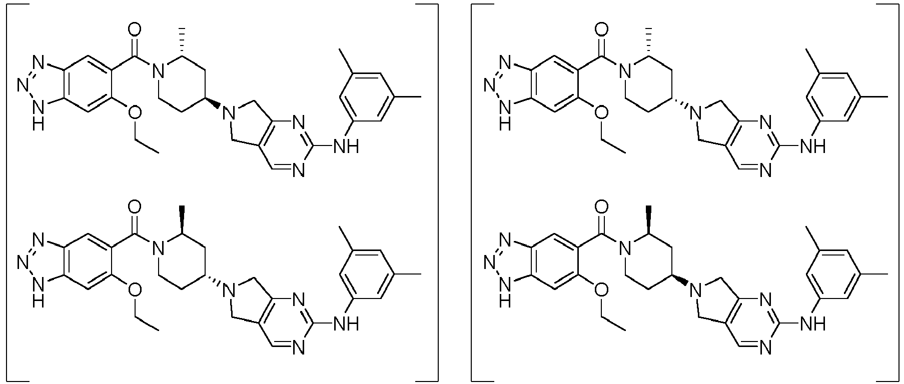

To a solution of Int 83 (126 mg, 0.42 mmol, 1.0 eq.) in DCM (2 mL) were added Int 2011 (100 mg, 0.42 mmol, 1.0 eq.), AcOH (100 mg, 1.68 mmol, 4.0 eq.), activated 4 Å molecular sieves (100 mg) and NaBH(OAc)₃ (177 mg, 0.83 mmol, 2.0 eq.) and the mixture was stirred at RT for 5 h. The reaction medium was concentrated to dryness and the residue was purified by preparative HPLC to afford 2 fractions containing 1 diasteromer each. Each of the obtained compounds was filtered on a SCX column eluting with DCM/MeOH 1/1 and then NH₃ 7N in MeOH. Each of the NH₃ phases were separately concentrated, the residues were triturated in DCM and heptane to afford Cpd 138 (racemic mixture of TRANS isomers, first eluting) and Cpd 139 (racemic mixture of CIS isomers, second eluting).

### 1.76. Cpd 142

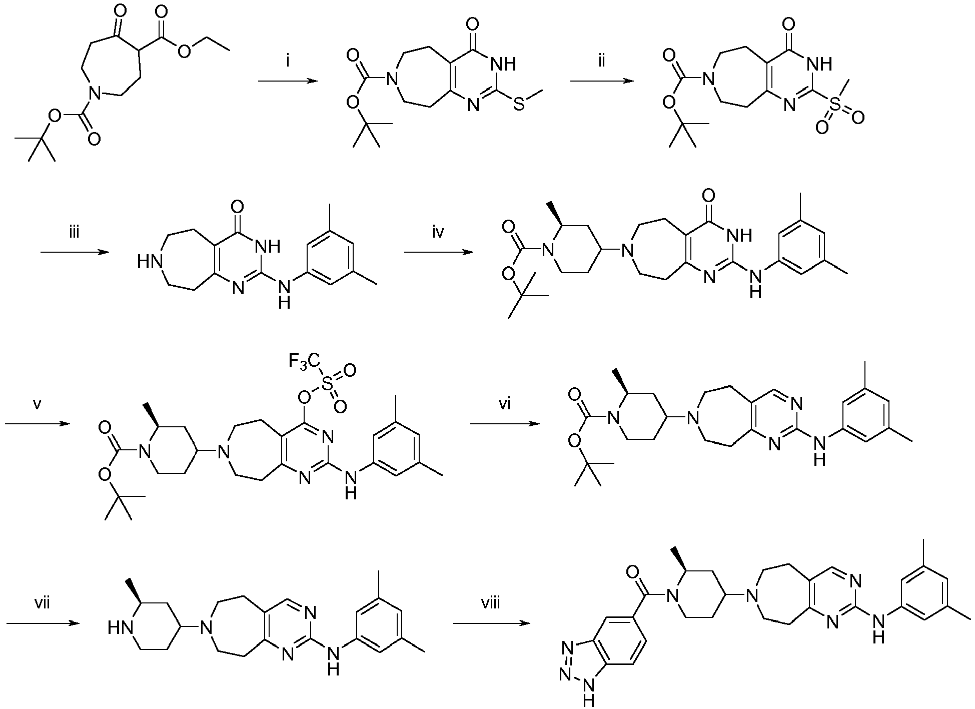

### 1.76.1. Step i

To a solution of 1-Tert-butyl 4-ethyl 5-oxoazepane-1,4-dicarboxylate (CAS# 141642-82-2; 860 mg, 3.01 mmol, 1.0 eq.) in EtOH (13 mL) were added NaOEt (21% in EtOH, 3.6 mL, 9.04 mmol, 3.0 eq.) and thiourea (344 mg, 4.52 mmol, 1.5 eq.) and the mixture was stirred at 90 °C for 15 h. Then at RT, MeI (245 µL, 3.92 mmol, 1.3 eq.) was added and the reaction medium was stirred at RT for 1 h. The volatiles were evaporated in vacuo and the residue was taken up in a sat. NaHCO₃ aq. solution. The aqueous phase was extracted with EtOAc. The combined organic layers were dried over Na₂SO₄, filtered and concentrated to dryness to afford tert-butyl 2-methylsulfanyl-4-oxo-5,6,8,9-tetrahydro-3H-pyrimido[4,5-d]azepine-7-carboxylate.
LCMS: MW (calcd): 311.4; m/z MW (obsd): 312.4 (M+H)

### 1.76.2. Step ii

To a solution of tert-butyl 2-methylsulfanyl-4-oxo-5,6,8,9-tetrahydro-3H-pyrimido[4,5-d]azepine-7-carboxylate (720 mg, 2.31 mmol, 1.0 eq.) in DCM (23 mL) was added mCPBA (800 mg, 4.62 mmol, 2.0 eq.) and the mixture was stirred at RT for 2 h. Then more mCPBA (400 mg, 2.31 mmol, 1.0 eq.) was added and the mixture was stirred for another 2 h. The reaction medium was hydrolysed by a sat. NaHCO₃ aq. solution and was extracted with EtOAc and n-BuOH. The combined organic layers were dried over Na₂SO₄, filtered and concentrated to dryness. The aqueous phase was then concentrated to dryness and the residues from organic and aqueous phases were purified by flash chromatography on silica gel (eluting with DCM/MeOH) to obtain tert-butyl 2-methylsulfonyl-4-oxo-5,6,8,9-tetrahydro-3H-pyrimido[4,5-d]azepine-7-carboxylate.
LCMS: MW (calcd): 343.4; m/z MW (obsd): 344.4 (M+H)

### 1.76.3. Step iii

To a solution of tert-butyl 2-methylsulfonyl-4-oxo-5,6,8,9-tetrahydro-3H-pyrimido[4,5-d]azepine-7-carboxylate (395 mg, 1.15 mmol, 1.0 eq.) in toluene (38 mL) were added 3,5-dimethylaniline (209 mg, 1.72 mmol, 1.5 eq.) and APTS (396 mg, 2.30 mmol, 2.0 eq.) and the mixture was stirred at 125 °C for 16 h. The reaction medium was concentrated to dryness, the residue was taken up in water and washed with EtOAc. The aqueous phase was concentrated to dryness and the residue was purified by flash chromatography on silica gel (eluting with DCM/MeOH then DCM/ MeOH/NH3) then on a KP-NH flash chromatography column (eluting with DCM/MeOH) to afford 2-(3,5-dimethylanilino)-3,5,6,7,8,9-hexahydropyrimido[4,5-d]azepin-4-one.
LCMS: MW (calcd): 284.4; m/z MW (obsd): 285.4 (M+H)

### 1.76.4. Step iv

2-(3,5-dimethylanilino)-3,5,6,7,8,9-hexahydropyrimido[4,5-d]azepin-4-one (90 mg, 0.32 mmol, 1.0 eq.) and tert-butyl (2S)-2-methyl-4-oxo-piperidine-1-carboxylate (81 mg, 0.38 mmol, 1.2 eq.) were placed in MeOH (2 mL) and AcOH (2 µL, 0.0032 mmol, 0.01 eq.) The mixture was stirred at RT for 10 min. Then NaBH₃CN (50 mg, 0.63 mmol, 2.0 eq.) was added and the mixture was stirred at RT for 3 h. DME (1 mL), tert-butyl (2S)-2-methyl-4-oxo-piperidine-1-carboxylate (81 mg, 0.38 mmol, 1.2 eq.) and NaBH₃CN (50 mg, 0.63 mmol, 2.0 eq.) were added and the reaction medium was stirred at RT overnight. Tert-butyl (2S)-2-methyl-4-oxo-piperidine-1-carboxylate (81 mg, 0.38 mmol, 1.2 eq.) and NaBH₃CN (50 mg, 0.63 mmol, 2.0 eq.) were added and the mixture was stirred at 50 °C overnight and at 90 °C for 6 h. More tert-butyl (2S)-2-methyl-4-oxo-piperidine-1-carboxylate (135 mg, 0.63 mmol, 2.0 eq.) and NaBH₃CN (50 mg, 0.63 mmol, 2.0 eq.) were added and the mixture was stirred at 70 °C overnight. Tert-butyl (2S)-2-methyl-4-oxo-piperidine-1-carboxylate (68 mg, 0.32 mmol, 1.0 eq.) was added and the mixture was stirred at 70 °C for 72 h. The reaction mixture was concentrated to dryness and the residue was purified by flash chromatography on silica gel (eluting with DCM/MeOH) to afford tert-butyl (2S)-4-[2-(3,5-dimethylanilino)-4-oxo-5,6,8,9-tetrahydro-3H-pyrimido[4,5-d]azepin-7-yl]-2-methyl-piperidine-1-carboxylate.
LCMS: MW (calcd): 481.64; m/z MW (obsd): 482.6 (M+H)

### 1.76.5. Step v

To a solution of tert-butyl (2S)-4-[2-(3,5-dimethylanilino)-4-oxo-5,6,8,9-tetrahydro-3H-pyrimido[4,5-d]azepin-7-yl]-2-methyl-piperidine-1-carboxylate (152 mg, 0.32 mmol, 1.0 eq.) in DCM (2 mL) were added DBU (47 µL, 0.32 mmol, 1.0 eq.), 1,1,1-trifluoro-N-phenyl-N-(trifluoromethylsulfonyl)methanesulfonamide (CAS# 37595-74-7; 112 mg, 0.32 mmol, 1.0 eq.) and DMAP (1 mg, 0.006 mmol, 0.02 eq.) and the mixture was stirred at RT for 8 h. Then trifluoromethylsulfonyl)methanesulfonamide (CAS# 37595-74-7; 112 mg, 0.32 mmol, 1.0 eq.), DBU (47 µL, 0.32 mmol, 1.0 eq.) and DMAP (1 mg, 0.006 mmol, 0.02 eq.) were added again and the mixture was stirred at RT overnight. The reaction medium was hydrolysed with water and extracted with DCM. The phases were separated using a phase separator and the organic layer was concentrated to dryness. The residue was purified by flash chromatography on silica gel (eluting with DCM/MeOH) to afford tert-butyl (2S)-4-[2-(3,5-dimethylanilino)-4-(trifluoromethylsulfonyloxy)-5,6,8,9-tetrahydropyrimido[4,5-d]azepin-7-yl]-2-methyl-piperidine-1-carboxylate.
LCMS: MW (calcd): 613.7; m/z MW (obsd): 614.6 (M+H)

### 1.76.6. Step vi

To a solution of tert-butyl (2S)-4-[2-(3,5-dimethylanilino)-4-(trifluoromethylsulfonyloxy)-5,6,8,9-tetrahydropyrimido[4,5-d]azepin-7-yl]-2-methyl-piperidine-1-carboxylate (128 mg, 0.21 mmol, 1.0 eq.) in DMF (2 mL) were added Pd(OAc)₂ (1 mg, 0.004 mmol, 0.02 eq.), dppf (5 mg, 0.008 mmol, 0.04 eq.), TEA (87 µL, 0.63 mmol, 3.0 eq.) and formic acid (15 µL, 0.42 mmol, 2.0 eq.) and the mixture was stirred at 50°C for 2 h. The reaction medium was concentrated to dryness and the residue was purified by flash chromatography on silica gel (eluting with heptane/EtOAc) to afford tert-butyl (2S)-4-[2-(3,5-dimethylanilino)-5,6,8,9-tetrahydropyrimido[4,5-d]azepin-7-yl]-2-methyl-piperidine-1-carboxylate.
LCMS: MW (calcd): 465.6; m/z MW (obsd): 466.6 (M+H)

### 1.76.7. Step vii

N-(3,5-dimethylphenyl)-7-[(2S)-2-methyl-4-piperidyl]-5,6,8,9-tetrahydropyrimido[4,5-d]azepin-2-amine is obtained from tert-butyl (2S)-4-[2-(3,5-dimethylanilino)-5,6,8,9-tetrahydropyrimido[4,5-d]azepin-7-yl]-2-methyl-piperidine-1-carboxylate following general method B.

### 1.76.8. Step viii

Cpd 142 is obtained from N-(3,5-dimethylphenyl)-7-[(2S)-2-methyl-4-piperidyl]-5,6,8,9-tetrahydropyrimido[4,5-d]azepin-2-amine following general method A with 1H-benzotriazole-5-carboxylic acid.

### 1.77. Cpd 146

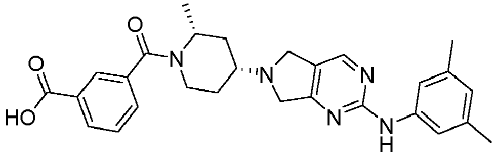

Cpd 145 (58 mg, 0.12 mmol, 1.0 eq.) was dissolved in a mixture of THF (0.5 mL) and water (150 µL). A 1N NaOH aq. solution (116 µL, 0.12 mmol, 1.0 eq.) was added and the mixture was stirred at RT overnight. Toluene was added and the reaction medium was concentrated to dryness. The residue was taken up in water, a 1N HCl aq. solution was added to reach pH 3 to 4. The obtained precipitate was filtered and the solid was dried to afford Cpd 146.

### 1.78. Cpd 153

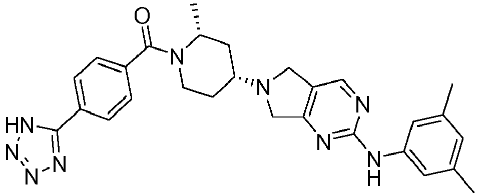

To a solution of 4-(1H-tetrazol-5-yl)benzoic acid (CAS# 34114-12-0; 57 mg, 0.30 mmol, 1.0 eq.) in DMF (4 mL) were added TEA (164 µL, 1.18 mmol, 4.0 eq.) and HATU (153 mg, 0.30 mmol, 1.0 eq.), the mixture was stirred at RT for 30 min and Int 39 (80 mg, 0.24 mmol, 1.0 eq.) was added. The reaction mixture was stirred at RT for 3 h. The reaction medium was quenched with a sat. NaHCO₃ aq. solution and the pH was adjusted to 4 with a 1N HCl aq. solution. The aqueous phase was extracted with EtOAc and the combined organic layers were dried over Na₂SO₄, filtered and concentrated to dryness. The residue was purified by flash chromatography on silica gel (eluting with DCM/MeOH) and twice by preparative HPLC to afford Cpd 153.

### 1.79. Cpd 158

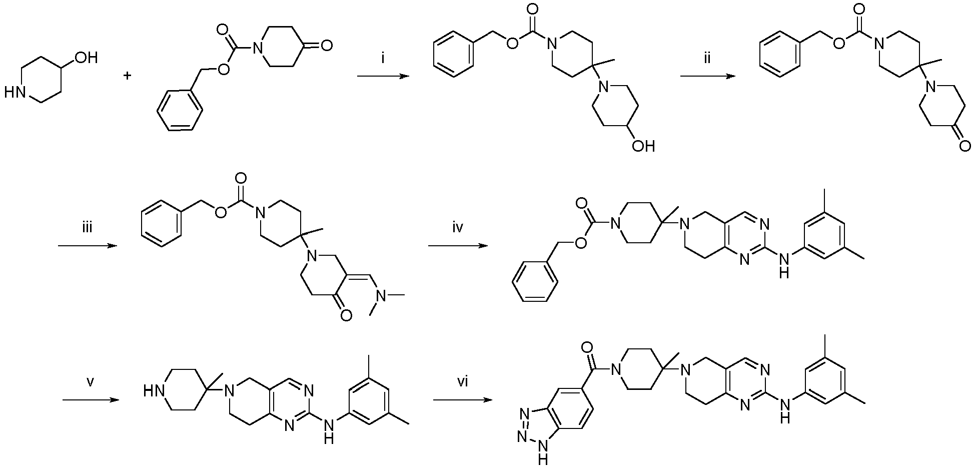

### 1.79.1. Step i

To a solution of piperidin-4-ol (1.16 g, 11.46 mmol, 1.0 eq.) in toluene (12 mL) were added 1H-triazole (890 mg, 12.61 mmol, 1.1 eq.) and benzyl 4-oxopiperidine-1-carboxylate (CAS# 4727-72-4; 2.54 g, 10.89 mmol, 0.95 eq.) and the mixture was stirred at reflux for 4 h using a Dean-Stark apparatus to remove water from the reaction medium. After cooling down to RT, a MeMgBr solution (3M in Et₂O, 14.5 mL, 3.8 eq.) was added to the reaction mixture while maintaining the temperature below 24 °C with an ice bath. At the end of the addition the bath was removed and the mixture was stirred at RT for 4 h. The reaction medium was diluted with water and extracted with EtOAc. The combined organic layers were dried over Na₂SO₄, filtered and concentrated to dryness. The residue was purified by flash chromatography on silica gel (eluting with DCM/MeOH) to afford benzyl 4-(4-hydroxy-1-piperidyl)-4-methyl-piperidine-1-carboxylate.
LCMS: MW (calcd): 332.4; m/z MW (obsd): 333.5 (M+H)

### 1.79.2. Step ii

To a solution of oxalyl chloride (116 µL, 1.37 mmol, 1.5 eq.) in DCM (2 mL) at -78 °C was added a cooled solution (-78 °C) of DMSO (193 µL, 2.71 mmol, 3.0 eq.) in DCM (1 mL). The mixture was stirred at -78 °C for 10 min and a solution of benzyl 4-(4-hydroxy-1-piperidyl)-4-methyl-piperidine-1-carboxylate (300 mg, 0.90 mmol, 1.0 eq.) in DCM (1 mL) followed by TEA (503 µL, 3.61 mmol, 4.0 eq.) were added. The reaction mixture was then stirred for another 20 min at -78 °C and then for 1 h at RT. The reaction was quenched by the addition of water. After extraction with DCM the combined organic layers were washed with brine, dried over Na₂SO₄, filtered and concentrated to dryness to afford benzyl 4-methyl-4-(4-oxo-1-piperidyl)piperidine-1-carboxylate.
LCMS: MW (calcd): 330.4; m/z MW (obsd): 331.4 (M+H)

### 1.79.3. Step iii

Benzyl 4-[(3Z)-3-(dimethylaminomethylene)-4-oxo-1-piperidyl]-4-methyl-piperidine-1-carboxylate is synthesized from benzyl 4-methyl-4-(4-oxo-1-piperidyl)piperidine-1-carboxylate following general method F.

### 1.79.4. Step iv

To a solution of benzyl 4-[(3Z)-3-(dimethylaminomethylene)-4-oxo-1-piperidyl]-4-methylpiperidine-1-carboxylate (427 mg, 1.11 mmol, 1.0 eq.) in EtOH (5 mL) were added potassium carbonate (460 mg, 3.33 mmol, 3.0 eq.) and Int_141 (251 mg, 1.11 mmol, 1.0 eq.) and the mixture was stirred at 100 °C for 4 h. The volatiles were evaporated and the resulting crude mixture was partitioned between water and EtOAc. The aqueous layer was extracted with EtOAc and the combined organic layers were dried over Na₂SO₄, filtered and concentrated to dryness. The residue was purified by flash chromatography on silica gel (eluting with heptane/EtOAc) to afford benzyl 4-[2-(3,5-dimethylanilino)-7,8-dihydro-5H-pyrido[4,3-d]pyrimidin-6-yl] -4-methyl-piperidine-1 -carboxylate.

### 1.79.5. Step v

To benzyl 4-[2-(3,5-dimethylanilino)-7,8-dihydro-5H-pyrido[4,3-d]pyrimidin-6-yl]-4-methylpiperidine-1-carboxylate (90 mg, 0.19 mmol, 1.0 eq.) in MeOH (10 mL) was added Pd/C (10%, 180 mg) and the mixture was stirred at RT under H₂ atmosphere for 4 h. The reaction medium was filtered on Celite® and the filtrate was concentrated to afford N-(3,5-dimethylphenyl)-6-(4-methyl-4-piperidyl)-7,8-dihydro-5H-pyrido[4,3-d]pyrimidin-2-amine.

### 1.79.6. Step vi

Cpd 158 was obtained from N-(3,5-dimethylphenyl)-6-(4-methyl-4-piperidyl)-7,8-dihydro-5H-pyrido[4,3-d]pyrimidin-2-amine following general method A with 1H-benzotriazole-5-carboxylic acid.

### 1.80. Cpd 160

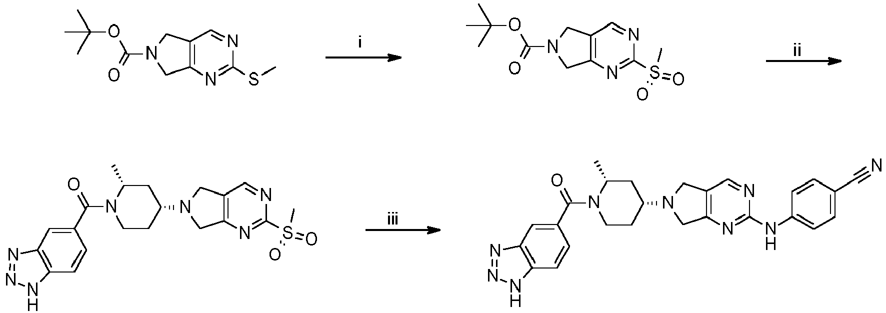

### 1.80.1. Step i

To a solution of tert-butyl 2-methylsulfanyl-5,7-dihydropyrrolo[3,4-d]pyrimidine-6-carboxylate (CAS# 365996-86-7; 6.8 g, 25.4 mmol, 1.0 eq.) in DCM (150 mL) was added mCPBA (12.9 g, 56 mmol, 2.2 eq.) and the mixture was stirred at RT for 18 h. The reaction medium was quenched with a 1N NaOH aqeous solution and the aqueous phase was extracted with DCM. The combined organic layers were dried over Na₂SO₄, filtered and concentrated to dryness to afford tert-butyl 2-methylsulfonyl-5,7-dihydropyrrolo[3,4-d]pyrimidine-6-carboxylate.
LCMS: MW (calcd): 299.3; m/z MW (obsd): 300.3 (M+H)

### 1.80.2. Step ii

Tert-butyl 2-methylsulfonyl-5,7-dihydropyrrolo[3,4-d]pyrimidine-6-carboxylate (4 g, 13.36 mmol, 1.0 eq.) was dissolved in a mixture of DCM and TFA (5/1, 60 mL) and the mixture was stirred at RT for 2 h. The solvents were concentrated to dryness. The residue was then added to a solution of Int 131 (2.86 g, 11.07 mmol, 0.8 eq.) in DME (100 mL) containing activated 4 Å molecular sieves. The mixture was stirred at RT for 10 min and NaBH(OAc)₃ (7.1 g, 33.5 mmol, 3.0 eq.) was added. The reaction was stirred at RT for 2 h, the solvents were evaporated and the residue was taken up in a sat. NaOAc aq. solution. The aqueous phase was extracted with DCM and the combined organic layers were dried over Na₂SO₄, filtered and concentrated to dryness. The crude material was purified by flash chromatography on silica gel (eluting with DCM/Acetone) to afford 1H-benzotriazol-5-yl-[(2R,4R)-2-methyl-4-(2-methylsulfonyl-5,7-dihydropyrrolo[3,4-d]pyrimidin-6-yl)-1-piperidyl]methanone.
LCMS: MW (calcd): 441.5; m/z MW (obsd): 442.4 (M+H)

### 1.80.3. Step iii

A mixture of 1H-benzotriazol-5-yl-[(2R,4R)-2-methyl-4-(2-methylsulfonyl-5,7-dihydropyrrolo[3,4-d]pyrimidin-6-yl)-1-piperidyl]methanone (100 mg, 0.23 mmol, 1.0 eq.) and 4-cyanoaniline (230 mg, 1.94 mmol, 8.6 eq.) was stirred at 50°C in a sealed vial for 2 h. Then 0.1 mL of DMAC were added and the mixture was stirred at 60°C for 2 more h. Then APTS (40 mg, 0.23 mmol, 1.0 eq.) and toluene (0.2 mL) were added and the reaction was stirred at 90°C for 15 h. Solvents were removed in vacuo and the residue was purified by preparative HPLC to afford Cpd 160.

### 1.81. Cpd 164 and Cpd 169

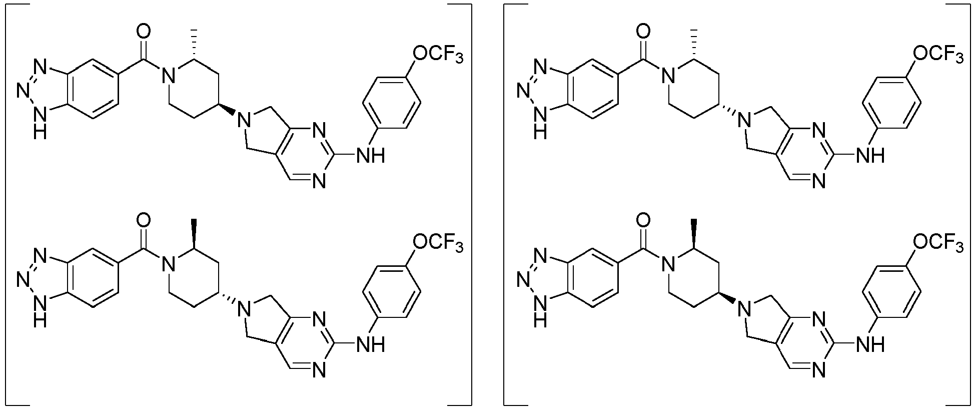

To a solution of Int 131 (169 mg, 0.45 mmol, 1.0 eq.) and Int 2077 (166 mg, 0.5 mmol, 1.1 eq.) in THF (1.7 mL) was added activated 4 Å molecular sieves (800 mg) and the suspension was stirred at RT for 10 min. Then NaBH(OAc)₃ (127 mg, 0.6 mmol, 1.2 eq.) was added and the mixture was stirred at RT overnight. AcOH (2 drops) was added and the mixture was stirred at RT for another 24 h. More NaBH(OAc)₃ (127 mg, 0.6 mmol, 1.2 eq.) was added and the suspension was stirred for 3 days at RT. The reaction medium was quenched with a sat. NaHCO₃ aq. solution and was extracted with DCM. The combined organic layers were washed with brine, dried over MgSO₄, filtered and concentrated to dryness. The residue was purified by preparative HPLC to afford Cpd 164 (racemic mixture of TRANS isomers, first eluted) and Cpd 169 (racemic mixture of CIS isomers, second eluted).

### 1.82. Cpd 245

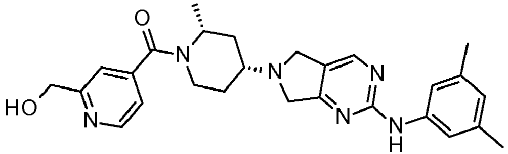

To a solution of Cpd 283 (250 mg, 0.50 mmol, 1.0 eq.) in THF (10 mL) at 0°C was added LiBH₄ (2 M in THF, 1.25 mL, 2.5 mmol, 5.0 eq.) and the mixture was stirred at RT overnight. The reaction medium was poured on a sat. NH₄Cl aq. solution and was extracted with DCM. The organic layer was filtered on Clarcel® and the filtrated was concentrated to dryness. The residue was purified by flash chromatography on silica gel (eluting with DCM/MeOH) to afford Cpd 245.

### 1.83. Cpd 267

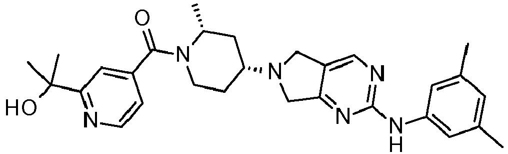

To a solution of Cpd 283 (65 mg, 0.12 mmol, 1.0 eq.) in THF (5 mL) at 0°C was added MeMgBr (3 M in Et₂O, 103 µL, 0.31 mmol, 2.5 eq.) dropwise and the mixture was stirred at RT for 2 h. Then at 0°C additional MeMgBr (3 M in Et₂O, 62 µL, 0.19 mmol, 1.5 eq.) was added and the reaction was run at RT for another 30 min. The reaction was quenched with a sat. NH₄Cl aq. solution and extracted with EtOAc. The combined organic layers were dried over Na₂SO₄, filtered and concentrated to dryness. The residue was purified by flash chromatography on silica gel (eluting with DCM/MeOH) to afford Cpd 267.

### 1.84. Cpd 275

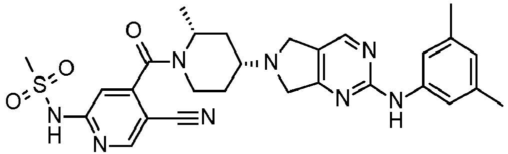

To a solution of Int 65 (45 mg, 0.19 mmol, 1.25 eq.) in DMF (2 mL) were added TEA (82 µL, 0.60 mmol, 4.0 eq.) and HATU (71 mg, 0.19 mmol, 1.25 eq.), the mixture was stirred at RT for 5 min and Int 39 (54 mg, 0.15 mmol, 1.0 eq.) was added. The reaction mixture was stirred at RT for 2 h. The reaction medium was quenched with water and was extracted with EtOAc. The aqueous phase was acidified to pH 4 with a 1N HCl aq. solution and was extracted with EtOAc. The combined organic layers were dried over Na₂SO₄, filtered and concentrated to dryness. The residue was purified by flash chromatography on silica gel (eluting with DCM/EtOAc) and by preparative HPLC to afford Cpd 275.

### 1.85. Cpd 283

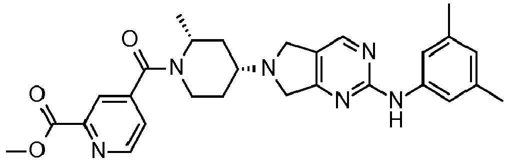

Int 39 (2 g, 4.87 mmol, 1.0 eq.) was suspended in a mixture of DCM (60 mL) and TEA (3.4 mL, 24.35 mmol, 5.0 eq.) and Int 72 (1 g, 4.9 mmol, 1.0 eq.) was added dropwise at 0°C. The reaction mixture was stirred at 0°C for 30 min and the medium was poured into a sat. NaOAc aq. solution. The aqueous phase was extracted with DCM, the combined organic layers were dried over Na₂SO₄, filtered and concentrated to dryness. The residue was purified by flash chromatography on silica gel (eluting with DCM/EtOAc) to afford Cpd 283.

### 1.86. Cpd 288

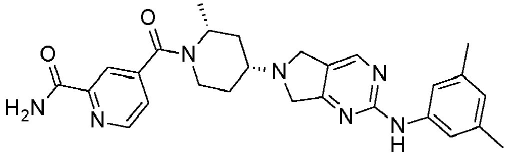

To DMF (2.5 mL) and DIPEA (64 µL, 0.37 mmol, 3.0 eq.) were added Cpd 276 (60 mg, 0.12 mmol, 1.0 eq.) and HATU (61 mg, 0.16 mmol, 1.3 eq.) and the mixture was stirred at RT for 15 min. Then NH₄Cl (66 mg, 1.23 mmol, 10 eq.) and DIPEA (214 µL, 1.23 mmol, 10 eq.) were added and the reaction mixture was stirred at RT for 1 h. Water was added and the precipitate was filtered, washed with water and Et₂O and dried. The solid was then purified by flash chromatography on silica gel (eluting with DCM/MeOH) to afford Cpd 288.

### 1.87. Cpd 290

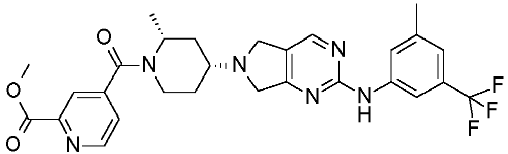

Int 120 (500 mg, 1.08 mmol, 1.0 eq.) was suspended in a mixture of DCM (10 mL) and TEA (0.8 mL, 5.4 mmol, 5.0 eq.) and Int 72 (220 mg, 1.1 mmol, 1.02 eq.) was added dropwise at 0°C. The reaction mixture was stirred at 0°C for 30 min and the medium was poured into a sat. NaOAc aq. solution. The aqueous phase was extracted with DCM, the combined organic layers were dried over Na₂SO₄, filtered and concentrated to dryness. The residue was purified by flash chromatography on silica gel (eluting with DCM/EtOAc) to afford Cpd 290.

### 1.88. Cpd 291

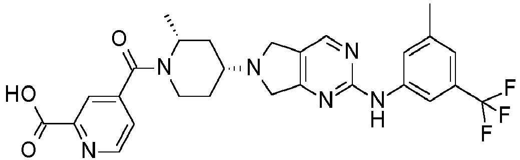

To a solution of Cpd 290 (170 mg, 0.31 mmol, 1.0 eq.) in MeOH (10 mL) was added a 2N NaOH aq. solution (10 drops, excess) and the mixture was stirred at RT for 4 h. MeOH was then removed from the reaction medium and the obtained aqueous phase was acidified with acetic acid to pH 6. The precipitate was filtered, the solid was washed with water and dried to afford Cpd 291.

### 1.89. Cpd 292

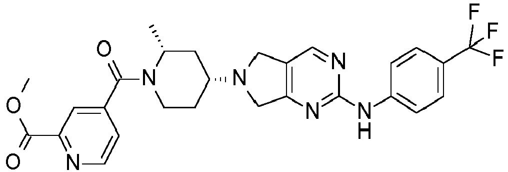

To a solution of Int 122 (320 mg, 0.71 mmol, 1.0 eq.) in DCM (20 mL) and TEA (0.5 mL, 3.55 mmol, 5.0 eq.) at 0°C was slowly added Int 72 and the mixture was stirred at 0°C for 30 min. The reaction medium was washed with a sat. NH₄Cl aq. solution and the organic phase was dried over Na₂SO₄, filtered and concentrated to dryness. The residue was purified by flash chromatography on silica gel (eluting with EtOAc) to afford Cpd 292.

### 1.90. Cpd 293

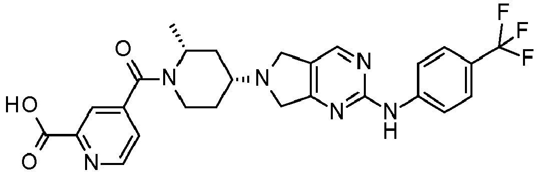

To a solution of Cpd 292 (180 mg, 0.33 mmol, 1.0 eq.) in MeOH (5 mL) was added NaOH (2N in water, 0.5 mL, 1 mmol, 3.0 eq.) and the mixture was stirred at RT for 4 h. MeOH was removed in vacuo, the reaction medium was diluted with water and acidified with acetic acid until precipitation occurs. The precipitate was filtered, washed with water and dried to afford Cpd 293.

### 1.91. Cpd 294

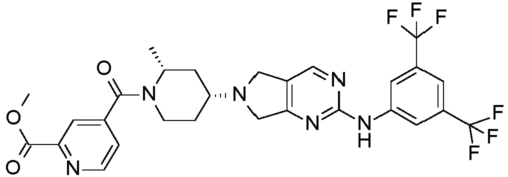

Int 124 (1.58 g, 3.04 mmol, 1.0 eq.) was suspended in a mixture of DCM (100 mL) and TEA (2.2 mL, 15.2 mmol, 5.0 eq.) and Int 72 (670 mg, 3.35 mmol, 1.1 eq.) was added dropwise at 0°C. The reaction mixture was stirred at 0°C for 1 h and the medium was poured into a sat. NaCl aq. solution. The aqueous phase was extracted with DCM, the combined organic layers were dried over Na₂SO₄, filtered and concentrated to dryness. The residue was purified by flash chromatography on silica gel (eluting with EtOAc/MeOH) to afford Cpd 294.

### 1.92. Cpd 295

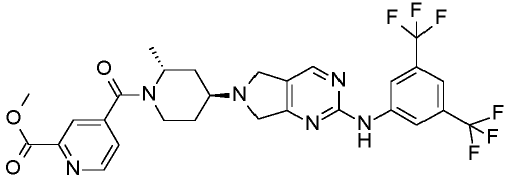

Int 126 (510 mg, 0.98 mmol, 1.0 eq.) was suspended in a mixture of DCM (10 mL) and TEA (0.7 mL, 4.9 mmol, 5.0 eq.) and Int 72 (210 mg, 1.08 mmol, 1.1 eq.) was added dropwise at 0°C. The reaction mixture was stirred at 0°C for 1 h and the medium was poured into a sat. NaCl aq. solution. The aqueous phase was extracted with DCM, the combined organic layers were dried over Na₂SO₄, filtered and concentrated to dryness. The residue was purified by flash chromatography on silica gel (eluting with EtOAc/MeOH) to afford Cpd 295.

### 1.93. Cpd 296

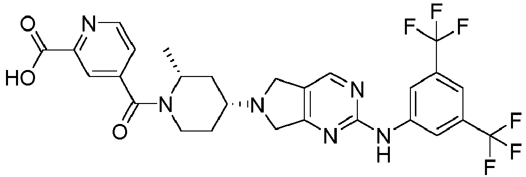

To a solution of Cpd 294 (1 g, 1.64 mmol, 1.0 eq.) in MeOH (50 mL) was added NaOH (2N in water, 4 mL, 8 mmol, 5.0 eq.) and the mixture was stirred at RT for 2 h. MeOH was removed in vacuo, the reaction medium was diluted with water and acidified with acetic acid until precipitation occurs. The precipitate was filtered, washed with water and dried to afford Cpd 296.

### 1.94. Cpd 297

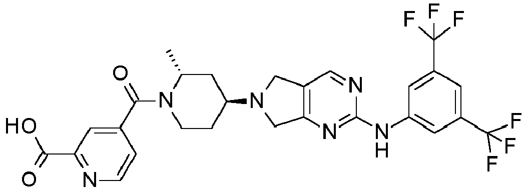

To a solution of Cpd 295 (300 mg, 0.49 mmol, 1.0 eq.) in MeOH (8 mL) was added a 2N NaOH aq. solution (2 mL, 4 mmol, 8.0 eq.) and the mixture was stirred at RT for 2 h. MeOH was then removed from the reaction medium and the obtained aqueous phase was acidified with acetic acid to pH 6. The precipitate was filtered, washed with water and dried. The solid was taken up in acetone, a precipitate forms and was filtered to afford Cpd 297.

### 1.95. Cpd 298

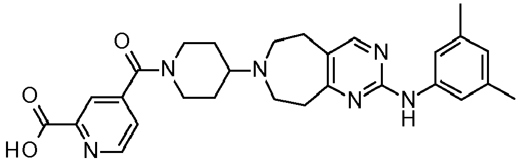

To a solution of Cpd 289 (21 mg, 0.04 mmol, 1.0 eq.) in MeOH (300 µL) was added a 2N NaOH aq. solution (102 µL, 0.2 mmol, 5.0 eq.) and the mixture was stirred at RT for 2 h. MeOH was then removed from the reaction medium and the obtained aqueous phase was acidified with acetic acid to pH 5. The aqueous phase was extracted with EtOAc and n-BuOH, the combined organic layers were dried over Na₂SO₄, filtered and concentrated to dryness. The residue was purified by preparative HPLC to afford Cpd 298.

### 1.96. Cpd 303

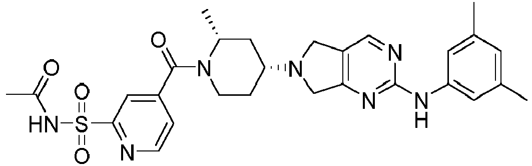

To a solution of Cpd 300 (50 mg, 0.10 mmol, 1.0 eq.) in DCM (1 mL) were added acetyl chloride (8 µL, 0.1 mmol, 1.0 eq.) and TEA (26 µL, 0.19 mmol, 2.0 eq.) and the mixture was stirred at RT overnight. The reaction medium was concentrated to dryness and the residue was purified by preparative HPLC followed by 2 purifications by flash chromatography on silica gel (eluting with DCM/MeOH) to afford a solid that was then triturated in Et₂O. The obtained compound was taken up in MeOH (500 µL) and a 1N NaOH aq. solution (500 µL) and the mixture was allowed to stir at RT for 1 h before being concentrated to dryness. The residue was taken up in a sat. NH₄Cl aq. solution, extracted with DCM. The combined organic layers were dried over Na₂SO₄, filtered and concentrated to dryness to afford Cpd 303.

### 1.97. Cpd 308

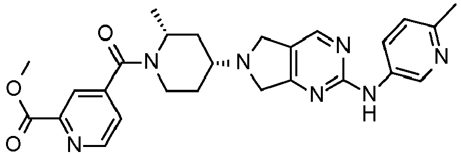

Int 129 (450 mg, 1.14 mmol, 1.0 eq.) was suspended in a mixture of DCM (10 mL) and TEA (0.8 mL, 5.7 mmol, 5.0 eq.) and Int 72 (250 mg, 1.25 mmol, 1.1 eq.) in DCM (5 mL) was added dropwise at 0°C. The reaction mixture was stirred at 0°C for 30 min and the medium was poured into a sat. NH₄Cl aq. solution. The aqueous phase was extracted with DCM, the combined organic layers were dried over Na₂SO₄, filtered and concentrated to dryness. The residue was purified by flash chromatography on silica gel (eluting with DCM/MeOH) to afford Cpd 308.

### 1.98. Cpd 309

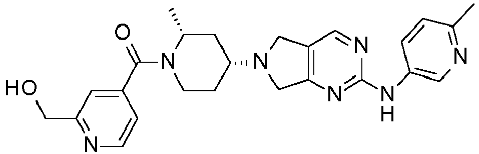

Cpd 308 (340 mg, 0.7 mmol, 1.0 eq.) was dissolved in MeOH (10 mL) and NaBH₄ (310 mg, 8.4 mmol, 12 eq.) was added portion wise (100 mg every h). After 3 h at RT the reaction medium was quenched with a sat. NH₄Cl aq. solution and the aqueous phase was extracted with DCM. The combined organic layers were dried over Na₂SO₄, filtered and concentrated to dryness. The residue was purified by flash chromatography on silica gel (eluting with DCM/MeOH) to afford Cpd 309.

### 1.99. Cpd 311

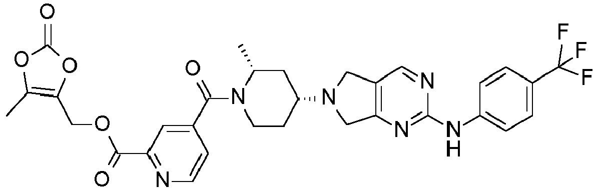

Cpd 293 (200 mg, 0.38 mmol, 1.0 eq.), cesium carbonate (370 mg, 1.14 mmol, 3.0 eq.), NaI (cat.) and 4-(chloromethyl)-5-methyl-1,3-dioxol-2-one (CAS# 80841-78-7; 140 mg, 0.94 mmol, 2.5 eq.) were placed in DMF (5 mL) and the mixture was stirred at 50°C for 2 h. The reaction medium was poured into water and extracted with DCM. The combined organic layers were dried over Na₂SO₄, filtered and concentrated to dryness. The residue was purified by flash chromatography on silica gel (eluting with EtOAc) to afford Cpd 311.

### 1.100. Cpd 314

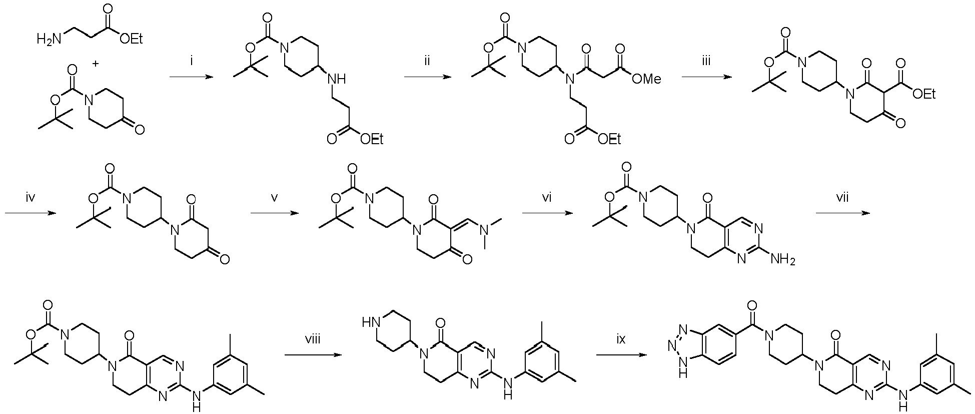

### 1.100.1. Step i

To a solution of tert-butyl 4-oxopiperidine-1-carboxylate (2.0 g, 10 mmol, 1.0 eq.) in MeOH (80 mL) were added ethyl 3-aminopropanoate.HCl (1.75 g, 11 mmol, 1.1 eq.), acetic acid (10.57 mL, 10 mmol, 1.0 eq.) and NaBH(OAc)₃ portionwise (2.33 g, 11 mmol, 1.1 eq.). The suspension was stirred at RT for 20 h before being concentrated in vacuo. The residue was purified by flash chromatography on silica gel (eluting with DCM/MeOH) to afford tert-butyl 4-[(3-ethoxy-3-oxo-propyl)amino]piperidine-1-carboxylate.

### 1.100.2. Step ii

To a solution of tert-butyl 4-[(3-ethoxy-3-oxo-propyl)amino]piperidine-1-carboxylate (2 g, 6.6 mmol, 1.0 eq.) in DCM (40 mL) at 0°C were added methyl 3-chloro-3-oxo-propanoate (0.86 mL, 8.0 mmol, 1.2 eq.) and TEA (1.1 mL, 8.0 mmol, 1.2 eq.) and the mixture was stirred at 0°C for 1.5 h. The reaction medium was then diluted with water and extracted with DCM. The combined organic layers were dried avec MgSO₄, filtered and concentrated to dryness. The residue was purified by flash chromatography on silica gel (eluting with DCM/MeOH) to afford tert-butyl 4-[(3-ethoxy-3-oxo-propyl)-(3-methoxy-3-oxo-propanoyl)amino]piperidine-1-carboxylate.
LCMS: MW (calcd): 400.48; m/z MW (obsd): 401.6 (M+H)

### 1.100.3. Step iii: ethyl 1-(1-tert-butoxycarbonyl-4-piperidyl)-2,4-dioxo-piperidine-3-carboxylate

A solution of NaOEt in EtOH was freshly prepared by dissolution of 121 mg of sodium (5.25 mmol, 1.05 eq.) in 5 mL of EtOH and tert-butyl 4-[(3-ethoxy-3-oxo-propyl)-(3-methoxy-3-oxo-propanoyl)amino]piperidine-1-carboxylate (2 g, 5 mmol, 1.0 eq.) in EtOH (5 mL) was added. The reaction mixture was refluxed for 2 h before being concentrated to dryness. The crude residue was taken up in DCM and washed with a 0.1N HCl aq. solution. The organic layer was dried over MgSO₄, filtered and concentrated to dryness to afford ethyl 1-(1-tert-butoxycarbonyl-4-piperidyl)-2,4-dioxo-piperidine-3-carboxylate.
LCMS: MW (calcd): 368.4; m/z MW (obsd): 369.5 (M+H)

### 1.100.4. Step iv

To a solution of ethyl 1-(1-tert-butoxycarbonyl-4-piperidyl)-2,4-dioxo-piperidine-3-carboxylate (1.55 g, 4.21 mmol, 1.0 eq.) in ACN (20 mL) was added water (20 mL) and the mixture was refluxed for 6 h. The reaction mixture was concentrated to dryness, taken up in toluene and concentrated in vacuo (twice). The residue was purified by flash chromatography on silica gel (eluting with heptane/EtOAc) to tert-butyl 4-(2,4-dioxo-1-piperidyl)piperidine-1-carboxylate.
LCMS: MW (calcd): 296.4; m/z MW (obsd): 297.4 (M+H)

### 1.100.5. Step v

Tert-butyl 4-[(3E)-3-(dimethylaminomethylene)-2,4-dioxo-1-piperidyl]piperidine-1-carboxylate was obtained from tert-butyl 4-(2,4-dioxo-1-piperidyl)piperidine-1-carboxylate following general method F.
LCMS: MW (calcd): 351.5; m/z MW (obsd): 352.5 (M+H)

### 1.100.6. Step vi

Tert-butyl 4-(2-amino-5-oxo-7,8-dihydropyrido[4,3-d]pyrimidin-6-yl)piperidine-1-carboxylate was obtained from tert-butyl 4-[(3E)-3-(dimethylaminomethylene)-2,4-dioxo-1-piperidyl]piperidine-1-carboxylate following general method E.
LCMS: MW (calcd): 347.4; m/z MW (obsd): 348.5 (M+H)

### 1.100.7. Step vii

Tert-butyl 4-[2-(3,5-dimethylanilino)-5-oxo-7,8-dihydropyrido[4,3-d]pyrimidin-6-yl]piperidine-1-carboxylate was obtained from tert-butyl 4-(2-amino-5-oxo-7,8-dihydropyrido[4,3-d]pyrimidin-6-yl)piperidine-1-carboxylate following general method D with 1-bromo-3,5-dimethyl-benzene.
LCMS: MW (calcd): 451.6; m/z MW (obsd): 452.6 (M+H)

### 1.100.8. Step viii

2 2-(3,5-dimethylanilino)-6-(4-piperidyl)-7,8-dihydropyrido[4,3-d]pyrimidin-5-one was obtained from tert-butyl 4-[2-(3,5-dimethylanilino)-5-oxo-7,8-dihydropyrido[4,3-d]pyrimidin-6-yl]piperidine-1-carboxylate following general method B.

### 1.100.9. Step ix

Cpd 314 was obtained from 2-(3,5-dimethylanilino)-6-(4-piperidyl)-7,8-dihydropyrido[4,3-d]pyrimidin-5-one and 1H-benzotriazole-5-carboxylic acid following general method A.

### 1.101. Cpd 316

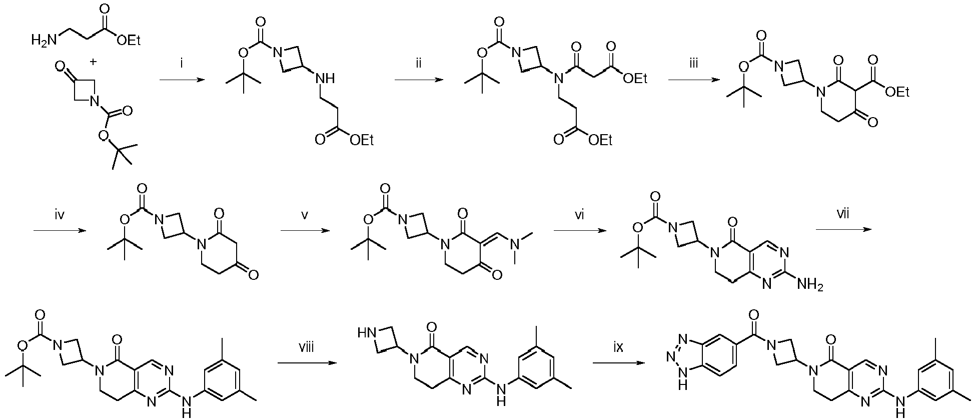

### 1.101.1. Step i

To a solution of tert-butyl 3-oxoazetidine-1-carboxylate (4.0 g, 18.76 mmol, 1.0 eq.) in DCM (100 mL) were added activated 4 Å molecular sieves (8 g), ethyl 3-aminopropanoate (3.95 g, 25.7 mmol, 1.1 eq.), NaOAc (2.2 g, 26.87 mmol, 1.2 eq.) and NaBH(OAc)₃ portionwise (7.43 g, 35.05 mmol, 1.5 eq.) and the mixture was stirred at RT for 12 h. The reaction medium was diluted with water and the phases were separated. The aqueous phase was extracted with DCM. The combined organic layers were washed with a sat. NaHCO₃ aq. solution, dried over MgSO₄, filtered and concentrated to dryness. The residue was purified by flash chromatography on silica gel (eluting with heptane/EtOAc) to afford tert-butyl 3-[(3-ethoxy-3-oxo-propyl)amino] azetidine-1 -carboxylate.
LCMS: MW (calcd): 272.4; m/z MW (obsd): 273.4 (M+H)

### 1.101.2. Step ii

To a solution of tert-butyl 3-[(3-ethoxy-3-oxo-propyl)amino]azetidine-1-carboxylate (3.7 g, 13.64 mmol, 1.0 eq.) in DCM (50 mL) at 0°C were added ethyl 3-chloro-3-oxo-propanoate (2.24 g, 16.37 mmol, 1.2 eq.) and TEA (2.3 mL, 16.37 mmol, 1.2 eq.) and the mixture was stirred at 0°C for 1 h and at RT for 30 min. The reaction medium was then diluted with DCM and washed with water. The organic layer was dried avec MgSO₄, filtered and concentrated to dryness. The residue was purified by flash chromatography on silica gel (eluting with heptane/EtOAc) to afford tert-butyl 3-[(3-ethoxy-3-oxo-propanoyl)-(3-ethoxy-3-oxo-propyl)amino] azetidine-1 -carboxylate.
LCMS: MW (calcd): 386.4; m/z MW (obsd): 387.5 (M+H)

### 1.101.3. Step iii

A solution of NaOEt in EtOH was freshly prepared by dissolution of 1.45 g of sodium in 150 mL of EtOH. Tert-butyl 3-[(3-ethoxy-3-oxo-propanoyl)-(3-ethoxy-3-oxo-propyl)amino]azetidine-1-carboxylate (4.5 g, 11.59 mmol, 1.0 eq.) was dissolved in 29 mL of the above NaOEt solution in EtOH (1.05 eq.) and the mixture was refluxed for 30 min. The reaction medium was then diluted with DCM and washed with a 0.5 M HCl aq. solution and with water. The organic layer was dried over MgSO₄, filtered and concentrated to dryness to afford ethyl 1-(1-tert-butoxycarbonylazetidin-3-yl)-2,4-dioxo-piperidine-3-carboxylate.
LCMS: MW (calcd): 340.4; m/z MW (obsd): 341.5 (M+H)

### 1.101.4. Step iv

To a solution of ethyl 1-(1-tert-butoxycarbonylazetidin-3-yl)-2,4-dioxo-piperidine-3-carboxylate (1 g, 2.94 mmol, 1.0 eq.) in ACN (150 mL) was added water (150 mL) and the mixture was refluxed for 3 h. The reaction mixture was concentrated to dryness. The residue was purified by flash chromatography on silica gel (eluting with heptane/EtOAc) to afford tert-butyl 3-(2,4-dioxo-1-piperidyl)azetidine-1-carboxylate.
LCMS: MW (calcd): 268.3; m/z MW (obsd): 269.4 (M+H)

### 1.101.5. Step v

tert-butyl 3-[(3E)-3-(dimethylaminomethylene)-2,4-dioxo-1-piperidyl]azetidine-1-carboxylate was obtained from tert-butyl 3-(2,4-dioxo-1-piperidyl)azetidine-1-carboxylate following general method F.
LCMS: MW (calcd): 323.4; m/z MW (obsd): 324.4 (M+H)

### 1.101.6. Step vi

Tert-butyl 3-(2-amino-5-oxo-7,8-dihydropyrido[4,3-d]pyrimidin-6-yl)azetidine-1-carboxylate was obtained from tert-butyl 3-[(3E)-3-(dimethylaminomethylene)-2,4-dioxo-1-piperidyl]azetidine-1-carboxylate following general method E.
LCMS: MW (calcd): 319.4; m/z MW (obsd): 320.4 (M+H)

### 1.101.7. Step vii

Tert-butyl 3-[2-(3,5-dimethylanilino)-5-oxo-7,8-dihydropyrido[4,3-d]pyrimidin-6-yl]azetidine-1-carboxylate was obtained from tert-butyl 3-(2-amino-5-oxo-7,8-dihydropyrido[4,3-d]pyrimidin-6-yl)azetidine-1-carboxylate following general method D with 1-bromo-3,5-dimethyl-benzene.
LCMS: MW (calcd): 423.5; m/z MW (obsd): 424.5 (M+H)

### 1.101.8. Step viii

To a solution of tert-butyl 3-[2-(3,5-dimethylanilino)-5-oxo-7,8-dihydropyrido[4,3-d]pyrimidin-6-yl]azetidine-1-carboxylate (25 mg, 0.06 mmol, 1.0 eq.) in 1,4-dioxane (0.5 mL) was added HCl (4N in 1,4-dioxane, 0.2 mL, 0.9 mmol, 15 eq.) and the mixture was stirred at RT for 4 h. The reaction medium was concentrated to dryness, the residue was taken up in Et₂O and the solvent was evaporated in vacuo (done twice). The residue was taken up in ACN (3 mL), potassium carbonate (83 mg, 0.6 mmol, 10 eq.) was added and the mixture was refluxed for 14 h. The reaction medium was filtered, the solid rinsed with DCM and the filtrate was concentrated to dryness to afford 6-(azetidin-3-yl)-2-(3,5-dimethylanilino)-7,8-dihydropyrido[4,3-d]pyrimidin-5-one.
LCMS: MW (calcd): 323.4; m/z MW (obsd): 324.4 (M+H)

### 1.101.9. Step ix

Cpd 316 was obtained from 6-(azetidin-3-yl)-2-(3,5-dimethylanilino)-7,8-dihydropyrido[4,3-d]pyrimidin-5-one and 1H-benzotriazole-5-carboxylic acid following general method A.

**Table II. Intermediates used towards the compounds of the invention**

| **Int#** | **Structure** | **Name** | **SM** | **Mtd** | **MW** | **MS Mes'd** |
|---|---|---|---|---|---|---|
| 1 | 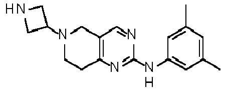 | 6-(azetidin-3-yl)-N-(3,5-dimethylphenyl)-7,8-dihydro-5H-pyrido[4,3-d]pyrimidin-2-amine | Int 2001 + CAS# 398489-26-4 | C + B | 309.4 | 310.3 |
| 2 | 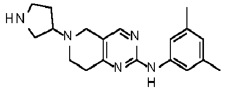 | N-(3,5-dimethylphenyl)-6-pyrrolidin-3-yl-7,8-dihydro-5H-pyrido[4,3-d]pyrimidin-2-amine | Int 2001 + CAS# 101385-93-7 | C + B | 323.4 | 324.3 |
| 3 | 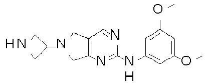 | 6-(azetidin-3-yl)-N-(3,5-dimethoxyphenyl)-5,7-dihydropyrrolo[3,4-d]pyrimidin-2-amine | Int 2003 + CAS# 398489-26-4 | C + B | 327.4 | 328.3 |
| 4 | 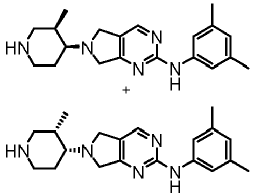 | N-(3,5-dimethylphenyl)-6-((3R,4S)-3-methylpiperidin-4-yl)-6,7-dihydro-5H-pyrrolo[3,4-d]pyrimidin-2-amine | Int 1011 | B | 337.5 | 338.4 |
| 5 | 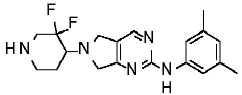 | 6-(3,3-difluoro-4-piperidyl)-N-(3,5-dimethylphenyl)-5,7-dihydropyrrolo[3,4-d]pyrimidin-2-amine | Int 2011 + CAS# 1215071-17-2 | C + B | 359.4 | 360.4 |
| 10 | 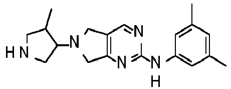 | N-(3,5-dimethylphenyl)-6-(4-methylpyrrolidin-3-yl)-5,7-dihydropyrrolo[3,4-d]pyrimidin-2-amine | Int 2011 + CAS# 362706-25-0 | C + B | 323.4 | 324.4 |
| 11 | 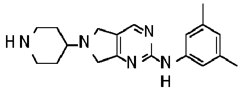 | N-(3,5-dimethylphenyl)-6-(4-piperidyl)-5,7-dihydropyrrolo[3,4-d]pyrimidin-2-amine | Int 2011 + CAS# 79099-07-3 | C + B | 323.4 | 324.4 |
| 13 | 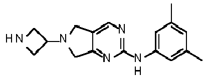 | 6-(azetidin-3-yl)-N-(3,5-dimethylphenyl)-5,7-dihydropyrrolo[3,4-d]pyrimidin-2-amine | Int 2011 + CAS# 398489-26-4 | C + B | 295.4 | 296.4 |
| 14 | 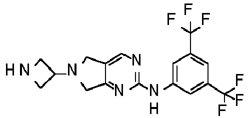 | 6-(azetidin-3-yl)-N-[3,5-bis(trifluoromethyl)phenyl]-5,7-dihydropyrrolo[3,4-d]pyrimidin-2-amine | Int 2014 + CAS# 398489-26-4 | C + B | 403.3 | 404.4 |
| 15 | 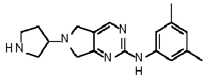 | N-(3,5-dimethylphenyl)-6-pyrrolidin-3-yl-5,7-dihydropyrrolo[3,4-d]pyrimidin-2-amine | Int 2011 + CAS# 101385-93-7 | C + B | 309.4 | 310.4 |
| 17 | 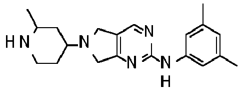 | N-(3,5-dimethylphenyl)-6-(2-methyl-4-piperidyl)-5,7-dihydropyrrolo[3,4-d]pyrimidin-2-amine | Int 2011 + CAS# 362704-66-3 | C + B | 337.5 | 338.4 |
| 19 | 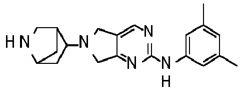 | 6-(2-azabicyclo[2.2.1]heptan-5-yl)-N-(3,5-dimethylphenyl)-5,7-dihydropyrrolo[3,4-d]pyrimidin-2-amine | Int 2011 + CAS# 198835-06-2 | C + B | 335.4 | 336.3 |
| 21 | 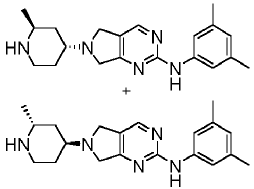 | N-(3,5-dimethylphenyl)-6-[(2R,4S/2S,4R)-2-methyl-4-piperidyl]-5,7-dihydropyrrolo[3,4-d]pyrimidin-2-amine | Int 2011 + CAS# 190906-92-4 | C + B | 337.5 | 338.4 |
| 25 | 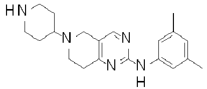 | N-(3,5-dimethylphenyl)-6-(4-piperidyl)-7,8-dihydro-5H-pyrido[4,3-d]pyrimidin-2-amine | Int 2001 + CAS# 79099-07-3 | C + B | 337.5 | 338.2 |
| 26 | 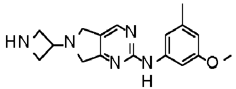 | 6-(azetidin-3-yl)-N-(3-methoxy-5-methyl-phenyl)-5,7-dihydropyrrolo[3,4-d]pyrimidin-2-amine | Int 2026 + CAS# 398489-26-4 | C + B | 311.4 | 312.3 |
| 29 | 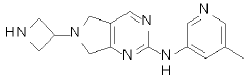 | 6-(azetidin-3-yl)-N-(5-methyl-3-pyridyl)-5,7-dihydropyrrolo[3,4-d]pyrimidin-2-amine | Int 2029 + CAS# 398489-26-4 | C + B | 282.3 | 283.2 |
| 30 | 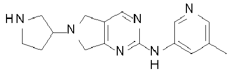 | N-(5-methyl-3-pyridyl)-6-pyrrolidin-3-yl-5,7-dihydropyrrolo[3,4-d]pyrimidin-2-amine | Int 2029 + CAS# 101385-93-7 | C + B | 296.4 | 297.3 |
| 31 | 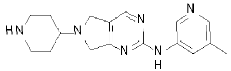 | N-(5-methyl-3-pyridyl)-6-(4-piperidyl)-5,7-dihydropyrrolo[3,4-d]pyrimidin-2-amine | Int 2029 + CAS# 79099-07-3 | C + B | 310.4 | 311.3 |
| 32 | 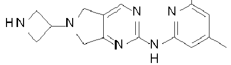 | 6-(azetidin-3-yl)-N-(4,6-dimethyl-2-pyridyl)-5,7-dihydropyrrolo[3,4-d]pyrimidin-2-amine | Int 2032 + CAS# 398489-26-4 | C + B | 296.4 | 297.3 |
| 33 | 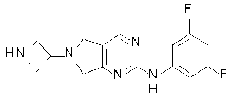 | 6-(azetidin-3-yl)-N-(3,5-difluorophenyl)-5,7-dihydropyrrolo[3,4-d]pyrimidin-2-amine | Int 2033 + CAS# 398489-26-4 | C + B | 303.3 | 304.2 |
| 34 | 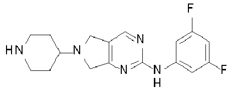 | N-(3,5-difluorophenyl)-6-(4-piperidyl)-5,7-dihydropyrrolo[3,4-d]pyrimidin-2-amine | Int 2033 + CAS# 79099-07-3 | C + B | 331.4 | 332.2 |
| 37 | 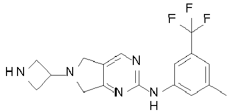 | 6-(azetidin-3-yl)-N-[3-methyl-5-(trifluoromethyl)phenyl]-5,7-dihydropyrrolo[3,4-d]pyrimidin-2-amine | Int 3037 + CAS# 398489-26-4 | B + C + B | 349.4 | 350.3 |
| 38 | 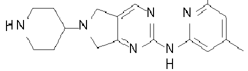 | N-(4,6-dimethyl-2-pyridyl)-6-(4-piperidyl)-5,7-dihydropyrrolo[3,4-d]pyrimidin-2-amine | Int 2032 + CAS# 79099-07-3 | C + B | 324.4 | 325.1 |
| 39 | 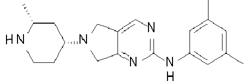 | N-(3,5-dimethylphenyl)-6-[(2R,4R)-2-methyl-4-piperidyl]-5,7-dihydropyrrolo[3,4-d]pyrimidin-2-amine | Int 2011 + CAS# 790667-43-5 | C + B | 337.5 | 338.1 |
| 40 | 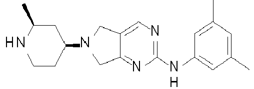 | N-(3,5-dimethylphenyl)-6-[(2S,4S)-2-methyl-4-piperidyl]-5,7-dihydropyrrolo[3,4-d]pyrimidin-2-amine | Int 2011 + CAS# 790667-49-1 | C + B | 337.5 | 338.1 |
| 41 | 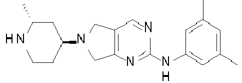 | N-(3,5-dimethylphenyl)-6-[(2R,4S)-2-methyl-4-piperidyl]-5,7-dihydropyrrolo[3,4-d]pyrimidin-2-amine | Int 2011 + CAS# 790667-43-5 | C + B | 337.5 | 338.1 |
| 43 | 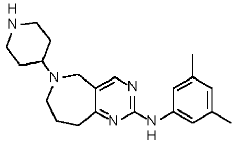 | N-(3,5-dimethylphenyl)-6-(4-piperidyl)-5,7,8,9-tetrahydropyrimido[5,4-c]azepin-2-amine | Int 1043 | B | 351.5 | 352.1 |
| 45 | 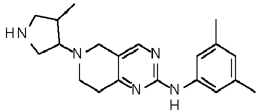 | N-(3,5-dimethylphenyl)-6-(4-methylpyrrolidin-3-yl)-7,8-dihydro-5H-pyrido[4,3-d]pyrimidin-2-amine | Int 2001 + CAS 362706-25-0 | C + B | 337.5 | 338.2 |
| 46 | 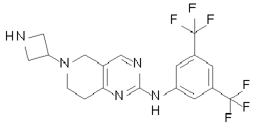 | 6-(azetidin-3-yl)-N-[3,5-bis(trifluoromethyl)phenyl]-7,8-dihydro-5H-pyrido[4,3-d]pyrimidin-2-amine | Int 4001 + CAS# 328-70-1 + CAS# 398489-26-4 | D + B + C + B | 417.4 | 418.2 |
| 47 | 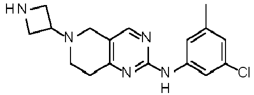 | 6-azetidin-3-yl)-N-(3-chloro-5-methyl-phenyl)-7,8-dihydro-5H-pyrido[4,3-d]pyrimidin-2-amine | Int 4001 + CAS# 329944-72-1 + CAS# 398489-26-4 | D + B + C + B | 329.8 | 330.3 - 332.3 |
| 48 | 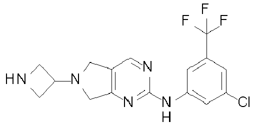 | 6-(azetidin-3-yl)-N-[3-chloro-5-(trifluoromethyl)phenyl]-5,7-dihydropyrrolo[3,4-d]pyrimidin-2-amine | Int 4011 + CAS# 928783-85-1 + CAS# 398489-26-4 | D + B + C + B | 369.8 | 370.2 - 372.2 |
| 49 | 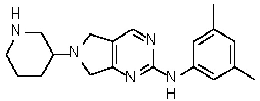 | N-(3,5-dimethylphenyl)-6-(3-piperidyl)-5,7-dihydropyrrolo[3,4-d]pyrimidin-2-amine | Int 2011 + CAS# 98977-36-7 | C + B | 323.4 | 324.4 |
| 52 | 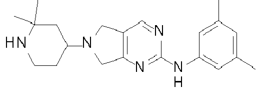 | N-(3,5-dimethylphenyl)-6-(2,2-dimethyl-4-piperidyl)-5,7-dihydropyrrolo[3,4-d]pyrimidin-2-amine | Int 2011 + CAS# 346593-03-1 | C + B | 351.5 | 352.5 |
| 53 | 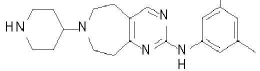 | N-(3,5-dimethylphenyl)-7-(4-piperidyl)-5,6,8,9-tetrahydropyrimido[4,5-d]azepin-2-amine | CAS# 141642-82-2 | Desc' d | 351.5 | 352.5 |
| 57 | 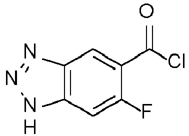 | 6-fluoro-1H-benzotriazole-5-carbonyl chloride | CAS# 1427081-62-6 | Desc' d | 199.6 | no data |
| 58 | 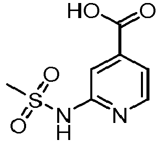 | 2-(methanesulfonamido)pyridine-4-carboxylic acid | CAS# 6937-03-7 | Desc' d | 216.2 | 217.2 |
| 59 | 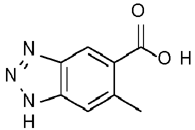 | 6-methyl-1H-benzotriazole-5-carboxylic acid | Int 137 | Desc' d | 177.2 | 178.2 |
| 60 | 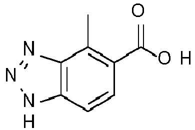 | 4-methyl-1H-benzotriazole-5-carboxylic acid | Int 138 | Desc' d | 177.2 | 178.3 |
| 61 | 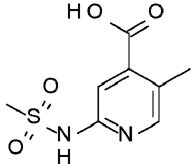 | 2-(methanesulfonamido)-5-methyl-pyridine-4-carboxylic acid | Int 139 | Desc' d | 230.2 | 231.1 |
| 62 | 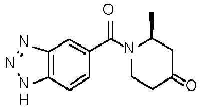 | (2S)-1-(1H-benzotriazole-5-carbonyl)-2-methyl-piperidin-4-one | CAS# 23814-12-2 + CAS# 790667 -45-7 | A | 258.3 | 259.3 |
| 63 | 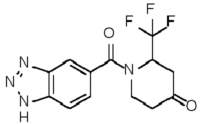 | 1-(1H-benzotriazole-5-carbonyl)-2-(trifluoromethyl)piperidin-4-one | CAS# 1245648-32-1 + CAS# 46053-85-4 | Desc' d | 312.2 | 313.3 |
| 64 | 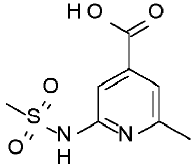 | 2-(methanesulfonamido)-6-methyl-pyridine-4-carboxylic acid | CAS# 5398-44-7 | Desc' d | 230.2 | 231.2 |
| 65 | 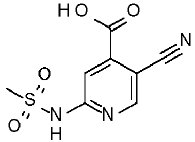 | 5-cyano-2-(methanesulfonamido)pyridine-4-carboxylic acid | Int 139 | Desc' d | 241.2 | 242.2 |
| 66 | 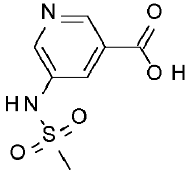 | 5-(methanesulfonamido)pyridine-3-carboxylic acid | CAS# 24242-19-1 | Desc' d | 216.2 | no data |
| 67 | 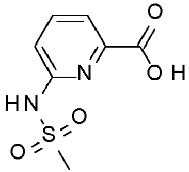 | 6-(methanesulfonamido)pyridine-2-carboxylic acid | CAS# 23628-31-1 | Desc' d | 216.2 | no data |
| 68 | 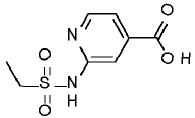 | 2-(ethylsulfonylamino)pyridin e-4-carboxylic acid | CAS# 6937-03-7 | Desc' d | 230.2 | 231.1 |
| 69 | 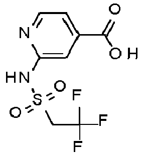 | 2-(2,2,2-trifluoroethylsulfonylamino)pyridine-4-carboxylic acid | CAS# 6937-03-7 | Desc' d | 284.2 | no data |
| 70 | 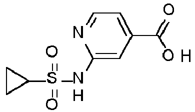 | 2-(cyclopropylsulfonylamino)pyridine-4-carboxylic acid | CAS# 26156-48-9 | Desc' d | 242.3 | 243.1 |
| 71 | 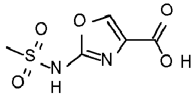 | 2-(methanesulfonamido)oxazole-4-carboxylic acid | CAS# 177760-52-0 | Desc' d | 206.2 | no data |
| 72 | 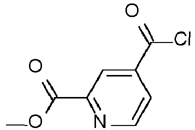 | methyl 4-chlorocarbonylpyridine-2-carboxylate | CAS# 24195-10-6 | Desc' d | 199.6 | no data |
| 73 | 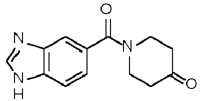 | 1-(1H-benzimidazole-5-carbonyl)piperidin-4-one | CAS# 15788-16-6 + CAS# 41979-39-9 | A | 243.3 | 244.3 |
| 74 | 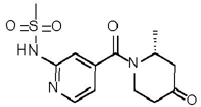 | N-[4-[(2R)-2-methyl-4-oxo-piperidine-1-carbonyl]-2-pyridyl]methanesulfonamide | Int 58 + CAS# 1434126-97-2 | A | 311.4 | 312.3 |
| 75 | 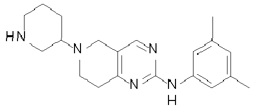 | N-(3,5-dimethylphenyl)-6-(3-piperidyl)-7,8-dihydro-5H-pyrido[4,3-d]pyrimidin-2-amine | Int 2011 + CAS# 98977-36-7 | C + B | 337.5 | 338.4 |
| 76 | 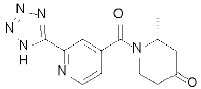 | (2R)-2-methyl-1-[2-(1H-tetrazol-5-yl)pyridine-4-carbonyl]piperidin-4-one | Int 128 | Desc' d | 286.3 | 287.4 |
| 77 | 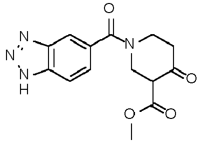 | methyl 1-(1H-benzotriazole-5-carbonyl)-4-oxo-piperidine-3-carboxylate | CAS# 23814-12-2 + CAS# 71486-53-8 | A | 302.3 | 303.3 |
| 78 | 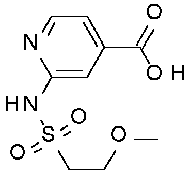 | 2-(2-methoxyethylsulfonylamino)pyridine-4-carboxylic acid | CAS# 6937-03-7 | Desc' d | 260.3 | no data |
| 79 | 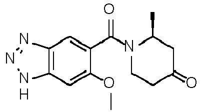 | (2S)-1-(6-methoxy-1H-benzotriazole-5-carbonyl)-2-methyl-piperidin-4-one | CAS# 790667-45-7 + CAS# 59338-92-0 | A | 288.3 | 289.3 |
| 80 | 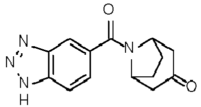 | 8-(1H-benzotriazole-5-carbonyl)-8-azabicyclo[3.2.1]octan-3-one | CAS# 25602-68-0 + CAS# 23814-12-2 | A | 270.3 | 271.3 |
| 81 | 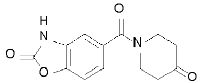 | 5-(4-oxopiperidine-1-carbonyl)-3H-1,3-benzoxazol-2-one | CAS# 41979-39-9 + CAS# 54903-16-1 | A | 260.2 | 261.3 |
| 82 | 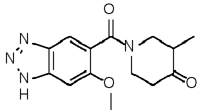 | 1-(6-methoxy-1H-benzotriazole-5-carbonyl)-3-methyl-piperidin-4-one | CAS# 4629-78-1 + CAS# 59338-92-0 | A | 288.3 | 289.4 |
| 83 | 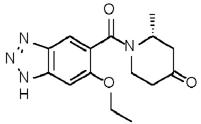 | (2R)-1-(6-ethoxy-1H-benzotriazole-5-carbonyl)-2-methyl-piperidin-4-one | CAS# 213627-49-7 + CAS# 1434126-97-2 | A | 302.3 | 303.4 |
| 84 | 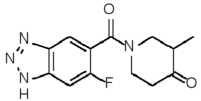 | 1-(6-fluoro-1H-benzotriazole-5-carbonyl)-3-methyl-piperidin-4-one | CAS# 1427081-62-6 + CAS# 4629-78-1 | A | 276.3 | 277.3 |
| 85 | 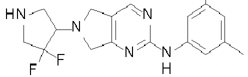 | 6-(4,4-difluoropyrrolidin-3-yl)-N-(3,5-dimethylphenyl)-5,7-dihydropyrrolo[3,4-d]pyrimidin-2-amine | Int 2011 + CAS# 1215071-16-1 | C + B | 345.4 | 346.4 |
| 88 | 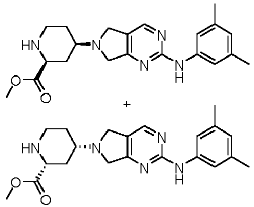 | methyl (2S,4R/2R,4S) -4-[2-(3,5-dimethyl anilino)-5,7-dihydro pyrrolo[3,4-d]pyrimidin-6-yl]piperidine-2-carboxylate | Int 2011 + CAS# 81357-18-8 | C + B | 381.5 | 382.5 |
| 89 | 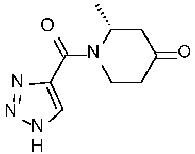 | (2R)-2-methyl-1-(1H-triazole-4-carbonyl)piperidin-4-one | CAS# 1434126-97-2 + CAS# 16681-70-2 | A | 208.2 | 209.3 |
| 103 | 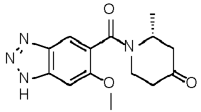 | (2R)-1-(6-methoxy-1H-benzotriazole-5-carbonyl)-2-methyl-piperidin-4-one | CAS# 1434126-97-2 + CAS# 59338-92-0 | Desc' d | 288.3 | 289.3 |
| 104 | 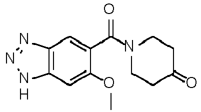 | 1-(6-methoxy-1H-benzotriazole-5-carbonyl)piperidin-4-one | CAS# 41979-39-9 + CAS# 59338-92-0 | Desc' d | 274.3 | 275.3 |
| 105 | 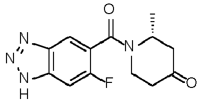 | (2R)-1-(6-fluoro-1H-benzotriazole-5-carbonyl)-2-methyl-piperidin-4-one | CAS# 1434126-97-2 + CAS# 1427081-62-6 | Desc' d | 276.3 | no data |
| 106 | 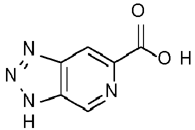 | 3H-triazolo[4,5-c]pyridine-6-carboxylic acid | CAS# 850689-13-3 | Desc' d | 164.1 | no data |
| 108 | 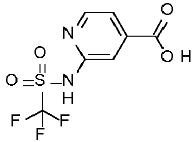 | 2-(trifluoromethylsulfonylamino)pyridine-4-carboxylic acid | CAS# 6937-03-7 | Desc' d | 270.2 | no data |
| 111 | 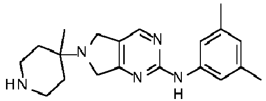 | N-(3,5-dimethylphenyl)-6-(4-methyl-4-piperidyl)-5,7-dihydropyrrolo[3,4-d]pyrimidin-2-amine | CAS# 79099-07-3 | Desc' d | 337.5 | 338.5 |
| 113 | 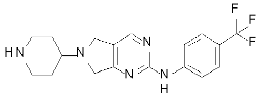 | 6-(4-piperidyl)-N-[4-(trifluoromethyl)phenyl]-5,7-dihydropyrrolo[3,4-d]pyrimidin-2-amine | Int 2016 + CAS# 79099-07-3 | C + B | 363.4 | 364.4 |
| 119 | 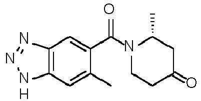 | (2R)-2-methyl-1 -(6-methyl-1H-benzotriazole-5-carbonyl)piperidin-4-one | Int 59 + CAS# 1434126-97-2 | A | 272.3 | 273.3 |
| 120 | 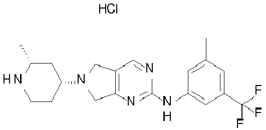 | 6-[(2R,4R)-2-methyl-4-piperidyl]-N-[3-methyl-5-(trifluoromethyl)phenyl]-5,7-dihydropyrrolo[3,4-d]pyrimidin-2-amine;hydrochloride | Int 2071 + CAS# 790667-43-5 | C + B | 391.4 | 392.5 |
| 122 | 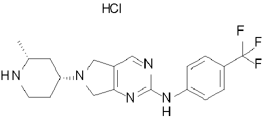 | 6-[(2R,4R)-2-methyl-4-piperidyl]-N-[4-(trifluoromethyl)phenyl]-5,7-dihydropyrrolo[3,4-d]pyrimidin-2-amine;hydrochloride | Int 2016 + CAS# 790667-43-5 | C + B | 377.4 | 378.2 |
| 124 | 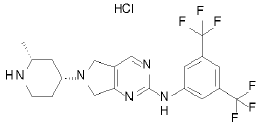 | N-[3,5-bis(trifluoromethyl)phenyl]-6-[(2R,4R)-2-methyl-4-piperidyl]-5,7-dihydropyrrolo[3,4-d]pyrimidin-2-amine;hydrochloride | Int 2014 + CAS# 790667-43-5 | C + B | 445.4 | 446.5 |
| 126 | 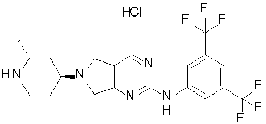 | N-[3,5-bis(trifluoromethyl)phenyl]-6-[(2R,4S)-2-methyl-4-piperidyl]-5,7-dihydropyrrolo[3,4-d]pyrimidin-2-amine;hydrochloride | Int 2014 + CAS# 790667-43-5 | C + B | 445.4 | 446.2 |
| 129 | 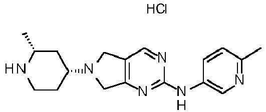 | 6-[(2R,4R)-2-methyl-4-piperidyl]-N-(6-methyl-3-pyridyl)-5,7-dihydropyrrolo[3,4-d]pyrimidin-2-amine;hydrochloride | Int 130 | B | 324.4 | 325.2 |
| 130 | 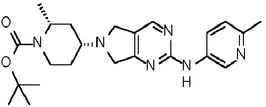 | tert-butyl (2R,4R)-2-methyl-4-[2-[(6-methyl-3-pyridyl)amino]-5,7-dihydropyrrolo[3,4-d]pyrimidin-6-yl]piperidine-1-carboxylate | Int 2056 + CAS# 790667-43-5 | Desc' d | 424.5 | 425.4 |
| 131 | 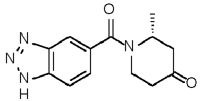 | (2R)- 1 -(1 H-benzotriazole-5-carbonyl)-2-methyl-piperidin-4-one | CAS# 23814-12-2 + CAS# 1434126-97-2 | Desc' d | 258.3 | 259.3 |
| 134 | 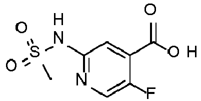 | 5-fluoro-2-(methanesulfonamido)pyridine-4-carboxylic acid | CAS# 885588-12-5 | Desc' d | 234.2 | no data |
| 128 | 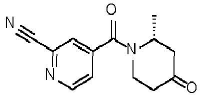 | 4-[(2R)-2-methyl-4-oxo-piperidine-1-carbonyl]pyridine-2-carbonitrile | CAS# 53234-55-2 + CAS# 1434126-97-2 | A | 243.3 | no data |
| 135 | 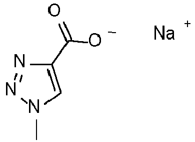 | sodium; 1-methyltriazole-4-carboxylate | CAS# 57362-82-0 | Desc' d | 127.1 | no UPL C data |
| 137 | 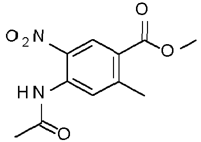 | methyl 4-acetamido-2-methyl-5-nitro-benzoate | CAS# 103204-69-9 | Desc' d | 252.2 | 253.3 |
| 138 | 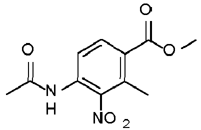 | methyl 4-acetamido-2-methyl-3-nitro-benzoate | CAS# 103204-69-9 | Desc' d | 252.2 | 253.3 |
| 139 | 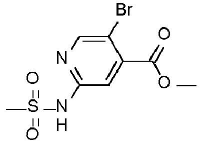 | methyl 5-bromo-2-(methanesulfonamido)pyridine-4-carboxylate | CAS# 882499-87-8 | Desc' d | 309.1 | 309.1 311.1 |
| 141 | 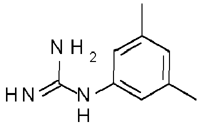 | 1-(3,5-dimethylphenyl)guanidine | CAS# 108-69-0 | Desc' d | 163.2 | 164.5 |
| 1011 | 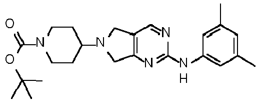 | tert-butyl 4-[2-(3,5-dimethylanilino)-5,7-dihydropyrrolo[3,4-d]pyrimidin-6-yl]piperidine-1-carboxylate | Int 2011 + CAS# 2091951-57-2 | C | 423.6 | 424.4 |
| 1043 | 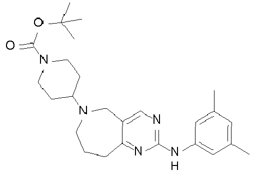 | tert-butyl 4-[2-(3,5-dimethylanilino)-5,7,8,9-tetrahydropyrimido[5,4-c]azepin-6-yl]piperidine-1-carboxylate | Int 2043 + CAS# 79099-07-3 | Desc' d | 451.6 | 452.1 |
| 1044 | 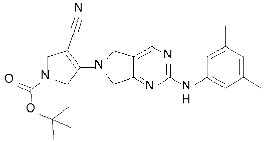 | tert-butyl 3-cyano-4-[2-(3,5-dimethylanilino)-5,7-dihydropyrrolo[3,4-d]pyrimidin-6-yl]-2,5-dihydropyrrole-1-carboxylate | Int 2011 + CAS# 175463-32-8 | Desc' d | 432.5 | 433.4 |
| 2001 | 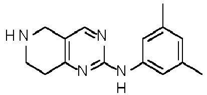 | N-(3,5-dimethylphenyl)-5,6,7,8-tetrahydropyrido[4,3-d]pyrimidin-2-amine | Int 3001 | B | 254.3 | 255.2 |
| 2003 | 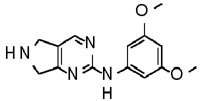 | N-(3,5-dimethoxyphenyl)-6,7-dihydro-5H-pyrrolo[3,4-d]pyrimidin-2-amine | Int 4011 + CAS# 20469-65-2 | D + B | 272.3 | 273.2 |
| 2007 | 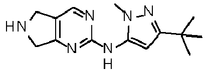 | N-(5-tert-butyl-2-methyl-pyrazol-3-yl)-6,7-dihydro-5H-pyrrolo[3,4-d]pyrimidin-2-amine | Int 3007 | B | 272.3 | 273.3 |
| 2011 | 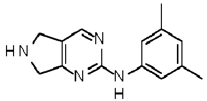 | N -(3,5-dimethylphenyl)-6,7-dihydro-5H-pyrrolo[3,4-d]pyrimidin-2-amine | Int 3011 | B | 240.3 | 241.2 |
| 2014 | 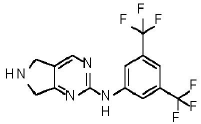 | N-[3,5-bis(trifluoromethyl)phenyl]-6,7-dihydro-5H-pyrrolo[3,4-d]pyrimidin-2-amine | Int 4011 + CAS# 328-70-1 | D + B | 348.2 | 349.4 |
| 2016 | 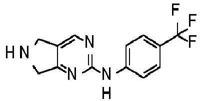 | N-[4-(trifluoromethyl)phenyl]-6,7-dihydro-5H-pyrrolo[3,4-d]pyrimidin-2-amine | Int 4011 + CAS# 402-43-7 | D + B | 280.2 | 281.2 |
| 2026 | 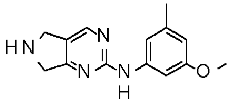 | N-(3-methoxy-5-methylphenyl)-6,7-dihydro-5H-pyrrolo[3,4-d]pyrimidin-2-amine | Int 4011 + CAS# 29578-83-4 | D + B | 256.3 | 257.2 |
| 2029 | 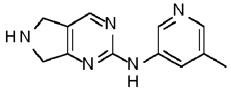 | N-(5-methyl-3-pyridyl)-6,7-dihydro-5H-pyrrolo[3,4-d]pyrimidin-2-amine | Int 4011 + CAS# 3430-16-8 | D + B | 227.3 | 228.2 |
| 2032 | 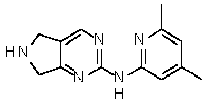 | N-(4,6-dimethyl-2-pyridyl)-6,7-dihydro-5H-pyrrolo[3,4-d]pyrimidin-2-amine | Int 4011 + CAS# 4926-26-5 | D + B | 241.3 | 242.2 |
| 2033 | 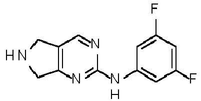 | N-(3,5-difluorophenyl)-6,7-dihydro-5H-pyrrolo[3,4-d]pyrimidin-2-amine | Int 4011 + CAS# 461-96-1 | D + B | 248.2 | 249.1 |
| 2035 | 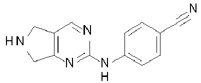 | 4-(6,7-dihydro-5H-pyrrolo[3,4-d]pyrimidin-2-ylamino)benzonitrile | Int 4011 + CAS# 623-00-7 | C + B | 237.3 | 238.1 |
| 2043 | 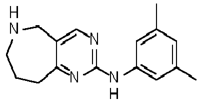 | N-(3 ,5-dimethylphenyl)-6,7,8,9-tetrahydro-5H-pyrimido [5,4-c] azepin-2-amine | CAS# 188975-88-4 + CAS# 556-96-7 | F + E + D + B | 268.4 | 269.4 |
| 2049 | 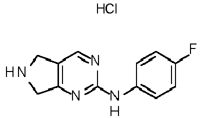 | N-(4-fluorophenyl)-6,7-dihydro-5H-pyrrolo[3,4-d]pyrimidin-2-amine;hydrochloride | Int 4011 + CAS# 460-00-4 | D + B | 230.2 | 231.0 |
| 2050 | 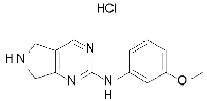 | N-(3-methoxyphenyl)-6,7-dihydro-5H-pyrrolo[3,4-d]pyrimidin-2-amine;hydrochloride | Int 4011 + CAS# 2398-37-0 | D + B | 242.3 | 243.3 |
| 2052 | 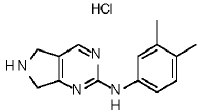 | N-(3,4-dimethylphenyl)-6,7-dihydro-5H-pyrrolo[3,4-d]pyrimidin-2-amine;hydrochloride | Int 4011 + CAS# 583-71-1 | D + B | 240.3 | 241.5 |
| 2053 | 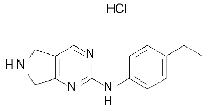 | N-(4-ethylphenyl)-6,7-dihydro-5H-pyrrolo[3,4-d]pyrimidin-2-amine;hydrochloride | Int 4011 + CAS# 1585-07-5 | D + B | 240.3 | 241.6 |
| 2055 | 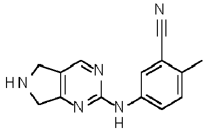 | 5-(6,7-dihydro-5H-pyrrolo[3,4-d]pyrimidin-2-ylamino)-2-methyl-benzonitrile | Int 4011 + CAS# 156001-51-3 | D + B | 251.3 | 252.3 |
| 2056 | 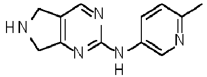 | N-(6-methyl-3-pyridyl)-6,7-dihydro-5H-pyrrolo[3,4-d]pyrimidin-2-amine | Int 4011 + CAS# 3430-13-5 | D + B | 227.3 | 228.3 |
| 2057 | 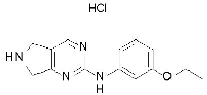 | N-(3-ethoxyphenyl)-6,7-dihydro-5H-pyrrolo[3,4-d]pyrimidin-2-amine;hydrochloride | Int 4011 + CAS# 2655-84-7 | D + B | 256.3 | 257.5 |
| 2058 | 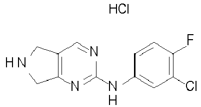 | N-(3-chloro-4-fluorophenyl)-6,7-dihydro-5H-pyrrolo[3,4-d]pyrimidin-2-amine;hydrochloride | Int 4011 + CAS# 60811-21-4 | D + B | 264.7 | 265.3 267.3 |
| 2059 | 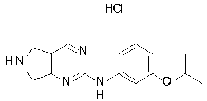 | N-(3-isopropoxyphenyl)-6,7-dihydro-5H-pyrrolo[3,4-d]pyrimidin-2-amine;hydrochloride | Int 4011 + CAS# 131738-73-3 | D + B | 270.3 | 271.5 |
| 2060 | 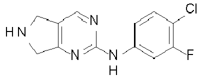 | N-(4-chloro-3-fluorophenyl)-6,7-dihydro-5H-pyrrolo[3,4-d]pyrimidin-2-amine;hydrochloride | Int 4011 + CAS# 60811-18-9 | D + B | 264.7 | 265.3 |
| 2061 | 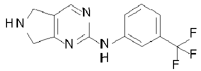 | N-[3-(trifluoromethyl)phenyl]-6,7-dihydro-5H-pyrrolo[3,4-d]pyrimidin-2-amine;hydrochloride | Int 4011 + CAS# 401-78-5 | D + B | 280.2 | 281.3 |
| 2062 | 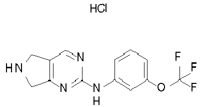 | N-[3-(trifluoromethoxy)phenyl]-6,7-dihydro-5H-pyrrolo[3,4-d]pyrimidin-2-amine;hydrochloride | Int 4011 + CAS# 2252-44-0 | D + B | 296.2 | 297.2 |
| 2063 | 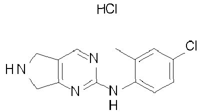 | N-(4-chloro-2-methylphenyl)-6,7-dihydro-5H-pyrrolo[3,4-d]pyrimidin-2-amine;hydrochloride | Int 4011 + CAS# 14495-51-3 | D + B | 260.7 | 261.2 |
| 2064 | 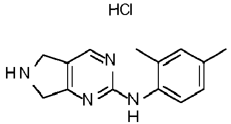 | N-(2,4-dimethylphenyl)-6,7-dihydro-5H-pyrrolo[3,4-d]pyrimidin-2-amine,hydrochloride | Int 4011 + CAS# 583-70-0 | D + B | 240.3 | 241.3 |
| 2065 | 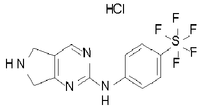 | N-[4-(pentafluoro-λ⁶-sulfanyl)phenyl]-6,7-dihydro-5H-pyrrolo[3,4-d]pyrimidin-2-amine;hydrochloride | Int 4011 + CAS# 774-93-6 | D + B | 338.3 | 339.5 |
| 2066 | 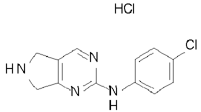 | N-(4-chlorophenyl)-6,7-dihydro-5H-pyrrolo[3,4-d]pyrimidin-2-amine;hydrochloride | Int 4011 + CAS# 106-39-8 | D + B | 246.7 | 247.5 |
| 2067 | 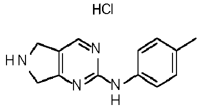 | N-(p-tolyl)-6,7-dihydro-5H-pyrrolo[3,4-d]pyrimidin-2-amine;hydrochloride | Int 4011 + CAS# 106-38-7 | D + B | 226.3 | 227.3 |
| 2068 | 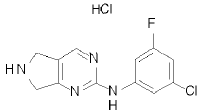 | N-(3-chloro-5-fluorophenyl)-6,7-dihydro-5H-pyrrolo[3,4-d]pyrimidin-2-amine;hydrochloride | Int 4011 + CAS# 33863-76-2 | D + B | 264.7 | 265.5 267.2 |
| 2069 | 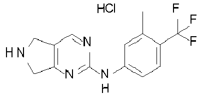 | N-[3-methyl-4-(trifluoromethyl)phenyl]-6,7-dihydro-5H-pyrrolo[3,4-d]pyrimidin-2-amine;hydrochloride | Int 4011 + CAS# 936092-88-5 | D + B | 294.3 | 295.5 |
| 2070 | 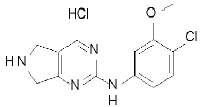 | N-(4-chloro-3-methoxyphenyl)-6,7-dihydro-5H-pyrrolo[3,4-d]pyrimidin-2-amine;hydrochloride | Int 4011 + CAS# 16817-43-9 | D + B | 276.7 | 277.3 |
| 2071 | 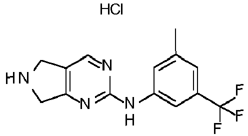 | N-[3-methyl-5-(trifluoromethyl)phenyl]-6,7-dihydro-5H-pyrrolo[3,4-d]pyrimidin-2-amine;hydrochloride | Int 4011 + CAS# 86845-28-5 | D + B | 294.3 | 295.3 |
| 2072 | 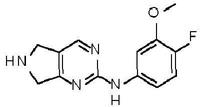 | N-(4-fluoro-3-methoxyphenyl)-6,7-dihydro-5H-pyrrolo[3,4-d]pyrimidin-2-amine | Int 4011 + CAS# 103291-07-2 | D + B | 260.3 | 261.6 |
| 2073 | 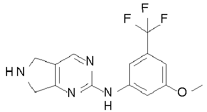 | N-[3-methoxy-5-(trifluoromethyl)phenyl]-6,7-dihydro-5H-pyrrolo[3,4-d]pyrimidin-2-amine | Int 4011 + CAS# 627527-23-5 | D + B | 310.3 | 311.4 |
| 2074 | 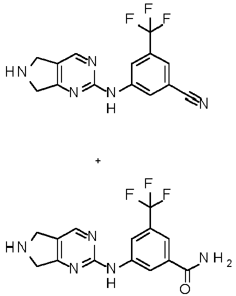 | 3-(6,7-dihydro-5H-pyrrolo[3,4-d]pyrimidin-2-ylamino)-5-(trifluoromethyl)benzonitrile / 3-(6,7-dihydro-5H-pyrrolo[3,4-d]pyrimidin-2-ylamino)-5-(trifluoromethyl)benzamide mixture | Int 4011 + CAS# 691877-03-9 | Desc' d | 305.3 + 323.3 | 306.3 + 324.4 |
| 2076 | 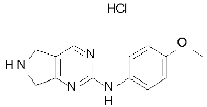 | N-(4-methoxyphenyl)-6,7-dihydro-5H-pyrrolo[3,4-d]pyrimidin-2-amine;hydrochloride | Int 4011 + CAS# 104-92-7 | D + B | 242.3 | no data |
| 2077 | 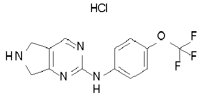 | N-[4-(trifluoromethoxy)phenyl]-6,7-dihydro-5H-pyrrolo[3,4-d]pyrimidin-2-amine;hydrochloride | Int 4011 + CAS# 407-14-7 | D + B | 296.2 | 297.3 |
| 2078 | 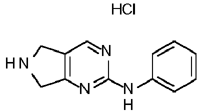 | N-phenyl-6,7-dihydro-5H-pyrrolo[3,4-d]pyrimidin-2-amine;hydrochloride | Int 4011 + CAS# 108-86-1 | D + B | 212.3 | 213.0 |
| 2079 | 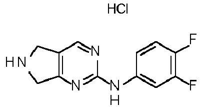 | N-(3,4-difluorophenyl)-6,7-dihydro-5H-pyrrolo[3,4-d]pyrimidin-2-amine;hydrochloride | Int 4011 + CAS# 348-61-8 | D + B | 248.2 | 249.3 |
| 2080 | 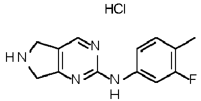 | N-(3-fluoro-4-methylphenyl)-6,7-dihydro-5H-pyrrolo[3,4-d]pyrimidin-2-amine;hydrochloride | Int 4011 + CAS# 51436-99-8 | D + B | 244.3 | 245.6 |
| 2081 | 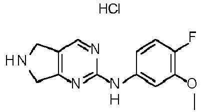 | N-(4-fluoro-3-methoxyphenyl)-6,7-dihydro-5H-pyrrolo[3,4-d]pyrimidin-2-amine;hydrochloride | Int 4011 + CAS# 103291-07-2 | D + B | 260.3 | 261.3 |
| 2082 | 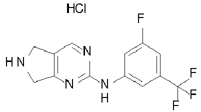 | N-[3-fluoro-5-(trifluoromethyl)phenyl]-6,7-dihydro-5H-pyrrolo[3,4-d]pyrimidin-2-amine;hydrochloride | Int 4011 + CAS# 130723-13-6 | D + B | 298.2 | 299.2 |
| 2083 | 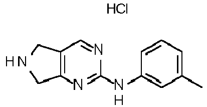 | N-(m-tolyl)-6,7-dihydro-5H-pyrrolo[3,4-d]pyrimidin-2-amine;hydrochloride | Int 4011 + CAS# 591-17-3 | D + B | 226.3 | 227.3 |
| 2084 | 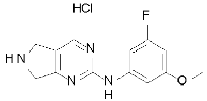 | N-(3-fluoro-5-methoxyphenyl)-6,7-dihydro-5H-pyrrolo[3,4-d]pyrimidin-2-amine;hydrochloride | Int 4011 + CAS# 29578-39-0 | D + B | 260.3 | 261.3 |
| 2085 | 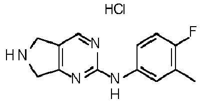 | N-(4-fluoro-3-methylphenyl)-6,7-dihydro-5H-pyrrolo[3,4-d]pyrimidin-2-amine;hydrochloride | Int 4011 + CAS# 51437-00-4 | D + B | 244.3 | 245.0 |
| 2086 | 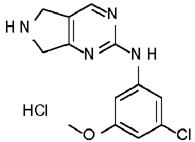 | N-(3-chloro-5-methoxyphenyl)-6,7-dihydro-5H-pyrrolo[3,4-d]pyrimidin-2-amine;hydrochloride | Int 3086 | B | 276.7 | 277.2 |
| 3086 | 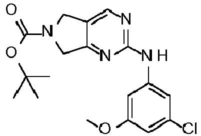 | tert-butyl 2-(3-chloro-5-methoxy-anilino)-5,7-dihydropyrrolo[3,4-d]pyrimidine-6-carboxylate | Int 4011 + CAS# 51437-00-4 | D | 376.8 | 377.3 |
| 2087 | 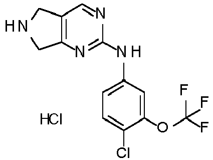 | N-[4-chloro-3-(trifluoromethoxy)phenyl]-6,7-dihydro-5H-pyrrolo[3,4-d]pyrimidin-2-amine;hydrochloride | Int 3087 | B | 330.7 | 331.2 |
| 2088 | 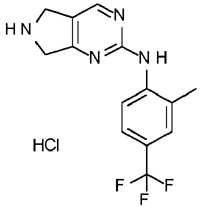 | N-[2-methyl-4-(trifluoromethyl)phenyl]-6,7-dihydro-5H-pyrrolo[3,4-d]pyrimidin-2-amine;hydrochloride | Int 4011 + CAS# 929000-62-4 | D + B | 294.3 | 295.4 |
| 2089 | 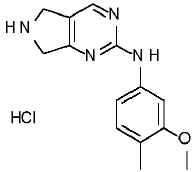 | N-(3-methoxy-4-methylphenyl)-6,7-dihydro-5H-pyrrolo[3,4-d]pyrimidin-2-amine;hydrochloride | Int 4011 + CAS# 67868-73-9 | D + B | 256.3 | 257.3 |
| 2090 | 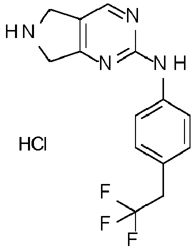 | N-[4-(2,2,2-trifluoroethyl)phenyl]-6,7-dihydro-5H-pyrrolo[3,4-d]pyrimidin-2-amine;hydrochloride | Int 4011 + CAS# 155820-88-5 | D + B | 294.3 | 295.6 |
| 2091 | 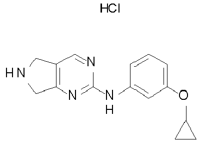 | N-[3-(cyclopropoxy)phenyl]-6,7-dihydro-5H-pyrrolo[3,4-d]pyrimidin-2-amine;hydrochloride | Int 4011 + CAS# 1035690-22-2 | D + B | 268.3 | 269.5 |
| 2092 | 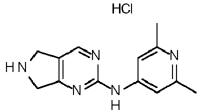 | N-(2,6-dimethyl-4-pyridyl)-6,7-dihydro-5H-pyrrolo[3,4-d]pyrimidin-2-amine;hydrochloride | Int 4011 + CAS# 5093-70-9 | D + B | 241.3 | 242.2 |
| 2095 | 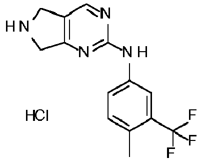 | N-[4-methyl-3-(trifluoromethyl)phenyl]-6,7-dihydro-5H-pyrrolo[3,4-d]pyrimidin-2-amine;hydrochloride | Int 4011 + CAS# 86845-27-4 | D + B | 294.3 | 295.0 |
| 3001 | 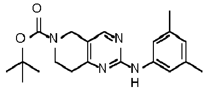 | tert-butyl 2-(3,5-dimethylanilino)-7,8-dihydro-5H-pyrido[4,3-d]pyrimidine-6-carboxylate | Int 4001 + CAS# 556-96-7 | D | 354.4 | 353.3 |
| 3007 | 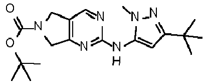 | tert-butyl 2-[(5-tert-butyl-2-methyl-pyrazol-3-yl)amino]-5,7-dihydropyrrolo[3,4-d]pyrimidine-6-carboxylate | Int 5011 | Desc' d | 372.5 | 373.3 |
| 3011 | 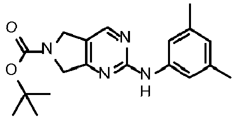 | tert-butyl 2-(3,5-dimethylanilino)-5,7-dihydropyrrolo[3,4-d]pyrimidine-6-carboxylate | Int 4011 + CAS# 556-96-7 | Desc' d | 340.4 | 341.4 |
| 3037 | 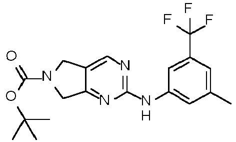 | tert-butyl 2-[3-methyl-5-(trifluoromethyl)anilino]-5,7-dihydropyrrolo[3,4-d]pyrimidine-6-carboxylate | Int 4011 + CAS# 86845-28-5 | Desc' d | 394.4 | 395.2 |
| 3087 | 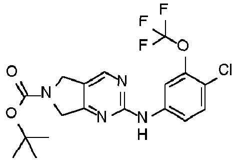 | tert-butyl 2-[4-chloro-3-(trifluoromethoxy)anilino]-5,7-dihydropyrrolo[3,4-d]pyrimidine-6-carboxylate | Int 4011 + CAS# 406232-79-9 | D | 430.8 | 431.3 |
| 4001 | 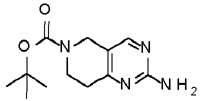 | tert-butyl 2-amino-7,8-dihydro-5H-pyrido[4,3-d]pyrimidine-6-carboxylate | Int 5001 | E | 250.3 | 251.1 |
| 4011 | 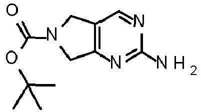 | tert-butyl 2-amino-5,7-dihydropyrrolo[3,4-d]pyrimidine-6-carboxylate | Int 5011 | Desc' d | 236.3 | 237.3 |
| 5001 | 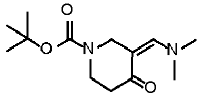 | tert-butyl (3Z)-3-(dimethylaminomethylene)-4-oxo-piperidine-1-carboxylate | CAS# 79099-07-3 | F | 254.3 | 255.2 |
| 5011 | 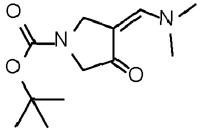 | tert-butyl (3Z)-3-(dimethylaminomethylene)-4-oxo-pyrrolidine-1-carboxylate | CAS# 101385-93-7 | F | 240.3 | 241.3 |

| | | | | | | |
|---|---|---|---|---|---|---|
| SM = Starting Material Mtd = Method, MS Mes'd = Mesured mass NA = not measured Desc'd= described above | | | | | | |

**Table III. Illustrative compounds of the invention**

| **Cpd #** | **Structure** | **Name** | **SM** | **Mtd** | **MW** | **MS Mes'd** |
|---|---|---|---|---|---|---|
| 1 | 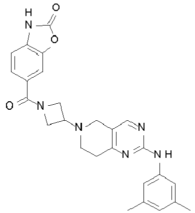 | 6-[3-[2-(3,5-dimethylanilino)-7,8-dihydro-5H-pyrido[4,3-d]pyrimidin-6-yl]azetidine-1-carbonyl]-3H-1,3-benzoxazol-2-one | Int 1 + CAS# 54903-16-1 | A | 470.5 | 471.3 |
| 2 | 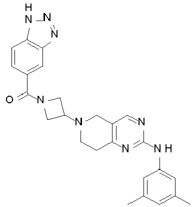 | 1H-benzotriazol-5-yl-[3-[2-(3,5-dimethylanilino)-7,8-dihydro-5H-pyrido[4,3-d]pyrimidin-6-yl]azetidin-1-yl]methanone | Int 1 + CAS# 23814-12-2 | A | 454.5 | 455.3 |
| 3 | 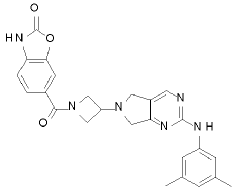 | 6-[3-[2-(3,5-dimethylanilino)-5,7-dihydropyrrolo[3,4-d]pyrimidin-6-yl]azetidine-1-carbonyl]-3H-1,3-benzoxazol-2-one | Int 13 + CAS# 54903-16-1 | example | 456.5 | 457.4 |
| 4 | 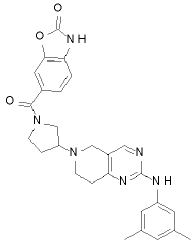 | 6-[3-[2-(3,5-dimethylanilino)-7,8-dihydro-5H-pyrido[4,3-d]pyrimidin-6-yl]pyrrolidine-1-carbonyl]-3H-1,3-benzoxazol-2-one | Int 2 + CAS# 54903-16-1 | A | 484.5 | 485.3 |
| 5 | 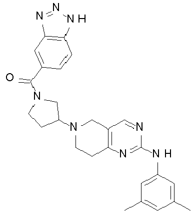 | 1H-benzotriazol-5-yl-[3-[2-(3,5-dimethylanilino)-7,8-dihydro-5H-pyrido[4,3-d]pyrimidin-6-yl]pyrrolidin-1-yl]methanone | Int 2 + CAS# 23814-12-2 | A | 468.6 | 469.4 |
| 6 | 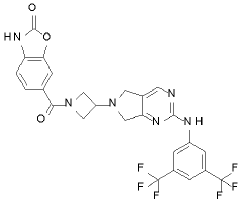 | 6-[3-[2-[3,5-bis(trifluoromethyl)anilino]-5,7-dihydropyrrolo[3,4-d]pyrimidin-6-yl]azetidine-1-carbonyl]-3H-1,3-benzoxazol-2-one | Int 14 + CAS# 54903-16-1 | A | 564.4 | 565.4 |
| 7 | 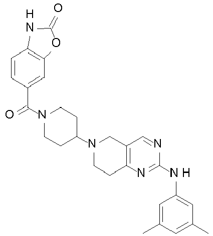 | 6-[4-[2-(3,5-dimethylanilino)-7,8-dihydro-5H-pyrido[4,3-d]pyrimidin-6-yl]piperidine-1-carbonyl]-3H-1,3-benzoxazol-2-one | Int 25 + CAS# 54903-16-1 | A | 498.6 | 499.2 |
| 8 | 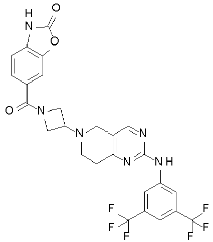 | 6-[3-[2-[3,5-bis(trifluoromethyl)anilino]-7,8-dihydro-5H-pyrido[4,3-d]pyrimidin-6-yl]azetidine-1-carbonyl]-3H-1,3-benzoxazol-2-one | Int 46 + CAS# 23814-12-2 | A | 578.5 | 579.2 |
| 9 | 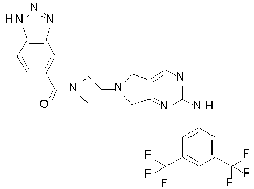 | 1H-benzotriazol-5-yl-[3-[2-[3,5-bis(trifluoromethyl)anilino]-5,7-dihydropyrrolo[3,4-d]pyrimidin-6-yl]azetidin-1-yl]methanone | Int 14 + CAS# 23814-12-2 | A | 548.4 | 549.4 |
| 10 | 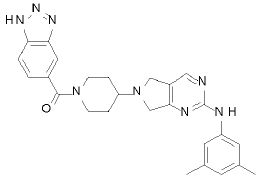 | 1H-benzotriazol-5-yl-[4-[2-(3,5-dimethylanilino)-5,7-dihydropyrrolo[3,4-d]pyrimidin-6-yl]-1-piperidyl]methanone | Int 11 + CAS# 23814-12-2 | A | 468.6 | 469.2 |
| 11 | 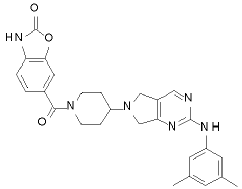 | 6-[4-[2-(3,5-dimethylanilino)-5,7-dihydropyrrolo[3,4-d]pyrimidin-6-yl]piperidine-1-carbonyl]-3H-1,3-benzoxazol-2-one | Int 11 + CAS# 54903-16-1 | A | 484.5 | 485.2 |
| 12 | 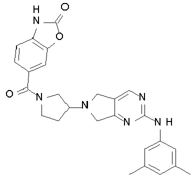 | 6-[3-[2-(3,5-dimethylanilino)-5,7-dihydropyrrolo[3,4-d]pyrimidin-6-yl]pyrrolidine-1-carbonyl]-3H-1,3-benzoxazol-2-one | Int 15 + CAS# 54903-16-1 | A | 470.5 | 471.4 |
| 13 | 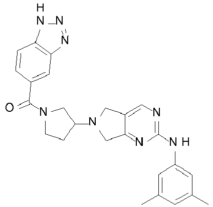 | 1H-benzotriazol-5-yl-[3-[2-(3,5-dimethylanilino)-5,7-dihydropyrrolo[3,4-d]pyrimidin-6-yl]pyrrolidin-1-yl]methanone | Int 15 + CAS# 23814-12-2 | A | 454.5 | 455.4 |
| 14 | 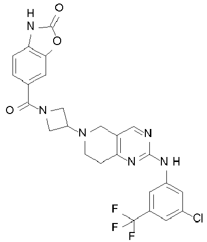 | 6-[3-[2-[3-chloro-5-(trifluoromethyl)anilino]-7,8-dihydro-5H-pyrido[4,3-d]pyrimidin-6-yl]azetidine-1-carbonyl]-3H-1,3-benzoxazol-2-one | Int 46 + CAS# 54903-16-1 | A | 544.9 | 545.3 |
| 15 | 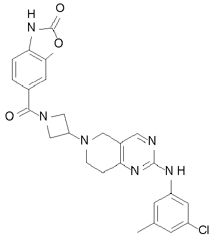 | 6-[3-[2-(3-chloro-5-methyl-anilino)-7,8-dihydro-5H-pyrido[4,3-d]pyrimidin-6-yl]azetidine-1-carbonyl]-3H-1,3-benzoxazol-2-one | Int 47 + CAS# 54903-16-1 | A | 490.9 | 491.3 + 493.3 |
| 16 | 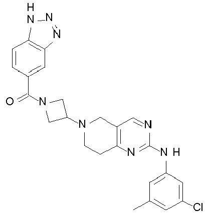 | 1H-benzotriazol-5-yl-[3-[2-(3-chloro-5-methyl-anilino)-7,8-dihydro-5H-pyrido[4,3-d]pyrimidin-6-yl]azetidin-1-yl]methanone | Int 47 + CAS# 23814-12-2 | A | 474.9 | 475.3 + 477.3 |
| 17 | 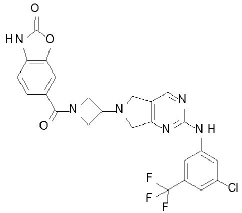 | 6-[3-[2-[3-chloro-5-(trifluoromethyl)anilino]-5,7-dihydropyrrolo[3,4-d]pyrimidin-6-yl]azetidine-1-carbonyl]-3H-1,3-benzoxazol-2-one | Int 48 + CAS# 54903-16-1 | A | 530.9 | 531.2 + 533.2 |
| 18 | 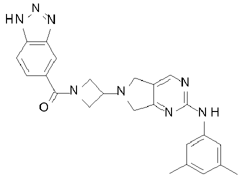 | 1H-benzotriazol-5-yl-[3-[2-(3,5-dimethylanilino)-5,7-dihydropyrrolo[3,4-d]pyrimidin-6-yl]azetidin-1-yl]methanone | Int 13 + CAS# 23814-12-2 | A | 440.5 | 441.4 |
| 19 | 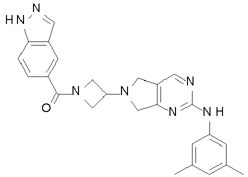 | [3-[2-(3,5-dimethylanilino)-5,7-dihydropyrrolo[3,4-d]pyrimidin-6-yl]azetidin-1-yl]-(1H-indazol-5-yl)methanone | Int 13 + CAS# 61700-61-6 | A | 439.5 | 440.4 |
| 20 | 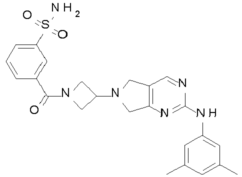 | 3-[3-[2-(3,5-dimethylanilino)-5,7-dihydropyrrolo[3,4-d]pyrimidin-6-yl]azetidine-1-carbonyl]benzenesulfonamide | Int 13 + CAS# 636-76-0 | A | 478.6 | 479.5 |
| 21 | 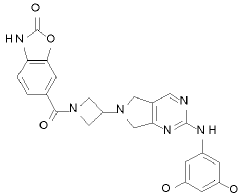 | 6-[3-[2-(3,5-dimethoxyanilino)-5,7-dihydropyrrolo[3,4-d]pyrimidin-6-yl]azetidine-1-carbonyl]-3H-1,3-benzoxazol-2-one | Int 3 + CAS# 54903-16-1 | A | 488.5 | 489.7 |
| 22 | 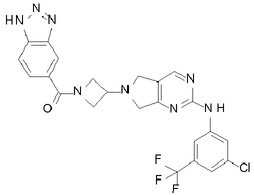 | 1H-benzotriazol-5-yl-[3-[2-[3-chloro-5-(trifluoromethyl)anilino]-5,7-dihydropyrrolo[3,4-d]pyrimidin-6-yl]azetidin-1-yl]methanone | Int 48 + CAS# 23814-12-2 | A | 514.9 | 515.6 + 517.6 |
| 23 | 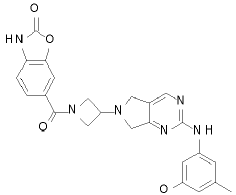 | 6-[3-[2-(3-methoxy-5-methyl-anilino)-5,7-dihydropyrrolo[3,4-d]pyrimidin-6-yl]azetidine-1-carbonyl]-3H-1,3-benzoxazol-2-one | Int 26 + CAS# 54903-16-1 | A | 472.5 | 473.1 |
| 24 | 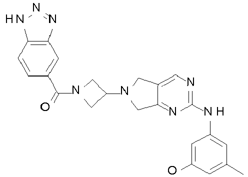 | 1H-benzotriazol-5-yl-[3-[2-(3-methoxy-5-methyl-anilino)-5,7-dihydropyrrolo[3,4-d]pyrimidin-6-yl]azetidin-1-yl]methanone | Int 26 + CAS# 23814-12-2 | A | 456.5 | 457.1 |
| 25 | 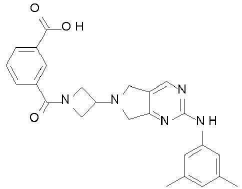 | 3-[3-[2-(3,5-dimethylanilino)-5,7-dihydropyrrolo[3,4-d]pyrimidin-6-yl]azetidine-1-carbonyl]benzoic acid | Int 13 + CAS# 121-91-5 | exampl e | 443.5 | 444.4 |
| 26 | 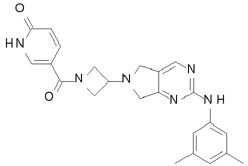 | 5-[3-[2-(3,5-dimethylanilino) -5,7-dihydropyrrolo[3,4-d]pyrimidin-6-yl]azetidine-1-carbonyl]-1H-pyridin-2-one | Int 13 + CAS# 5006-66-6 | A | 416.5 | 417.4 |
| 27 | 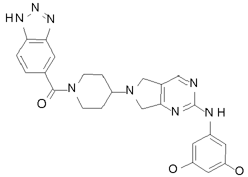 | 1H-benzotriazol-5-yl-[4-[2-(3,5-dimethoxyanilino)-5,7-dihydropyrrolo[3,4-d]pyrimidin-6-yl]-1-piperidyl]methanone | Int 2003 + CAS# 79099-07-3 + CAS# 23814-12-2 | C + B + A | 500.6 | 501.2 |
| 28 | 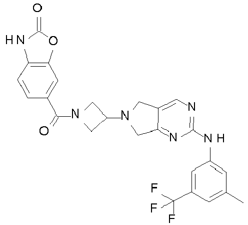 | 6-[3-[2-[3-methyl-5-(trifluoromethyl)anilino]-5,7-dihydropyrrolo[3,4-d]pyrimidin-6-yl]azetidine-1-carbonyl]-3H-1,3-benzoxazol-2-one | Int 37 + CAS# 54903-16-1 | A | 510.5 | 511.1 |
| 29 | 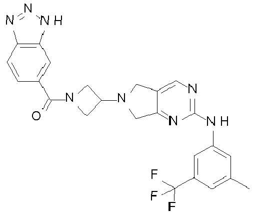 | 3H-benzotriazol-5-yl-[3-[2-[3-methyl-5-(trifluoromethyl)anilino]-5,7-dihydropyrrolo[3,4-d]pyrimidin-6-yl]azetidin-1-yl]methanone | Int 37 + CAS# 23814-12-2 | A | 494.5 | 495.1 |
| 30 | 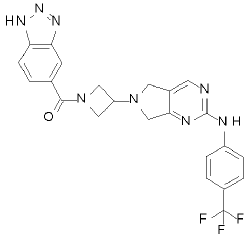 | 1H-benzotriazol-5-yl-[3-[2-[4-(trifluoromethyl)anilino]-5,7-dihydropyrrolo[3,4-d]pyrimidin-6-yl]azetidin-1-yl]methanone | Int 2016 + CAS# 398489-26-4 + CAS# 23814-12-2 | C + B + A | 480.4 | 481.4 |
| 31 | 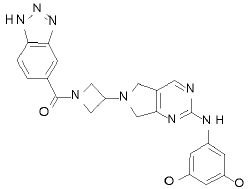 | 1H-benzotriazol-5-yl-[3-[2-(3,5-dimethoxyanilino)-5,7-dihydropyrrolo[3,4-d]pyrimidin-6-yl]azetidin-1-yl]methanone | Int 3 + CAS# 23814-12-2 | A | 472.5 | 473.2 |
| 32 | 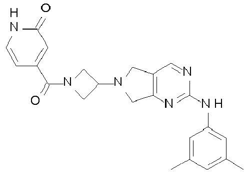 | 4-[3-[2-(3,5-dimethylanilino)-5,7-dihydropyrrolo[3,4-d]pyrimidin-6-yl]azetidine-1-carbonyl]-1H-pyridin-2-one | Int 13 + CAS# 22282- 72-0 | A | 416.5 | 417.4 |
| 33 | 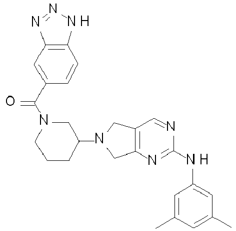 | 1H-benzotriazol-5-yl-[3-[2-(3,5-dimethylanilino)-5,7-dihydropyrrolo[3,4-d]pyrimidin-6-yl]-1-piperidyl]methanone | Int 49 + CAS# 23814-12-2 | A | 468.6 | 469.3 |
| 34 | 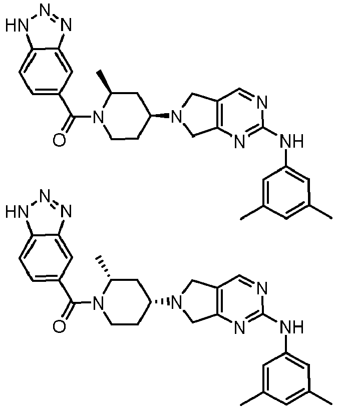 | 1H-benzotriazol-5-yl-[(2S,4S/2R,4R)-4-[2-(3,5-dimethylanilino)-5,7-dihydropyrrolo[3,4-d]pyrimidin-6-yl]-2-methyl-1-piperidyl]methanone | Int 17 + CAS# 23814-12-2 | A | 482.6 | 483.4 |
| 35 | 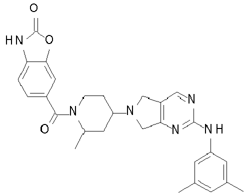 | 6-[4-[2-(3,5-dimethylanilino)-5,7-dihydropyrrolo[3,4-d]pyrimidin-6-yl]-2-methyl-piperidine-1-carbonyl]-3H-1,3-benzoxazol-2-one | Int 17 + CAS# 54903-16-1 | A | 498.6 | 499.4 |
| 36 | 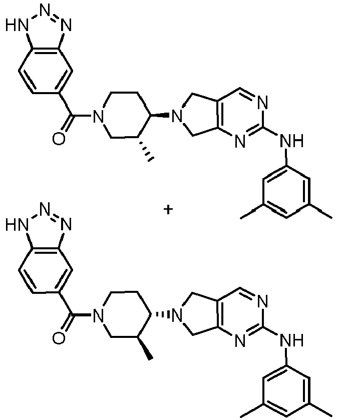 | 1H-benzotriazol-5-yl-[(3R,4S/3S,4R)-4-[2-(3,5-dimethylanilino)-5,7-dihydropyrrolo[3,4-d]pyrimidin-6-yl]-3-methyl-1-piperidyl]methanone | Int 4 + CAS# 23814-12-2 | A | 482.6 | 483.5 |
| 37 | 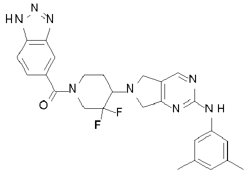 | 1H-benzotriazol-5-yl-[4-[2-(3,5-dimethylanilino)-5,7-dihydropyrrolo[3,4-d]pyrimidin-6-yl]-3,3-difluoro-1-piperidyl]methanone | Int 2011 + CAS# 1215071-17-2 + CAS# 23814-12-2 | C + B + A | 504.5 | 505.4 |
| 38 | 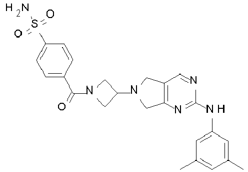 | 4-[3-[2-(3,5-dimethylanilino)-5,7-dihydropyrrolo[3,4-d]pyrimidin-6-yl]azetidine-1-carbonyl]benzenesulfonamide | Int 13 + CAS# 138-41-0 | A | 478.6 | 479.4 |
| 39 | 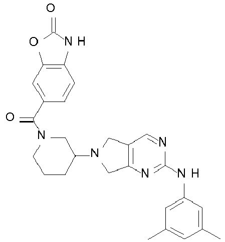 | 6-[3-[2-(3,5-dimethylanilino)-5,7-dihydropyrrolo[3,4-d]pyrimidin-6-yl]piperidine-1-carbonyl]-3H-1,3-benzoxazol-2-one | Int 49 + CAS# 54903-16-1 | A | 484.5 | 485.4 |
| 40 | 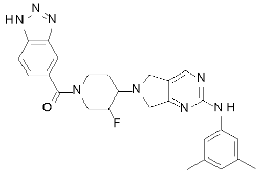 | 1H-benzotriazol-5-yl-[4-[2-(3,5-dimethylanilino)-5,7-dihydropyrrolo[3,4-d]pyrimidin-6-yl]-3-fluoro-1-piperidyl]methanone | Int 2011 + CAS# 211108-50-8 + CAS# 23814-12-2 | C + B + A | 486.5 | 487.4 |
| 41 | 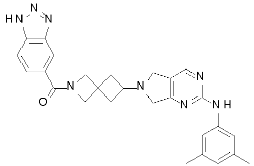 | 1H-benzotriazol-5-yl-[6-[2-(3,5-dimethylanilino)-5,7-dihydropyrrolo[3,4-d]pyrimidin-6-yl]-2-azaspiro[3.3]heptan-2-yl]methanone | Int 2011 + CAS# 1181816-12-5 + CAS# 23814-12-2 | C + B + A | 480.6 | 481.4 |
| 42 | 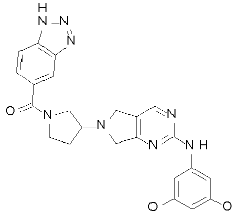 | 1H-benzotriazol-5-yl-[3-[2-(3,5-dimethoxyanilino)-5,7-dihydropyrrolo[3,4-d]pyrimidin-6-yl]pyrrolidin-1-yl]methanone | Int 2003 + CAS# 101385-93-7 + CAS# 23814-12-2 | C + B + A | 486.5 | 487.1 |
| 43 | 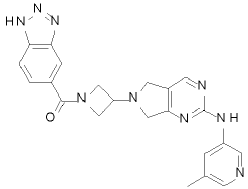 | 1H-benzotriazol-5-yl-[3-[2-[(5-methyl-3-pyridyl)amino]-5,7-dihydropyrrolo[3,4-d]pyrimidin-6-yl]azetidin-1-yl]methanone | Int 29 + CAS# 23814-12-2 | A | 427.5 | 428.1 |
| 44 | 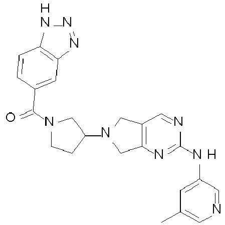 | 1H-benzotriazol-5-yl-[3-[2-[(5-methyl-3-pyridyl)amino]-5,7-dihydropyrrolo[3,4-d]pyrimidin-6-yl]pyrrolidin-1-yl]methanone | Int 30 + CAS# 23814-12-2 | A | 441.5 | 442.1 |
| 45 | 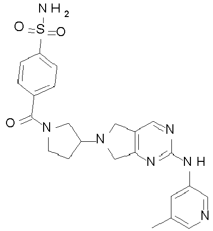 | 4-[3-[2-[(5-methyl-3-pyridyl)amino]-5,7-dihydropyrrolo[3,4-d]pyrimidin-6-yl]pyrrolidine-1-carbonyl]benzenesulfonamide | Int 30 + CAS# 138-41-0 | A | 479.6 | 480.1 |
| 46 | 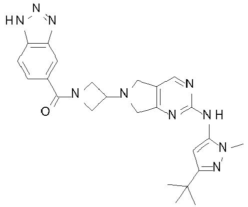 | 1H-benzotriazol-5-yl-[3-[2-[(5-tert-butyl-2-methyl-pyrazol-3-yl)amino]-5,7-dihydropyrrolo[3,4-d]pyrimidin-6-yl]azetidin-1-yl]methanone | Int 2007 + CAS# 398489-26-4 + CAS# 23814-12-2 | C + B + A | 472.5 | 473.4 |
| 47 | 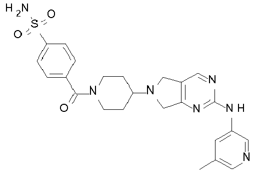 | 4-[4-[2-[(5-methyl-3-pyridyl)amino]-5,7-dihydropyrrolo[3,4-d]pyrimidin-6-yl]piperidine-1-carbonyl]benzenesulfonamide | Int 31 + CAS# 138-41-0 | A | 493.6 | 494.1 |
| 48 | 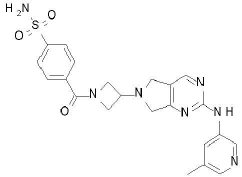 | 4-[3-[2-[(5-methyl-3-pyridyl)amino]-5,7-dihydropyrrolo[3,4-d]pyrimidin-6-yl]azetidine-1-carbonyl]benzenesulfonamide | Int 29 + CAS# 138-41-0 | A | 465.5 | 466.1 |
| 49 | 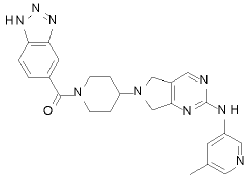 | 1H-benzotriazol-5-yl-[4-[2-[(5-methyl-3-pyridyl)amino]-5,7-dihydropyrrolo[3,4-d]pyrimidin-6-yl]-1-piperidyl]methanone | Int 31 + CAS# 23814-12-2 | A | 455.5 | 456.1 |
| 50 | 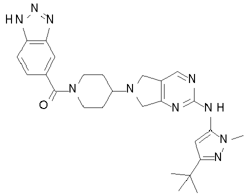 | 1H-benzotriazol-5-yl-[4-[2-[(5-tert-butyl-2-methyl-pyrazol-3-yl)amino]-5,7-dihydropyrrolo[3,4-d]pyrimidin-6-yl]-1-piperidyl]methanone | Int 2007 + CAS# 79099-07-3 + CAS# 23814-12-2 | C + B + A | 500.6 | 501.5 |
| 51 | 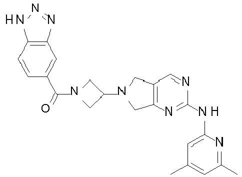 | 1H-benzotriazol-5-yl-[3-[2-[(4,6-dimethyl-2-pyridyl)amino]-5,7-dihydropyrrolo[3,4-d]pyrimidin-6-yl]azetidin-1-yl]methanone | Int 32 + CAS# 23814-12-2 | A | 441.5 | 442.1 |
| 52 | 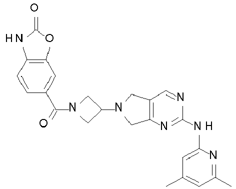 | 6-[3-[2-[(4,6-dimethyl-2-pyridyl)amino]-5,7-dihydropyrrolo[3,4-d]pyrimidin-6-yl]azetidine-1-carbonyl]-3H-1,3-benzoxazol-2-one | Int 32 + CAS# 54903-16-1 | A | 457.5 | 458.1 |
| 53 | 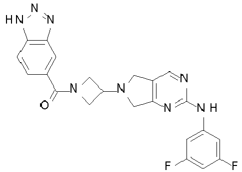 | 1H-benzotriazol-5-yl-[3-[2-(3,5-difluoroanilino)-5,7-dihydropyrrolo[3,4-d]pyrimidin-6-yl]azetidin-1-yl]methanone | Int 33 + CAS# 23814-12-2 | A | 448.4 | 449.1 |
| 54 | 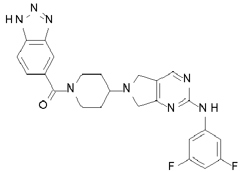 | 1H-benzotriazol-5-yl-[4-[2-(3,5-difluoroanilino)-5,7-dihydropyrrolo[3,4-d]pyrimidin-6-yl]-1-piperidyl]methanone | Int 34 + CAS# 23814-12-2 | A | 476.5 | 477.1 |
| 55 | 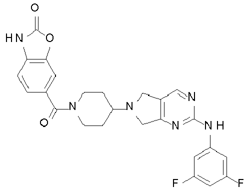 | 6-[4-[2-(3,5-difluoroanilino)-5,7-dihydropyrrolo[3,4-d]pyrimidin-6-yl]piperidine-1-carbonyl]-3H-1,3-benzoxazol-2-one | Int 34 + CAS# 54903-16-1 | A | 492.5 | 493.1 |
| 56 | 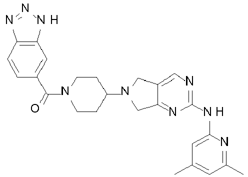 | 3H-benzotriazol-5-yl-[4-[2-[(4,6-dimethyl-2-pyridyl)amino]-5,7-dihydropyrrolo [3,4-d]pyrimidin-6-yl]-1-piperidyl]methanone | Int 38 + CAS# 23814-12-2 | A | 469.5 | 470.1 |
| 57 | 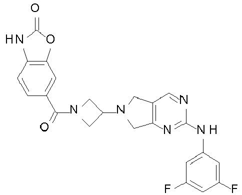 | 6-[3-[2-(3,5-difluoroanilino)-5,7-dihydropyrrolo[3,4-d]pyrimidin-6-yl]azetidine-1-carbonyl]-3H-1,3-benzoxazol-2-one | Int 33 + CAS# 54903-16-1 | A | 464.4 | 465.1 |
| 58 | 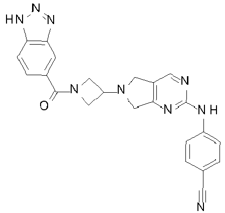 | 4-[[6-[1-(1H-benzotriazole-5-carbonyl)azetidin-3-yl]-5,7-dihydropyrrolo[3,4-d]pyrimidin-2-yl]amino]benzonitrile | Int 2035 + CAS# 398489-26-4 + CAS# 23814-12-2 | C + B + A | 437.5 | 438.2 |
| 59 | 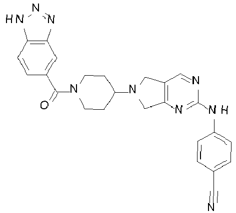 | 4-[[6-[1-(1H-benzotriazole-5-carbonyl)-4-piperidyl]-5,7-dihydropyrrolo[3,4-d]pyrimidin-2-yl]amino]benzonitrile | Int 2035 + CAS# 79099-07-3 + CAS# 23814-12-2 | C + B + A | 465.5 | 466.2 |
| 60 | 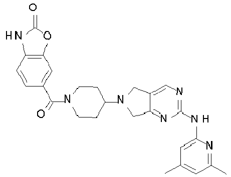 | 6-[4-[2-[(4,6-dimethyl-2-pyridyl)amino]-5,7-dihydropyrrolo[3,4-d]pyrimidin-6-yl]piperidine-1-carbonyl]-3H-1,3-benzoxazol-2-one | Int 38 + CAS# 54903-16-1 | exampl e | 485.5 | 486.2 |
| 61 | 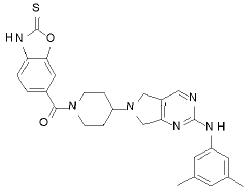 | [4-[2-(3,5-dimethylanilino)-5,7-dihydropyrrolo[3,4-d]pyrimidin-6-yl]-1-piperidyl]-(2-thioxo-3H-1,3-benzoxazol-6-yl)methanone | Int 11 + CAS# 108085-62-7 | A | 500.6 | 501.3 |
| 62 | 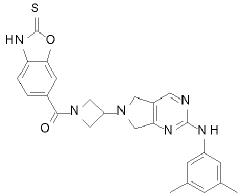 | [3-[2-(3,5-dimethylanilino)-5,7-dihydropyrrolo[3,4-d]pyrimidin-6-yl]azetidin-1-yl]-(2-thioxo-3H-1,3-benzoxazol-6-yl)methanone | Int 13 + CAS# 108085-62-7 | A | 472.6 | 473.3 |
| 63 | 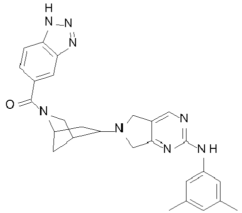 | 1H-benzotriazol-5-yl-[5-[2-(3,5-dimethylanilino)-5,7-dihydropyrrolo[3,4-d]pyrimidin-6-yl]-2-azabicyclo[2.2.1]heptan-2-yl]methanone | Int 19 + CAS# 23814-12-2 | A | 480.6 | 481.4 |
| 64 | 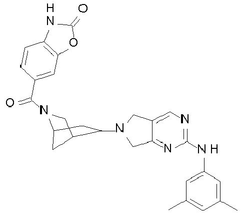 | 6-[5-[2-(3,5-dimethylanilino)-5,7-dihydropyrrolo[3,4-d]pyrimidin-6-yl]-2-azabicyclo[2.2.1]heptane-2-carbonyl]-3H-1,3-benzoxazol-2-one | Int 19 + CAS# 54903-16-1 | A | 496.6 | 497.4 |
| 65 | 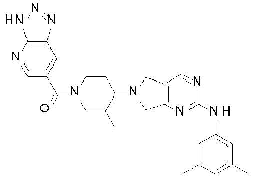 | [4-[2-(3,5-dimethylanilino)-5,7-dihydropyrrolo[3,4-d]pyrimidin-6-yl]-3-methyl-1-piperidyl]-(3H-triazolo[4,5-b]pyridin-6-yl)methanone | Int 2011 + CAS# 181269-69-2 + CAS# 1260385-82-7 | C + B + A | 483.6 | 484.4 |
| 66 | 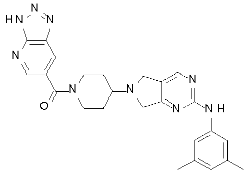 | [4-[2-(3,5-dimethylanilino)-5,7-dihydropyrrolo[3,4-d]pyrimidin-6-yl]-1-piperidyl]-(3H-triazolo[4,5-b]pyridin-6-yl)methanone | Int 11 + CAS# 1260385-82-7 | exampl e | 469.5 | 470.2 |
| 67 | 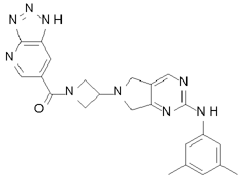 | [3-[2-(3,5-dimethylanilino)-5,7-dihydropyrrolo[3,4-d]pyrimidin-6-yl]azetidin-1-yl]-(1H-triazolo[4,5-b]pyridin-6-yl)methanone | Int 13 + CAS# 1260385-82-7 | exampl e | 441.5 | 442.1 |
| 68 | 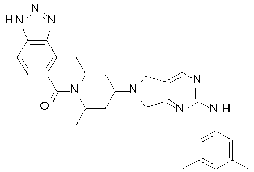 | 1H-benzotriazol-5-yl-[4-[2-(3,5-dimethylanilino)-5,7-dihydropyrrolo[3,4-d]pyrimidin-6-yl]-2,6-dimethyl-1-piperidyl]methanone | Int 2011 + CAS# 604010-24-4 + CAS# 23814-12-2 | C + B + A | 496.6 | 496.5 |
| 69 | 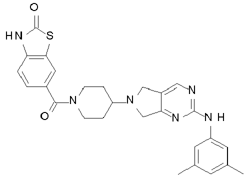 | 6-[4-[2-(3,5-dimethylanilino)-5,7-dihydropyrrolo[3,4-d]pyrimidin-6-yl]piperidine-1-carbonyl]-3H-1,3-benzothiazol-2-one | Int 11 + CAS# 99615-68-6 | A | 500.6 | 501.2 |
| 70 | 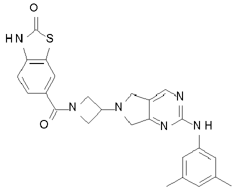 | 6-[3-[2-(3,5-dimethylanilino)-5,7-dihydropyrrolo[3,4-d]pyrimidin-6-yl]azetidine-1-carbonyl]-3H-1,3-benzothiazol-2-one | Int 13 + CAS# 99615-68-6 | A | 472.6 | 473.2 |
| 71 | 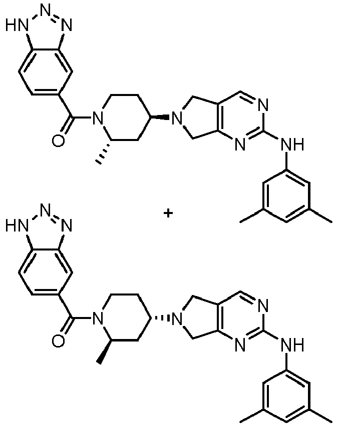 | 1H-benzotriazol-5-yl-[(2S,4R/2R,4S)-4-[2-(3,5-dimethylanilino)-5,7-dihydropyr rolo[3,4-d]pyrimidin-6-yl]-2-methyl-1-piperidyl]methanone | Int 21 + CAS# 23814-12-2 | A | 482.6 | 483.5 |
| 72 | 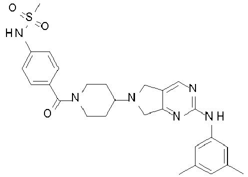 | N-[4-[4-[2-(3,5-dimethylanilino)-5,7-dihydropyrrolo[3,4-d]pyrimidin-6-yl]piperidine-1-carbonyl]phenyl]methanesulfonamide | Int 11 + CAS# 7151-76-0 | A | 520.6 | 521.4 |
| 73 | 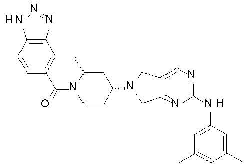 | 1H-benzotriazol-5-yl-[(2R,4R)-4-[2-(3,5-dimethylanilino)-5,7-dihydropyrrolo[3,4-d]pyrimidin-6-yl]-2-methyl-1-piperidyl]methanone | Int 39 + CAS# 23814-12-2 | A | 482.6 | 483.1 |
| 74 | 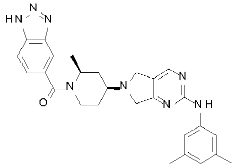 | 1H-benzotriazol-5-yl-[(2S,4S)-4-[2-(3,5-dimethylanilino)-5,7-dihydropyrrolo[3,4-d]pyrimidin-6-yl]-2-methyl-1-piperidyl]methanone | Int 40 + CAS# 23814-12-2 | A | 482.6 | 483.1 |
| 75 | 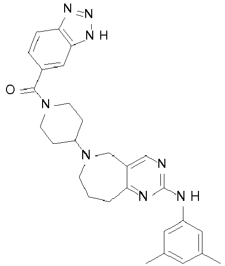 | 3H-benzotriazol-5-yl-[4-[2-(3,5-dimethylanilino)-5,7,8,9-tetrahydropyrimido[5,4-c]azepin-6-yl]-1-piperidyl]methanone | Int 43 + CAS# 23814-12-2 | A | 496.6 | 497.1 |
| 76 | 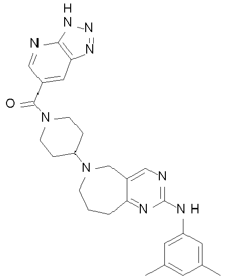 | [4-[2-(3,5-dimethylanilino)-5,7,8,9-tetrahydropyrimido[5,4-c]azepin-6-yl]-1-piperidyl]-(3H-triazolo[4,5-b]pyridin-6-yl)methanone | Int 43 + CAS# 1260385-82-7 | exampl e | 497.6 | 498.1 |
| 77 | 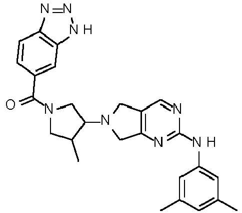 | 3H-benzotriazol-5-yl-[3-[2-(3,5-dimethylanilino)-5,7-dihydropyrrolo[3,4-d]pyrimidin-6-yl]-4-methyl-pyrrolidin-1-yl]methanone | Int 10 + CAS# 23814-12-2 | A | 468.6 | 469.4 |
| 78 | 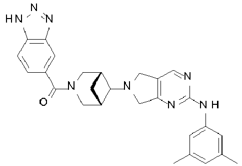 | 1H-benzotriazol-5-yl-[(1R,5S)-6-[2-(3,5-dimethylanilino)-5,7-dihydropyrrolo[3,4-d]pyrimidin-6-yl]-3-azabicyclo[3.1.1]heptan-3-yl]methanone | Int 2001 + CAS# 1251013-26-9 + CAS# 23814-12-2 | C + B + A | 480.6 | 481.4 |
| 79 | 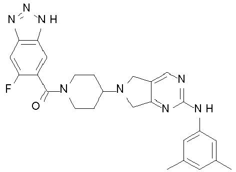 | [4-[2-(3,5-dimethylanilino)-5,7-dihydropyrrolo[3,4-d]pyrimidin-6-yl]-1-piperidyl]-(6-fluoro-3H-benzotriazol-5-yl)methanone | Int 11 + CAS# 1427081-62-6 | A | 486.5 | 487.4 |
| 80 | 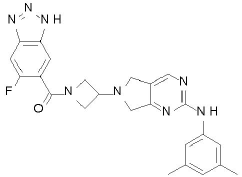 | [3-[2-(3,5-dimethylanilino)-5,7-dihydropyrrolo[3,4-d]pyrimidin-6-yl]azetidin-1-yl]-(6-fluoro-3H-benzotriazol-5-yl)methanone | Int 13 + CAS# 1427081-62-6 | A | 458.5 | 459.4 |
| 81 | 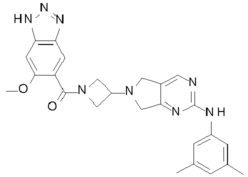 | [3-[2-(3,5-dimethylanilino)-5,7-dihydropyrrolo[3,4-d]pyrimidin-6-yl]azetidin-1-yl]-(6-methoxy-1H-benzotriazol-5-yl)methanone | Int 13 + CAS# 59338-92-0 | A | 470.5 | 471.4 |
| 82 | 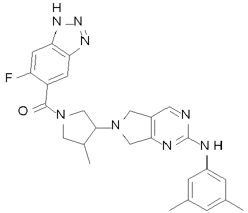 | [3-[2-(3,5-dimethylanilino)-5,7-dihydropyrrolo[3,4-d]pyrimidin-6-yl]-4-methyl-pyrrolidin-1-yl]-(6-fluoro-1H-benzotriazol-5-yl)methanone | Int 10+ CAS# 1427081-62-6 | A | 486.5 | 487.4 |
| 83 | 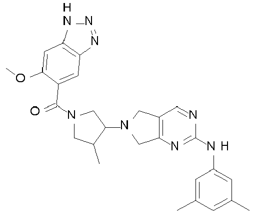 | [3-[2-(3,5-dimethylanilino)-5,7-dihydropyrrolo[3,4-d]pyrimidin-6-yl] -4-methyl-pyrrolidin-1-yl]-(6-methoxy-1H-benzotriazol-5-yl)methanone | Int 10 + CAS# 59338-92-0 | A | 498.6 | 499.5 |
| 85 | 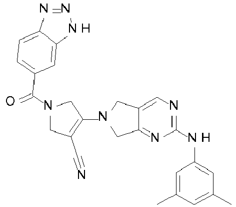 | 1-(3H-benzotriazole-5-carbonyl)-4-[2-(3,5-dimethylanilino)-5,7-dihydropyrrolo[3,4-d]pyrimidin-6-yl]-2,5-dihydropyrrole-3-carbonitrile | Int 1044 + CAS# 23814-12-2 | C + B + A | 477.5 | 478.4 |
| 86 | 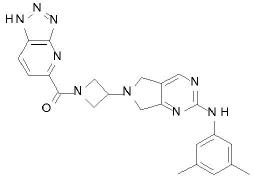 | [3-[2-(3,5-dimethylanilino)-5,7-dihydropyrrolo[3,4-d]pyrimidin-6-yl]azetidin-1-yl]-(1H-triazolo[4,5-b]pyridin-5-yl)methanone | Int 13 + CAS 1216149-55-1 | example | 441.5 | 442.3 |
| 87 | 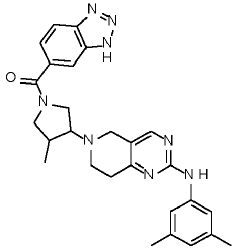 | 3H -benzotriazol-5-yl-[3-[2-(3,5-dimethylanilino)-7,8-dihydro-5H-pyrido[4,3-d]pyrimidin-6-yl] -4-methyl-pyrrolidin-1-yl]methanone | Int 45 + CAS# 23814-12-2 | A | 482.6 | 483.3 |
| 88 | 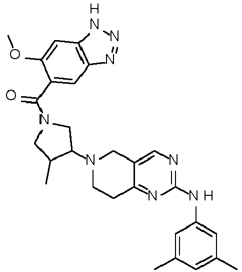 | [3-[2-(3,5-dimethylanilino)-7,8-dihydro-5H-pyrido[4,3-d]pyrimidin-6-yl]-4-methyl-pyrrolidin-1-yl]-(6-methoxy-1H-benzotriazol-5-yl)methanone | Int 45 + CAS# 59338-92-0 | A | 512.6 | 513.4 |
| 92 | 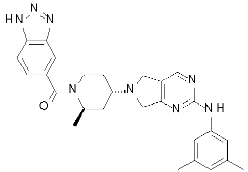 | 1H-benzotriazol-5-yl-[(2R,4S)-4-[2-(3,5-dimethylanilino)-5,7-dihydropyrrolo[3,4-d]pyrimidin-6-yl]-2-methyl-1-piperidyl]methanone | Int 2011 + CAS# 790667-43-5 + CAS# 23814-12-2 | C + B + A | 482.6 | 483.7 |
| 93 | 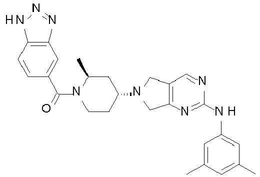 | 1H-benzotriazol-5-yl-[(2S,4R)-4-[2-(3,5-dimethylanilino)-5,7-dihydropyrrolo[3,4-d]pyrimidin-6-yl]-2-methyl-1-piperidyl]methanone | Int 21 + CAS# 23814-12-2 | A | 482.6 | 483.6 |
| 94 | 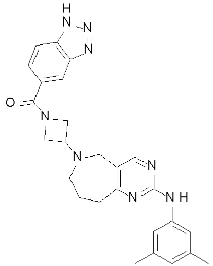 | 1H-benzotriazol-5-yl-[3-[2-(3,5-dimethylanilino)-5,7,8,9-tetrahydropyrimido[5,4-c]azepin-6-yl]azetidin-1-yl]methanone | Int 2043 + CAS# 398489-26-4 + CAS# 23814-12-2 | C + B + A | 468.6 | 469.6 |
| 95 | 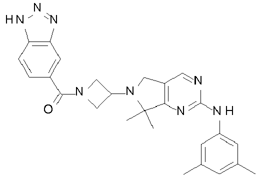 | 1H-benzotriazol-5-yl-[3-[2-(3,5-dimethylanilino)-7,7-dimethyl-5H-pyrrolo[3,4-d]pyrimidin-6-yl]azetidin-1-yl]methanone | CAS# 1215295-96-7 + CAS# 398489-26-4 + CAS# 23814-12-2 | F + E + D + B + C + B + A | 468.6 | 469.6 |
| 96 | 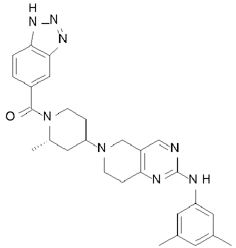 | 1H-benzotriazol-5-yl-[(2S)-4-[2-(3,5-dimethylanilino)-7,8-dihydro-5H-pyrido[4,3-d]pyrimidin-6-yl]-2-methyl-1-piperidyl]methanone | Int 62 + Int 2001 | example | 496.6 | 497.6 |
| 97 | 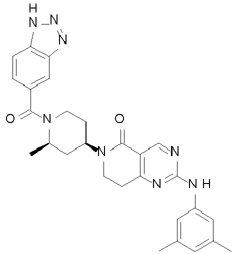 | 6-[(2R,4R)-1-(1H-benzotriazole-5-carbonyl)-2-methyl-4-piperidyl]-2-(3,5-dimethylanilino)-7,8-dihydropyrido[4,3-d]pyrimidin-5-one | CAS# 790667-43-5 | example | 510.6 | 511.6 |
| 98 | 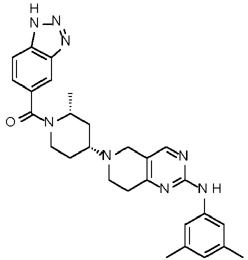 | 1H-benzotriazol-5-yl-[(2R,4R)-4-[2-(3,5-dimethylanilino)-7,8-dihydro-5H-pyrido[4,3-d]pyrimidin-6-yl]-2-methyl-1-piperidyl]methanone | Int 2001 + CAS# 790667-43-5 + CAS# 23814-12-2 | C + B + A | 496.6 | 497.6 |
| 99 | 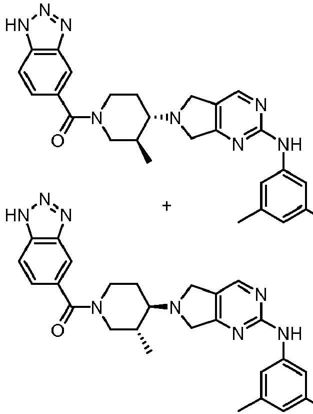 | 1H-benzotriazol-5-yl-[(3S,4S/3R,4R)-4-[2-(3,5-dimethylanilino)-5,7-dihydropyrrolo[3,4-d]pyrimidin-6-yl]-3-methyl-1-piperidyl]methanone | Int 2011 + CAS# 181269-69-2 + CAS# 23814-12-2 | C + B + A | 482.6 | 483.5 |
| 100 | 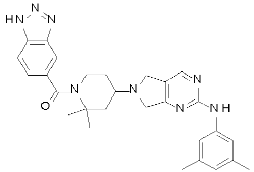 | 1H-benzotriazol-5-yl-[4-[2-(3,5-dimethylanilino)-5,7-dihydropyrrolo[3,4-d]pyrimidin-6-yl]-2,2-dimethyl-1-piperidyl]methanone | Int 52 + CAS# 46053-85-4 | example | 496.6 | 497.6 |
| 101 | 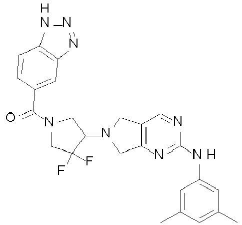 | 1H-benzotriazol-5-yl-[4-[2-(3,5-dimethylanilino)-5,7-dihydropyrrolo[3,4-d]pyrimidin-6-yl]-3,3-difluoro-pyrrolidin-1-yl]methanone | Int 85 + CAS# 23814-12-2 | example | 490.5 | 491.5 |
| 102 | 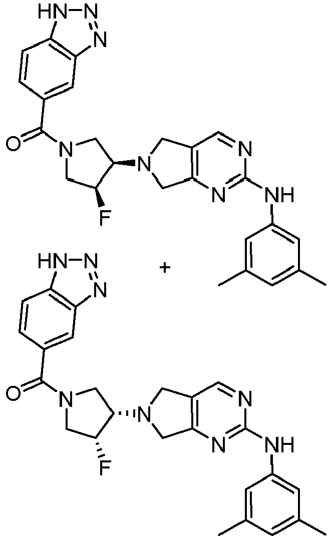 | 1H-benzotriazol-5-yl-[(3R,4S/3S,4R)-3-[2-(3,5-dimethylanilino)-5,7-dihydropyrrolo[3,4-d]pyrimidin-6-yl] -4-fluoro-pyrrolidin-1-yl]methanone | Int 2011 + CAS# 845894-03-3 + CAS# 23814-12-2 | C + B + A | 472.5 | 473.5 |
| 103 | 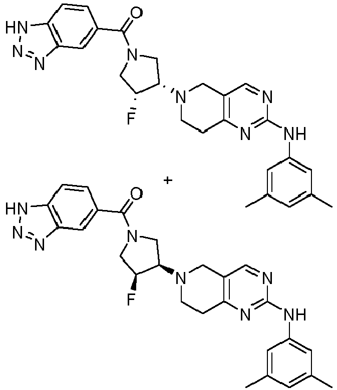 | 1H-benzotriazol-5-yl-[(3S,4R/3R,4S)-3-[2-(3,5-dimethylanilino)-7,8-dihydro-5H-pyrido[4,3-d]pyrimidin-6-yl] -4-fluoro-pyrrolidin-1-yl]methanone | Int 2011 + CAS# 845894-03-3 + CAS# 23814-12-2 | C + B + A | 486.5 | 487.5 |
| 104 | 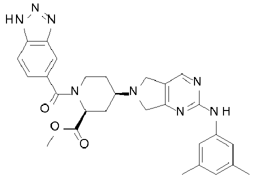 | methyl (2S,4R)-1-(1H-benzotriazole-5-carbonyl)-4-[2-(3,5-dimethylanilino)-5,7-dihydropyrrolo[3,4-d]pyrimidin-6-yl]pipe ridine-2-carboxylate | Int 88 + CAS# 23814-12-2 | example | 526.6 | 527.6 |
| 105 | 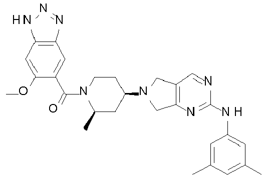 | [(2R,4R)-4-[2-(3,5-dimethylanilino)-5,7-dihydropyrrolo[3,4-d]pyrimidin-6-yl]-2-methyl-1-piperidyl]-(6-methoxy-1H-benzotriazol-5-yl)methanone | Int 2011 + Int 103 | C | 512.6 | 513.6 |
| 106 | 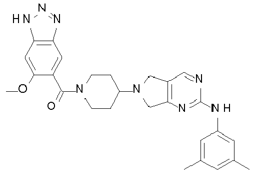 | [4-[2-(3,5-dimethylanilino)-5,7-dihydropyrrolo[3,4-d]pyrimidin-6-yl]-1-piperidyl]-(6-methoxy-1H-benzotriazol-5-yl)methanone | Int 2011 + Int 104 | C | 498.6 | 499.5 |
| 107 | 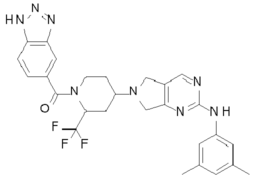 | 1H-benzotriazol-5-yl-[4-[2-(3,5-dimethylanilino)-5,7-dihydropyrrolo[3,4-d]pyrimidin-6-yl]-2-(trifluoromethyl)-1-piperidyl]methanone | Int 2011 + Int 63 | C | 536.6 | 537.6 |
| 108 | 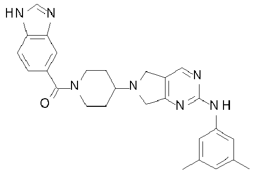 | 1H-benzimidazol-5-yl-[4-[2-(3,5-dimethylanilino)-5,7-dihydropyrrolo[3,4-d]pyrimidin-6-yl]-1-piperidyl]methanone | Int 2011 + Int 73 | example | 467.6 | 468.6 |
| 109 | 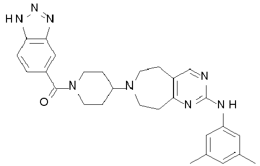 | 1H-benzotriazol-5-yl-[4-[2-(3,5-dimethylanilino)-5,6,8,9-tetrahydropyrimido[4,5-d]azepin-7-yl]-1-piperidyl]methanone | Int 53 + CAS# 23814-12-2 | A | 496.6 | 497.5 |
| 110 | 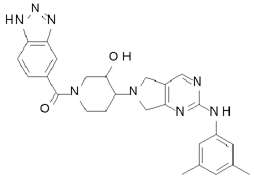 | 1H-benzotriazol-5-yl-[4-[2-(3,5-dimethylanilino)-5,7-dihydropyrrolo[3,4-d]pyrimidin-6-yl]-3-hydroxy-1-piperidyl]methanone | Int 2011 + CAS# 161157-50-2 | example | 484.6 | 485.5 |
| 111 | 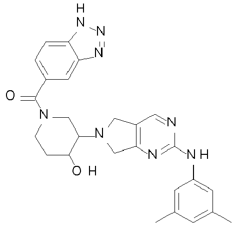 | 1H-benzotriazol-5-yl-[3-[2-(3,5-dimethylanilino)-5,7-dihydropyrrolo[3,4-d]pyrimidin-6-yl]-4-hydroxy-1-piperidyl]methanone | Int 2011 + CAS# 161157-50-2 | example | 484.6 | 485.5 |
| 112 | 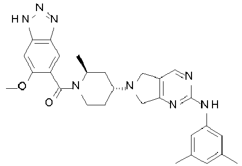 | [(2S,4R)-4-[2-(3,5-dimethylanilino)-5,7-dihydropyrrolo[3,4-d]pyrimidin-6-yl]-2-methyl-1-piperidyl]-(6-methoxy-1H-benzotriazol-5-yl)methanone | Int 2011 + Int 79 | C | 512.6 | 513.5 |
| 113 | 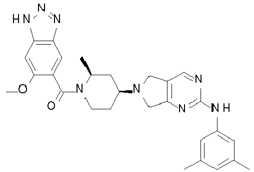 | [(2S,4S)-4-[2-(3,5-dimethylanilino)-5,7-dihydropyrrolo[3,4-d]pyrimidin-6-yl]-2-methyl-1-piperidyl]-(6-methoxy-1H-benzotriazol-5-yl)methanone | Int 2011 + Int 79 | C | 512.6 | 513.2 |
| 114 | 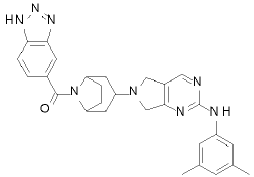 | 1H-benzotriazol-5-yl-[3-[2-(3,5-dimethylanilino)-5,7-dihydropyrrolo[3,4-d]pyrimidin-6-yl]-8-azabicyclo[3.2.1]octan-8-yl]methanone | Int 2011 + Int 80 | C | 494.6 | 495.6 |
| 115 | 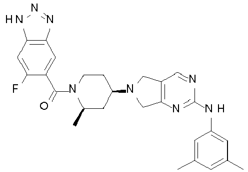 | [(2R,4R)-4-[2-(3,5-dimethylanilino)-5,7-dihydropyrrolo[3,4-d]pyrimidin-6-yl]-2-methyl-1-piperidyl]-(6-fluoro-1H-benzotriazol-5-yl)methanone | Int 39 + CAS# 1427081-62-6 | example | 500.6 | 501.5 |
| 116 | 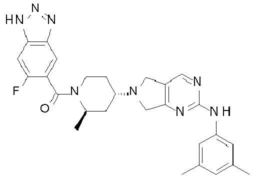 | [(2R,4S)-4-[2-(3,5-dimethylanilino)-5,7-dihydropyrrolo[3,4-d]pyrimidin-6-yl]-2-methyl-1-piperidyl]-(6-fluoro-1H-benzotriazol-5-yl)methanone | Int 2011 + Int 105 | C | 500.6 | 501.5 |
| 117 | 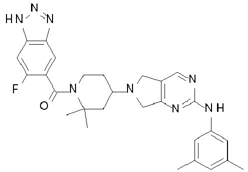 | [4-[2-(3,5-dimethylanilino)-5,7-dihydropyrrolo[3,4-d]pyrimidin-6-yl]-2,2-dimethyl-1-piperidyl]-(6-fluoro-1H-benzotriazol-5-yl)methanone | Int 52 + Int 57 | example | 514.6 | 515.6 |
| 118 | 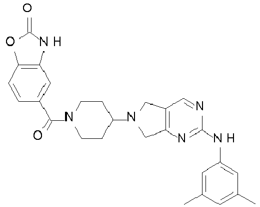 | 5-[4-[2-(3,5-dimethylanilino)-5,7-dihydropyrrolo[3,4-d]pyrimidin-6-yl]piperidine-1-carbonyl]-3H-1,3-benzoxazol-2-one | Int 2011 + Int 81 | example | 484.5 | 485.5 |
| 119 | 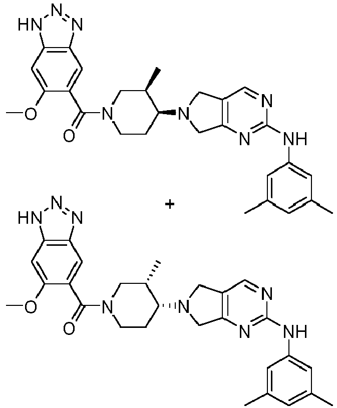 | [(3R,4S/3S,4R)-4-[2-(3,5-dimethylanilino)-5,7-dihydropyrrolo[3,4-d]pyrimidin-6-yl]-3-methyl-1-piperidyl]-(6-methoxy-1H-benzotriazol-5-yl)methanone | Int 2011 + Int 82 | C | 512.6 | 513.6 |
| 120 | 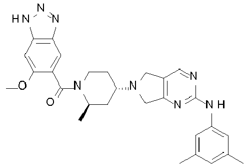 | [(2R,4S)-4-[2-(3,5-dimethylanilino)-5,7-dihydropyrrolo[3,4-d]pyrimidin-6-yl]-2-methyl-1-piperidyl]-(6-methoxy-1H-benzotriazol-5-yl)methanone | Int 2011 + Int 103 | C | 512.6 | 513.6 |
| 121 | 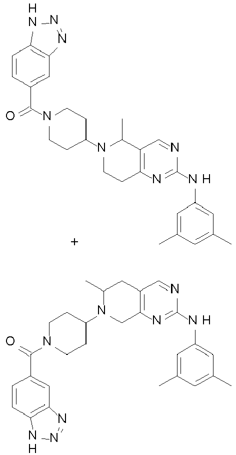 | 1H-benzotriazol-5-yl-[4-[2-(3,5-dimethylanilino)-5-methyl-7,8-dihydro-5H-pyrido[4,3-d]pyrimidin-6-yl]-1-piperidyl]methanone / 1H-benzotriazol-5-yl-[4-[2-(3,5-dimethylanilino)-6-methyl-6,8-dihydro-5H-pyrido[3,4-d]pyrimidin-7-yl]-1-pipe ridyl]methanone mixture | CAS# 190906-92-4 | example | 496.6 | 497.5 |
| 122 | 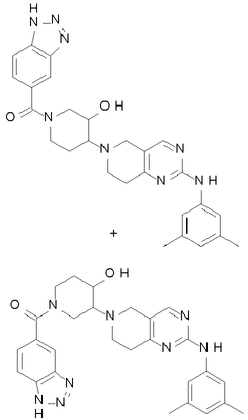 | 1H-benzotriazol-5-yl-[4-[2-(3,5-dimethylanilino)-7,8-dihydro-5H-pyrido[4,3-d]pyrimidin-6-yl]-3-hydroxy-1-piperidyl]methanone/1H-benzotriazol-5-yl-[3-[2-(3,5-dimethylanilino)-7,8-dihydro-5H-pyrido[4,3-d]pyrimidin-6-yl]-4-hydroxy-1-piperidyl]methanone mixture | Int 2011 + CAS# 161157-50-2 | example | 498.6 | 499.5 |
| 123 | 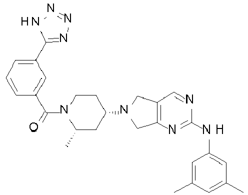 | [(2S,4S)-4-[2-(3,5-dimethylanilino)-5,7-dihydropyrrolo[3,4-d]pyrimidin-6-yl]-2-methyl-1-piperidyl]-[3-(1H-tetrazol-5-yl)phenyl]methanone | Int 40 + CAS# 73096-39-6 | A | 509.6 | 510.6 |
| 124 | 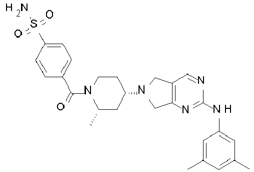 | 4-[(2S,4S)-4-[2-(3,5-dimethylanilino)-5,7-dihydropyrrolo[3,4-d]pyrimidin-6-yl]-2-methyl-piperidine-1-carbonyl]benzenesulfonamide | Int 40 + CAS# 138-41-0 | example | 520.6 | 521.5 |
| 125 | 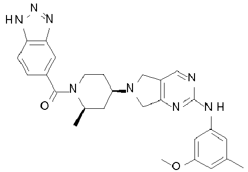 | 1H-benzotriazol-5-yl-[(2R,4R)-4-[2-(3-methoxy-5-methyl-anilino)-5,7-dihydropyrrolo[3,4-d]pyrimidin-6-yl]-2-methyl-1-piperidyl]methanone | Int 131 + Int 2026 | example | 498.6 | 499.5 |
| 126 | 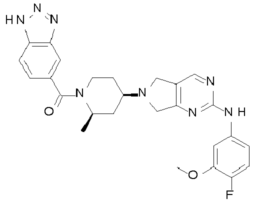 | 1H-benzotriazol-5-yl-[(2R,4R)-4-[2-(4-fluoro-3-methoxy-anilino)-5,7-dihydropyrrolo[3,4-d]pyrimidin-6-yl]-2-methyl-1-piperidyl]methanone | Int 131 + Int 2072 | C | 502.5 | 503.5 |
| 127 | 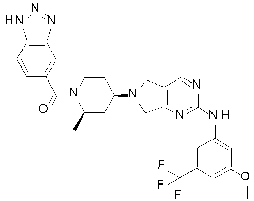 | 1H-benzotriazol-5-yl-[(2R,4R)-4-[2-[3-methoxy-5-(trifluoromethyl)anilino]-5,7-dihydropyrrolo[3,4-d]pyrimidin-6-yl]-2-methyl-1-piperidyl]methanone | Int 131 + Int 2073 | C | 552.6 | 553.5 |
| 128 | 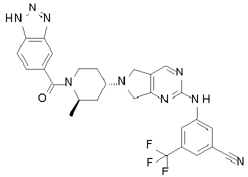 | 3-[[6-[(2R,4S)-1-(1H-benzotriazole-5-carbonyl)-2-methyl-4-piperidyl]-5,7-dihydropyrrolo[3,4-d]pyrimidin-2-yl]amino]-5-(trifluoromethyl)benzonitrile | Int 131 + Int 2074 | C | 547.5 | 548.5 |
| 129 | 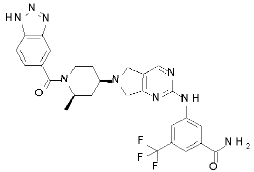 | 3-[[6-[(2R,4R)-1-(1H-benzotriazole-5-carbonyl)-2-methyl-4-piperidyl]-5,7-dihydropyrrolo[3,4-d]pyrimidin-2-yl]amino]-5-(trifluoromethyl)benzamide | Int 131 + Int 2074 | C | 565.5 | 566.5 |
| 130 | 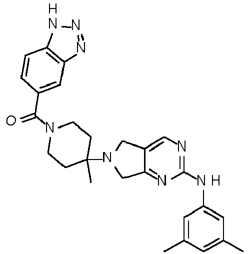 | 1H-benzotriazol-5-yl-[4-[2-(3,5-dimethylanilino)-5,7-dihydropyrrolo[3,4-d]pyrimidin-6-yl]-4-methyl-1-piperidyl]methanone | Int 111 + CAS# 23814-12-2 | example | 482.6 | 483.5 |
| 131 | 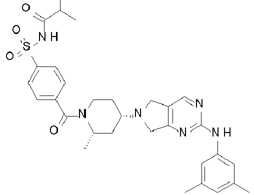 | N-[4-[(2S,4S)-4-[2-(3,5-dimethylanilino)-5,7-dihydropyrrolo[3,4-d]pyrimidin-6-yl]-2-methyl-piperidine-1-carbonyl]phenyl]sulfonyl-2-methyl-propanamide | Cpd 124 + CAS# 79-31-2 | example | 590.7 | 591.6 |
| 132 | 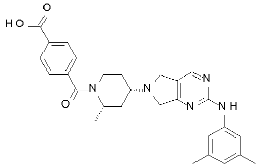 | 4-[(2S,4S)-4-[2-(3,5-dimethylanilino)-5,7-dihydropyrrolo[3,4-d]pyrimidin-6-yl]-2-methyl-piperidine-1-carbonyl]benzoic acid | Int 40 + CAS# 100-21-0 | example | 485.6 | 486.6 |
| 133 | 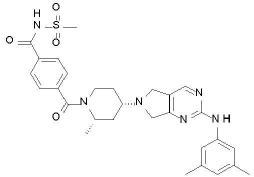 | 4-[(2S,4S)-4-[2-(3,5-dimethylanilino)-5,7-dihydropyrrolo[3,4-d]pyrimidin-6-yl]-2-methyl-piperidine-1-carbonyl]-N-methylsulfonylbenzamide | Cpd 132 + CAS# 3144-09-0 | example | 562.7 | 563.6 |
| 134 | 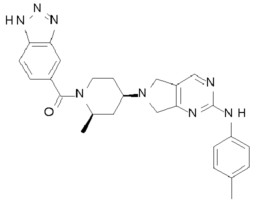 | 1H-benzotriazol-5-yl-[(2R,4R)-2-methyl-4-[2-(4-methylanilino)-5,7-dihydropyrrolo[3,4-d]pyrimidin-6-yl]-1-piperidyl]methanone | Int 131 + Int 2067 | C | 468.6 | 469.4 |
| 135 | 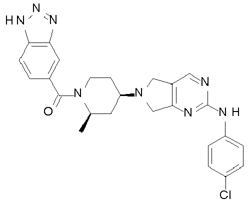 | 1H-benzotriazol-5-yl-[(2R,4R)-4-[2-(4-chloroanilino)-5,7-dihydropyrrolo[3,4-d]pyrimidin-6-yl]-2-methyl-1-piperidyl]methanone | Int 131 + Int 2066 | C | 489.0 | 489.5 |
| 136 | 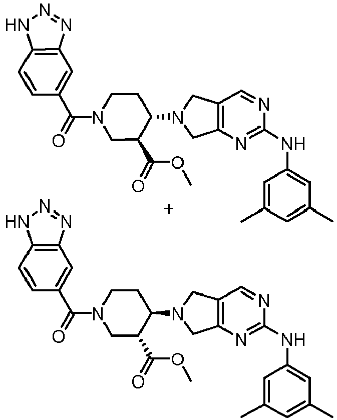 | methyl (3S,4S)-1-(1H-benzotriazole-5-carbonyl)-4-[2-(3,5-dimethylanilino)-5,7-dihydropyrrolo[3,4-d]pyrimidin-6-yl]piperidine-3-carboxylate | Int 77 + Int 2011 | example | 526.6 | 527.5 |
| 137 | 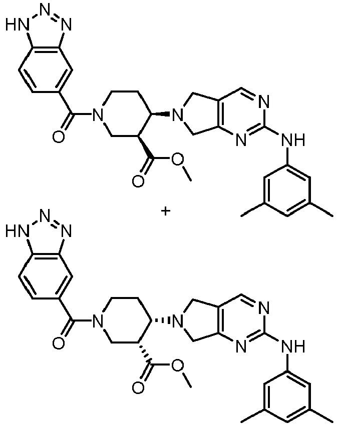 | methyl (3S,4R/3R,4S)-1-(1H-benzotriazole-5-carbonyl)-4-[2-(3,5-dimethylanilino)-5,7-dihydropyrrolo[3,4-d]pyrimidin-6-yl]piperidine-3-carboxylate | Int 77 + Int 2011 | example | 526.6 | 527.5 |
| 138 | 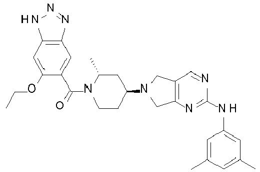 | [(2R,4S)-4-[2-(3,5-dimethylanilino)-5,7-dihydropyrrolo[3,4-d]pyrimidin-6-yl]-2-methyl-1-piperidyl]-(6-ethoxy-1H-benzotriazol-5-yl)methanone | Int 83 + Int 2011 | example | 526.6 | 527.5 |
| 139 | 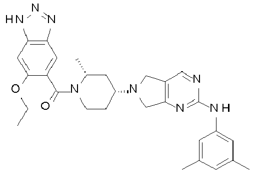 | [(2R,4R)-4-[2-(3,5-dimethylanilino)-5,7-dihydropyrrolo[3,4-d]pyrimidin-6-yl]-2-methyl-1-piperidyl]-(6-ethoxy-1H-benzotriazol-5-yl)methanone | Int 83 + Int 2011 | example | 526.6 | 527.5 |
| 140 | 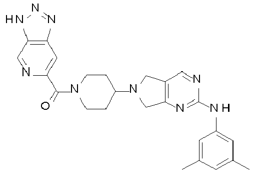 | [4-[2-(3,5-dimethylanilino)-5,7-dihydropyrrolo[3,4-d]pyrimidin-6-yl]-1-piperidyl]-(3H-triazolo[4,5-c]pyridin-6-yl)methanone | Int 11 + Int 106 | A | 469.5 | 470.5 |
| 141 | 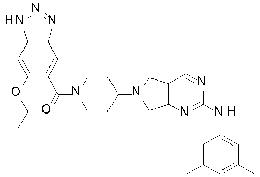 | [4-[2-(3,5-dimethylanilino)-5,7-dihydropyrrolo[3,4-d]pyrimidin-6-yl]-1-piperidyl]-(6-ethoxy-1H-benzotriazol-5-yl)methanone | Int 11 + Int 107 | A | 512.6 | 513.5 |
| 142 | 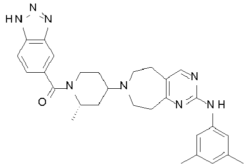 | 1H-benzotriazol-5-yl-[(2S)-4-[2-(3,5-dimethylanilino)-5,6,8,9-tetrahydropyrimido[4,5-d]azepin-7-yl]-2-methyl-1-piperidyl]methanone | CAS# 141642-82-2 | example | 510.6 | 511.5 |
| 143 | 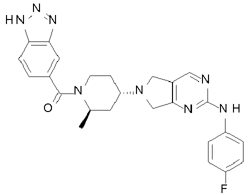 | 1H-benzotriazol-5-yl-[(2R,4S)-4-[2-(4-fluoroanilino)-5,7-dihydropyrrolo[3,4-d]pyrimidin-6-yl]-2-methyl-1-piperidyl]methanone | Int 131 + Int 2049 | C | 472.5 | 473.5 |
| 144 | 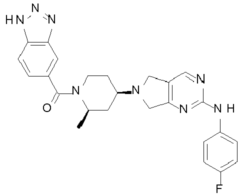 | 1H-benzotriazol-5-yl-[(2R,4R)-4-[2-(4-fluoroanilino)-5,7-dihydropyrrolo[3,4-d]pyrimidin-6-yl]-2-methyl-1-piperidyl] methanone | Int 131 + Int 2049 | C | 472.5 | 473.5 |
| 145 | 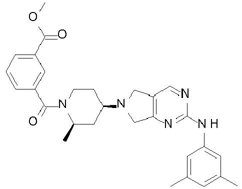 | methyl 3-[(2R,4R)-4-[2-(3,5-dimethyl anilino)-5,7-dihydropyrrolo[3,4-d]pyrimidin-6-yl]-2-methyl-piperidine-1-carbonyl]benzoate | Int 39 + CAS# 1877-71-0 | A | 499.6 | 500.5 |
| 146 | 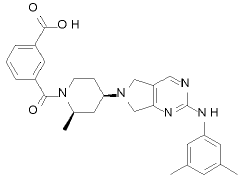 | 3-[(2R,4R)-4-[2-(3,5-dimethylanilino)-5,7-dihydropyrrolo[3,4-d]pyrimidin-6-yl]-2-methyl-piperidine-1-carbonyl]benzoic acid | Cpd 145 | example | 485.6 | 486.5 |
| 147 | 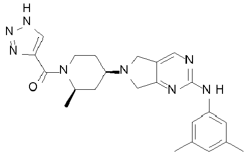 | [(2R,4R)-4-[2-(3,5-dimethylanilino)-5,7-dihydropyrrolo[3,4-d]pyrimidin-6-yl]-2-methyl-1-piperidyl]-(1H-triazol-4-yl)methanone | Int 39 + CAS# 16681-70-2 | A | 432.5 | 433.5 |
| 148 | 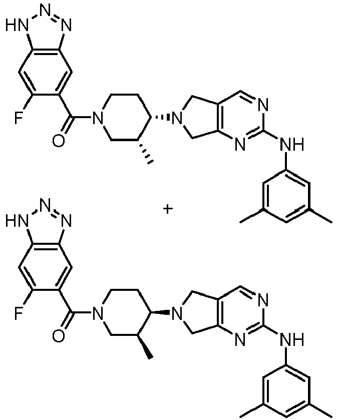 | [(3R,4S/3S,4R)-4-[2-(3,5-dimethylanilino)-5,7-dihydropyrrolo[3,4-d]pyrimidin-6-yl]-3-methyl-1-piperidyl]-(6-fluoro-1H-benzotriazol-5-yl)methanone | Int 004 + CAS# 1427081-62-6 | A | 500.6 | 501.5 |
| 149 | 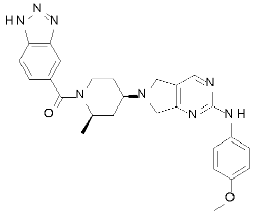 | 1H-benzotriazol-5-yl-[(2R,4R)-4-[2-(4-methoxyanilino)-5,7-dihydropyrrolo[3,4-d]pyrimidin-6-yl]-2-methyl-1-piperidyl] methanone | Int 131 + Int 2076 | C | 484.6 | 485.5 |
| 150 | 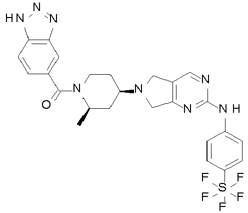 | 1H-benzotriazol-5-yl-[(2R,4R)-2-methyl-4-[2-[4-(pentafluoro-λ⁶-sulfanyl)anilino]-5,7-dihydropyrrolo[3,4-d]pyrimidin-6-yl]-1-piperidyl]methanone | Int 131 + Int 2065 | C | 580.6 | 581.5 |
| 151 | 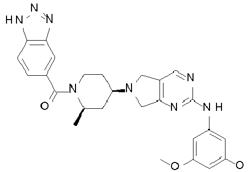 | 1H-benzotriazol-5-yl-[(2R,4R)-4-[2-(3,5-dimethoxyanilino)-5,7-dihydropyrrolo[3,4-d]pyrimidin-6-yl]-2-methyl-1-piperidyl]methanone | Int 131 + Int 2003 | C | 514.6 | 515.6 |
| 152 | 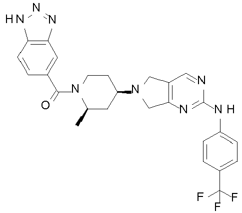 | 1H-benzotriazol-5-yl-[(2R,4R)-2-methyl-4-[2-[4-(trifluoromethyl)anilino]-5,7-dihydropyrrolo[3,4-d]pyrimidin-6-yl]-1-piperidyl]methanone | Int 131 + Int 2016 | C | 522.5 | 523.4 |
| 153 | 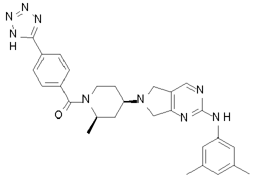 | [(2R,4R)-4-[2-(3,5-dimethylanilino)-5,7-dihydropyrrolo[3,4-d]pyrimidin-6-yl]-2-methyl-1-piperidyl]-[4-(1H-tetrazol-5-yl)phenyl]methanone | Int 39 + CAS# 34114-12-0 | example | 509.6 | 510.5 |
| 154 | 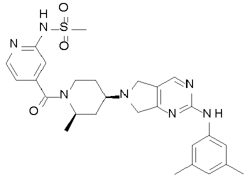 | N-[4-[(2R,4R)-4-[2-(3,5-dimethylanilino)-5,7-dihydropyrrolo[3,4-d]pyrimidin-6-yl]-2-methyl-piperidine-1-carbonyl]-2-pyridyl]methanesulfonamide | Int 39 + Int 58 | A | 535.7 | 536.5 |
| 155 | 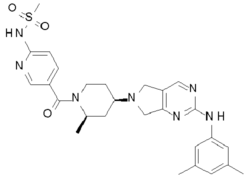 | N-[5-[(2R,4R)-4-[2-(3,5-dimethylanilino)-5,7-dihydropyrrolo[3,4-d]pyrimidin-6-yl]-2-methyl-piperidine-1-carbonyl]-2-pyridyl]methanesulfon amide | Int 39 + CAS# 960324-86-1 | A | 535.7 | 536.5 |
| 156 | 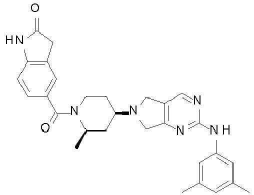 | 5-[(2R,4R)-4-[2-(3,5-dimethylanilino)-5,7-dihydropyrrolo[3,4-d]pyrimidin-6-yl]-2-methyl-piperidine-1-carbonyl]indolin-2-one | Int 39 + CAS# 102359-00-2 | A | 496.6 | 497.4 |
| 157 | 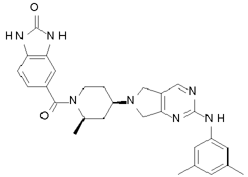 | 5-[(2R,4R)-4-[2-(3,5-dimethylanilino)-5,7-dihydropyrrolo[3,4-d]pyrimidin-6-yl]-2-methyl-piperidine-1-carbonyl]-1,3-dihydrobenzimidazol-2-one | Int 39 + CAS# 23814-14-4 | A | 497.6 | 497.5 |
| 158 | 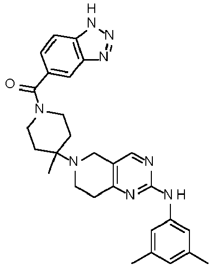 | 1H-benzotriazol-5-yl-[4-[2-(3,5-dimethylanilino)-7,8-dihydro-5H-pyrido[4,3-d]pyrimidin-6-yl]-4-methyl-1-piperidyl]methanone | CAS# 5382-16-1 + CAS# 19099-93-5 | example | 496.6 | 497.4 |
| 159 | 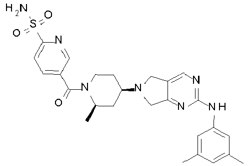 | 5-[(2R,4R)-4-[2-(3,5-dimethylanilino)-5,7-dihydropyrrolo[3,4-d]pyrimidin-6-yl]-2-methyl-piperidine-1-carbonyl]pyridine-2-sulfonamide | Int 39 + CAS# 285135-56-0 | A | 521.6 | 522.4 |
| 160 | 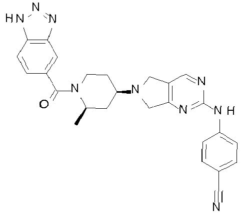 | 4-[[6-[(2R,4R)-1-(1H-benzotriazole-5-carbonyl)-2-methyl-4-piperidyl]-5,7-dihydropyrrolo[3,4-d]pyrimidin-2-yl]amino]benzonitrile | CAS# 365996-86-7 | example | 479.5 | 480.4 |
| 161 | 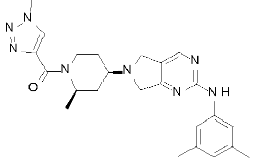 | [(2R,4R)-4-[2-(3,5-dimethylanilino)-5,7-dihydropyrrolo[3,4-d]pyrimidin-6-yl]-2-methyl-1-piperidyl]-(1-methyltriazol-4-yl)methanone | Int 39 + Int 135 | A | 446.5 | 447.5 |
| 162 | 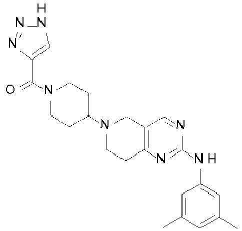 | [4-[2-(3,5-dimethylanilino)-7,8-dihydro-5H-pyrido[4,3-d]pyrimidin-6-yl]-1-piperidyl]-(1H-triazol-4-yl)methanone | Int 25 + CAS# 16681-70-2 | A | 432.5 | 433.5 |
| 163 | 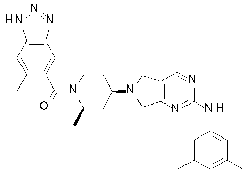 | [(2R,4R)-4-[2-(3,5-dimethylanilino)-5,7-dihydropyrrolo[3,4-d]pyrimidin-6-yl]-2-methyl-1-piperidyl]-(6-methyl-1H-benzotriazol-5-yl)methanone | Int 39 + Int 59 | A | 496.6 | 497.5 |
| 164 | 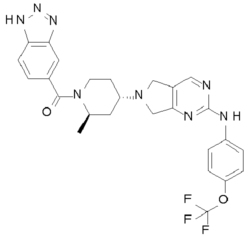 | 1H-benzotriazol-5-yl-[(2R,4S)-2-methyl-4-[2-[4-(trifluoromethoxy)anilino]-5,7-dihydropyrrolo[3,4-d]pyrimidin-6-yl]-1-piperidyl]methanone | Int 131 + Int 2077 | example | 538.5 | 539.4 |
| 165 | 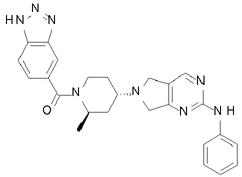 | [(2R,4S)-4-(2-anilino-5,7-dihydropyrrolo[3,4-d]pyrimidin-6-yl)-2-methyl-1-piperidyl]-(1H-benzotriazol-5-yl)methanone | Int 131 + Int 2078 | C | 454.5 | 455.4 |
| 166 | 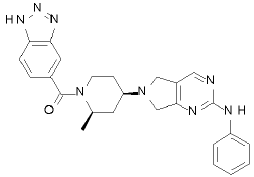 | [(2R,4R)-4-(2-anilino-5,7-dihydropyrrolo[3,4-d]pyrimidin-6-yl)-2-methyl-1-piperidyl]-(1H-benzotriazol-5-yl)methanone | Int 131 + Int 2078 | C | 454.5 | 455.4 |
| 167 | 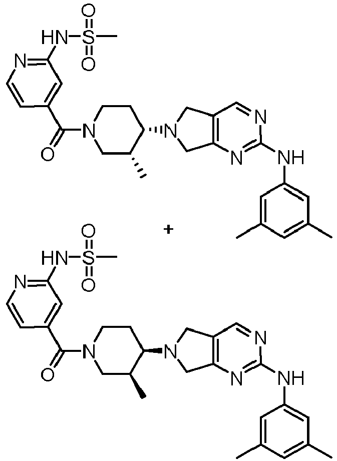 | N-[4-[(3R,4S/3S,4R)-4-[2-(3,5-dimethylanilino)-5,7-dihydropyrrolo[3,4-d]pyrimidin-6-yl]-3-methyl-piperidine-1-carbonyl]-2-pyridyl]methanesulfonamide | Int 55 + Int 58 | A | 535.7 | 536.4 |
| 168 | 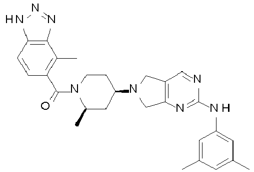 | [(2R,4R)-4-[2-(3,5-dimethylanilino)-5,7-dihydropyrrolo[3,4-d]pyrimidin-6-yl]-2-methyl-1-piperidyl]-(4-methyl-1H-benzotriazol-5-yl)methanone | Int 39 + Int 60 | A | 496.6 | 497.5 |
| 169 | 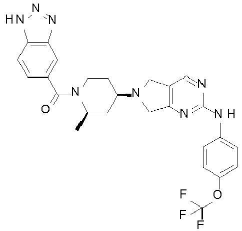 | 1H-benzotriazol-5-yl-[(2R,4R)-2-methyl-4-[2-[4-(trifluoromethoxy)anilino]-5,7-dihydropyrrolo[3,4-d]pyrimidin-6-yl]-1-piperidyl]methanone | Int 131 + Int 2077 | example | 538.5 | 539.4 |
| 170 | 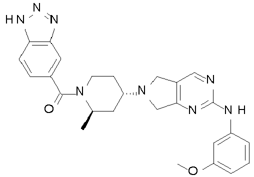 | 1H-benzotriazol-5-yl-[(2R,4S)-4-[2-(3-methoxyanilino)-5,7-dihydropyrrolo[3,4-d]pyrimidin-6-yl]-2-methyl-1-piperidyl]methanone | Int 131 + Int 2050 | C | 484.6 | 485.4 |
| 171 | 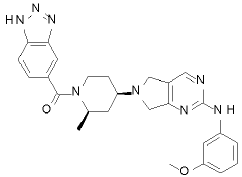 | 1H-benzotriazol-5-yl-[(2R,4R)-4-[2-(3-methoxyanilino)-5,7-dihydropyrrolo[3,4-d]pyrimidin-6-yl]-2-methyl-1-piperidyl]methanone | Int 131 + Int 2050 | C | 484.6 | 485.4 |
| 172 | 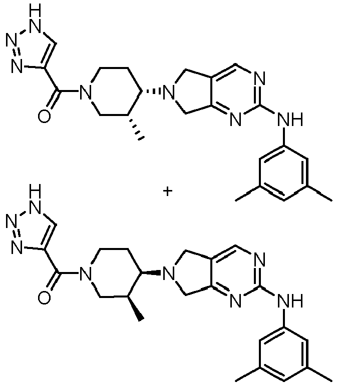 | [(3R,4S/3S,4R)-4-[2-(3,5-dimethylanilino)-5,7-dihydropyrrolo[3,4-d]pyrimidin-6-yl]-3-methyl-1-piperidyl]-(1H-triazol-4-yl)methanone | Int 2011 + CAS# 181269-69-2 + CAS# 16681-70-2 | C + B + A | 432.5 | 433.4 |
| 173 | 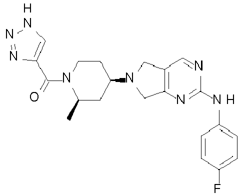 | [(2R,4R)-4-[2-(4-fluoroanilino)-5,7-dihydropyrrolo[3,4-d]pyrimidin-6-yl]-2-methyl-1-piperidyl]-(1H-triazol-4-yl)methanone | Int 89 + Int 2049 | C | 422.5 | 423.4 |
| 174 | 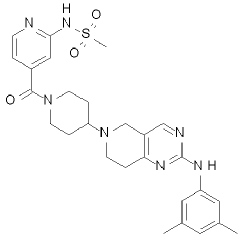 | N-[4-[4-[2-(3,5-dimethylanilino)-7,8-dihydro-5H-pyrido[4,3-d]pyrimidin-6-yl]piperidine-1-carbonyl]-2-pyridyl]methanesulfonamide | Int 25 + Int 58 | A | 535.7 | 536.5 |
| 175 | 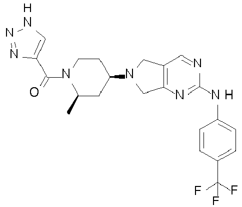 | [(2R,4R)-2-methyl-4-[2-[4-(trifluoromethyl)anilino]-5,7-dihydropyrrolo[3,4-d]pyrimidin-6-yl]-1-piperidyl]-(1H-triazol-4-yl)methanone | Int 89 + Int 2016 | C | 472.5 | 473.4 |
| 176 | 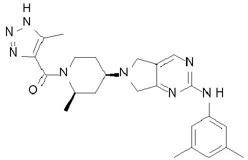 | [(2R,4R)-4-[2-(3,5-dimethylanilino)-5,7-dihydropyrrolo[3,4-d]pyrimidin-6-yl]-2-methyl-1-piperidyl]-(5-methyl-1H-triazol-4-yl)methanone | Int 39 + CAS# 89166-02-9 | A | 446.5 | 447.5 |
| 177 | 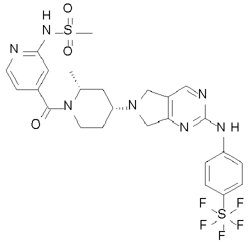 | N-[4-[(2R,4R)-2-methyl-4-[2-[4-(pentafluoro-λ⁶-sulfanyl)anilino]-5,7-dihydropyrrolo[3,4-d]pyrimidin-6-yl]piperidine-1-carbonyl]-2-pyridyl]methanesulfonamide | Int 74 + Int 2065 | C | 633.7 | 634.4 |
| 178 | 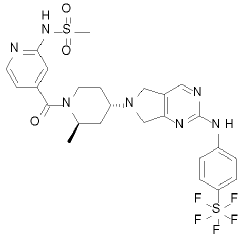 | N-[4-[(2R,4S)-2-methyl-4-[2-[4-(pentafluoro-λ⁶-sulfanyl)anilino]-5,7-dihydropyrrolo[3,4-d]pyrimidin-6-yl]piperidine-1-carbonyl]-2-pyridyl]methanesulfonamide | Int 74 + Int 2065 | C | 633.7 | 634.4 |
| 179 | 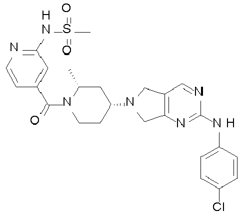 | N-[4-[(2R,4R)-4-[2-(4-chloroanilino)-5,7-dihydropyrrolo[3,4-d]pyrimidin-6-yl]-2-methyl-piperidine-1-carbonyl]-2-pyridyl]methanesulfonamide | Int 74 + Int 2066 | C | 542.1 | 542.4 |
| 180 | 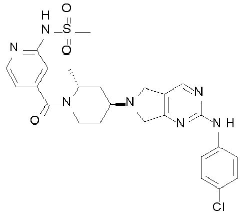 | N-[4-[(2R,4S)-4-[2-(4-chloroanilino)-5,7-dihydropyrrolo[3,4-d]pyrimidin-6-yl]-2-methyl-piperidine-1-carbonyl]-2-pyridyl]methanesulfonamide | Int 74 + Int 2066 | C | 542.1 | 542.4 |
| 181 | 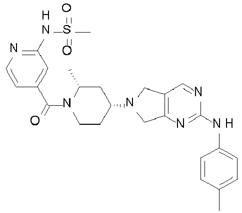 | N-[4-[(2R,4R)-2-methyl-4-[2-(4-methylanilino)-5,7-dihydropyrrolo[3,4-d]pyrimidin-6-yl]piperidine-1-carbonyl]-2-pyridyl]methanesulfonamide | Int 74 + Int 2067 | C | 521.6 | 522.5 |
| 182 | 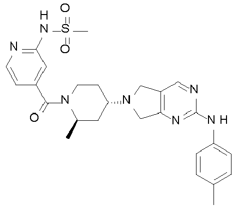 | N-[4-[(2R,4S)-2-methyl-4-[2-(4-methylanilino)-5,7-dihydropyrrolo[3,4-d]pyrimidin-6-yl]piperidine-1-carbonyl]-2-pyridyl]methanesulfonamide | Int 74 + Int 2067 | C | 521.6 | 522.5 |
| 183 | 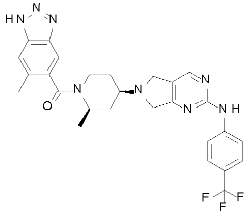 | (6-methyl-1H-benzotriazol-5-yl)-[(2R,4R)-2-methyl-4-[2-[4-(trifluoromethyl)anilino]-5,7-dihydropyrrolo[3,4-d]pyrimidin-6-yl]-1-piperidyl]methanone | Int 119 + Int 2016 | C | 536.6 | 537.5 |
| 184 | 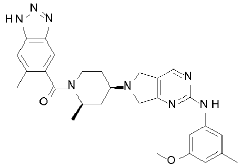 | [(2R,4R)-4-[2-(3-methoxy-5-methyl-anilino)-5,7-dihydropyrrolo[3,4-d]pyrimidin-6-yl]-2-methyl-1-piperidyl]-(6-methyl-1H-benzotriazol-5-yl)methanone | Int 119 + Int 2026 | C | 512.6 | 513.5 |
| 185 | 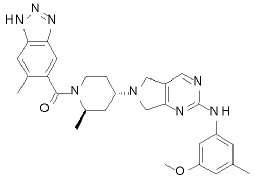 | [(2R,4S)-4-[2-(3-methoxy-5-methyl-anilino)-5,7-dihydropyrrolo[3,4-d]pyrimidin-6-yl]-2-methyl-1-piperidyl]-(6-methyl-1H-benzotriazol-5-yl)methanone | Int 119 + Int 2026 | C | 512.6 | 513.5 |
| 186 | 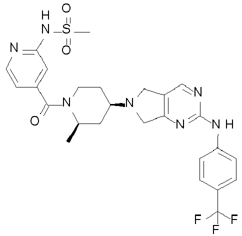 | N-[4-[(2R,4R)-2-methyl-4-[2-[4-(trifluoromethyl)anilino]-5,7-dihydropyrrolo[3,4-d]pyrimidin-6-yl]piperidine-1-carbonyl]-2-pyridyl]methanesulfonamide | Int 74 + Int 2016 | C | 575.6 | 576.4 |
| 187 | 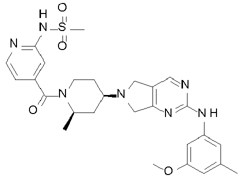 | N-[4-[(2R,4R)-4-[2-(3-methoxy-5-methyl-anilino)-5,7-dihydropyrrolo[3,4-d]pyrimidin-6-yl]-2-methyl-piperidine-1-carbonyl]-2-pyridyl]methanesulfonamide | Int 74 + Int 2026 | C | 551.7 | 552.5 |
| 188 | 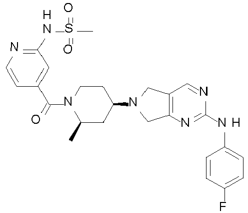 | N-[4-[(2R,4R)-4-[2-(4-fluoroanilino)-5,7-dihydropyrrolo[3,4-d]pyrimidin-6-yl]-2-methyl-piperidine-1-carbonyl]-2-pyridyl]methanesulfonamide | Int 74 + Int 2049 | C | 525.6 | 526.4 |
| 189 | 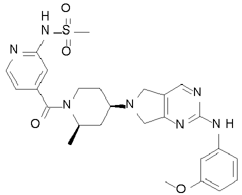 | N-[4-[(2R,4R)-4-[2-(3-methoxyanilino)-5,7-dihydropyrrolo[3,4-d]pyrimidin-6-yl]-2-methyl-piperidine-1-carbonyl]-2-pyridyl]methanesulfonamide | Int 74 + Int 2050 | C | 537.6 | 538.5 |
| 190 | 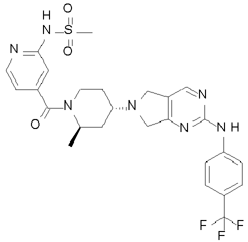 | N-[4-[(2R,4S)-2-methyl-4-[2-[4-(trifluoromethyl)anilino]-5,7-dihydropyrrolo[3,4-d]pyrimidin-6-yl]piperidine-1-carbonyl]-2-pyridyl]methanesulfonamide | Int 74 + Int 2016 | C | 575.6 | 576.4 |
| 191 | 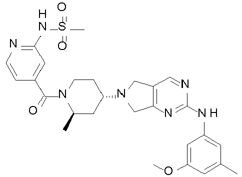 | N-[4-[(2R,4S)-4-[2-(3-methoxy-5-methyl-anilino)-5,7-dihydropyrrolo[3,4-d]pyrimidin-6-yl]-2-methyl-piperidine-1-carbonyl]-2-pyridyl]methanesulfonamide | Int 74 + Int 2026 | C | 551.7 | 552.4 |
| 192 | 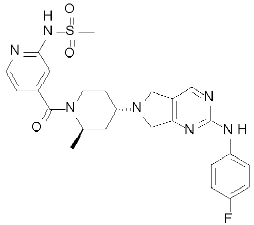 | N-[4-[(2R,4S)-4-[2-(4-fluoroanilino)-5,7-dihydropyrrolo[3,4-d]pyrimidin-6-yl]-2-methyl-piperidine-1-carbonyl]-2-pyridyl]methanesulfonamide | Int 74 + Int 2049 | C | 525.6 | 526.4 |
| 193 | 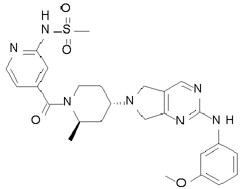 | N-[4-[(2R,4S)-4-[2-(3-methoxyanilino)-5,7-dihydropyrrolo[3,4-d]pyrimidin-6-yl]-2-methyl-piperidine-1-carbonyl]-2-pyridyl]methanesulfonamide | Int 74 + Int 2050 | C | 537.6 | 538.5 |
| 194 | 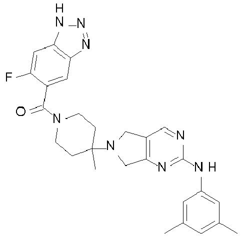 | [4-[2-(3,5-dimethylanilino)-5,7-dihydropyrrolo[3,4-d]pyrimidin-6-yl]-4-methyl-1-piperidyl]-(6-fluoro-1H-benzotriazol-5-yl)methanone | Int 111 + CAS# 1427081-62-6 | A | 500.6 | 501.4 |
| 195 | 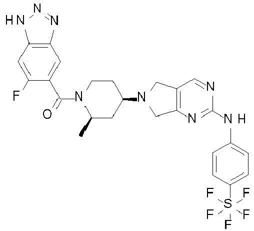 | (6-fluoro-1H-benzotriazol-5-yl)-[(2R,4R)-2-methyl-4-[2-[4-(pentafluoro-λ⁶-sulfanyl)anilino]-5,7-dihydropyrrolo[3,4-d]pyrimidin-6-yl]-1-piperidyl]methanone | Int 105 + Int 2065 | C | 598.6 | 599.4 |
| 196 | 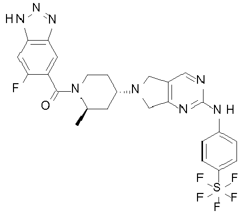 | (6-fluoro-1H-benzotriazol-5-yl)-[(2R,4S)-2-methyl-4-[2-[4-(pentafluoro-λ⁶-sulfanyl)anilino] -5,7-dihydropyrrolo[3,4-d]pyrimidin-6-yl]-1-piperidyl]methanone | Int 105 + Int 2065 | C | 598.6 | 599.4 |
| 197 | 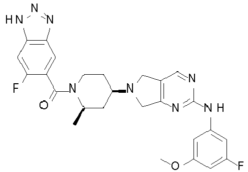 | (6-fluoro-1H-benzotriazol-5-yl)-[(2R,4R)-4-[2-(3-fluoro-5-methoxy-anilino)-5,7-dihydropyrrolo[3,4-d]pyrimidin-6-yl]-2-methyl-1-piperidyl] methanone | Int 131 + Int 2003 | C | 520.5 | 521.4 |
| 198 | 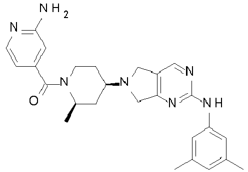 | (2-amino-4-pyridyl)-[(2R,4R)-4-[2-(3,5-dimethylanilino)-5,7-dihydropyrrolo[3,4-d]pyrimidin-6-yl]-2-methyl-1-piperidyl]methanone | Int 39 + CAS# 13362-28-2 | A | 457.6 | 458.5 |
| 199 | 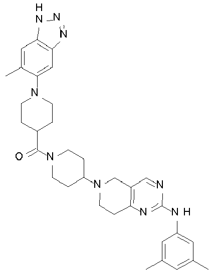 | [4-[2-(3,5-dimethylanilino)-7,8-dihydro-5H-pyrido[4,3-d]pyrimidin-6-yl]-1-piperidyl]-[1-(6-methyl-1H-benzotriazol-5-yl)-4-piperidyl]methanone | Int 25 + Int 136 | A | 579.7 | 580.6 |
| 200 | 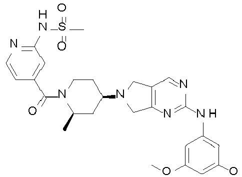 | N-[4-[(2R,4R)-4-[2-(3,5-dimethoxyanilino)-5,7-dihydropyrrolo[3,4-d]pyrimidin-6-yl]-2-methyl-piperidine-1-carbonyl]-2-pyridyl]methanesulfonamide | Int 74 + Int 2003 | C | 567.7 | 568.5 |
| 201 | 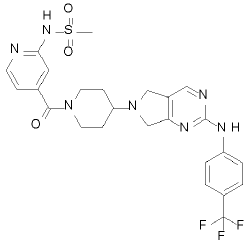 | N-[4-[4-[2-[4-(trifluoromethyl)anilino]-5,7-dihydropyrrolo[3,4-d]pyrimidin-6-yl]piperidine-1-carbonyl]-2-pyridyl]methanesulfonamide | Int 113 + Int 58 | A | 561.6 | 562.4 |
| 202 | 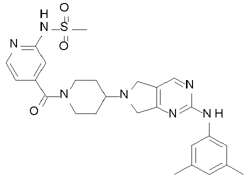 | N-[4-[4-[2-(3,5-dimethylanilino)-5,7-dihydropyrrolo[3,4-d]pyrimidin-6-yl]piperidine-1-carbonyl]-2-pyridyl]methanesulfonamide | Int 11 + Int 58 | A | 521.6 | 522.5 |
| 203 | 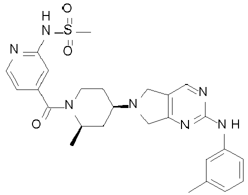 | N-[4-[(2R,4R)-2-methyl-4-[2-(3-methylanilino)-5,7-dihydropyrrolo[3,4-d]pyrimidin-6-yl]piperidine-1-carbonyl]-2-pyridyl]methanesulfonamide | Int 74 + Int 2083 | C | 521.6 | 522.4 |
| 204 | 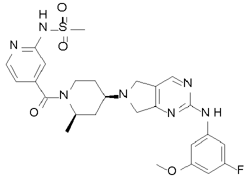 | N-[4-[(2R,4R)-4-[2-(3-fluoro-5-methoxy-anilino)-5,7-dihydropyrrolo[3,4-d]pyrimidin-6-yl] -2-methyl-piperidine-1-carbonyl]-2-pyridyl]methanesulfonamide | Int 74 + Int 2084 | C | 555.6 | 556.4 |
| 205 | 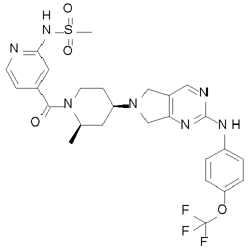 | N-[4-[(2R,4R)-2-methyl-4-[2-[4-(trifluoromethoxy)anilino]-5,7-dihydropyrrolo[3,4-d]pyrimidin-6-yl]piperidine-1-carbonyl]-2-pyridyl]methanesulfonamide | Int 74 + Int 2077 | C | 591.6 | 592.3 |
| 206 | 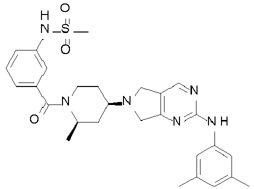 | N-[3-[(2R,4R)-4-[2-(3,5-dimethylanilino)-5,7-dihydropyrrolo[3,4-d]pyrimidin-6-yl]-2-methyl-piperidine-1-carbonyl]phenyl]methanesulfonamide | Int 39 + CAS# 28547-13-9 | A | 534.7 | 535.4 |
| 207 | 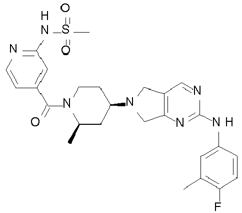 | N-[4-[(2R,4R)-4-[2-(4-fluoro-3-methyl-anilino)-5,7-dihydropyrrolo[3,4-d]pyrimidin-6-yl]-2-methyl-piperidine-1-carbonyl]-2-pyridyl]methanesulfonamide | Int 74 + Int 2085 | C | 539.6 | 540.4 |
| 208 | 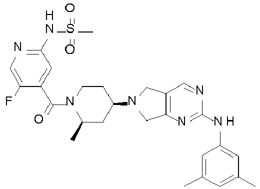 | N-[4-[(2R,4R)-4-[2-(3,5-dimethylanilino)-5,7-dihydropyrrolo[3,4-d]pyrimidin-6-yl]-2-methyl-piperidine-1-carbonyl]-5-fluoro-2-pyridyl]methanesulfonamide | Int 39 + Int 134 | A | 553.7 | 554.4 |
| 209 | 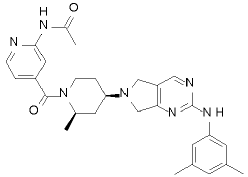 | N-[4-[(2R,4R)-4-[2-(3,5-dimethylanilino)-5,7-dihydropyrrolo[3,4-d]pyrimidin-6-yl]-2-methyl-piperidine-1-carbonyl]-2-pyridyl]acetamide | Int 39 + CAS# 54221-95-3 | A | 499.6 | 500.4 |
| 210 | 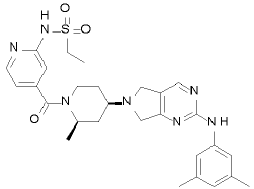 | N-[4-[(2R,4R)-4-[2-(3,5-dimethylanilino)-5,7-dihydropyrrolo[3,4-d]pyrimidin-6-yl]-2-methyl-piperidine-1-carbonyl]-2-pyridyl]ethanesulfonamide | Int 39 + Int 68 | A | 549.7 | 550.5 |
| 211 | 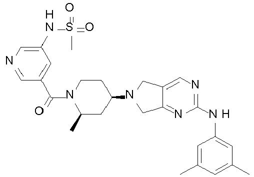 | N-[5-[(2R,4R)-4-[2-(3,5-dimethylanilino)-5,7-dihydropyrrolo[3,4-d]pyrimidin-6-yl]-2-methyl-piperidine-1-carbonyl]-3-pyridyl]methanesulfonamide | Int 39 + Int 66 | A | 535.7 | 536.4 |
| 212 | 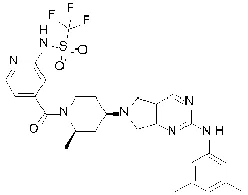 | N-[4-[(2R,4R)-4-[2-(3,5-dimethylanilino)-5,7-dihydropyrrolo[3,4-d]pyrimidin-6-yl]-2-methyl-piperidine-1-carbonyl]-2-pyridyl]-1,1,1-trifluoro-methanesulfonamide | Int 39 + Int 108 | A | 589.6 | 590.4 |
| 213 | 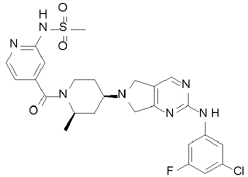 | N-[4-[(2R,4R)-4-[2-(3-chloro-5-fluoro-anilino)-5,7-dihydropyrrolo[3,4-d]pyrimidin-6-yl]-2-methyl-piperidine-1-carbonyl]-2-pyridyl]methanesulfonamide | Int 74 + Int 2068 | C | 560.0 | 560.3 |
| 214 | 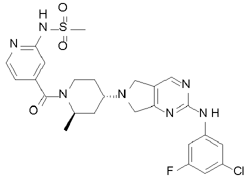 | N-[4-[(2R,4S)-4-[2-(3-chloro-5-fluoro-anilino)-5,7-dihydropyrrolo[3,4-d]pyrimidin-6-yl]-2-methyl-piperidine-1-carbonyl]-2-pyridyl]methanesulfonamide | Int 74 + Int 2068 | C | 560.0 | 560.3 |
| 215 | 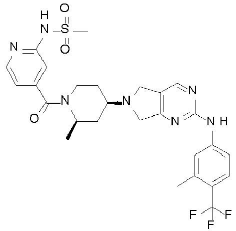 | N-[4-[(2R,4R)-2-methyl-4-[2-[3-methyl-4-(trifluoromethyl)anilino]-5,7-dihydropyrrolo[3,4-d]pyrimidin-6-yl]piperidine-1-carbonyl]-2-pyridyl]methanesulfonamide | Int 74 + Int 2069 | C | 589.6 | 590.4 |
| 216 | 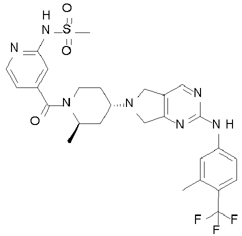 | N-[4-[(2R,4S)-2-methyl-4-[2-[3-methyl-4-(trifluoromethyl)anilino]-5,7-dihydropyrrolo[3,4-d]pyrimidin-6-yl]piperidine-1-carbonyl]-2-pyridyl]methanesulfonamide | Int 74 + Int 2069 | C | 589.6 | 590.4 |
| 217 | 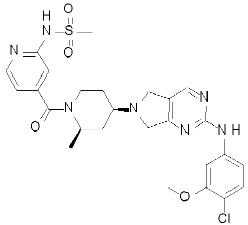 | N-[4-[(2R,4R)-4-[2-(4-chloro-3-methoxy-anilino)-5,7-dihydropyrrolo[3,4-d]pyrimidin-6-yl]-2-methyl-piperidine-1-carbonyl]-2-pyridyl]methanesulfonamide | Int 74 + Int 2070 | C | 572.1 | 572.3 |
| 218 | 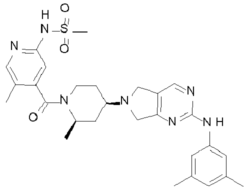 | N-[4-[(2R,4R)-4-[2-(3,5-dimethylanilino)-5,7-dihydropyrrolo[3,4-d]pyrimidin-6-yl]-2-methyl-piperidine-1-carbonyl]-5-methyl-2-pyridyl]methanesulfonamide | Int 39 + Int 61 | A | 549.7 | 500.5 |
| 219 | 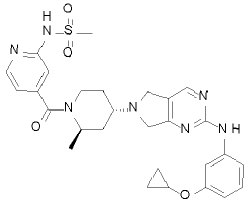 | N-[4-[(2R,4S)-4-[2-[3-(cyclopropoxy)anilino]-5,7-dihydropyrrolo[3,4-d]pyrimidin-6-yl]-2-methyl-piperidine-1-carbonyl]-2-pyridyl]methanesulfonamide | Int 74 + Int 2091 | C | 563.7 | 564.4 |
| 220 | 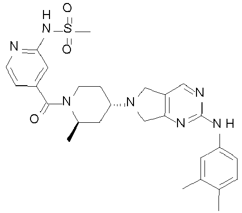 | N-[4-[(2R,4S)-4-[2-(3,4-dimethylanilino)-5,7-dihydropyrrolo[3,4-d]pyrimidin-6-yl]-2-methyl-piperidine-1-carbonyl]-2-pyridyl]methanesulfonamide | Int 74 + Int 2052 | C | 535.7 | 536.4 |
| 221 | 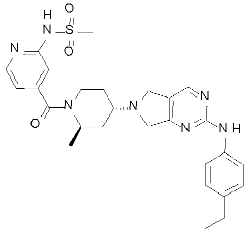 | N-[4-[(2R,4S)-4-[2-(4-ethylanilino)-5,7-dihydropyrrolo[3,4-d]pyrimidin-6-yl]-2-methyl-piperidine-1-carbonyl]-2-pyridyl]methanesulfonamide | Int 74 + Int 2053 | C | 535.7 | 536.5 |
| 222 | 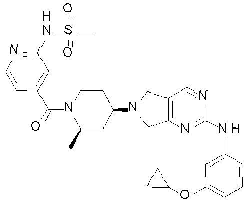 | N-[4-[(2R,4R)-4-[2-[3-(cyclopropoxy)anilino]-5,7-dihydropyrrolo[3,4-d]pyrimidin-6-yl]-2-methyl-piperidine-1-carbonyl]-2-pyridyl]methanesulfonamide | Int 74 + Int 2091 | C | 563.7 | 564.4 |
| 223 | 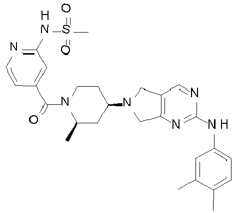 | N-[4-[(2R,4R)-4-[2-(3,4-dimethylanilino)-5,7-dihydropyrrolo[3,4-d]pyrimidin-6-yl]-2-methyl-piperidine-1-carbonyl]-2-pyridyl]methanesulfonamide | Int 74 + Int 2052 | C | 535.7 | 536.5 |
| 224 | 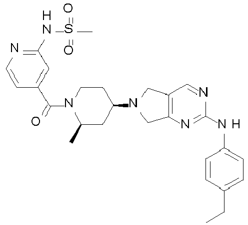 | N-[4-[(2R,4R)-4-[2-(4-ethylanilino)-5,7-dihydropyrrolo[3,4-d]pyrimidin-6-yl]-2-methyl-piperidine-1-carbonyl]-2-pyridyl]methanesulfonamide | Int 74 + Int 2053 | C | 535.7 | 536.5 |
| 225 | 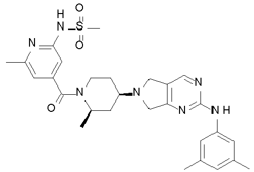 | N-[4-[(2R,4R)-4-[2-(3,5-dimethylanilino)-5,7-dihydropyrrolo[3,4-d]pyrimidin-6-yl]-2-methyl-piperidine-1-carbonyl]-6-methyl-2-pyridyl]methanesulfonamide | Int 39 + Int 64 | A | 549.7 | 550.5 |
| 226 | 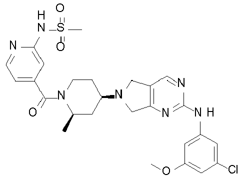 | N-[4-[(2R,4R)-4-[2-(3-chloro-5-methoxy-anilino)-5,7-dihydropyrrolo[3,4-d]pyrimidin-6-yl]-2-methyl-piperidine-1-carbonyl]-2-pyridyl]methanesulfonamide | Int 74 + Int 2086 | C | 572.1 | 572.3 + 574.4 |
| 227 | 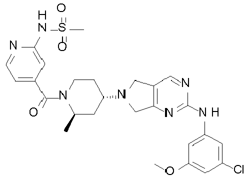 | N-[4-[(2R,4S)-4-[2-(3-chloro-5-methoxy-anilino)-5,7-dihydropyrrolo[3,4-d]pyrimidin-6-yl]-2-methyl-piperidine-1-carbonyl]-2-pyridyl]methanesulfonamide | Int 74 + Int 2086 | C | 572.1 | 572.4 + 574.4 |
| 228 | 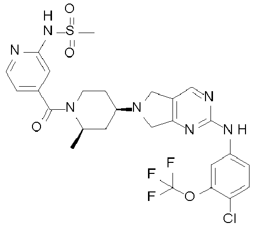 | N-[4-[(2R,4R)-4-[2-[4-chloro-3-(trifluoromethoxy)anilino]-5,7-dihydropyrrolo[3,4-d]pyrimidin-6-yl]-2-methyl-piperidine-1-carbonyl]-2-pyridyl]methanesulfonamide | Int 74 + Int 2087 | C | 626.1 | 626.4 + 628.3 |
| 229 | 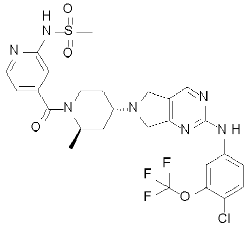 | N-[4-[(2R,4S)-4-[2-[4-chloro-3-(trifluoromethoxy)anilino]-5,7-dihydropyrrolo[3,4-d]pyrimidin-6-yl]-2-methyl-piperidine-1-carbonyl]-2-pyridyl]methanesulfonamide | Int 74 + Int 2087 | C | 626.1 | 626.4 + 628.3 |
| 230 | 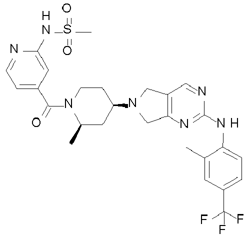 | N-[4-[(2R,4R)-2-methyl-4-[2-[2-methyl-4-(trifluoromethyl)anilino]-5,7-dihydropyrrolo[3,4-d]pyrimidin-6-yl]piperidine-1-carbonyl]-2-pyridyl]methanesulfonamide | Int 74 + Int 2088 | C | 589.6 | 590.4 |
| 231 | 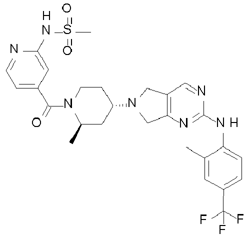 | N-[4-[(2R,4S)-2-methyl-4-[2-[2-methyl-4-(trifluoromethyl)anilino]-5,7-dihydropyrrolo[3,4-d]pyrimidin-6-yl]piperidine-1-carbonyl]-2-pyridyl]methanesulfonamide | Int 74 + Int 2088 | C | 589.6 | 590.4 |
| 232 | 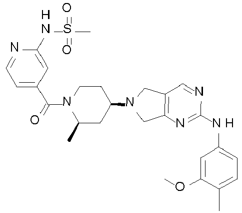 | N-[4-[(2R,4R)-4-[2-(3-methoxy-4-methyl-anilino)-5,7-dihydropyrrolo[3,4-d]pyrimidin-6-yl]-2-methyl-piperidine-1-carbonyl]-2-pyridyl]methanesulfonamide | Int 74 + Int 2089 | C | 551.7 | 552.4 |
| 233 | 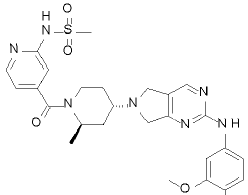 | N-[4-[(2R,4S)-4-[2-(3-methoxy-4-methyl-anilino)-5,7-dihydropyrrolo[3,4-d]pyrimidin-6-yl]-2-methyl-piperidine-1-carbonyl]-2-pyridyl]methanesulfonamide | Int 74 + Int 2089 | C | 551.7 | 552.4 |
| 234 | 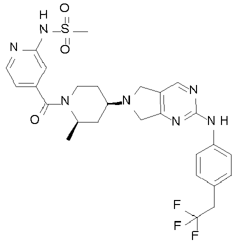 | N-[4-[(2R,4R)-2-methyl-4-[2-[4-(2,2,2-trifluoroethyl)anilino]-5,7-dihydropyrrolo[3,4-d]pyrimidin-6-yl]piperidine-1-carbonyl]-2-pyridyl]methanesulfonamide | Int 74 + Int 2090 | C | 589.6 | 590.5 |
| 235 | 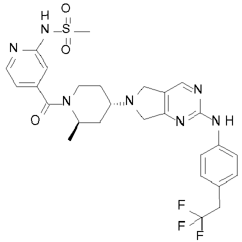 | N-[4-[(2R,4S)-2-methyl-4-[2-[4-(2,2,2-trifluoroethyl)anilino]-5,7-dihydropyrrolo[3,4-d]pyrimidin-6-yl]piperidine-1-carbonyl]-2-pyridyl]methanesulfonamide | Int 74 + Int 2090 | C | 589.6 | 590.3 |
| 236 | 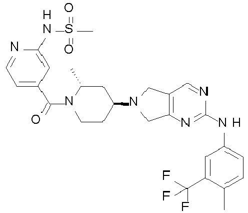 | N-[4-[(2R,4S)-2-methyl-4-[2-[4-methyl-3-(trifluoromethyl)anilino]-5,7-dihydropyrrolo[3,4-d]pyrimidin-6-yl]piperidine-1-carbonyl]pyridin-2-yl]methanesulfonamide | Int 74 + Int 2095 | C | 589.6 | 590.4 |
| 237 | 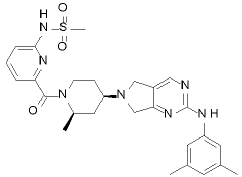 | N-[6-[(2R,4R)-4-[2-(3,5-dimethylanilino)-5,7-dihydropyrrolo[3,4-d]pyrimidin-6-yl]-2-methyl-piperidine-1-carbonyl]-2-pyridyl]methanesulfonamide | Int 39 + Int 67 | A | 535.7 | 536.4 |
| 238 | 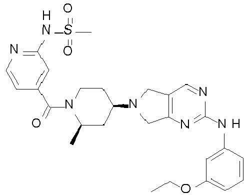 | N-[4-[(2R,4R)-4-[2-(3-ethoxyanilino)-5,7-dihydropyrrolo[3,4-d]pyrimidin-6-yl]-2-methyl-piperidine-1-carbonyl]-2-pyridyl]methanesulfonamide | Int 74 + Int 2057 | C | 551.7 | 552.4 |
| 239 | 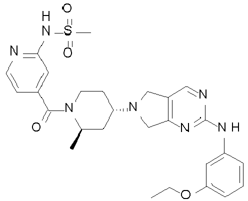 | N-[4-[(2R,4S)-4-[2-(3-ethoxyanilino)-5,7-dihydropyrrolo[3,4-d]pyrimidin-6-yl]-2-methyl-piperidine-1-carbonyl]-2-pyridyl]methanesulfonamide | Int 74 + Int 2057 | C | 551.7 | 552.4 |
| 240 | 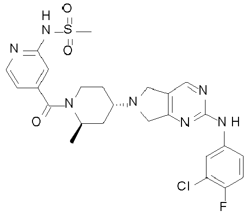 | N-[4-[(2R,4S)-4-[2-(3-chloro-4-fluoro-anilino)-5,7-dihydropyrrolo[3,4-d]pyrimidin-6-yl]-2-methyl-piperidine-1-carbonyl]-2-pyridyl]methanesulfonamide | Int 74 + Int 2058 | C | 560.0 | 560.3 |
| 241 | 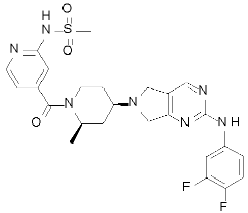 | N-[4-[(2R,4R)-4-[2-(3,4-difluoroanilino)-5,7-dihydropyrrolo[3,4-d]pyrimidin-6-yl]-2-methyl-piperidine-1-carbonyl]-2-pyridyl]methanesulfonamide | Int 74 + Int 2079 | C | 543.6 | 544.3 |
| 242 | 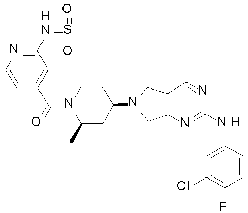 | N-[4-[(2R,4R)-4-[2-(3-chloro-4-fluoro-anilino)-5,7-dihydropyrrolo[3,4-d]pyrimidin-6-yl]-2-methyl-piperidine-1-carbonyl]-2-pyridyl]methanesulfonamide | Int 74 + Int 2058 | C | 560.0 | 560.3 |
| 243 | 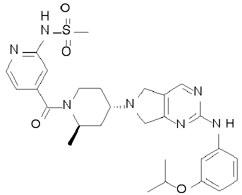 | N-[4-[(2R,4S)-4-[2-(3-isopropoxyanilino)-5,7-dihydropyrrolo[3,4-d]pyrimidin-6-yl]-2-methyl-piperidine-1-carbonyl]-2-pyridyl]methanesulfonamide | Int 74 + Int 2059 | C | 565.7 | 566.4 |
| 244 | 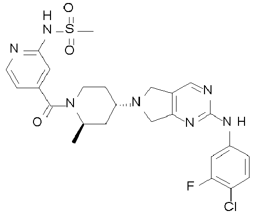 | N-[4-[(2R,4S)-4-[2-(4-chloro-3-fluoro-anilino)-5,7-dihydropyrrolo[3,4-d]pyrimidin-6-yl]-2-methyl-piperidine-1-carbonyl]-2-pyridyl]methanesulfonamide | Int 74 + Int 2060 | C | 560.0 | 560.3 |
| 245 | 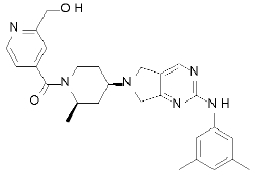 | [(2R,4R)-4-[2-(3,5-dimethylanilino)-5,7-dihydropyrrolo[3,4-d]pyrimidin-6-yl]-2-methyl-1-piperidyl]-[2-(hydroxymethyl)-4-pyridyl]methanone | Cpd 283 | example | 472.6 | 473.6 |
| 246 | 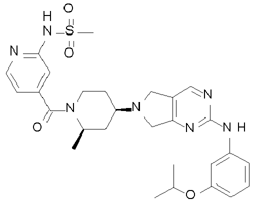 | N-[4-[(2R,4R)-4-[2-(3-isopropoxyanilino)-5,7-dihydropyrrolo[3,4-d]pyrimidin-6-yl]-2-methyl-piperidine-1-carbonyl]-2-pyridyl]methanesulfonamide | Int 74 + Int 2059 | C | 565.7 | 566.4 |
| 247 | 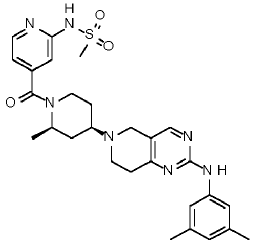 | N-[4-[(2R,4R)-4-[2-(3,5-dimethylanilino)-7,8-dihydro-5H-pyrido[4,3-d]pyrimidin-6-yl]-2-methyl-piperidine-1-carbonyl]-2-pyridyl]methanesulfonamide | Int 74 + Int 2011 | C | 549.7 | 550.5 |
| 248 | 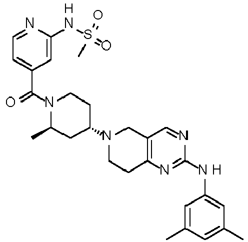 | N-[4-[(2R,4S)-4-[2-(3,5-dimethylanilino)-7,8-dihydro-5H-pyrido[4,3-d]pyrimidin-6-yl]-2-methyl-piperidine-1-carbonyl]-2-pyridyl]methanesulfonamide | Int 74 + Int 2011 | C | 549.7 | 550.4 |
| 249 | 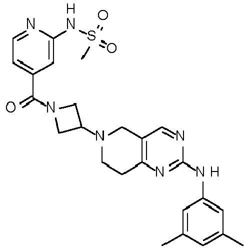 | N-[4-[3-[2-(3,5-dimethylanilino)-7,8-dihydro-5H-pyrido[4,3-d]pyrimidin-6-yl]azetidine-1-carbonyl]-2-pyridyl]methanesulfonamide | Int 1 + Int 58 | A | 507.6 | 508.4 |
| 250 | 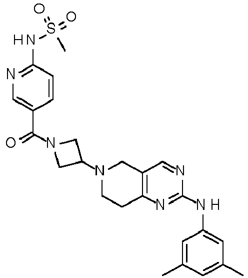 | N-[5-[3-[2-(3,5-dimethylanilino)-7,8-dihydro-5H-pyrido[4,3-d]pyrimidin-6-yl]azetidine-1-carbonyl]-2-pyridyl]methanesulfonamide | Int 1 + CAS# 960324-86-1 | A | 507.6 | 508.4 |
| 251 | 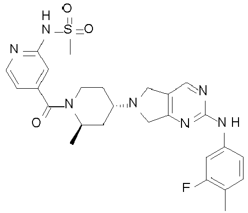 | N-[4-[(2R,4S)-4-[2-(3-fluoro-4-methyl-anilino)-5,7-dihydropyrrolo[3,4-d]pyrimidin-6-yl]-2-methyl-piperidine-1-carbonyl]-2-pyridyl]methanesulfonamide | Int 74 + Int 2080 | C | 539.6 | 540.4 |
| 252 | 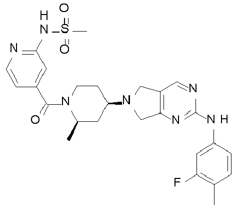 | N-[4-[(2R,4R)-4-[2-(3-fluoro-4-methyl-anilino)-5,7-dihydropyrrolo[3,4-d]pyrimidin-6-yl]-2-methyl-piperidine-1-carbonyl]-2-pyridyl]methanesulfonamide | Int 74 + Int 2080 | C | 539.6 | 540.4 |
| 253 | 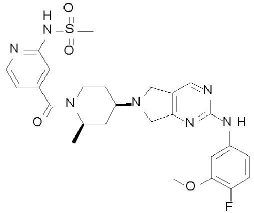 | N-[4-[(2R,4R)-4-[2-(4-fluoro-3-methoxy-anilino)-5,7-dihydropyrrolo[3,4-d]pyrimidin-6-yl]-2-methyl-piperidine-1-carbonyl]-2-pyridyl]methanesulfonamide | Int 74 + Int 2081 | C | 555.6 | 556.4 |
| 254 | 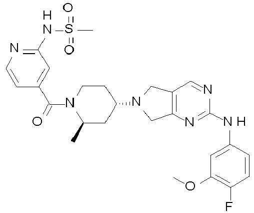 | N-[4-[(2R,4S)-4-[2-(4-fluoro-3-methoxy-anilino)-5,7-dihydropyrrolo[3,4-d]pyrimidin-6-yl]-2-methyl-piperidine-1-carbonyl]-2-pyridyl]methanesulfonamide | Int 74 + Int 2081 | C | 555.6 | 556.4 |
| 255 | 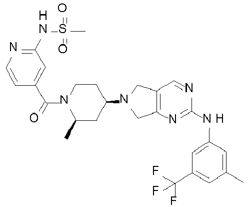 | N-[4-[(2R,4R)-2-methyl-4-[2-[3-methyl-5-(trifluoromethyl)anilino]-5,7-dihydropyrrolo[3,4-d]pyrimidin-6-yl]piperidine-1-carbonyl]-2-pyridyl]methanesulfonamide | Int 74 + Int 2071 | C | 589.6 | 590.5 |
| 256 | 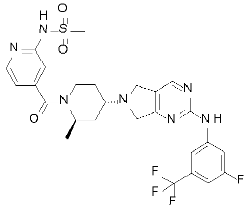 | N-[4-[(2R,4S)-4-[2-[3-fluoro-5-(trifluoromethyl)anilino]-5,7-dihydropyrrolo[3,4-d]pyrimidin-6-yl]-2-methyl-piperidine-1-carbonyl]-2-pyridyl]methanesulfonamide | Int 74 + Int 2082 | C | 593.6 | 594.4 |
| 257 | 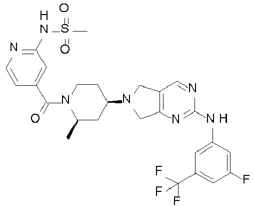 | N-[4-[(2R,4R)-4-[2-[3-fluoro-5-(trifluoromethyl)anilino]-5,7-dihydropyrrolo[3,4-d]pyrimidin-6-yl]-2-methyl-piperidine-1-carbonyl]-2-pyridyl]methanesulfonamide | Int 74 + Int 2082 | C | 593.6 | 594.5 |
| 258 | 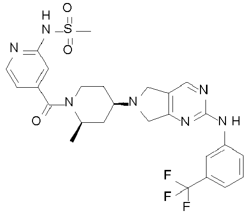 | N-[4-[(2R,4R)-2-methyl-4-[2-[3-(trifluoromethyl)anilino]-5,7-dihydropyrrolo[3,4-d]pyrimidin-6-yl]piperidine-1-carbonyl]-2-pyridyl]methanesulfonamide | Int 74 + Int 2061 | C | 575.6 | 576.5 |
| 259 | 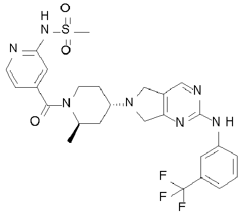 | N-[4-[(2R,4S)-2-methyl-4-[2-[3-(trifluoromethyl)anilino]-5,7-dihydropyrrolo[3,4-d]pyrimidin-6-yl]piperidine-1-carbonyl]-2-pyridyl]methanesulfonamide | Int 74 + Int 2061 | C | 575.6 | 576.5 |
| 260 | 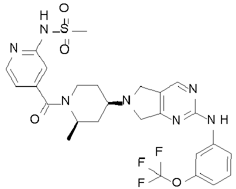 | N-[4-[(2R,4R)-2-methyl-4-[2-[3-(trifluoromethoxy)anilino]-5,7-dihydropyrrolo[3,4-d]pyrimidin-6-yl]piperidine-1-carbonyl]-2-pyridyl]methanesulfonamide | Int 74 + Int 2062 | C | 591.6 | 592.5 |
| 261 | 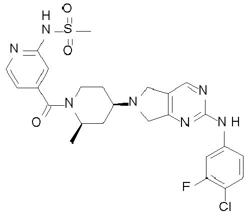 | N-[4-[(2R,4R)-4-[2-(4-chloro-3-fluoro-anilino)-5,7-dihydropyrrolo[3,4-d]pyrimidin-6-yl]-2-methyl-piperidine-1-carbonyl]-2-pyridyl]methanesulfonamide | Int 74 + Int 2060 | C | 560.0 | 560.3 |
| 262 | 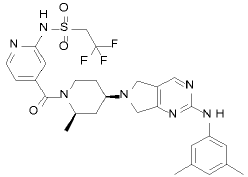 | N-[4-[(2R,4R)-4-[2-(3,5-dimethylanilino)-5,7-dihydropyrrolo[3,4-d]pyrimidin-6-yl]-2-methyl-piperidine-1-carbonyl]-2-pyridyl]-2,2,2-trifluoroethanesulfonamide | Int 39 + Int 69 | A | 603.7 | 604.4 |
| 263 | 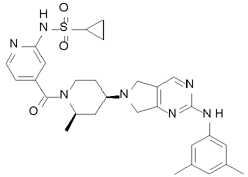 | N-[4-[(2R,4R)-4-[2-(3,5-dimethylanilino)-5,7-dihydropyrrolo[3,4-d]pyrimidin-6-yl]-2-methyl-piperidine-1-carbonyl]-2-pyridyl]cyclopropanesulfonamide | Int 39 + Int 70 | A | 561.7 | 562.5 |
| 264 | 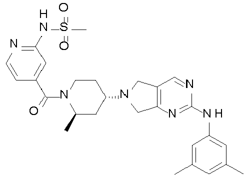 | N-[4-[(2R,4S)-4-[2-(3,5-dimethylanilino)-5,7-dihydropyrrolo[3,4-d]pyrimidin-6-yl]-2-methyl-piperidine-1-carbonyl]-2-pyridyl]methanesulfonamide | Int 74 + Int 2011 | C | 535.7 | 536.5 |
| 265 | 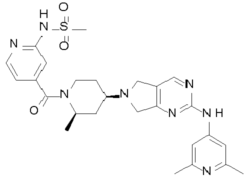 | N-[4-[(2R,4R)-4-[2-[(2,6-dimethyl-4-pyridyl)amino]-5,7-dihydropyrrolo[3,4-d]pyrimidin-6-yl]-2-methyl-piperidine-1-carbonyl]-2-pyridyl]methanesulfonamide | Int 74 + Int 2092 | C | 536.6 | 537.5 |
| 266 | 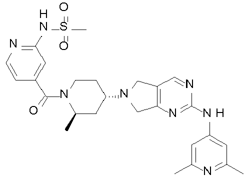 | N-[4-[(2R,4S)-4-[2-[(2,6-dimethyl-4-pyridyl)amino]-5,7-dihydropyrrolo[3,4-d]pyrimidin-6-yl]-2-methyl-piperidine-1-carbonyl]-2-pyridyl]methanesulfonamide | Int 74 + Int 2092 | C | 536.6 | 537.5 |
| 267 | 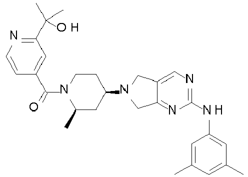 | [(2R,4R)-4-[2-(3,5-dimethylanilino) -5,7-dihydropyrrolo[3,4-d]pyrimidin-6-yl]-2-methyl-1-piperidyl]-[2-(1-hydroxy-1-methyl-ethyl)-4-pyridyl]methanone | Cpd 283 | example | 500.6 | 501.5 |
| 268 | 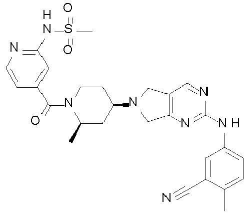 | N-[4-[(2R,4R)-4-[2-(3-cyano-4-methyl-anilino)-5,7-dihydropyrrolo[3,4-d]pyrimidin-6-yl]-2-methyl-piperidine-1-carbonyl]-2-pyridyl]methanesulfonamide | Int 74 + Int 2055 | C | 546.6 | 547.4 |
| 269 | 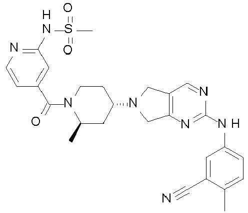 | N-[4-[(2R,4S)-4-[2-(3-cyano-4-methyl-anilino)-5,7-dihydropyrrolo[3,4-d]pyrimidin-6-yl]-2-methyl-piperidine-1-carbonyl]-2-pyridyl]methanesulfonamide | Int 74 + Int 2055 | C | 546.6 | 547.4 |
| 270 | 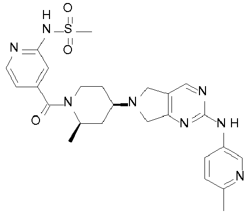 | N-[4-[(2R,4R)-2-methyl-4-[2-[(6-methyl-3-pyridyl)amino]-5,7-dihydropyrrolo[3,4-d]pyrimidin-6-yl]piperidine-1-carbonyl]-2-pyridyl]methanesulfonamide | Int 74 + Int 2056 | C | 522.6 | 523.4 |
| 271 | 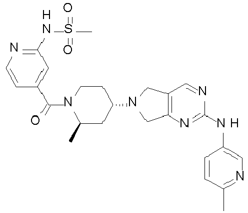 | N-[4-[(2R,4S)-2-methyl-4-[2-[(6-methyl-3-pyridyl)amino]-5,7-dihydropyrrolo[3,4-d]pyrimidin-6-yl]piperidine-1-carbonyl]-2-pyridyl]methanesulfonamide | Int 74 + Int 2056 | C | 522.6 | 523.4 |
| 272 | 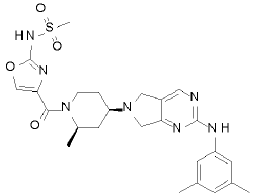 | N-[4-[(2R,4R)-4-[2-(3,5-dimethylanilino)-5,7-dihydropyrrolo[3,4-d]pyrimidin-6-yl]-2-methyl-piperidine-1-carbonyl]oxazol-2-yl]methanesulfonamide | Int 39 + Int 71 | A | 525.6 | 526.4 |
| 273 | 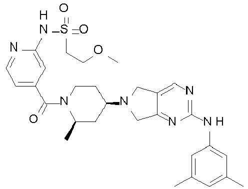 | N-[4-[(2R,4R)-4-[2-(3,5-dimethylanilino)-5,7-dihydropyrrolo[3,4-d]pyrimidin-6-yl]-2-methyl-piperidine-1-carbonyl]-2-pyridyl]-2-methoxyethane sulfonamide | Int 39 + Int 78 | A | 579.7 | 580.5 |
| 274 | 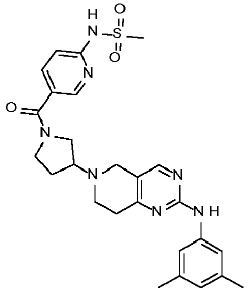 | N-[5-[3-[2-(3,5-dimethylanilino)-7,8-dihydro-5H-pyrido[4,3-d]pyrimidin-6-yl]pyrrolidine-1-carbonyl]-2-pyridyl]methanesulfonamide | Int 2 + CAS# 960324-86-1 | A | 521.6 | 522.5 |
| 275 | 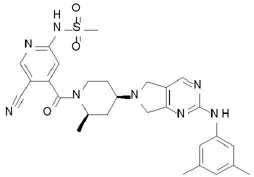 | N-[5-cyano-4-[(2R,4R)-4-[2-(3,5-dimethylanilino)-5,7-dihydropyrrolo[3,4-d]pyrimidin-6-yl]-2-methyl-piperidine-1-carbonyl]-2-pyridyl]methanesulfonamide | Int 39 + Int 65 | example | 560.7 | 561.5 |
| 276 | 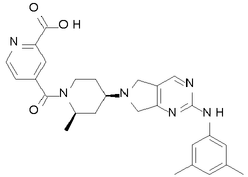 | 4-[(2R,4R)-4-[2-(3,5-dimethylanilino)-5,7-dihydropyrrolo[3,4-d]pyrimidin-6-yl]-2-methyl-piperidine-1-carbonyl]pyridine-2-carboxylic acid | Cpd 283 | G | 486.6 | 487.5 |
| 277 | 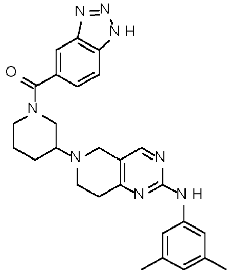 | 1H-benzotriazol-5-yl-[3-[2-(3,5-dimethylanilino)-7,8-dihydro-5H-pyrido[4,3-d]pyrimidin-6-yl]-1-piperidyl]methanone | Int 75 + CAS# 23814-12-2 | A | 482.6 | 483.5 |
| 278 | 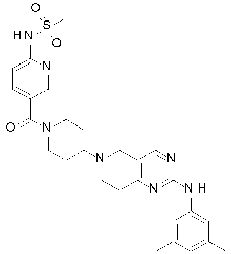 | N-[5-[4-[2-(3,5-dimethylanilino)-7,8-dihydro-5H-pyrido[4,3-d]pyrimidin-6-yl]piperidine-1-carbonyl]-2-pyridyl]methanesulfonamide | Int 25 + CAS# 960324-86-1 | A | 535.7 | 536.5 |
| 279 | 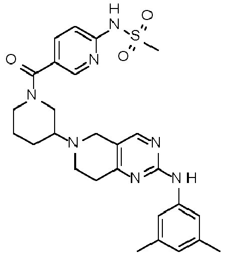 | N-[5-[3-[2-(3,5-dimethylanilino)-7,8-dihydro-5H-pyrido[4,3-d]pyrimidin-6-yl]piperidine-1-carbonyl]-2-pyridyl]methanesulfonamide | Int 75 + CAS# 960324-86-1 | A | 535.7 | 536.5 |
| 280 | 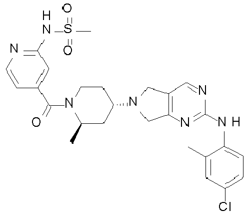 | N-[4-[(2R,4S)-4-[2-(4-chloro-2-methyl-anilino)-5,7-dihydropyrrolo[3,4-d]pyrimidin-6-yl]-2-methyl-piperidine-1-carbonyl]-2-pyridyl]methanesulfonamide | Int 74 + Int 2063 | C | 556.1 | 556.5 |
| 281 | 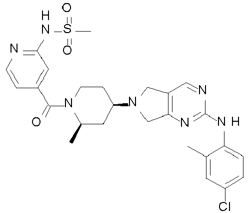 | N-[4-[(2R,4R)-4-[2-(4-chloro-2-methyl-anilino)-5,7-dihydropyrrolo[3,4-d]pyrimidin-6-yl]-2-methyl-piperidine-1-carbonyl]-2-pyridyl]methanesulfonamide | Int 74 + Int 2063 | C | 556.1 | 556.5 |
| 282 | 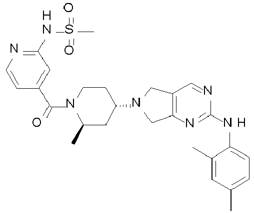 | N-[4-[(2R,4S)-4-[2-(2,4-dimethylanilino)-5,7-dihydropyrrolo[3,4-d]pyrimidin-6-yl]-2-methyl-piperidine-1-carbonyl]-2-pyridyl]methanesulfonamide | Int 74 + Int 2064 | C | 535.7 | 536.5 |
| 283 | 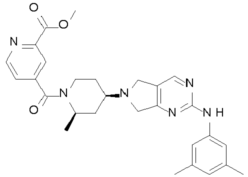 | methyl 4-[(2R,4R)-4-[2-(3,5-dimethylanilino)-5,7-dihydropyrrolo[3,4-d]pyrimidin-6-yl]-2-methyl-piperidine-1-carbonyl]pyridine-2-carboxylate | Int 39 + Int 72 | example | 500.6 | 501.5 |
| 284 | 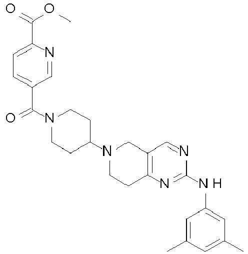 | methyl 5-[4-[2-(3,5-dimethylanilino)-7,8-dihydro-5H-pyrido[4,3-d]pyrimidin-6-yl]piperidine-1-carbonyl]pyridine-2-carboxylate | Int 25 + CAS# 17874-76-9 | A | 500.6 | 501.5 |
| 285 | 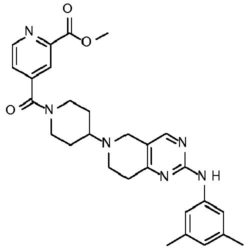 | methyl 4-[4-[2-(3,5-dimethylanilino)-7,8-dihydro-5H-pyrido[4,3-d]pyrimidin-6-yl]piperidine-1-carbonyl]pyridine-2-carboxylate | Int 25 + CAS# 24195-10-6 | A | 500.6 | 501.5 |
| 286 | 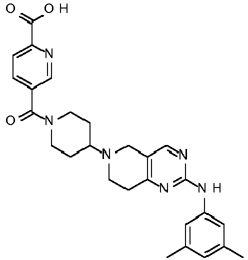 | 5-[4-[2-(3,5-dimethylanilino)-7,8-dihydro-5H-pyrido[4,3-d]pyrimidin-6-yl]piperidine-1-carbonyl]pyridine-2-carboxylic acid | Cpd 274 | G | 486.6 | 487.5 |
| 287 | 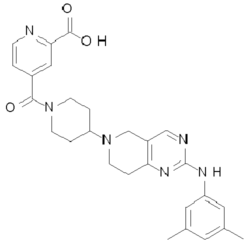 | 4-[4-[2-(3,5-dimethylanilino)-7,8-dihydro-5H-pyrido[4,3-d]pyrimidin-6-yl]piperidine-1-carbonyl]pyridine-2-carboxylic acid | Cpd 285 | G | 486.6 | 487.5 |
| 288 | 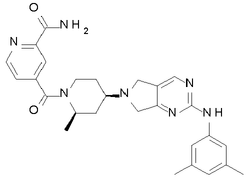 | 4-[(2R,4R)-4-[2-(3,5-dimethylanilino)-5,7-dihydropyrrolo[3,4-d]pyrimidin-6-yl]-2-methyl-piperidine-1-carbonyl]pyridine-2-carboxamide | Cpd 286 | example | 485.6 | 486.5 |
| 289 | 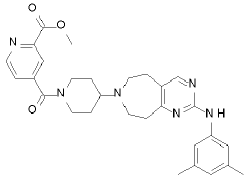 | methyl 4-[4-[2-(3,5-dimethylanilino)-5,6,8,9-tetrahydropyrimido[4,5-d]azepin-7-yl]piperidine-1-carbonyl]pyridine-2-carboxylate | Int 53 + CAS# 24195-10-6 | A | 514.6 | 515.3 |
| 290 | 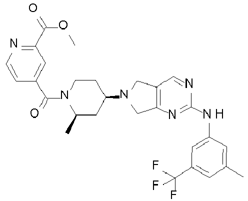 | methyl 4-[(2R,4R)-2-methyl-4-[2-[3-methyl-5-(trifluoromethyl)anilino]-5,7-dihydropyrrolo[3,4-d]pyrimidin-6-yl]piperidine-1-carbonyl]pyridine-2-carboxylate | Int 120 + Int 72 | example | 554.6 | 555.6 |
| 291 | 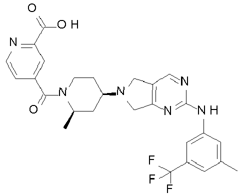 | 4-[(2R,4R)-2-methyl-4-[2-[3-methyl-5-(trifluoromethyl)anilino]-5,7-dihydropyrrolo[3,4-d]pyrimidin-6-yl]piperidine-1-carbonyl]pyridine-2-carboxylic acid | Cpd 290 | example | 540.5 | 541.3 |
| 292 | 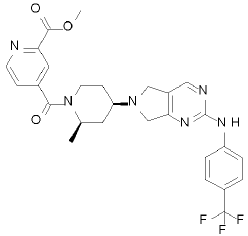 | methyl 4-[(2R,4R)-2-methyl-4-[2-[4-(trifluoromethyl)anilino]-5,7-dihydropyrrolo[3,4-d]pyrimidin-6-yl]piperidine-1-carbonyl]pyridine-2-carboxylate | Int 122 + Int 72 | example | 540.5 | 541.7 |
| 293 | 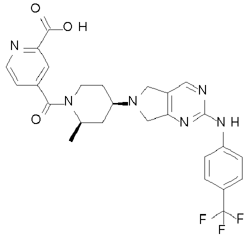 | 4-[(2R,4R)-2-methyl-4-[2-[4-(trifluoromethyl)anilino]-5,7-dihydropyrrolo[3,4-d]pyrimidin-6-yl]piperidine-1-carbonyl]pyridine-2-carboxylic acid | Cpd 292 | example | 526.5 | 527.5 |
| 294 | 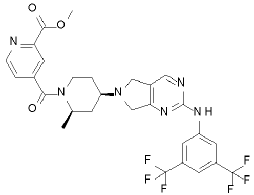 | methyl 4-[(2R,4R)-4-[2-[3,5-bis(trifluoromethyl)anilino]-5,7-dihydropyrrolo[3,4-d]pyrimidin-6-yl]-2-methyl-piperidine-1-carbonyl]pyridine-2-carboxylate | Int 124 + Int 72 | example | 608.5 | 609.6 |
| 295 | 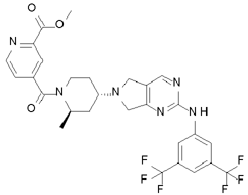 | methyl 4-[(2R,4S)-4-[2-[3,5-bis(trifluoromethyl)anilino]-5,7-dihydropyrrolo[3,4-d]pyrimidin-6-yl]-2-methyl-piperidine-1-carbonyl]pyridine-2-carboxylate | Int 126 + Int 72 | example | 608.5 | 609.6 |
| 296 | 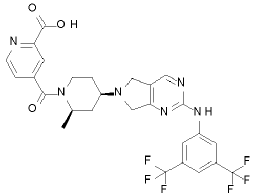 | 4-[(2R,4R)-4-[2-[3,5-bis(trifluoromethyl)anilino]-5,7-dihydropyrrolo[3,4-d]pyrimidin-6-yl]-2-methyl-piperidine-1-carbonyl]pyridine-2-carboxylic acid | Cpd 294 | example | 594.5 | 595.6 |
| 297 | 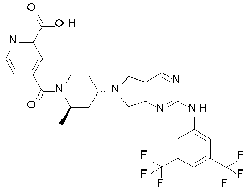 | 4-[(2R,4S)-4-[2-[3,5-bis(trifluoromethyl)anilino]-5,7-dihydropyrrolo[3,4-d]pyrimidin-6-yl]-2-methyl-piperidine-1-carbonyl]pyridine-2-carboxylic acid | Cpd 295 | example | 594.5 | 595.6 |
| 298 | 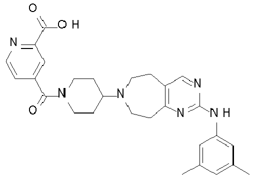 | 4-[4-[2-(3,5-dimethylanilino)-5,6,8,9-tetrahydropyrimido[4,5-d]azepin-7-yl]piperidine-1-carbonyl]pyridine-2-carboxylic acid | Cpd 289 | example | 500.6 | 501.6 |
| 299 | 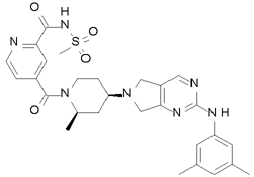 | 4-[(2R,4R)-4-[2-(3,5-dimethylanilino)-5,7-dihydropyrrolo[3,4-d]pyrimidin-6-yl]-2-methyl-piperidine-1-carbonyl]-N-methylsulfonyl-pyridine-2-carboxamide | Cpd 276 | A | 563.7 | 564.5 |
| 300 | 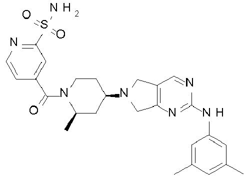 | 4-[(2R,4R)-4-[2-(3,5-dimethylanilino)-5,7-dihydropyrrolo[3,4-d]pyrimidin-6-yl]-2-methyl-piperidine-1-carbonyl]pyridine-2-sulfonamide | Int 39 + CAS# 1308677-82-8 | A | 521.6 | 522.6 |
| 301 | 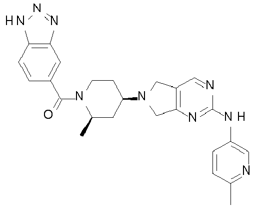 | 1H-benzotriazol-5-yl-[(2R,4R)-2-methyl-4-[2-[(6-methyl-3-pyridyl)amino]-5,7-dihydropyrrolo[3,4-d]pyrimidin-6-yl]-1-piperidyl]methanone | Int 74 + Int 2056 | C | 469.5 | 470.3 |
| 302 | 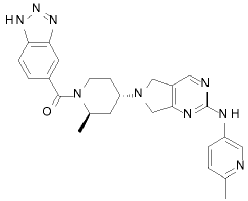 | 1H-benzotriazol-5-yl-[(2R,4S)-2-methyl-4-[2-[(6-methyl-3-pyridyl)amino]-5,7-dihydropyrrolo[3,4-d]pyrimidin-6-yl]-1-piperidyl]methanone | Int 74 + Int 2056 | C | 469.5 | 470.4 |

| **Cpc #** | **Structure** | **Name** | **SM** | **Mtd** | **MW** | **MS Mes'd** |
|---|---|---|---|---|---|---|
| 303 | 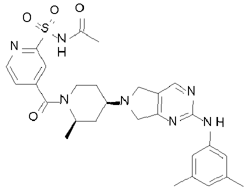 | N-[[4-[(2R,4R)-4-[2-(3,5-dimethylanilino)-5,7-dihydropyrrolo[3,4-d]pyrimidin-6-yl]-2-methyl-piperidine-1-carbonyl]-2-pyridyl]sulfonyl]acetamide | Cpd 300 | example | 563.7 | 564.5 |
| 304 | 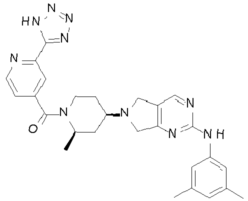 | [(2R,4R)-4-[2-(3,5-dimethylanilino)-5,7-dihydropyrrolo[3,4-d]pyrimidin-6-yl]-2-methyl-1-piperidyl]-[2-(1H-tetrazol-5-yl)-4-pyridyl]methanone | Int 76 + Int 2011 | C | 510.6 | 511.6 |
| 305 | 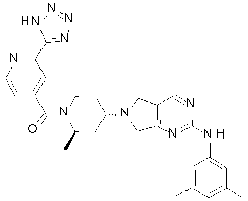 | [(2R,4S)-4-[2-(3,5-dimethylanilino)-5,7-dihydropyrrolo[3,4-d]pyrimidin-6-yl]-2-methyl-1-piperidyl]-[2-(1H-tetrazol-5-yl)-4-pyridyl]methanone | Int 76 + Int 2011 | C | 510.6 | 511.6 |
| 306 | 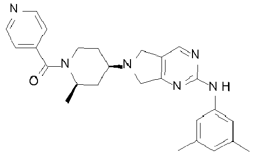 | [(2R,4R)-4-[2-(3,5-dimethylanilino)-5,7-dihydropyrrolo[3,4-d]pyrimidin-6-yl]-2-methyl-1-piperidyl]-(4-pyridyl)methanone | Int 39 + CAS# 55-22-1 | A | 442.6 | 443.5 |

| **Cpd #** | **Structure** | **Name** | **SM** | **Mtd** | **MW** | **MS Mes'd** |
|---|---|---|---|---|---|---|
| 308 | 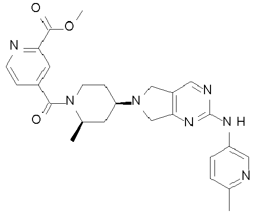 | methyl 4-[(2R,4R)-2-methyl-4-[2-[(6-methyl-3-pyridyl)amino]-5,7-dihydropyrrolo[3,4-d]pyrimidin-6-yl]piperidine-1-carbonyl]pyridine-2-carboxylate | Int 129 + Int 72 | example | 487.6 | 488.5 |
| 309 | 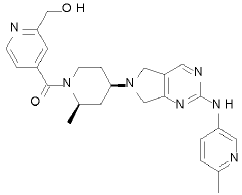 | [2-(hydroxymethyl)-4-pyridyl]-[(2R,4R)-2-methyl-4-[2-[(6-methyl-3-pyridyl)amino]-5,7-dihydropyrrolo[3,4-d]pyrimidin-6-yl]-1-piperidyl]methanone | Cpd 308 | example | 459.5 | 460.5 |
| 310 | 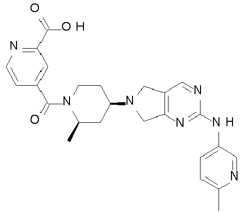 | 4-[(2R,4R)-2-methyl-4-[2-[(6-methyl-3-pyridyl)amino]-5,7-dihydropyrrolo[3,4-d]pyrimidin-6-yl]piperidine-1-carbonyl]pyridine-2-carboxylic acid | Cpd 308 | G | 473.5 | 474.5 |
| 311 | 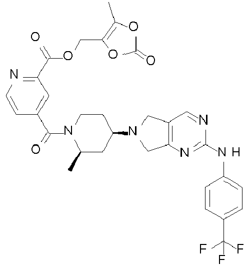 | (5-methyl-2-oxo-1,3-dioxol-4-yl)methyl 4-[(2R,4R)-2-methyl-4-[2-[4-(trifluoromethyl)anilino]-5,7-dihydropyrrolo[3,4-d]pyrimidin-6-yl]piperidine-1-carbonyl]pyridine-2-carboxylate | Cpd 293 | example | 638.6 | 639.6 |
| 313 | 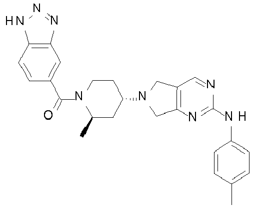 | 1H-benzotriazol-5-yl-[(2R,4S)-2-methyl-4-[2-(4-methylanilino)-5,7-dihydropyrrolo[3,4-d]pyrimidin-6-yl]-1-piperidyl]methanone | Int 131 + Int 2067 | C | 468.6 | 469.4 |
| 314 | 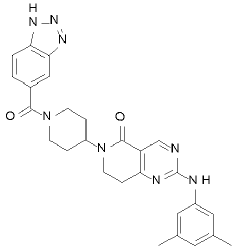 | 6-[1-(1H-benzotriazole-5-carbonyl)-4-piperidyl]-2-(3,5-dimethylanilino)-7,8-dihydropyrido[4,3-d]pyrimidin-5-one | CAS# 79099-07-3 | example | 496.6 | 497.6 |
| 315 | 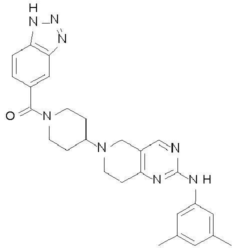 | 1H-benzotriazol-5-yl-[4-[2-(3,5-dimethylanilino)-7,8-dihydro-5H-pyrido[4,3-d]pyrimidin-6-yl]-1-piperidyl]methanone | Int 25 + CAS# 23814-12-2 | A | 482.6 | 483.2 |
| 316 | 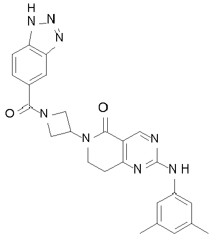 | 6-[1-(1H-benzotriazole-5-carbonyl)azetidin-3-yl]-2-(3,5-dimethylanilino)-7,8-dihydropyrido[4,3-d]pyrimidin-5-one | CAS# 398489-26-4 | example | 468.5 | 469.5 |
| 317 | 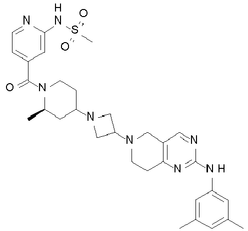 | N-[4-[(2R)-4-[3-[2-(3,5-dimethylanilino)-7,8-dihydro-5H-pyrido[4,3-d]pyrimidin-6-yl]azetidin-1-yl]-2-methyl-piperidine-1-carbonyl]-2-pyridyl]methanesulfonamide | Int 1 + Int 74 | C | 604.8 | 605.5 |

| | | | | | | |
|---|---|---|---|---|---|---|
| SM = Starting Material, Mtd = Method, MS Mes'd = Mesured mass | | | | | | |

**Table IV. NMR data of illustrative compounds of the invention**

| **Cpd#** | **NMR data** |
|---|---|
| 1 | ¹H NMR (500 MHz, DMSO-d₆) δ 11.92 (bs, 1H), 9.30 (s, 1H), 8.20 (s, 1H), 7.57 (d, 1H), 7.52 (dd, 1H), 7.38 (s, 2H), 7.15 (d, 1H), 6.56 (s, 1H), 4.51-3.92 (m, 4H), 3.46 (s, 2H), 3.44-3.37 (m, 1H), 2.83-2.63 (m, 4H), 2.23 (s, 6H) |
| 3 | ¹H NMR (300 MHz, DMSO-d₆) δ 11.93 (bs, 1H), 9.43(s, 1H), 8.32 (s, 1H), 7.57 (s, 1H), 7.52 (d, 1H), 7.38 (s, 2H), 7.13 (d, 1H), 6.56 (s, 1H), 4.49-4.31 (m, 2H), 4.26-3.99 (m, 2H), 3.91-3.22 (m, 5H), 2.24 (s, 6H) |
| 10 | ¹H NMR (300 MHz, DMSO-d₆) δ 15.91 (bs, 1H), 9.41(s, 1H), 8.34 (s, 1H), 8.02-7.94 (m, 2H), 7.48 (d, 1H), 7.38 (s, 2H), 6.58 (s, 1H), 4.41-4.31 (m, 1H), 3.91-3.82 (m, 4H), 3.73-3.49 (m, 1H), 3.25-3.12 (m, 2H), 2.81-2.70 (m, 1H), 2.24 (s, 6H), 2.09-1.77 (m, 2H), 1.63-1.43 (m, 2H) |
| 34 | ¹H NMR (400 MHz, DMSO-d₆) δ 9.39 (s, 1H), 8.31 (s, 1H), 8.03-7.82 (m, 2H), 7.52-7.38 (m, 1H), 7.38-7.20 (m, 2H), 6.87-6.35 (m, 2H), 4.43-4.12 (m, 2H), 3.95-3.66 (m, 5H), 2.95-2.74 (m, 1H), 2.21 (s, 6H), 1.99-1.63 (m, 4H), 1.41 (d, 3H) |
| 73 | ¹H NMR (500 MHz, DMSO-d₆) δ 15.89 (bs, 1H), 9.40 (s, 1H), 8.32 (s, 1H), 7.98-7.95 (m, 1H), 7.93 (s, 1H), 7.45-7.43 (m, 1H), 7.36 (s, 2H), 6.57 (s, 1H), 4.26 (bs, 1H), 3.91-3.76 (m, 4H), 3.72 (bs, 1H), 3.39-3.25 (m, 1H), 2.89-2.82 (m, 1H), 2.22 (s, 6H), 1.94-1.81 (m, 3H), 1.78-1.75 (m, 1H), 1.42 (d, 3H) |
| 78 | ¹H NMR (300 MHz, DMSO-d₆) δ 15.97 (bs, 1H), 9.42 (s, 1H), 8.32 (s, 1H), 8.11-7.85 (m, 2H), 7.43 (d, 1H), 7.36 (s, 2H), 6.57 (s, 1H), 3.99-3.87 (m, 1H), 3.85-3.72 (m, 4H), 3.68-3.53 (m, 2H), 3.47-3.31 (m, 3H), 3.98-3.92 (m, 1H), 2.23 (s, 6H), 1.84-1.77 (m, 1H), 1.52-1.48 (m, 1H) |
| 115 | ¹H NMR (400 MHz, CD₃OD) δ 8.25 (s, 1H), 8.17-7.88 (m, 1H), 7.82-7.60 (m, 1H), 7.30-7.18 (m, 2H), 6.79-6.61 (m, 1H), 4.03-3.81 (m, 4H), 3.72-3.46 (m, 2H), 3.07-2.73 (m, 2H), 2.30 (s, 6H), 2.19-1.72 (m, 4H), 1.63-1.41 (m, 3H) |
| 121 | ¹H NMR (300 MHz, CD₃OD) δ 8.21 (s, 1H), 8.09 (s, 1H), 7.92 (s, 1H), 7.83-7.60 (m, 2H), 7.14-6.89 (m, 2H), 6.43 (s, 1H), 4.49-4.23 (m, 1H), 3.75-3.40 (m, 2H), 3.25-3.10 (m, 3H), 3.04-2.77 (m, 4H), 2.74-2.41 (m, 4H), 2.06 (s, 6H), 1.14 (d, 3H) |
| 154 | ¹H NMR (400 MHz, CDCl₃) δ 8.45-8.32 (m, 1H), 8.28 (s, 1H), 7.29-7.28 (m, 1H), 7.27-7.24 (m, 2H), 7.19 (s, 1H), 6.94 (dd, 1H), 6.81-6.63 (m, 1H), 4.44-3.78 (m, 4H), 3.77-3.26 (m, 6H), 3.07-2.83 (m, 1H), 2.44-2.26 (m, 6H), 2.11-1.71 (m, 4H), 1.54 (d, 3H) |
| 158 | ¹H NMR (400 MHz, CD₃OD) δ 8.15 (s, 1H), 8.09-7.89 (m, 2H), 7.68-7.44 (m, 1H), 7.30-7.13 (m, 2H), 6.66 (s, 1H), 3.85-3.43 (m, 5H), 3.00-2.72 (m, 5H), 2.29 (s, 6H), 2.19-2.02 (m, 1H), 2.02-1.88 (m, 1H), 1.77-1.67 (m, 1H), 1.67-1.49 (m, 1H), 1.14 (s, 3H) |
| 169 | ¹H NMR (400 MHz, CD₃OD) δ 8.33 (s, 1H), 8.21 (s, 1H), 8.07-7.91 (m, 2H), 7.86-7.70 (m, 2H), 7.62-7.48 (m, 1H), 7.32-7.07 (m, 2H), 4.17-3.94 (m, 4H), 3.53-3.35 (m, 1H), 3.28-3.02 (m, 2H), 2.34-1.89 (m, 2H), 1.89-1.49 (m, 2H), 1.49-1.25 (m, 3H) |
| 270 | ¹H NMR (300 MHz, CDCl₃) δ 8.60 (d, 1H), 8.38 (d, 1H), 8.25 (s, 1H), 8.05 (dd, 1H), 7.47 (s, 1H), 7.34-7.21 (m, 2H), 7.12 (d, 1H), 6.94 (d, 1H), 5.23-4.59 (m, 1H), 4.14-3.79 (m, 4H), 3.18 (s, 3H), 2.97 (d, 2H), 2.52 (s, 3H), 2.22-1.37 (m, 5H), 1.41-1.23 (m, 3H) |
| 276 | ¹H NMR (300 MHz, DMSO-d₆) δ 9.42 (s, 1H), 8.78 (s, 1H), 8.33 (s, 1H), 7.95 (s, 1H), 7.60 (s, 1H), 7.52-7.26 (m, 2H), 6.58 (s, 1H), 3.95-3.72 (m, 4H), 3.45-3.19 (m, 2H), 2.97-2.76 (m, 1H), 2.44 (s, 2H), 2.23 (s, 6H), 2.03-1.59 (m, 4H), 1.42 (d, 3H) |
| 304 | ¹H NMR (400 MHz, CD₃OD) δ 8.78 (d, 1H), 8.26 (s, 1H), 8.09 (s, 1H), 7.41 (dd, 1H), 7.30-7.26 (m, 2H), 6.68 (s, 1H), 3.98-3.89 (m, 4H), 3.40-3.35 (m, 2H), 2.98-2.91 (m, 1H), 2.30 (s, 6H), 2.08-2.04 (m, 2H), 2.00-1.95 (m, 3H), 1.54 (d, 3H) |

### BIOLOGICAL EXAMPLES

### Example 2. In vitro assays

### 2.1. Human ENPP2 (hENPP2) assay

The principle of the assay conswasts in quantifying the released choline with an enzymatic method using choline oxidase and peroxidase. Choline oxydation by choline oxydase releases betaine and peroxide. The latter was quantified in presence of HRP that converts the peroxide detection agent TOOS (CAS# 82692-93-1) and 4-aminoantipyrine into quinoneimine dye. The appearance of quinoneimine dye was measured spectrophotometrically at 555 nm and was proportional to the amount of choline released by ENPP2. Inhibition of ENPP2 results in a decrease of the signal.

Compound IC₅₀ values were determined in a hENPP2 (UniProtKB/SwwassProt Sequence ref Q13822) biochemical assay using LPC as substrate.

5 µL of a dilution series of compound, starting from 50 µM highest concentration, 1/3 dilution, was added to the wells. hENPP2 was used at a final concentration of 1 µg/mL or 3 µg/mL (it will be appreciated by the skilled person that the potency read out was independent of the enzyme concentration). The enzyme was diluted in 50 mM Trwas-HCl pH 8.5, 500 mM NaCl, 5 mM KCl, 10 mM CaCl₂, 0.1% fatty acid free BSA in a total volume of 10 µL. The reaction was started by the addition of 10 µL of 150 µM LPC (palmitoyl 16:0; Avanti Polar Lipids, Inc., Cat# 855675) diluted in the same buffer as described above and the mixture was incubated at 37°C for 30 min. The reaction was terminated and choline quantified by the addition of a 25 µL of a mixture containing 0.6 U/mL of choline oxidase, 0.6 U/mL of peroxydase, 1.8 mM TOOS, 1.2 mM amino-antipyrine, 20 mM EGTA (stop-developer solution) diluted in the buffer described above. Luminescence was read on an Envision plate reader (PerkinElmer, Inc.) after an incubation of 30 min at room temperature (excitation 555 nm).

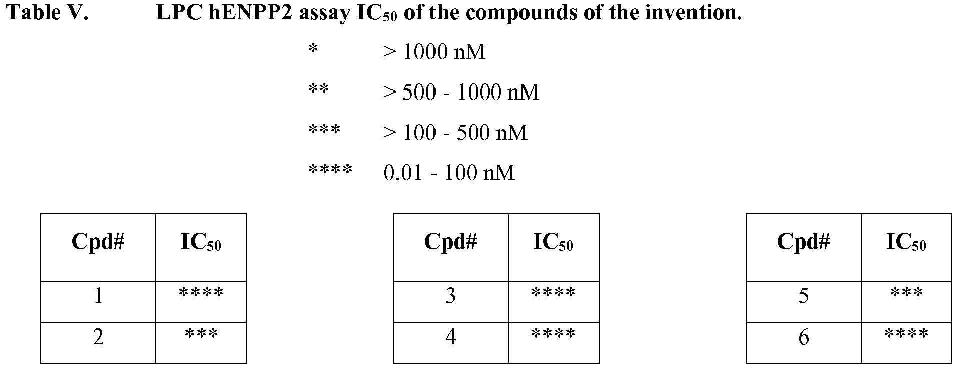

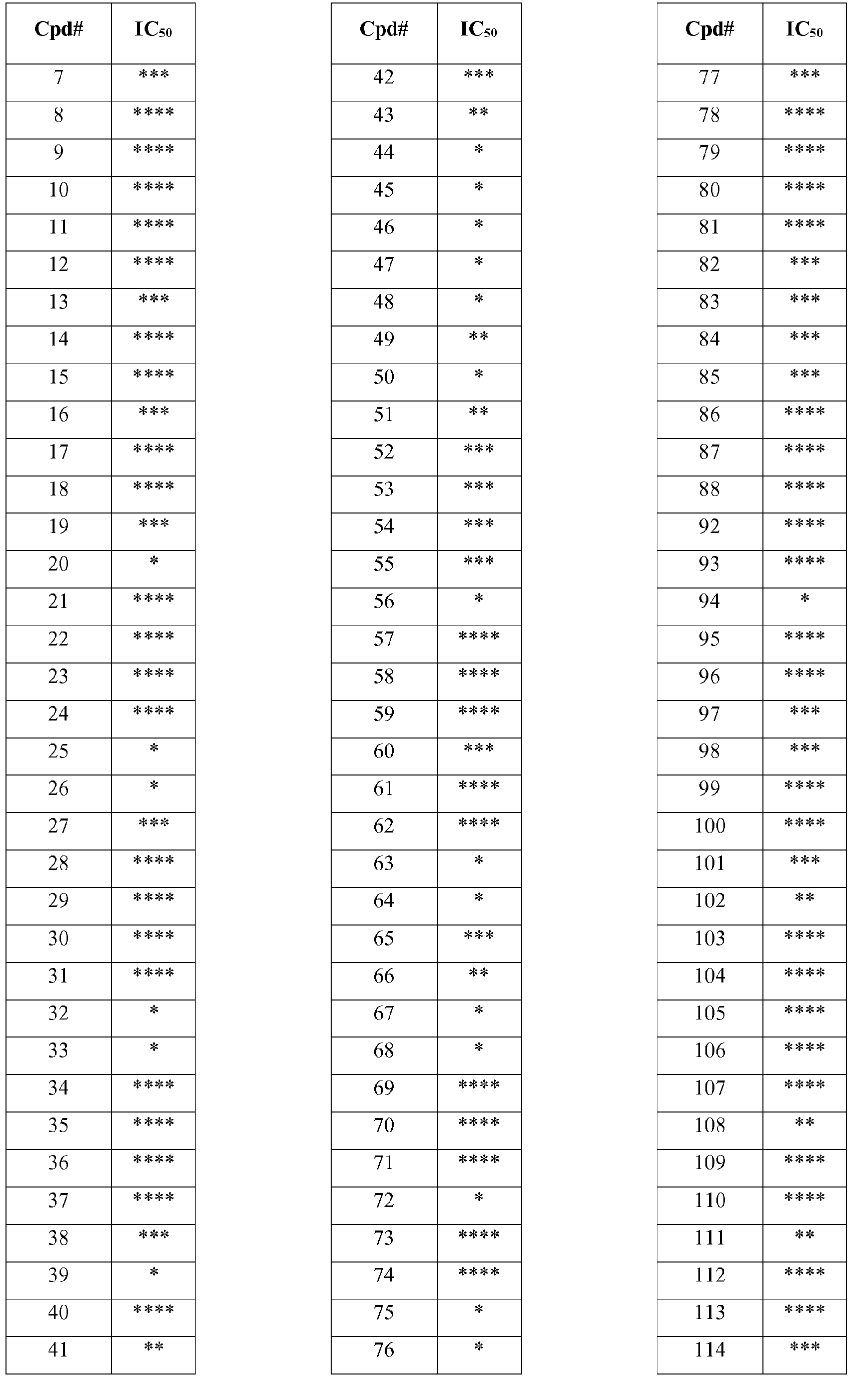

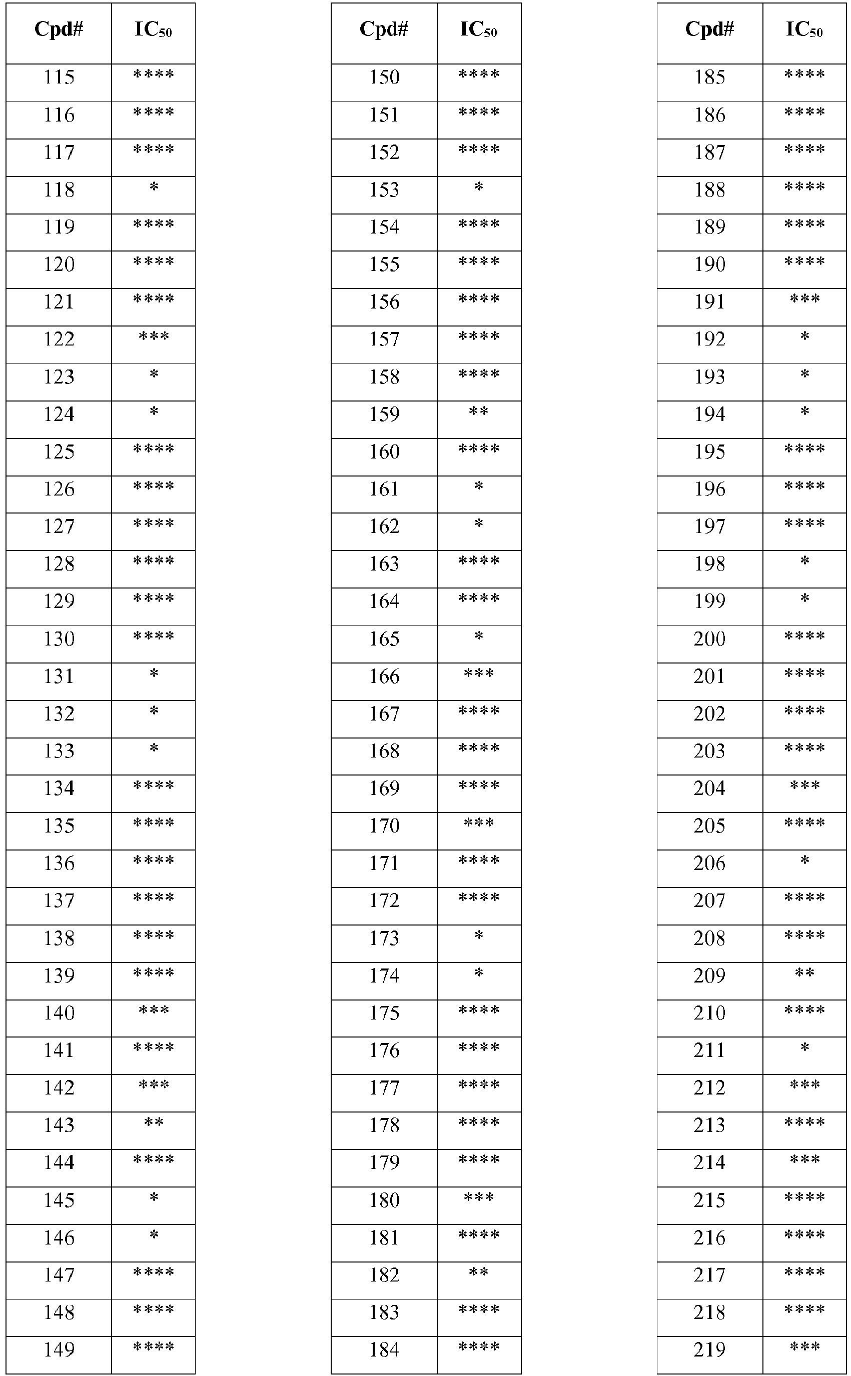

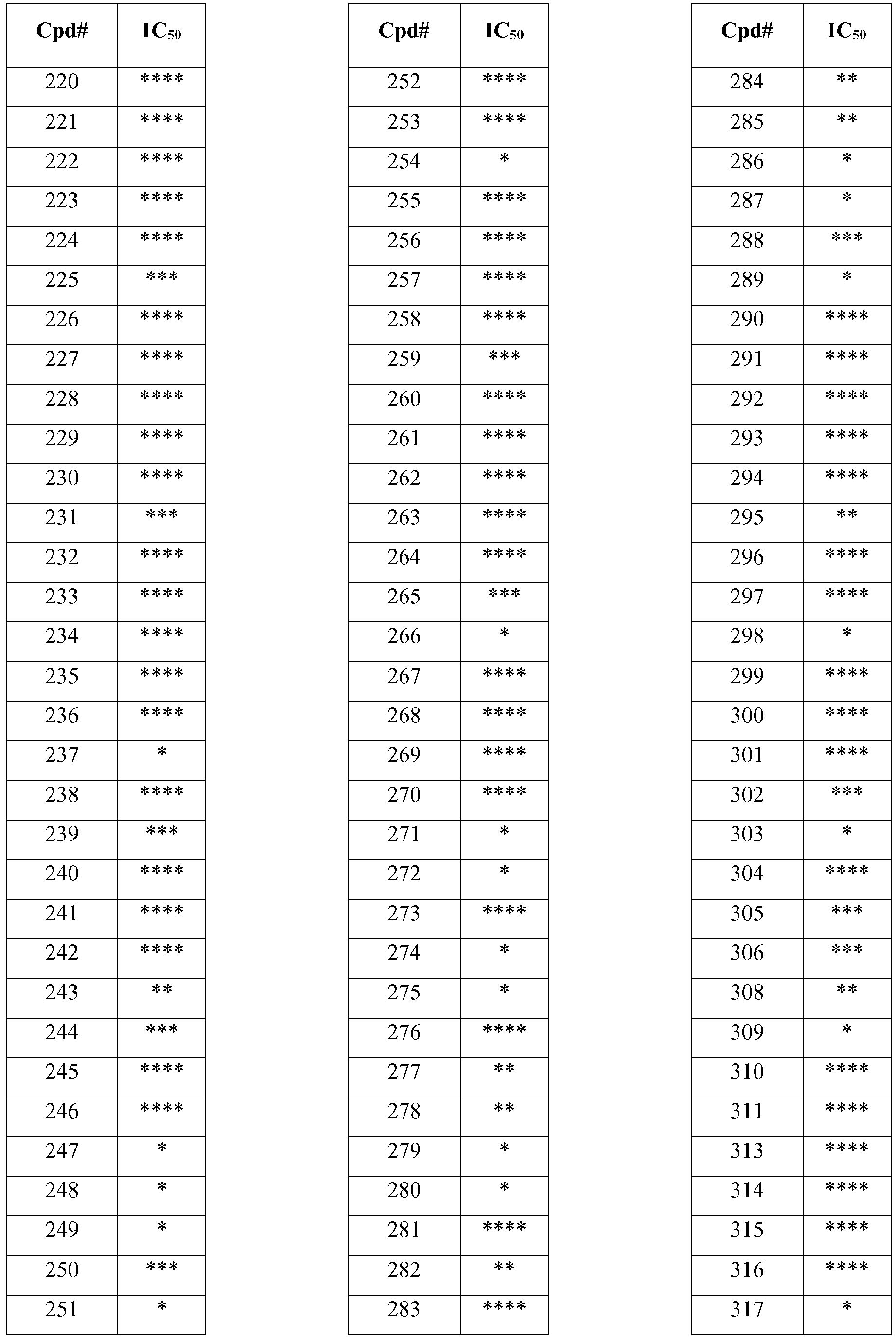

### Example 3. Whole blood assay

### 3.1. Rat LPA assay

Whole blood was collected from rats in sodium heparin tube, then after centrifugation at 3000 rpm for 15 min at 4°C, plasma was stored at -80°C. Compounds were diluted in DMSO in concentration dependent manner then 0.5 µL were dispensed into 96-well plate placed in ice. Plasma was defrosted on ice then 49.5 µL of plasma were added into the well containing 0.5 µL of compound (1% DMSO final). Plates were covered with a polypropylene lid and incubated at 37°C, 5% CO₂, for 2 h under gentle shaking (except controls which were stored at -20°C).

At the end of the incubation, controls were defrosted in ice and transferred in the incubated plates for LC-MS/MS analysis. For the analysis, plasma proteins from a 10 µL incubated plates were precipitated with an excess of methanol containing the internal standard, LPA 17:0 (Avanti Polar Lipids, Inc., Cat# 857127). After centrifugation, the corresponding supernatant was injected on a C18 column. Analytes were eluted out of the column under isocratic conditions. An API5500 QTRAP mass spectrometer (ABSciex™) was used for the detection of LPA 18:2. Relative quantities were evaluated based on the peak area.

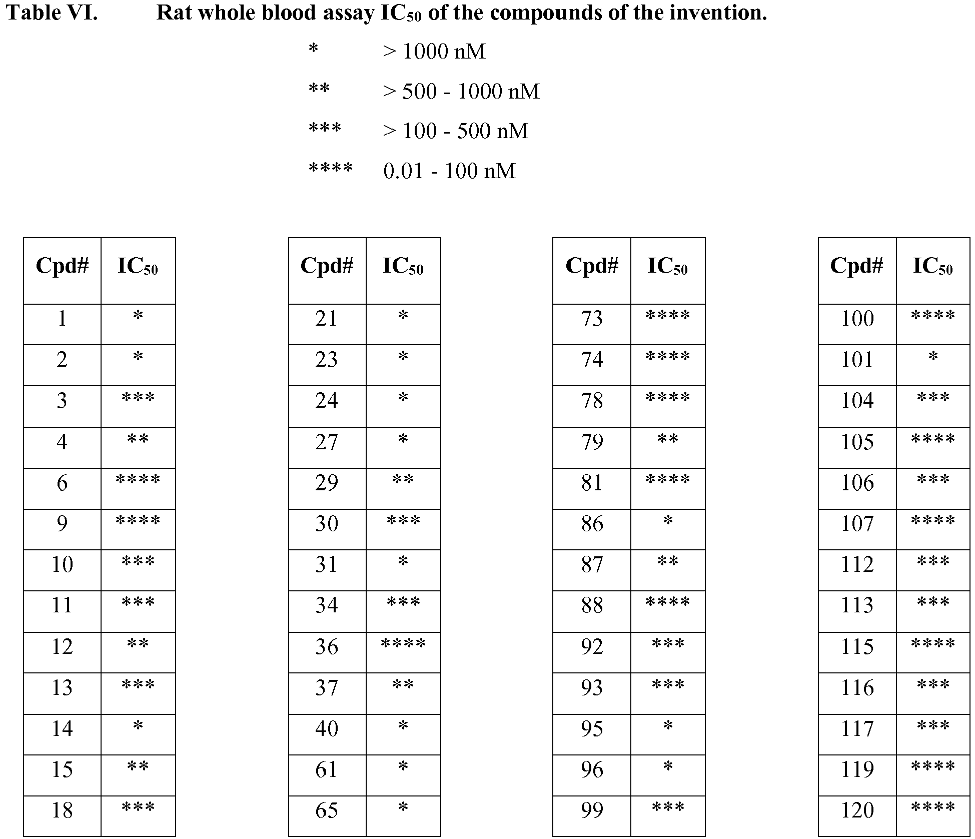

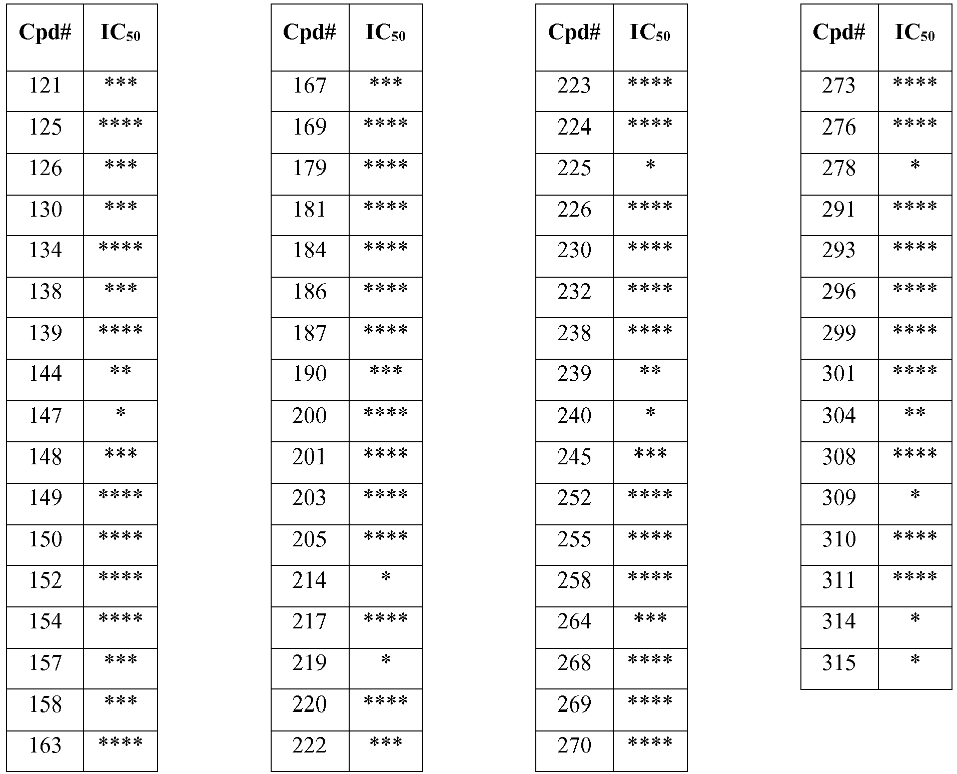

### 3.2. Human LPA assay

Blood was collected from healthy volunteers who gave informed consent, into sodium heparin tubes, then gently inverted several times to prevent clotting. Tubes were centrifuged at 3000 rpm for 15 min at 4°C then plasma was stored at -80°C. Compounds were diluted in DMSO in concentration dependent manner then 0.5 µL were dispensed into 96-well plate placed in ice. Plasma was defrosted on ice then 49.5 µL of plasma were added into the well containing 0.5 µL of compound (1% DMSO final). Plates were covered with a polypropylene lid and incubated at 37°C, 5% CO₂, for 2 h under gentle shaking (except controls which were stored at -20°C).

At the end of the incubation, controls were defrosted in ice and transferred in the incubated plates for LC-MS/MS analysis. For the analysis, plasma proteins from a 10 µL incubated plates were precipitated with an excess of methanol containing the internal standard, LPA 17:0 (Avanti Polar Lipids, Inc., Cat# 857127). After centrifugation, the corresponding supernatant was injected on a C18 column. Analytes were eluted out of the column under isocratic conditions. An API5500 QTRAP mass spectrometer (ABSciex™) was used for the detection of LPA 18:2. Relative quantities were evaluated based on the peak area.

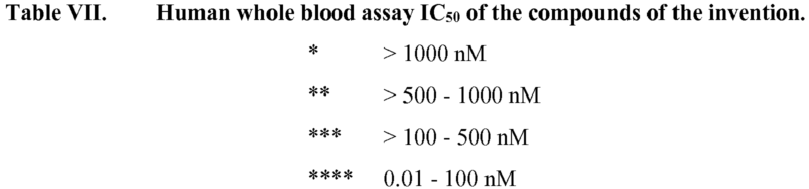

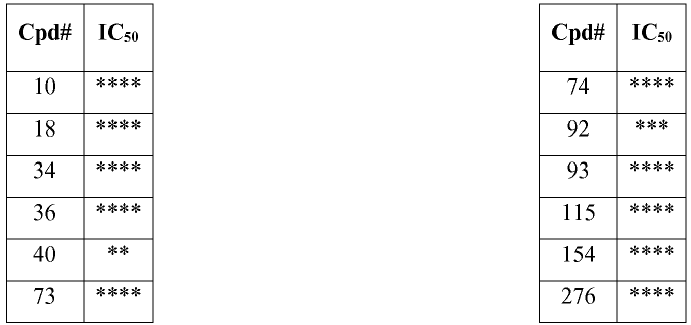

### Example 4. In vivo assays

### 4.1. Bleomycin (BLM) model

### 4.1.1. Study description

The murine bleomycin induced pulmonary fibrosis model is a widely used standard animal model of pulmonary fibrosis (Walters & Kleeberger 2001). Bleomycin administration results in oxidative stress and acute inflammation with the subsequent onset of pulmonary fibrosis. Epithelial cell injury with reactive hyperplasia, basement membrane damage, clusters of spindle-shaped mesenchymal cells resembling fibroblastic foci, and interstitial as well as intra-alveolar fibrosis are histological characteristics of the bleomycin model similar to those of IPF.

### 4.1.2. Protocol

For the purpose of the induction of pulmonary fibrosis 8 weeks old C57BL/6 mice with an average weight of ∼25 g randomly grouped in experimental groups were used.

In the model, fibrosis was induced by a single intranasal (i.n) administration of bleomycin at a dose of about 1 mg/kg (volume of 50 µL/mouse) on Day 1, and the duration of the experiment was 21 days post challenge.

Vehicle solutions were prepared as follows:
- For MC 0.5%, by adding 2.5 g of carboxymethyl cellulose into 500 mL Aqua distillate (0.5%)
- For 0.1% Natrosol, by dissolving 0.1 g of hydroxyethylcellulose (Natrosol) in 100 mL of purified water

The Sham group received Phosphate buffered saline (PBS) dissolved in the vehicle solution.

Nintedanib (CAS#656247-15-5) formulation was prepared daily in a 0.1% Natrosol solution to a final concentration of 6 mg/mL giving a dose of 60 mg/kg. Formulations was vortexed and placed in an ultrasonic bath for approximately 5 min, then was administered p.o. with about 7.5 h interval between two administrations. Prior to dosing, animals were weighed and the amount administered adjusted according to individual weights.

Pre-weighed amounts of test compounds were dissolved in PEG 400/0.5 % MC (20/80, v/v). The formulation was prepared by weighing the test compound and then adding the required volume of firstly PEG, vortexed, and then 0.5% MC to full volume. Formulations were vortexed and placed in an ultrasonic bath for approximately 5 min. Prior to dosing, animals were weighed and the amount administered adjusted according to individual weights. The formulation was administered twice daily (b.i.d.) *per os* (*p.o.*) from Day 7 to Day 21 as described below:with about 7.5 h interval between two administrations.

**Table VIII. Study group overview**

| **Group #** | **Group** | **Purpose** | **Dose** | **Vehicle** |
|---|---|---|---|---|
| 1 | PBS + vehicle | Sham | - | PEG/ MC 0.5% |
| 2 | BLM + vehicle | Negative control | - | PEG/ MC 0.5% |
| 3 | BLM + Nintedanib | Positive control | 60 mg/kg | 0.1% Natrosol |
| 4 | BLM + test compound | Test | 10 mg/kg | PEG/ MC 0.5% |
| 5 | BLM + test compound | Test | 30 mg/kg | PEG/MC 0.5% |

Body weight and clinical signs were monitored throughout the experiment.

The main read-outs in the model were:
- Blood for PK analysis
- Body weight
- Lung weight
- Histopathological examination of lung tissue
- Ashcroft score on slides stained for collagen with Crossman's trichrome, as well as evaluation of collagen deposition using digital image analysis (CaloPix software, TRIBVN Healthcare, France) on collagen-1A1 stained slides (immunohistochemistry, IHC) (Ashcroft et al. 1988; Hübner et al. 2008; Matsuse et al. 1999).

**Table IX. Ashcroft scoring**

| **Score** | **Score description** |
|---|---|
| 1 | Normal lungs (no fibrosis) |
| 2 | Minimal fibrotic thickening of alveolar or bronchial walls (network of fine collagen fibrils) |
| 3 | Moderate fibrotic thickening of walls without obvious damage to lung architecture |
| 4 | Fibrosis with damage of pulmonary structure (coarse fibrous bands or small fibrous masses, intra-alveolar collagen fibrils) |
| 5 | Large fibrous area with severe distortion of lung structure |

### 4.1.3. Statistical analysis

Data were processed using MS Excel. Statistical analysis and graphical presentation were performed using GraphPad Prism software (version 5.04). One-way ANOVA was employed. Additionally, Mann-Whitney test for comparison of two data sets was applied. Differences between groups were considered statistically significant when p<0.05.

### 4.1.4. Results

When subjected to this assay, mice treated with illustrative Cpd 115 showed a significantly decrease in Ashcroft score at both doses in comparison to vehicle treated group (*p<0.05 vs. BLM/vehicle control; Mann-Whitney t-test).

### 4.2. Radiation induced fibrosis mice model

### 4.2.1. Study overview

Pneumonitis and lung fibrosis are the major radiation-induced complications following thoracic radiotherapy, which is one of the major treatment of lung and breast cancers, lymphomas and hematopoietic transplant conditioning.

The objective of this model is to evaluate the effect of a compound of the invention in lung fibrosis induced by radiation in mice (Bickelhaupt et al. 2017).

### 4.2.2. Animals

7 weeks old (18/22 gr) female C57BL/6J albinos mice from Charles River (France) are maintained on 12 h light/dark cycle at 22°C with *ad libitum* access to tap water and food.

### 4.2.3. Materials

The test compounds are dissolved/suspended in appropriate vehicle prior to using and the kept light-free, under agitation at room temperature.

An aliquot of the formulation (approx. 200 µL) is frozen at T0 (day of preparation) and all the formulations are checked (daily) for any change in aspect.

The dose volume administered is 10 mL/kg and the volume is adapted following mean (body weight (BW) of the group as follows: 200 µL if mean BW < 22.5 g, 250 µL if mean BW ≥ 22.5 g; 300 µL if mean BW > 27.5 g.

### 4.2.4. In vivo experimental procedure

On day 1 of week 1, the animals are exposed at the thorax to a 17 Gray irradiation dose, under isoflurane anesthesia.

At the beginning of week 18 post irradiation (Day 1), animals are randomized into 6 study groups (15 subjects per group) 1) sham (vehicle: methylcellulose (MC) 0.5%), 2) diseased (vehicle: methylcellulose (MC) 0.5%), 3) positive control (nintedanib dosed 60 mg/kg in 0.1% Natrosol (hydroxyethylcellulose)), and 4) test compound (dissolved in 0.1% Natrosol), and dosed p.o. q.d until Day 23.

Body weight are recorded once a week, and on Day 23, lung function measurement under anesthesia is realized by Flexivent (Devos et al. 2017) for all groups (6 successful measurements per group) before sacrifice.

Collagen I deposition - a fibrosis marker - is analysed by immunohistochemistry using lung section staining and the percentage of collagen positive area is determined for each animal lung section (Lomas et al. 2012; Martin et al. 1992).

### 4.3. Murine sclerodermatous chronic graft-versus-host disease (cGvHD)

### 4.3.1. Study overview

This inflammation driven fibrosis model reproduces the rapidly progressing diffuse cutaneous systemic scleroderma (SSc) observed in patients, and is used to evaluate the effect of the compounds of the invention on the pathology (Chen et al. 2017).

In this model, fibrosis is induced in BALB/c (H-2d) mice by allogeneic transplantation of bone marrow cells and splenocytes from B10.D2 (H-2d) donor mice (minor HLA mismatch).

### 4.3.2. Animals

BALB/c (H-2d) mice are purchased from Janvier Labs (Le Genest-Saint-Isle, France).

B10.D2 (H-2d) mice are purchased from Jackson Laboratory (Bar Harbor, ME, USA).

All mice are maintained in specific pathogen-free conditions with sterile pellet food and water and a normal day-night cycle.

### 4.3.3. Study protocol

Transplantation of tibial and femoral bone marrow cells and splenocytes is performed as follows: 8-weeks old mice (BALB/c (H-2d)) receives total body irradiation with 700 cGy. Six h after irradiation, all BALB/c (H-2d) recipients receive bone marrow from either BALB/c (H-2d) in a syngeneic or B10.D2 (H-2d) in an allogeneic transplantation manner. For transplantation, 5 x 10⁶ splenocytes and 1 x 10⁶ bone marrow cells from donor mice are resuspended in 0.2 mL of PBS and injected via tail veins.

Treatment is started 21 days after bone marrow transplantation and thus several days after the first clinically detectable manifestations of cGvHD in allogeneically transplanted mice.

The following study groups are made:
- Syngeneically transplanted, placebo-treated control group
   Syngeneic bone marrow and splenocyte transplantation (BALB/c (H-2d) → BALB/c (H-2d)). Application of the vehicle from day 21 to day 56 post-transplantation.
- Placebo-treated fibrosis group
   Allogeneic bone marrow and splenocyte transplantation (B10.D2 (H-2d) → BALB/c (H-2d)). Application of the vehicle from day 21 to day 56 post-transplantation.
- Control group to assess pretreatment change induced by allogeneic transplantation
   Allogeneic bone marrow and splenocyte transplantation (B10.D2 (H-2d) → BALB/c (H-2d)). Sacrifice at day 21, before treatment is initiated in the control groups.
- Treatment group 1
   Allogeneic bone marrow and splenocyte transplantation (B10.D2 (H-2d) → BALB/c (H-2d)). Application of low doses of test compound (10 mg/kg/bid p.o.) from day 21 to day 56 post transplantation.
- Treatment group 2
   Allogeneic bone marrow and splenocyte transplantation (B10.D2 (H-2d) → BALB/c (H-2d)). Application of high doses of test compound (30 mg/kg/ bid p.o.) from day 21 to day 56 post transplantation.
- Positive control group:
   Allogeneic bone marrow and splenocyte transplantation (B10.D2 (H-2d) → BALB/c (H-2d)). Application of 60 mg/kg qd nintedanib from day 21 to day 56 post transplantation.

### 4.3.4. Histological evaluation of skin fibrosis

Skin samples are fixed in 4 % formalin for 6 h and embedded in paraffin. 5 µm sections are cut and stained with hematoxylin and eosin, with Trichrome or with Sirius Red.

The dermal thickness is measured at 100-fold magnification by measuring the distance between the epidermal-dermal junction and the dermal-subcutaneous fat junction at four sites oer mouse. As for other readouts, the analyses are performed in a blinded manner.

### 4.3.5. Detection of myofibroblasts

Myofibroblasts are characterized by the expression of α-smooth muscle actin (αSMA). Fibroblasts positive for αSMA are detected by incubation with monoclonal anti-aSMA antibodies (clone 1A4, Sigma-Aldrich, Steinheim, Germany). The expression is visualized with horseradish peroxidase labeled secondary antibodies and 3,3-diaminobenzidine tetrahydrochloride (DAB) (Sigma-Aldrich). Monoclonal mouse IgG antibodies (Calbiochem, San Diego, CA, USA) are used for controls.

### 4.3.6. Hydroxyproline assay

The amount of collagen protein in skin samples is determined via hydroxyproline assay. After digestion of punch biopsies (∅ 3 mm) in 6 M HCl for three h at 120°C, the pH of the samples is adjusted to 6 with 6 M sodium hydroxide (NaOH). Afterwards, 0.06 M chloramine T is added to each sample and incubated for 20 min at room temperature. Next, 3.15 M perchloric acid and 20 % p-dimethylaminobenzaldehyde are added and samples are incubated for additional 20 min at 60°C. The absorbance is determined at 557 nm with a Spectra MAX 190 microplate spectrophotometer.

### 4.3.7. Clinical score of cutaneous cGvHD

Recipient mice are clinically monitored once daily from the day of transplantation to the indicated days after transplantation to determine the incidence and severity of cutaneous cGvHD as well as mobility, diarrhea and weight loss. The following scoring system for cutaneous cGvHD is used: healthy appearance = 0; skin lesions with alopecia < 1 cm² in area = 1; skin lesions with alopecia 1 - 2 cm² in area = 2; skin lesions with alopecia > 2 cm² in area = 3. Incidence is expressed as the percentage of mice that showed clinical manifestations.

### 4.3.8. Statistics

All data are presented as mean ± SD, and differences between the groups are tested for their statistical significance by paired student t-tests for related samples and Mann-Whitney U non-parametric test for non-related samples. P-values less than 0.05 are considered significant. P-values are expressed as follows: 0.05 > p > 0.01 as *; 0.01 > p > 0.001 as **; p < 0.001 as ***.

### 4.4. Rat Pain Model

### 4.4.1. Principle of the assay

The neuropathic pain rat model originally established by Seltzer et al. (Seltzer et al. 1990) is used to evaluate the effect of a test compound against tactile allodynia and contingent thermal hyperalgesia caused by partial sciatic nerve injury (PSNL) induced by sciatic nerve partial ligature.

The test compounds are administered to the treatment groups on a daily basis, starting from post-surgical day 3 (D3), and continued until the end point day on D15.

Gabapentin is used as a positive control. The effect in vivo of the test compounds, vehicle, and gabapentin on neuropathy are tested by a set of three tests: 1) 10 g von Frey filament test (10 g vF), 2) Hargreave's Plantar test, and 3) electronic von Frey test (evF).

### 4.4.2. Animals

Male Sprague-Dawley rats (250-300 g) were housed at 22 ± 1°C and in light-controlled environment (lights on from 7 am to 8 pm) with *ad libitum* access to food and water.

### 4.4.3. Protocol

Treatments were commenced on day 3 post PSNL (D3), and continued until the study end on D15. In addition to the baseline (pre-surgery) tests, all tests were executed on D3, D7, and D14.

On the test days, pre-dose allodynia was evaluated prior to dosings, and the effects of the administered compounds assessed 2 hours post dosing. On D10, plasma samples were taken from the rats receiving the test compound - Vehicle for pharmacokinetic assays. Sampling was performed prior to dosing, and subsequently 1, 3, and 6 hours post dosing.

The negative control group (n=15) is dosed daily q.d. with the vehicle (methyl cellulose, 0.5%, PEG 400, 0.9% saline), the positive control group (n=15) is dosed i.p. on D3, D7 and D14 with gabapentin (50 mg/kg), and the test compound group (n=15) is dosed daily p.o. b.i.d. (10 mg/kg).

### 4.4.4. Tactile allodynia -von Frey tests

Two von Frey test approaches are used to assess the congenital (referred to as baseline) tactile allodynia levels of the animals. In order to avoid false sensitization in the test results, the rats are subjected to a sufficient habituation period and two handling procedures prior to all baseline tests. In addition, the baseline von Frey tests take place at maximum of 2 days prior to the surgery.

In each test, the hind paw ipsilateral to the injury (left) is tested for each rat.

In the manual test approach (10 g vF), a pre-calibrated 10 g von Frey hair was used to exert five repeats of filament presentations. A minimum of three minutes interval gapped the repeated measurements. The results were recorded as percentages of positive reactions within five repeats of filament presentations. Similarly with the traditional vF, the baseline electronic von Frey test (evF) was carried out following the handling procedures, to evaluate the levels of congenital mechanical allodynia. The tests were executed by the evF equipment according to the manufacturer's instructions. The stimuli were yielded using a 0.2 mm diameter tip, by increasing the applied force linearly at a rate of 10 g/s, in 20 s of total measurement time. The actual force application was monitored in real time to ensure its linearity and thus the proper performance of the test in each measurement. The peak force (g), i.e. the lowest force evoking paw withdrawal was recorded, and the absolute paw withdrawal thresholds (PWTs) calculated as medians of triplicate measurements. A minimum of three minutes interval was used to separate the consecutive stimulus repeats to the same paw.

### 4.4.5. Heat hyperalgesia assessments (plantar test)

The potential hyperalgesia to heat stimulus in the ipsilateral paw was tested by the plantar test.

The contingency of heat hyperalgesia due to PSNL injury was assessed on the test days 2 hours post dosing. Animals were placed into acrylic test chambers standing on a glass pane. Following a short adaptation period, an infrared (IR) beam of the apparatus was directed onto the mid-plantar surface of the hind paw.

The beam was set to yield an IR intensity of 190 mW/cm². The paw withdrawal latencies were recorded and measured three times per paw, with a minimum of 5 minutes interval between the repeats. Minimum and maximum cut-off latencies were preset at 1 and 30 seconds, respectively.

### 4.4.6. Results

When dosed at 10 mg/kg p.o. bid, compound 115 showed a statistically significant reduction of allodynia and heat hyperalgesia.

### 4.5. LPS induced lung inflammation model

### 4.5.1. Overview

The aim of the assay is to assess the effect of a test compound in a mouse model of acute lung inflammation induced by intranasal instillation of LPS. The impact on the induced cells recruitment in lung is evaluated by measurement of white cells count in broncho-alveolar lavage (BAL) fluid.

### 4.5.2. Protocol

The animals (BALB/c J mice, 18-20 g) are obtained from Harlan Laboratories (Maison-Alfort, France). The animals are maintained on 12 hours light/dark cycle at 22°C with ad libitum access to tap water and food. Litters are changed twice a week. For each tested compound, a group of 10 subjects is used. In addition to the test compound-treated groups, a vehicle+LPS control group (inLPS), a non-treated group (intact), and a positive control dexamethasone treated group (DEX) are used.

LPS is dissolved in saline solution in order to obtain a final 10 µg/50 µL solution for intranasal instillation, and administered at 50 µL / mouse by intranasal instillation.

The test compounds are prepared in 15 mL PEG200 (9 mL) / H₂O (6 mL) to be dosed in a range of 0.3, 1, 3, 10, and 30 mg/kg, and then kept at room temperature in the dark, and are administered once (qd) or twice daily (bid) over 2 days.

Dexamethasone (10 mg/kg, bid, po) is used as a positive control.

On day 1, mice are anaesthetized by isoflurane inhalation. During breathing, LPS solution is instilled intra-nasally and mice are monitored until complete recovery from anesthesia.

On day 2, mice are anaesthetized by intra-peritoneal injection (under a volume of 10 mL/kg) of anaesthetic solution (18 mL NaCl 0.9% + 0.5 mL xylazine (5 mg/kg) + 1.5 mL ketamine (75 mg/kg)).

The trachea is cannulated with a catheter, and BAL is performed by 2 × 0.75 mL sterile PBS. The BAL fluid removed is shaken gently at room temperature before centrifugation at 1500 rpm during 10 min at 4°C.

The supernatant is removed and the cell pellet is suspended in 200 µL of PBS, kept on ice and total cell count is processed by hematologie analysis. Finally, mice are sacrificed under anesthesia.

### 4.5.3. Data analysis

For each readout, mean and sem are calculated. A difference statistically significant between intact or treated groups and in LPS Vehicle group is evaluated with Prism software using a one-way ANOVA (for treatment groups) followed by a Dunnett's multiple comparisons post-hoc test. *: p < 0.05; **: p < 0.01; ***: p < 0.001 versus LPS Vehicle group.

### FINAL REMARKS

It will be appreciated by those skilled in the art that the foregoing descriptions are exemplary and explanatory in nature, and intended to illustrate the invention and its preferred embodiments. Through routine experimentation, an artisan will recognize apparent modifications and variations that may be made without departing from the spirit of the invention. All such modifications coming within the scope of the appended claims are intended to be included therein. Thus, the invention is intended to be defined not by the above description, but by the following claims and their equivalents.

All publications, including but not limited to patents and patent applications, cited in this specification are herein incorporated by reference as if each individual publication are specifically and individually indicated to be incorporated by reference herein as though fully set forth.

It should be understood that factors such as the differential cell penetration capacity of the various compounds can contribute to discrepancies between the activity of the compounds in the *in vitro* biochemical and cellular assays.

At least some of the chemical names of compound of the invention as given and set forth in this application, may have been generated on an automated basis by use of a commercially available chemical naming software program, and have not been independently verified. Representative programs performing this function include the Lexichem naming tool sold by Open Eye Software, Inc. and the Autonom Software tool sold by MDL, Inc. In the instance where the indicated chemical name and the depicted structure differ, the depicted structure will control.

### REFERENCES

Ashcroft T, Simpson JM, Timbrell V. 1988. Simple method of estimating severity of pulmonary fibrosis on a numerical scale. J. Clin. Pathol. 41, 467-470.
Bickelhaupt S et al. 2017. Effects of CTGF Blockade on Attenuation and Reversal of Radiation-Induced Pulmonary Fibrosis. JNCI J. Natl. Cancer Inst. 109**.**
Bundgaard H. 1991. Novel Chemical Approaches in Prodrug Design. Drugs Future 16, 443-458.
Chen C-W et al. 2017. Pharmacological inhibition of porcupine induces regression of experimental skin fibrosis by targeting Wnt signalling. Ann. Rheum. Dis. 76, 773-778.
Devos FC et al. 2017. Forced expiration measurements in mouse models of obstructive and restrictive lung diseases. Respir. Res. 18, 123.
Hübner R-H et al. 2008. Standardized quantification of pulmonary fibrosis in histological samples. BioTechniques 44, 507-517.
Law S-H et al. 2019. An Updated Review of Lysophosphatidylcholine Metabolism in Human Diseases. Int. J. Mol. Sci. 20**.**
Lomas NJ et al. 2012. Idiopathic pulmonary fibrosis: immunohistochemical analysis provides fresh insights into lung tissue remodelling with implications for novel prognostic markers. Int. J. Clin. Exp. Pathol. 5, 58-71.
Maher TM et al. 2019. Rationale, design and objectives of two phase III, randomised, placebo-controlled studies of GLPG1690, a novel autotaxin inhibitor, in idiopathic pulmonary fibrosis (ISABELA 1 and 2). BMJ Open Respir. Res. 6**.**
Martin SG et al. 1992. Collagen metabolism in the murine colon following X irradiation. Radiat. Res. 130, 38-47.
Matralis AN, Afantitis A, Aidinis V. 2019. Development and therapeutic potential of autotaxin small molecule inhibitors: From bench to advanced clinical trials. Med. Res. Rev. 39, 976-1013.
Matsuse T et al. 1999. ICAM-1 mediates lung leukocyte recruitment but not pulmonary fibrosis in a murine model of bleomycin-induced lung injury. Eur. Respir. J. 13, 71-77.
Seltzer Z, Dubner R, Shir Y. 1990. A novel behavioral model of neuropathic pain disorders produced in rats by partial sciatic nerve injury. Pain 43, 205-218.
Sina C et al. 2009. G Protein-Coupled Receptor 43 Is Essential for Neutrophil Recruitment during Intestinal Inflammation. J. Immunol. 183, 7514-7522.
Tanaka M et al. 2006. Autotaxin Stabilizes Blood Vessels and Is Required for Embryonic Vasculature by Producing Lysophosphatidic Acid. J. Biol. Chem. 281, 25822-25830.
Walters DM, Kleeberger SR. 2001. Mouse Models of Bleomycin-Induced Pulmonary Fibrosis. in Current Protocols in Pharmacology. John Wiley & Sons, Inc.
Wirtz S et al. 2007. Chemically induced mouse models of intestinal inflammation. Nat. Protoc. 2, 541-546.
Wuts PGM, Greene TW. 2014. Greene's protective groups in organic synthesis Fifth edition., Wiley, Hoboken, NJ.

## Claims

1. A compound according to Formula I: 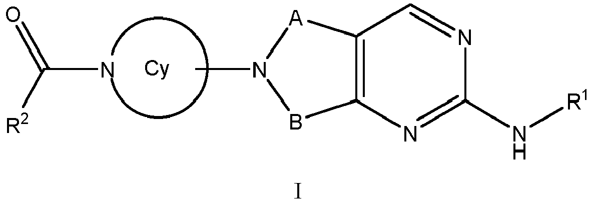 wherein
each A and B is independently C₁₋₄ alkylenyl optionally substituted with one oxo;
R¹ is:
- 6-10 membered monocyclic or fused bicyclic aryl, optionally substituted with one or more independently selected R^{3a} groups, or
- 5-10 membered monocyclic or fused bicyclic heteroaryl comprising one or more heteroatoms independently selected from N, O and S, which heteroaryl is optionally substituted with one or more independently selected R^{3b} groups;
R² is
- phenyl optionally substituted with one or more independently selected R⁵ groups, or
- 5-10 membered monocyclic or fused bicyclic heteroaryl comprising one or more heteroatoms independently selected from N, O and S, which heteroaryl is optionally substituted with one or more independently selected R⁵ groups;
each R^{3a} and R^{3b} is independently selected from:
- halo,
- C₁₋₄ alkyl optionally substituted with one or more independently selected halo,
- C₁₋₄ alkoxy optionally substituted with one or more independently selected halo,
- -SF₅,
- -CN,
- -O-C₃₋₇ cycloalkyl, and
- -C(=O)NH₂;
Cy is:
- 4-10 membered monocyclic, bridged bicyclic, or spiro bicyclic heterocycloalkyl comprising one N atom, which heterocycloalkyl is optionally substituted with one or more independently selected R⁴ groups, or
- 4-10 membered monocyclic, bridged bicyclic, or spiro bicyclic heterocycloalkenyl comprising one double bond and one N atom, which heterocycloalkenyl is optionally substituted with one or more independently selected R⁴ groups;
each R⁴ is independently selected from:
- C₁₋₄ alkyl optionally substituted with one or more independently selected halo,
- -C(=O)O-C₁₋₄ alkyl,
- halo,
- -CN, and
- -OH;
each R⁵ is independently selected from:
- -OH,
- -SH,
- halo,
- C₁₋₄ alkyl optionally substituted with one or more independently selected -OH, or halo,
- C₁₋₄ alkoxy optionally substituted with one or more independently selected -OH, or halo,
- -NH-S(=O)₂-C₁₋₄ alkyl, which alkyl is optionally substituted with one or more independently selected halo or C₁₋₄ alkoxy,
- -NH-S(=O)₂-C₃₋₇ cycloalkyl,
- -NH-C(=O)C₁₋₄ alkyl,
- -C(=O)OH,
- -C(=O)NH₂,
- -C(=O)O-C₁₋₄ alkyl, which alkyl is optionally substituted with one 5-6 membered monocyclic heterocycloalkenyl comprising one double bond and one or two O atoms, which heterocycloalkenyl is optionally substituted with one or more independently selected C₁₋₄ alkyl or oxo,
- -C(=O)-NH-S(=O)₂-C₁₋₄ alkyl,
- -S(=O)₂NH₂, and
- 5-6 membered monocyclic heteroaryl comprising one or more heteroatoms independently selected from N, O, and S;
or a pharmaceutically acceptable salt, or a solvate, or the solvate of a pharmaceutically acceptable salt thereof.

2. A compound or pharmaceutically acceptable salt thereof according to claim 1, wherein A is -CH₂-.

3. A compound or pharmaceutically acceptable salt thereof according to claim 1 or 2, wherein B is -CH₂- or -CH₂-CH₂-.

4. A compound or pharmaceutically acceptable salt thereof according to any one of claims 1-3, wherein Cy is azetidinyl, pyrrolidinyl, piperidinyl, azabicyclo[3.1.1]heptanyl, azaspiro[3.3]heptanyl, or azabicyclo[3.2.1]octanyl, each of which is substituted with one or more independently selected R⁴ groups.

5. A compound or pharmaceutically acceptable salt thereof according to claim 4, wherein R⁴ is -CH₃, -CH₂CH₃, -CF₃, -C(=O)O-CH₃, F, or -OH.

6. A compound or pharmaceutically acceptable salt thereof according to claim any one of claims 1-5, wherein R¹ is phenyl substituted with one or more independently selected R^{3a} groups.

7. A compound or pharmaceutically acceptable salt thereof according to claim 6, wherein R^{3a} is -CH₃, -CH₂CH₃, -CH(CH₃)₂, -CF₃, -CH₂CF₃, -OCH₃, -OCH₂CH₃, -OCH(CH₃)₂, -OCF₃, -OCH₂CF₃, -SF₅, -CN, -O-cyclopropyl, pyridinyl, or pyrazolyl.

8. A compound or pharmaceutically acceptable salt thereof according to claim any one of claims 1-7, wherein R¹ is:

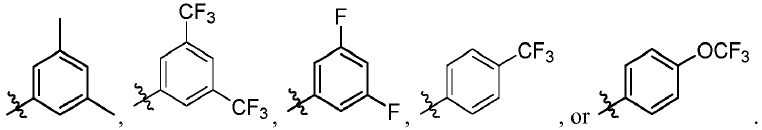

9. A compound or pharmaceutically acceptable salt thereof according to claim 1, wherein the compound is according to Formula IIIa or IIIb:

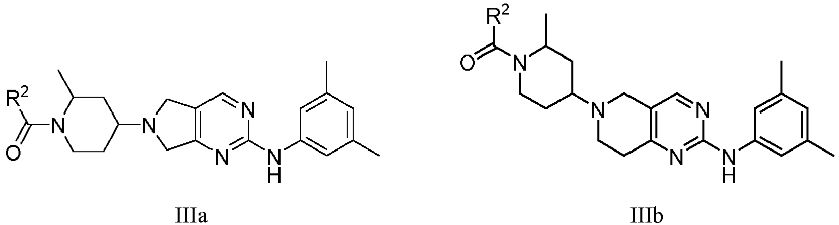

10. A compound or pharmaceutically acceptable salt thereof according to any one of claims 1-9, wherein R² is indazolyl, benzoimidazolyl, or benzotriazolyl.

11. A pharmaceutical composition comprising a pharmaceutically acceptable carrier and a pharmaceutically effective amount of a compound or pharmaceutically acceptable salt thereof according to any one of claims 1-10.

12. The pharmaceutical composition according to claim 11, comprising a further therapeutic agent.

13. A compound or pharmaceutically acceptable salt thereof according to any one of claims 1-10, or a pharmaceutical composition according to claim 11 or 12 for use in medicine.

14. A compound or pharmaceutically acceptable salt thereof according to claim any one of claims 1-10, or a pharmaceutical composition according to claim 11 or 12 for use in the prophylaxis and/or treatment of fibrotic diseases, proliferative diseases, inflammatory diseases, autoimmune diseases, respiratory diseases, cardiovascular diseases, neurodegenerative diseases, dermatological disorders, pain, and/or abnormal angiogenesis associated diseases.

15. The pharmaceutical composition according to claim 12, wherein the further therapeutic agent is an agent for the prophylaxis and/or treatment of fibrotic diseases, proliferative diseases, inflammatory diseases, autoimmune diseases, respiratory diseases, cardiovascular diseases, neurodegenerative diseases, dermatological disorders, pain, and/or abnormal angiogenesis associated diseases.
